# EUROPEAN PATENT APPLICATION

(11) **EP 4 450 503 A1**
(43) Date of publication of application: **23.10.2024**
(21) Application number: 22906654.3
(22) Date of filing: 15.12.2022
(51) Int. Cl.: C07D 471/12, C07D 498/12, A61K 31/4375, A61K 31/4985, A61P 35/00

(54) **AROMATIC HETEROCYCLIC COMPOUNDS, PREPARATION METHOD THEREFOR AND USES THEREOF**

(30) Priority: 15.12.2021 CN 202111536972; 24.04.2022 CN 202210449583
(71) Applicant: Innovstone Therapeutics Limited, Shanghai 201203 (CN)
(72) Inventor: SONG, Yunlong, Shanghai 201203 (CN); ZHOU, Yuanshu, Shanghai 201203 (CN); FU, Yiwei, Shanghai 201203 (CN); LI, Dapei, Shanghai 201203 (CN); WANG, Disha, Shanghai 201203 (CN); KOU, Hongyan, Shanghai 201203 (CN); ZHAO, Liang, Shanghai 201203 (CN); LU, Kai, Shanghai 201203 (CN); DONG, Weibing, Shanghai 201203 (CN); LAI, Qingqin, Shanghai 201203 (CN)
(74) Representative: Barrow, Nicholas Martin
(86) International application number: PCT/CN2022/139334
(87) International publication number: WO 2023/109909

(57) **Abstract**

Aromatic heterocyclic compounds as ENPP1 inhibitors, a preparation method therefor and the uses thereof. The compounds have structures as shown in formula (I).

## Description

### Technical Field

This invention relates to the field of medical technology, specifically, it relates to aromatic heterocyclic compounds as ENPP1 inhibitors and preparation methods and uses of the compounds.

### Background of Art

Cancer cells have chromosomal instability. As cancer cells cleavage, DNA fragments and even entire chromosomes may be duplicated, mutated, or completely lost. The more unstable the chromosomes are, the more likely DNA fragments will appear in inappropriate locations, e.g. floating in the cytoplasm outside the nucleus. Cells recognise these free DNA fragments as evidence of viral invasion, which in turn triggers an internal alarm within the cells, causing inflammation, and immune cells are recruited to the tumour site to launch an immune attack.

STING (stimulator of interferon genes) is widely expressed on the endoplasmic reticulum of various immune cells. It mainly recognizes cyclic dinucleotides (CDNs) to detect DNA leakage in the cytoplasm and stimulate an immune response.

DNA is usually present in the cell nucleus and mitochondria, and the presence of DNA in the cytoplasm is abnormal. cGAS can sense DNA in the cytoplasm and synthesize cyclic 2',3'-cGAMP from ATP or GTP. 2',3'-cGAMP binds to and activates STING, while ENPP1 is the only enzyme capable of hydrolyzing 2',3'-cGAMP. By hydrolyzing 2',3'-cGAMP, ENPP1 prevents signals from reaching immune cells. This process also releases the immunosuppressive molecule adenosine, which has a soothing effect on inflammation. Additionally, the expression of ENPP1 is associated with increased metastasis and resistance to immunotherapy.

ENPP1 is a type II transmembrane glycoprotein with nucleotide pyrophosphatase and phosphodiesterase activities, belonging to the ectonucleotide pyrophosphatase/phosphodiesterase (ENPP) family, which consists of seven functionally distinct proteins (1-7). The structure of ENPP1 has two N-terminal SMB domains (SMB1 and SMB2), two linker regions (L1 and L2), a catalytic domain, and a nuclease-like domain. ENPP1 is differentially expressed in immune cells, with low levels in NK cells, DCs, and macrophages, and high levels in neutrophils. ENPP1 is also expressed in a small subset of B cells that may be involved in regulating T cell activity. Its expression is elevated in M2 subtype macrophages, which play a role in tumour promotion. The expression of ENPP1 increases in astrocytoma, breast cancer, and head and neck tumours, and varies greatly among different tumour tissues. The specific high expression of ENPP1 in some tumours may be a driving factor for tumour immune escape and metastasis.

ENPP1 plays an important role in the immune response to multiple stimuli through the STING pathway, achieving high selectivity by selectively activating the STING signalling pathway in tumour cells and other cells in the tumour microenvironment. ENPP1 can also catalyze the hydrolysis of ATP to PPi and AMP, promoting the generation of adenosine, which has a strong immunosuppressive effect. Inhibiting ENPP1 can reduce adenosine production by inhibiting ATP hydrolysis, thereby lifting tumour immunosuppression.

While STING agonists non-selectively activate STING in cancer cells and host cells, ENPP1 limits the scope of STING activation to tumour tissues and the tumour microenvironment, enhancing anti-tumour immunity. Moreover, ENPP1 is selectively upregulated in metastatic and chromosomally unstable tumour cells. Systemic administration of ENPP1 inhibitors can interfere with the ability of diffused tumour cells to evade immune surveillance, bypassing the technical difficulties of intra-tumour administration of STING agonists.

The combination of ENPP1 inhibitors and radiotherapy has achieved good results in animal models, and there is also a certain synergistic effect with immune checkpoint inhibitors such as PD-1 and PARP inhibitors. Currently, the development of ENPP1 inhibitors is still in the preclinical research stage, but several pharmaceutical companies have published patents on ENPP1 inhibitors, such as WO2021061803A1, WO2021158829A1, WO2020190912A1, WO2019177971A1, and WO2019046778A1.

After heart injury, various cell populations are recruited to the heart. Using a mouse model of ischemic heart injury, it has been demonstrated that cardiomyocytes play a key role in heart repair by regulating nucleotide metabolism and the death of non-cardiomyocytes (Shen Li et al., 2022). Heart injury induces the expression of the ectonucleotide enzyme-ectonucleotide pyrophosphatase/phosphodiesterase 1 (ENPP1), which hydrolyzes extracellular ATP to form AMP. In response to AMP, cardiomyocytes release adenine and specific ribonucleosides, disrupting pyrimidine biosynthesis at the orotidine monophosphate (OMP) synthesis step and inducing genotoxic stress and p53-mediated proliferation of non-cardiomyocyte death. Since non-muscle cells are crucial for heart repair, rescuing pyrimidine biosynthesis by administering uridine or genetically targeting the ENPP1/AMP pathway can enhance repair after heart injury.

Although some small molecule ENPP1 inhibitors have been disclosed in existing technologies, there are currently no clinically approved small molecule drugs on the market. Therefore, there is still an urgent need to develop new compounds with better efficacy and pharmacokinetic results that have the potential to be marketed. This invention designs a series of compounds with new structures represented by general formulae and finds that compounds with such structures exhibit excellent effects and functions, which is of positive significance for the development of ENPP1 inhibitors.

### Summary of the Invention

The objective of this invention is to provide a compound with an aromatic heterocyclic structure as an ENPP1 inhibitor, a process for preparing the compound, and its use in treating ENPP 1-mediated diseases.

The first aspect of this invention provides a compound represented by the following formula (I), a stereoisomer or tautomer of the compound or a mixture thereof, a pharmaceutically acceptable salt, co-crystal, polymorph or solvate of the compound, or a stable isotope derivative, metabolite or prodrug of the compound: 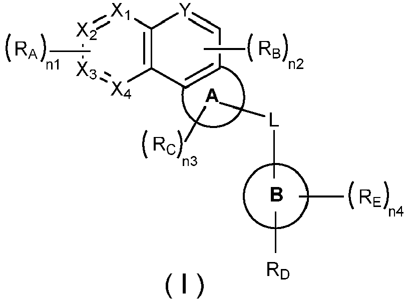
wherein X₁, X₂, X₃, X₄ and Y are each independently CH or N;
and when X₁, X₂, X₃ and X₄ are all CH, Y is not CH;
R_{A} is a substituent of the ring where X₁ is located, R_{B} is a substituent of the ring where Y is located, R_{A} and R_{B} are each independently hydrogen, deuterium, halogen, oxime, -CN, -OH, -SH, -NO₂, -NH₂, -Z-OR₁, -Z-SR₁, -Z-NR₂R₃, -Z-NR₂C(O)R₄, -Z-NR₂C(O)OR₁, -Z-C(O)R₄, -Z-C(O)OR₁, -Z-C(O)(CR₅R₆)ₙC(O)R₄, -Z-C(O)(CR₅R₆)ₙC(O)OR₁, - Z-C(O)NR₂R₃, -Z-S(O)₂R₄, -Z-S(O)₂NR₂R₃, C₁₋₆ alkyl, C₂-₆ alkenyl, C₂-₆ alkynyl, C₁₋₆ alkoxy, C₁₋₆ alkylthio, C₃-₈ cycloalkyl, C₃-₈ cycloalkenyl, 3-20 membered heterocyclyl, 5-16 membered heteroaryl; the alkyl, alkenyl, alkynyl, alkoxy, alkylthio, cycloalkyl, cycloalkenyl, heterocyclyl, or heteroaryl is optionally substituted by one or more substituents selected from deuterium, halogen, oxo, oxime, -CN, -OH, -NO₂, -NH₂, C₁₋₆ alkyl, phenyl, C₁₋₆ alkoxy, C₁₋₆ haloalkyl, C₁₋₆ haloalkoxy, and halophenyl;
n1 is 1, 2, 3 or 4;
n2 is 1 or 2;
ring A is 6-10 membered heterocyclyl or 6-12 membered heteroaryl;
R_{C} is a substituent of ring A, and each R_{C} is independently hydrogen, deuterium, halogen, oxo, oxime, -CN, -OH, - SH, -NO₂, -NH₂, -Z-OR₁, -Z-SR₁, -Z-NR₂R₃, -Z-NR₂C(O)R₄, -Z-NR₂C(O)OR₁, -Z-C(O)R₄, -Z-C(O)OR₁, -Z-C(O)(CR₅R₆)ₙC(O)R₄, -Z-C(O)(CR₅R₆)ₙC(O)OR₁, -Z-C(O)NR₂R₃, -Z-S(O)₂R₄, -Z-S(O)₂NR₂R₃, C₁₋₆ alkyl, C₂-₆ alkenyl, C₂-₆ alkynyl, C₁₋₆ alkoxy, C₁₋₆ alkylthio, C₃-₈ cycloalkyl, C₃-₈ cycloalkenyl, 3-20 membered heterocyclyl, C₆-₁₂ aryl, 5-16 membered heteroaryl; the alkyl, alkenyl, alkynyl, alkoxy, alkylthio, cycloalkyl, cycloalkenyl, heterocyclyl, aryl, or heteroaryl is optionally substituted by one or more substituents selected from deuterium, halogen, oxo, oxime, -CN, -OH, -NO₂, -NH₂, C₁₋₆ alkyl, phenyl, C₁₋₆ alkoxy, C₁₋₆ haloalkyl, C₁₋₆ haloalkoxy, and halophenyl;
n3 is 1, 2, 3, 4, 5, or 6;
Z is selected from a bond, C₁₋₃ alkylene, C₁₋₃ alkyleneoxy, and C₁₋₃ alkylenethio, the alkylene, alkyleneoxy, or alkylenethio is optionally substituted by one or more substituents selected from deuterium, halogen, oxo, -CN, -OH, and -NH₂;
ring B is C₃-₁₀ cycloalkyl, 3-20 membered heterocyclyl, C₆-₁₄ aryl or 5-16 membered heteroaryl;
R_{D} is selected from -W-OC(O)OR₁, -W-C(O)NR₂R₃, -W-C(O)NR₂OR₁, -W-OC(O)NR₂R₃, -W-NR₂C(O)R₄, -W-NR₂C(O)OR₁, -W-NR₂C(O)NR₂R₃, -W-S(O)₂R₄, -W-SO₂NR₂R₃, -W-S(O)=NR₂, -W-S(O)=NR₂NR₂R₃, -W- NR₂S(O)₂R₄, -W-OS(O)₂R₄, -W-NR₂S(O)₂NR₂R₃, -W-OS(O)₂NR₂R₃, -W-P(O)(OR₁)₂, -W-P(S)(OR₁)₂, -W-O-
P(S)(OR₁)₂, -W-B(OH)₂, and 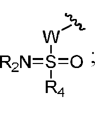 ; or R_{D} is selected from -W-OC(O)OR₁, -W-C(O)NR₂R₃, -W-C(O)NR₂OR₁,
-W-OC(O)NR₂R₃, -W-NR₂C(O)R₄, -W-NR₂C(O)OR₁, -W-NR₂C(O)NR₂R₃, -W-S(O)₂R₄, -W-SO₂NR₂R₃, -W-S(O)=NR₂, -W-S(O)=NR₂NR₂R₃, -W-NR₂S(O)₂R₄, -W-OS(O)₂R₄, -W-NR₂S(O)₂NR₂R₃, -W-OS(O)₂NR₂R₃, -W-P(O)(OR₁)₂, -W-P(S)(OR₁)₂, -W-O-P(S)(OR₁)₂, and -W-B(OH)₂;
each R_{E} is independently hydrogen, deuterium, halogen, oxo, oxime, carboxyl, -CN, -OH, -SH, -NO₂, -NH₂, -W-OR₁, -W-SR₁, -W-C(O)R₄, -W-C(O)OR₁, -W-OC(O)R₁, -W-OC(O)OR₁, -W-C(O)NR₂R₃, -W-C(O)NR₂OR₁, -W-OC(O)NR₂R₃, -W-NR₂R₃, -W-NR₂C(O)R₄, -W-NR₂C(O)OR₁, -W-NR₂C(O)NR₂R₃, -W-S(O)₂R₄, -W-SO₂NR₂R₃, - W-NR₂S(O)₂R₄, -W-OS(O)₂R₄, -W-NR₂S(O)₂NR₂R₃, -W-OS(O)₂NR₂R₃, -W-P(O)(OR₁)₂, -W-P(S)(OR₁)₂, -W-OP(S)(OR₁)₂, -W-B(OH)₂, C₁₋₆ alkyl, C₂-₆ alkenyl, C₂-₆ alkynyl, C₁₋₆ alkoxy, C₃-₈ cycloalkyl, C₃-₈ cycloalkenyl, 3-20 membered heterocyclyl, 5-16 membered heteroaryl; the alkyl, alkenyl, alkynyl, alkoxy, cycloalkyl, cycloalkenyl, heterocyclyl, or heteroaryl is optionally substituted by one or more substituents selected from deuterium, halogen, oxo, oxime, -CN, -OH, -NO₂, -NH₂, C₁₋₆ alkyl, phenyl, C₁₋₆ alkoxy, C₁₋₆ haloalkyl, C₁₋₆ haloalkoxy, and halophenyl; n is 1, 2, or 3;
n4 is 1, 2, 3, 4, 5, or 6;
W is selected from a bond, C₁₋₃ alkylene, C₁₋₃ alkyleneoxy, and C₁₋₃ alkylenethio, the alkylene, alkyleneoxy, or alkylenethio is optionally substituted by one or more substituents selected from deuterium, halogen, oxo, -CN, -OH, and -NH₂;
L is selected from a bond, -O-, -S-, C₁₋₆ alkylene, C₁₋₆ alkyleneoxy, C₃₋₆ cycloalkylene, C₁₋₆ alkylenethio, C₂-₆ alkenylene, and C₂-₆ alkynylene, the alkylene, alkyleneoxy, cycloalkylene, alkylenethio, alkenylene, or alkynylene is optionally substituted by one or more substituents selected from deuterium, halogen, C₁₋₃ alkyl, C₁₋₆ alkoxy, oxo, - CN, -OH, and -NH₂;
R₁ is independently at each occurrence hydrogen, C₁₋₆ alkyl, C₁₋₆ alkoxy, C₃-₈ cycloalkyl, phenyl, or 3-20 membered heterocyclyl, wherein the alkyl, alkoxy, cycloalkyl, phenyl, or heterocyclyl is optionally substituted by one or more substituents selected from halogen, cyano, hydroxy, amino, C₁₋₃ alkyl, C₁₋₄ alkoxy, phenyl, C₁₋₃ haloalkyl, C₁₋₄ haloalkoxy, and halophenyl;
R₂ and R₃ are independently at each occurrence hydrogen, C₁₋₆ alkyl, or C₁₋₆ alkoxy, wherein the alkyl, or alkoxy is optionally substituted by one or more substituents selected from halogen, deuterium, cyano, hydroxy, amino, carboxyl, C₁₋₃ alkyl, C₁₋₄ alkoxy, C₁₋₄ alkyl, C₃-₈ cycloalkyl, phenyl, C₁₋₃ haloalkyl, C₁₋₃ haloalkoxy, and halophenyl; R₄ is independently at each occurrence hydrogen, deuterium, C₁₋₆ alkyl, C₁₋₆ alkoxy, C₃-₈ cycloalkyl, phenyl, or 3-20 membered heterocyclyl, wherein the alkyl, alkoxy, cycloalkyl, phenyl, or heterocyclyl is optionally substituted by one or more substituents selected from halogen, cyano, hydroxy, amino, C₁₋₃ alkyl, C₁₋₄ alkoxy, phenyl, C₁₋₃ haloalkyl, C₁₋₃ haloalkoxy, and halophenyl,
R₅ and R₆ are independently hydrogen, C₁₋₃ alkyl, C₂-₆ alkenyl, C₂-₆ alkynyl, C₁₋₆ alkoxy, C₁₋₃ alkylthio, C₃₋₆ cycloalkyl, C₃₋₆ cycloalkenyl, 3-20 membered heterocyclyl, or 5-16 membered heteroaryl, wherein the alkyl, alkenyl, alkynyl, alkoxy, alkylthio, cycloalkyl, cycloalkenyl, heterocyclyl, or heteroaryl is optionally substituted by one or more substituents selected from deuterium, halogen, oxo, oxime, -CN, -OH, -NH₂, C₁₋₃ alkyl, and C₁₋₃ haloalkyl.

In the case that multiple R_{A}s, R_{B}s, Res or R_{E}s appear at the same time, R_{A}s, Ras, Res or R_{E}s can be identical or different from each other.

In a preferable embodiment according to the first aspect of this invention, X₁ is N, and X₂, X₃, and X₄ are all CH. In a preferable embodiment according to the first aspect of this invention, X₃ is N, and X₁, X₂ and X₄ are all CH.

In a preferable embodiment according to the first aspect of this invention, X₁ and X₃ are both N, and X₂ and X₄ are both CH.

In a preferable embodiment according to the first aspect of this invention, X₁, X₂, X₃, and X₄ are all CH.

In a preferable embodiment according to the first aspect of this invention, in any of the preceding embodiments of the first aspect, Y is CH; or Y is N.

In a preferable embodiment according to the first aspect of this invention, in any of the preceding embodiments of the first aspect, each R_{A} is independently hydrogen, deuterium, halogen, oxime, -CN, -OH, -SH, -NO₂, -NH₂, -Z-NR₂C(O)R₄, -Z-NR₂C(O)OR₁, 3-6 membered heterocyclyl, -Z-C(O)R₄, -Z-C(O)OR₁, -Z-C(O)NR₂R₃, -Z-S(O)₂R₄, - Z-S(O)₂NR₂R₃, C₁₋₆ alkyl, C₂-₆ alkenyl, C₂-₆ alkynyl, C₁₋₆ alkoxy, C₁₋₆ alkylthio, or C₃-₈ cycloalkyl, the alkyl, alkenyl, alkynyl, alkoxy, alkylthio, or cycloalkyl is optionally substituted by one or more substituents selected from deuterium, halogen, oxo, oxime, -CN, -OH, -NO₂, -NH₂, C₁₋₆ alkyl, phenyl, C₁₋₆ alkoxy, C₁₋₆h aloalkyl, C₁₋₆ haloalkoxy, and halophenyl;
or, each R_{A} is independently hydrogen, deuterium, halogen, oxime, -CN, -OH, -SH, -NO₂, -NH₂, -Z-NR₂C(O)R₄, - Z-NR₂C(O)OR₁, 3-6 membered heterocyclyl, -Z-C(O)R₄, -Z-C(O)OR₁, -Z-C(O)NR₂R₃, -Z-S(O)₂R₄, -Z-S(O)₂NR₂R₃, C₁₋₄ alkyl, C₂-₆ alkenyl, C₂-₆ alkynyl, C₁₋₆ alkoxy, C₁₋₆ alkylthio, or C₃₋₆ cycloalkyl, the alkyl, alkenyl, alkynyl, alkoxy, alkylthio, or cycloalkyl is optionally substituted by one or more substituents selected from deuterium, halogen, oxo, oxime, -CN, -OH, -NO₂, -NH₂, C₁₋₆ alkyl, phenyl, C₁₋₆ alkoxy, C₁₋₆ haloalkyl, C₁₋₆ haloalkoxy, and halophenyl;
or, each R_{A} is independently hydrogen, deuterium, halogen, oxime, -CN, -OH, -SH, -NO₂, -NH₂, -Z-NR₂C(O)R₄, - Z-NR₂C(O)OR₁, 3-6 membered heterocyclyl, -Z-C(O)R₄, -Z-C(O)OR₁, -Z-C(O)NR₂R₃, -Z-S(O)₂R₄, -Z-S(O)₂NR₂R₃, C₁₋₄ alkyl, C₂-₄ alkenyl, C₂-₄ alkynyl, C₁₋₃ alkoxy, C₁₋₃ alkylthio, or C₃₋₆ cycloalkyl, the alkyl, alkenyl, alkynyl, alkoxy, alkylthio, or cycloalkyl is optionally substituted by one or more substituents selected from deuterium, halogen, oxo, -CN, -OH, -NO₂, -NH₂, C₁₋₆ alkyl, C₁₋₆ alkoxy, C₁₋₆ haloalkyl, and C₁₋₆ haloalkoxy;
or, each R_{A} is independently hydrogen, deuterium, F, Cl, Br, oxime, -CN, -OH, -SH, -NO₂, -NH₂, -NR₂C(O)R₄, - NR₂C(O)OR₁, 3-6 membered heterocyclyl, -C(O)R₄, -C(O)OR₁, -C(O)NR₂R₃, -S(O)₂R₄, -S(O)₂NR₂R₃, methyl, ethyl, propyl, isopropyl, tert-butyl, ethenyl, ethynyl, methoxy, ethoxy, propoxy, isopropoxy, methylthio, ethylthio, propylthio, isopropylthio, cyclopropyl, cyclobutyl, trifluoromethyl, difluoromethyl, monofluoromethyl, trifluoroethyl, difluoroethyl, monofluoroethyl, hydroxypropyl, hydroxyethyl, hydroxymethyl, methoxypropyl, methoxyethyl, methoxymethyl, ethenyl, ethynyl, propenyl, or propynyl;
or, each R_{A} is independently hydrogen, F, Cl, Br, oxime, -CN, -OH, -NH₂, -NHC(O)C₁₋₃alkyl, -NHC(O)OC₁₋₃alkyl, 3-4 membered heterocyclyl, -C(O)C₁₋₃ alkyl, -C(O)OC₁₋₃ alkyl, -C(O)NHC₁₋₃ alkyl, -S(O)₂C₁₋₃ alkyl, -S(O)₂NH₂, - S(O)₂NHC₁₋₃ alkyl, methyl, ethyl, methoxy, ethoxy, methylthio, ethenyl, propenyl, ethynyl, cyclopropyl, or tert-butyl; or, R_{A} is selected from hydrogen, F, Cl, -CN, -NH₂, oxime, methyl, methoxy, ethoxy, methylthio, -OCD₃, - NHCOCH₃, -NHCOOCH₃, 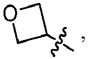 -COCH₃, -COOCH₃, -COCH₂OCH₃, -CONHCH₃, -SO₂CH₃, -SO₂NH₂, - CH=CH-CH₃, ethynyl, cyclopropyl, and tert-butyl.

In a preferable embodiment according to the first aspect of this invention, in any of the preceding embodiments of the first aspect, n1 is 1, 2, or 3; or, n1 is 1 or 2.

In a preferable embodiment according to the first aspect of this invention, in any of the preceding embodiments of the first aspect, each R_{B} is independently hydrogen, deuterium, halogen, oxime, -CN, -OH, -SH, -NO₂, -NH₂, C₁₋₃ alkyl, C₂-₆ alkenyl, C₂-₆ alkynyl, C₁₋₆ alkoxy, C₁₋₆ alkylthio, or C₃₋₆ cycloalkyl, the alkyl, alkenyl, alkynyl, alkoxy, alkylthio, or cycloalkyl is optionally substituted by one or more substituents selected from deuterium, halogen, oxo, oxime, -CN, -OH, -NO₂, -NH₂, C₁₋₆ alkyl, phenyl, C₁₋₆ alkoxy, C₁₋₆ haloalkyl, C₁₋₆ haloalkoxy, and halophenyl;
or, each R_{B} is independently hydrogen, deuterium, F, Cl, Br, -CN, -OH, -SH, -NO₂, -NH₂, methyl, ethyl, propyl, isopropyl, ethenyl, propenyl, ethynyl, propynyl, methoxy, ethoxy, propoxy, isopropoxy, methylthio, ethylthio, propylthio, isopropylthio, cyclopropyl, cyclobutyl, trifluoromethyl, difluoromethyl, monofluoromethyl, trifluoroethyl, difluoroethyl, monofluoroethyl, hydroxypropyl, hydroxyethyl, hydroxymethyl, methoxypropyl, methoxyethyl, or methoxymethyl;
or, each R_{B} is independently hydrogen, F, Cl, Br, -OH, -NH₂, methyl, ethyl, methoxy, ethoxy, ethenyl, or ethynyl; or, R_{B} is hydrogen, -NH₂, methyl, methoxy, or ethynyl.

In a preferable embodiment according to the first aspect of this invention, in any of the preceding embodiments of the first aspect, n2 is 1.

In a preferable embodiment according to the first aspect of this invention, in any of the preceding embodiments of the first aspect, ring A is 6-9 membered heterocyclyl or 6-12 membered heteroaryl;
or, ring A is 6-8 membered heterocyclyl or 6-8 membered heteroaryl;
or, ring A is 6-membered heterocyclyl or 6-membered heteroaryl;
or, ring A is piperidyl, hexahydropyrimidyl, piperazinyl, 1,3-oxazinanyl, morpholinyl, thiomorpholinyl, 1,3-thiazinyl, 1,2,3,6-tetrahydropyridyl, 1,2,3,6-tetrahydropyrazinyl, 1,4,5,6-tetrahydropyrimidyl, homopiperidyl, homopiperazinyl, homomorpholinyl, pyridyl, pyridazinyl, pyrimidyl, 1,4-diazepane, 1,5-diazocane, 1,4-oxazepane, 1,3-oxazepane, 1,4-oxazacyclooctane, 2,3,6,7-tetrahydro-1,4-diazepine;
or, ring A is 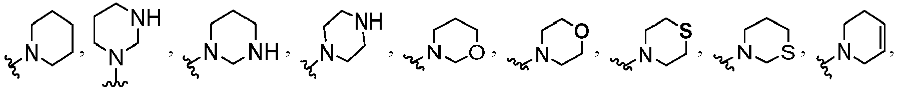 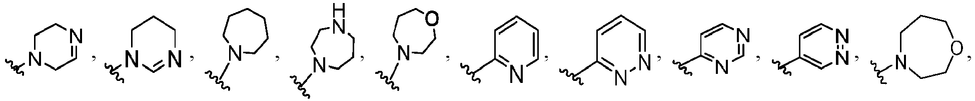 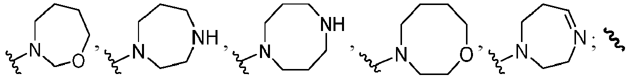 represents the attachment position of the L group;
or, ring A is 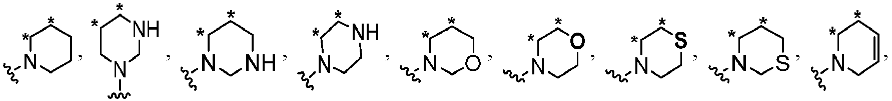 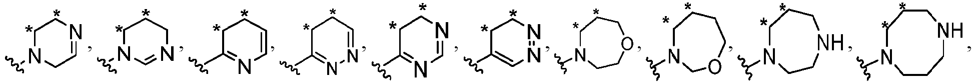 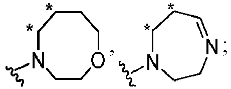 ** represents the site of fusing, 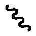 , represents the attachment position of the L group; or, when one or both of R_{C}s is oxo, the above-mentioned ring A substituted by one or two R_{C}s is 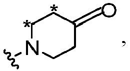 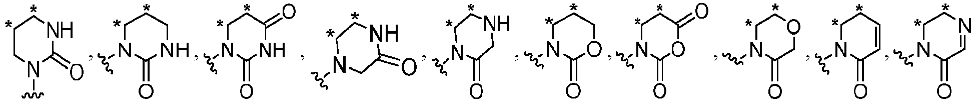 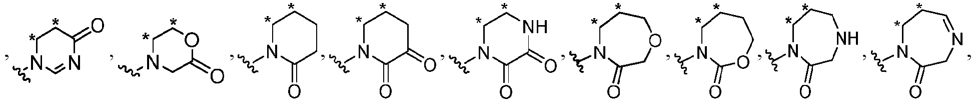 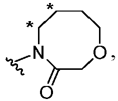 ** represents the site of fusing, 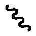 , represents the attachment position of the L group; or, the ring A substituted by Rc(s) is

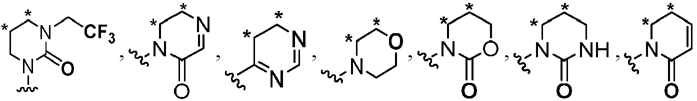

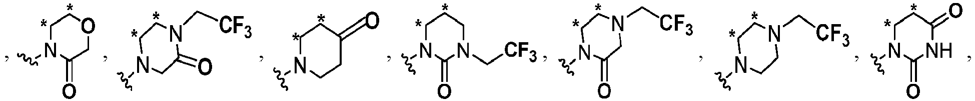

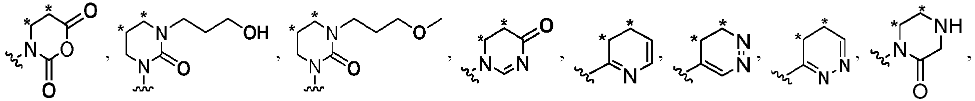

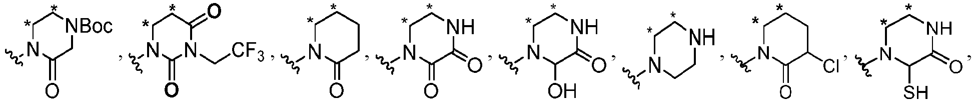

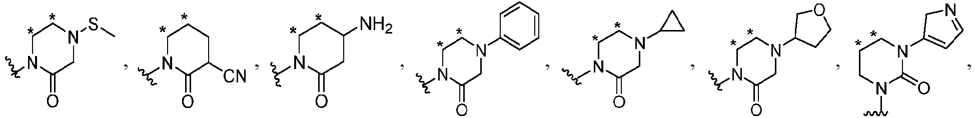

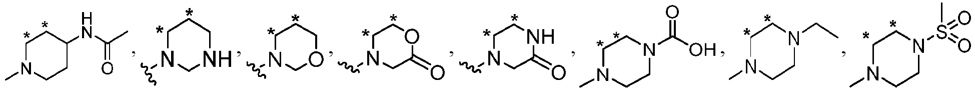

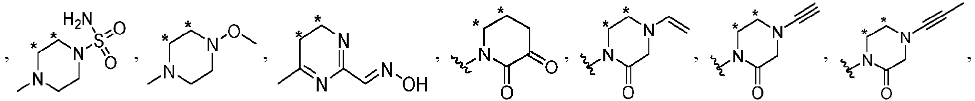

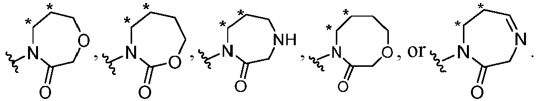

In a preferable embodiment according to the first aspect of this invention, in any of the preceding embodiments of the first aspect, each R_{C} is independently hydrogen, deuterium, halogen, oxo, oxime, -CN, -OH, -SH, -NO₂, -NH₂, - ZC(O)OR₁, -ZNR₂C(O)R₄, -Z-S(O)₂R₄, -Z-S(O)₂NR₂R₃, C₁₋₆ alkyl, C₂-₆ alkenyl, C₂-₆ alkynyl, C₁₋₆ alkoxy, C₁₋₆ alkylthio, C₃₋₆ cycloalkyl, 3-8 membered heterocyclyl, 5-8 membered heteroaryl, or C₆-₁₂ aryl; the alkyl, alkenyl, alkynyl, alkoxy, alkylthio, cycloalkyl, heterocyclyl, heteroaryl or aryl is optionally substituted by one or more substituents selected from hydrogen, deuterium, halogen, oxo, oxime, -CN, -OH, -NO₂, -NH₂, C₁₋₆ alkyl, phenyl, C₁₋₆ alkoxy, C₁₋₆ haloalkyl, C₁₋₆ haloalkoxy, and halophenyl;
or, each R_{C} is independently hydrogen, deuterium, halogen, oxo, oxime, -CN, -OH, -SH, -NO₂, -NH₂, -ZC(O)OR₁, - ZNR₂C(O)R₄, -Z-S(O)₂R₄, -Z-S(O)₂NR₂R₃, C₁₋₃ alkyl, C₂-₄ alkenyl, C₂-₄ alkynyl, C₁₋₆ alkoxy, C₁₋₆ alkylthio, C₃₋₆ cycloalkyl, 3-6 membered heterocyclyl, 5-8 membered heteroaryl, or C₆-₁₂ aryl; the alkyl, alkenyl, alkynyl, alkoxy, alkylthio, cycloalkyl, heterocyclyl, heteroaryl or aryl is optionally substituted by one or more substituents selected from hydrogen, deuterium, halogen, oxo, oxime, -CN, -OH, -NO₂, -NH₂, C₁₋₆ alkyl, C₁₋₆ alkoxy, C₁₋₆ haloalkyl, and C₁₋₆ haloalkoxy;
or, each R_{C} is independently hydrogen, halogen, oxo, oxime, -CN, -OH, -SH, -NH₂, -C(O)OC₁₋₆ alkyl, carboxyl, - NHC(O)C₁₋₆ alkyl, -S(O)₂C₁₋₆ alkyl, -S(O)₂NH₂, -CH₂S(O)₂C₁₋₆ alkyl, C₁₋₃ alkyl, C₂-₄ alkenyl, C₂-₄ alkynyl, C₁₋₃ alkoxy, C₁₋₃ alkylthio, C₃₋₆ cycloalkyl, 3-6 membered heterocyclyl, 5-8 membered heteroaryl, or C₆-₁₂ aryl; the alkyl, alkenyl, alkynyl, alkoxy, alkylthio, cycloalkyl, heterocyclyl, heteroaryl or aryl is optionally substituted by one or more substituents selected from halogen, oxo, oxime, -CN, -OH, -NO₂, -NH₂, C₁₋₃ alkyl, C₁₋₃ alkoxy, C₁₋₃ haloalkyl, and C₁₋₃ haloalkoxy;
or, each R_{C} is independently hydrogen, F, Cl, Br, oxo, oxime, -CN, -OH, -SH, -NH₂, -C(O)OC₁₋₄ alkyl, carboxyl, - NHC(O)C₁₋₄ alkyl, -S(O)₂C₁₋₄ alkyl, -S(O)₂NH₂, -CH₂S(O)₂C₁₋₄ alkyl, tetrahydrofuryl, pyrrolyl, phenyl, methyl, ethyl, n-propyl, isopropyl, cyclopropyl, cyclobutyl, cyclopentyl, cyclohexyl, ethenyl, propenyl, ethynyl, propynyl, methoxy, methylthio, trifluoromethyl, difluoromethyl, monofluoromethyl, trifluoroethyl, difluoroethyl, monofluoroethyl, hydroxypropyl, hydroxyethyl, hydroxymethyl, methoxypropyl, methoxyethyl, or methoxymethyl. or, each Rc is independently hydrogen, Cl, oxo, oxime, -CN, -OH, -SH, -NH₂, -Boc, -COCH₂OH, -NHC(O)CH₃, carboxyl, -S(O)₂CH₃, -S(O)₂NH₂, -CH₂S(O)₂CH₃, methoxy, methylthio, -CH₂CH₂CH₂OH, -CH₂CH₂CH₂OCH₃, ethyl, 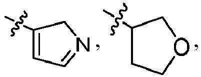 -CH₂CF₃, cyclopropyl, phenyl, or ethenyl.

In a preferable embodiment according to the first aspect of this invention, in any of the preceding embodiments of the first aspect, n3 is 1, 2, or 3.

In a preferable embodiment according to the first aspect of this invention, in any of the preceding embodiments of the first aspect, 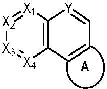 is selected from:

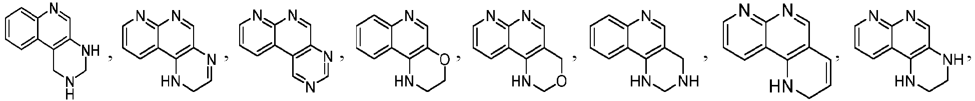

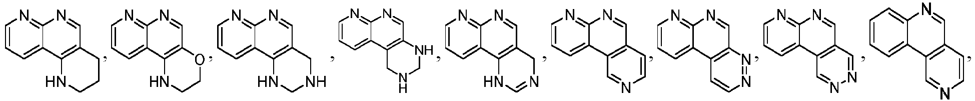

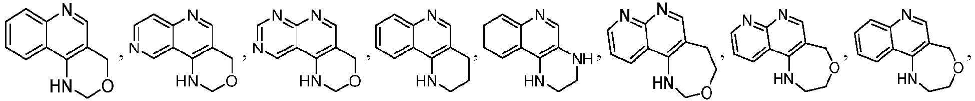

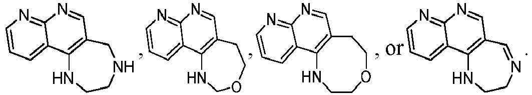

In a preferable embodiment according to the first aspect of this invention, in any of the preceding embodiments of the first aspect, ring B is C₅-₁₀ cycloalkyl, 3-20 membered heterocyclyl, C₆-₁₄ aryl or 5-16 membered heteroaryl;
or, ring B is C₅-₇ cycloalkyl, 5-7 membered monocyclic heterocyclyl, 5-14 membered spiro-heterocyclyl, 5-14 membered fused-heterocyclyl, C₆-₁₀ aryl, 5-6 membered monocyclic-heteroaryl or 5-14 membered fused-heteroaryl; or, ring B is C₆ cycloalkyl, C₇ cycloalkyl, 6-membered monocyclic heterocyclyl, 7-membered monocyclic heterocyclyl, 4-membered/4-membered spiro-heterocyclyl, 4-membered/5-membered spiro-heterocyclyl, 5-membered/4-membered spiro-heterocyclyl, 5-membered/5-membered spiro-heterocyclyl, 4-membered/6-membered spiro-heterocyclyl, 6-membered/4-membered spiro-heterocyclyl, 5-membered/6-membered spiro-heterocyclyl, 6-membered/5-membered spiro-heterocyclyl, 6-membered/6-membered spiro-heterocyclyl, 4-membered/4-membered fused-heterocyclyl, 4-membered/5-membered fused-heterocyclyl, 5-membered/4-membered fused-heterocyclyl, 5-membered/5-membered fused-heterocyclyl, 5-membered/6-membered fused-heterocyclyl, 6-membered/5-membered fused-heterocyclyl, 4-membered/6-membered fused-heterocyclyl, 6-membered/4-membered fused-heterocyclyl, 6-membered/6-membered fused-heterocyclyl, phenyl, naphthyl, 5-membered monocyclic-heteroaryl, 6-membered monocyclic-heteroaryl, 5-membered/5-membered fused-heteroaryl, 5-membered/6-membered fused-heteroaryl, 6-membered/5-membered fused-heteroaryl, 6-membered/6-membered fused-heteroaryl, the heteroatoms in the above-mentioned heterocyclyl, heteroaryl, fused-heterocyclyl, fused-heteroaryl, spiro-heterocyclyl are independently selected from O, N and S, the number of heteroatoms is 1, 2, or 3;
or, ring B is 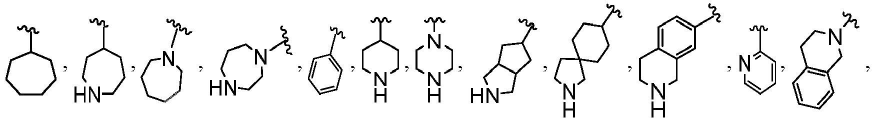 or

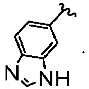

In a preferable embodiment according to the first aspect of this invention, in any of the preceding embodiments of the first aspect, R_{D} is selected from -W-C(O)NR₂R₃, -W-C(O)NR₂OR₁, -W-OC(O)NR₂R₃, -W-NR₂C(O)R₄, -W-NR₂C(O)OR₁, -W-NR₂C(O)NR₂R₃, -W-S(O)₂R₄, -W-SO₂NR₂R₃, -W-NR₂S(O)₂R₄, -W-OS(O)₂R₄, -W-NR₂S(O)₂NR₂R₃, -W-OS(O)₂NR₂R₃, -W-P(O)(OR₁)₂, -W-P(S)(OR₁)₂, -W-O-P(S)(OR₁)₂, -W-B(OH)₂, and 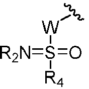 ; or R_{D} is selected from -W-C(O)NR₂R₃, -W-C(O)NR₂OR₁, -W-OC(O)NR₂R₃, -W-NR₂C(O)R₄, -W-NR₂C(O)OR₁, - W-NR₂C(O)NR₂R₃, -W-S(O)₂R₄, -W-SO₂NR₂R₃, -W-NR₂S(O)₂R₄, -W-OS(O)₂R₄, -W-NR₂S(O)₂NR₂R₃, -W-OS(O)₂NR₂R₃, -W-P(O)(OR₁)₂, -W-P(S)(OR₁)₂, -W-O-P(S)(OR₁)₂, and -W-B(OH)₂;
or, R_{D} is selected from -C₀₋₃ alkylene-C(O)NHOH, -C₀₋₃ alkylene-OC(O)NH₂, -C₀₋₃ alkylene-SO₂NH₂, -C₀₋₃ alkylene-NHS(O)zH, -C₀₋₃ alkylene-OS(O)zH, -C₀₋₃ alkylene-NHS(O)₂NH₂, -C₀₋₃ alkylene-OS(O)₂NH₂, -C₀₋₃ alkylene-P(O)(OH)₂, -C₀₋₃ alkylene-P(S)(OH)₂, -C₀₋₃ alkylene-O-P(S)(OH)₂, -C₀₋₃ alkylene-B(OH)₂, -C₀₋₃ alkylene-S(O)zOH, -C₀₋₃ alkylene-C(O)NH₂, -C₀₋₃ alkylene-S(O)₂C₁₋₃ alkyl, -C₀₋₃ alkylene-OS(O)₂-C₁₋₃ alkyl, -C₀₋₃ alkylene-S(O)₂-C₁₋₃ alkylene-C(O)OH, -C₀₋₃ alkylene-S(O)₂C₁₋₃ alkylene-NH₂, -C₀₋₃ alkylene-S(O)₂NHCH₂C(O)OH, -C₀₋₃ alkylene-NHS(O)₂CH₃, -C₀₋₃ alkylene-S(O)₂ND₂, -C₀₋₃ alkylene-S(O)₂NHNH₂, -C₀₋₃ alkylene-S(O)₂NHSC₁₋₃ alkyl, -C₀₋₃ alkylene-NHS(O)₂C₁₋₃ alkyl, -C₀₋₃ alkylene-NH-C(O)C₁₋₃ alkyl, -C₀₋₃ alkylene-NH-CONH₂, -C₀₋₃ alkylene-NH-COOC₁₋₃ alkyl, -C₀₋₃ alkylene-S(O)₂NHOH, and 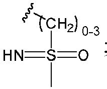 ; or R_{D} is selected from -C₀₋₃ alkylene-C(O)NHOH, -C₀₋₃ alkylene-OC(O)NH₂, -C₀₋₃ alkylene-SO₂NH₂, -C₀₋₃ alkylene-NHS(O)₂H, -C₀₋₃ alkylene-OS(O)₂H, -C₀₋₃ alkylene-NHS(O)₂NH₂, -C₀₋₃ alkylene-OS(O)₂NH₂, -C₀₋₃ alkylene-P(O)(OH)₂, -C₀₋₃ alkylene-P(S)(OH)₂, -C₀₋₃ alkylene-OP(S)(OH)₂, -C₀₋₃ alkylene-B(OH)₂, -C₀₋₃ alkylene-S(O)₂OH, -C₀₋₃ alkylene-C(O)NH₂, -C₀₋₃ alkylene-S(O)₂C₁₋₃ alkyl, -C₀₋₃ alkylene-OS(O)₂-C₁₋₃ alkyl, -C₀₋₃ alkylene-S(O)₂-C₁₋₃ alkylene-C(O)OH, -C₀₋₃ alkylene-S(O)₂C₁₋₃ alkylene-NH₂, -C₀₋₃ alkylene-S(O)₂NHCH₂C(O)OH, -C₀₋₃ alkylene-NHS(O)₂CH₃, -C₀₋₃ alkylene-S(O)₂ND₂, -C₀₋₃ alkyleneS(O)₂NHNH₂, -C₀₋₃ alkylene-S(O)₂NHSC₁₋₃ alkyl, -C₀₋₃ alkylene-NHS(O)₂C₁₋₃ alkyl, -C₀₋₃ alkylene-NH-C(O)C₁₋₃ alkyl, -C₀₋₃ alkylene-NH-CONH₂, -C₀₋₃ alkylene-NH-COOC₁₋₃ alkyl, and -C₀₋₃ alkylene-S(O)₂NHOH;
or, R_{D} is selected from -methylene-C(O)NHOH, -methylene-OC(O)NH₂, -methylene-SO₂NH₂, -methylene-NHS(O)₂H, -methylene-OS(O)₂H, -methylene-NHS(O)₂NH₂, -methylene-OS(O)₂NH₂, -methylene-P(O)(OH)₂, - methylene-P(S)(OH)₂, -methylene-O-P(S)(OH)₂, -methylene-B(OH)₂, -C(O)NHOH, -OC(O)NH₂, -SO₂NH₂, - NHS(O)₂H, -OS(O)₂H, -NHS(O)₂NH₂, -OS(O)₂NH₂, -P(O)(OH)₂, -P(S)(OH)₂, -O-P(S)(OH)₂, -B(OH)₂; -S(O)₂OH, -C(O)NH₂, -S(O)₂C₁₋₃ alkyl, -OS(O)₂C₁₋₃ alkyl, -S(O)₂C₁₋₃ alkylene-C(O)OH, -S(O)₂C₁₋₃ alkylene-NH₂, - S(O)₂NHCH₂C(O)OH, -NHS(O)₂CH₃, -S(O)₂ND₂, -S(O)₂NHNH₂, -methylene-NHS(O)₂C₁₋₃ alkyl, -methylene-NH-C(O)C₁₋₃ alkyl, -methylene-NH-CONH₂, -NH-COOC₁₋₃ alkyl, -S(O)₂NHOH, and ; 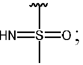 or, R_{D} is selected from - methylene-C(O)NHOH, -methylene-OC(O)NH₂, -methylene-SO₂NH₂, -methylene-NHS(O)₂H, -methylene-OS(O)₂H, -methylene-NHS(O)₂NH₂, -methylene-OS(O)₂NH₂, -methylene-P(O)(OH)₂, -methylene-P(S)(OH)₂, - methylene-O-P(S)(OH)₂, -methylene-B(OH)₂, -C(O)NHOH, -OC(O)NH₂, -SO₂NH₂, -NHS(O)₂H, -OS(O)₂H, - NHS(O)₂NH₂, -OS(O)₂NH₂, -P(O)(OH)₂, -P(S)(OH)₂, -O-P(S)(OH)₂, -B(OH)₂; -S(O)₂OH, -C(O)NH₂, -S(O)₂C₁₋₃ alkyl, -OS(O)₂C₁₋₃ alkyl, -S(O)₂C₁₋₃ alkylene-C(O)OH, -S(O)₂C₁₋₃ alkylene-NH₂, -S(O)₂NHCH₂C(O)OH, - NHS(O)₂CH₃, -S(O)₂ND₂, -S(O)₂NHNH₂, -methylene-NHS(O)₂C₁₋₃ alkyl, -methylene-NH-C(O)C₁₋₃ alkyl, - methylene-NH-CONH₂, -NH-COOC₁₋₃ alkyl, and -S(O)₂NHOH;
or, R_{D} is selected from -C(O)NHOH, -SO₂NH₂, -methylene-NHS(O)₂NH₂, -NHS(O)₂NH₂, -B(OH)₂, -P(O)(OH)₂, - OP(S)(OH)₂, -OS(O)₂NH₂, -S(O)₂OH, -C(O)NH₂, -S(O)₂CH₃, -OS(O)₂CH₃, -S(O)₂CH₂CH₂C(O)OH, - S(O)₂CH₂NH₂, -S(O)₂NHCH₂C(O)OH, -NHS(O)₂CH₃, -S(O)₂ND₂, -S(O)₂NHNH₂, -methylene-NHS(O)₂CH₃, - methylene-NH-C(O)CH₃, -methylene-NH-CONH₂, -NH-COOCH₃, -S(O)₂NHOH, and 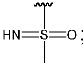 ; or, R_{D} is selected from -C(O)NHOH, -SO₂NH₂, -methylene-NHS(O)₂NH₂, -NHS(O)₂NH₂, -B(OH)₂, -P(O)(OH)₂, -OP(S)(OH)₂, - OS(O)₂NH₂, -S(O)₂OH, -C(O)NH₂, -S(O)₂CH₃, -OS(O)₂CH₃, -S(O)₂CH₂CH₂C(O)OH, -S(O)₂CH₂NH₂, - S(O)₂NHCH₂C(O)OH, -NHS(O)₂CH₃, -S(O)₂ND₂, -S(O)₂NHNH₂, -methylene-NHS(O)₂CH₃, -methylene-NH-C(O)CH₃, -methylene-NH-CONH₂, -NH-COOCH₃, and -S(O)₂NHOH.

In a preferable embodiment according to the first aspect of this invention, in any of the preceding embodiments of the first aspect, R_{E} is each independently hydrogen, deuterium, halogen, oxo, oxime, -CN, -OH, -SH, -NO₂, -NH₂, C₁₋₆ alkyl, C₂-₆ alkenyl, C₂-₆ alkynyl, C₁₋₆ alkoxy, or C₃-₈ cycloalkyl, the alkyl, alkenyl, alkynyl, alkoxy, or cycloalkyl is optionally substituted by one or more substituents selected from hydrogen, deuterium, halogen, oxo, oxime, -CN, -OH, -NO₂, -NH₂, C₁₋₆ alkyl, phenyl, C₁₋₆ alkoxy, C₁₋₆ haloalkyl, C₁₋₆haloalkoxy, and halophenyl;
or, R_{E} is each independently hydrogen, deuterium, halogen, oxo, oxime, -CN, -OH, -SH, -NO₂, -NH₂, C₁₋₃alkyl, C₁₋₃alkoxy, or C₃₋₆cycloalkyl, the alkyl, alkoxy, or cycloalkyl is optionally substituted by one or more substituents selected from hydrogen, deuterium, halogen, oxo, oxime, -CN, -OH, -NO₂, -NH₂, C₁₋₃alkyl, C₁₋₃ alkoxy, C₁₋₃ haloalkyl, and C₁₋₃ haloalkoxy;
or, R_{E} is each independently hydrogen, halogen, -OH, -SH, C₁₋₃alkyl, C₁₋₃alkoxy, or C₃₋₆ cycloalkyl, the alkyl, alkoxy, or cycloalkyl is optionally substituted by one or more substituents selected from hydrogen, deuterium, halogen, oxo, -CN, -OH, -NO₂, -NH₂, C₁₋₃ alkyl, C₁₋₃ alkoxy, C₁₋₃ haloalkyl, and C₁₋₃ haloalkoxy;
or, R_{E} is each independently hydrogen, F, Cl, Br, -OH, -SH, -methyl, ethyl, n-propyl, isopropyl, cyclopropyl, methoxy, methylthio, trifluoromethyl, difluoromethyl, monofluoromethyl, trifluoroethyl, difluoroethyl, monofluoroethyl, hydroxypropyl, hydroxyethyl, hydroxymethyl, methoxypropyl, methoxyethyl, or methoxymethyl; or, R_{E} is each independently hydrogen, deuterium, F, Cl, carboxyl, -CN, -NH₂, methyl, methylthio, -CF₃, methoxy, - CH₂NH₂, -CH₂OH, -CH₂NHOH, -CH=NOH, -CH₂CH₂OH, -CHF₂, -C(O)OCH₃, -C(O)CH₃, 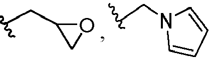 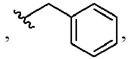 cyclopropyl, ethenyl, ethynyl, propynyl, ethyl, propenyl.

In a preferable embodiment according to the first aspect of this invention, in any of the preceding embodiments of the first aspect, n4 is 1, 2, 3 or 4; or, n4 is 1, 2, or 3.

In a preferable embodiment according to the first aspect of this invention, in any of the preceding embodiments of the first aspect, L is a bond, -O-, -S-, C₁₋₃ alkylene, C₁₋₃ alkyleneoxy, C₃₋₆ cycloalkylene, C₁₋₃ alkylenethio, C₂-₆ alkenylene, or C₂-₆ alkynylene, the alkylene, alkyleneoxy, cycloalkylene, alkylenethio, alkenylene, or alkynylene is optionally substituted by one or more substituents selected from deuterium, halogen, C₁₋₃ alkyl, C₁₋₃ alkoxy, oxo, - CN, -OH, and -NH₂;
or, L is a bond, -O-, -S-, methylene, ethylene, propylene, cyclopropylene, cyclobutylene, C₁₋₃alkyleneoxy, C₁₋₃alkylenethio, ethenylene, or ethynylene, the methylene, ethylene, propylene, cyclopropylene, cyclobutylene, alkyleneoxy, alkylenethio, ethenylene, or ethynylene is optionally substituted by one or more substituents selected from deuterium, halogen, methyl, ethyl, n-propyl, isopropyl, methoxy, ethoxy, propoxy, isopropoxy, oxo, -CN, -OH, and -NH₂;
or, L is a bond, -O-, -S-, cyclopropylene, methylenemethoxy, methylenemethyl, methylene-OH, methylene-NH₂, ethylene, or propylene;
or, L is a bond, -O-, -CH₂-,

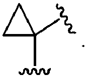

The second aspect of this invention provides a compound represented by the following formula (I), a stereoisomer or tautomer of the compound or a mixture thereof, a pharmaceutically acceptable salt, co-crystal, polymorph or solvate of the compound, or a stable isotope derivative, metabolite or prodrug of the compound: 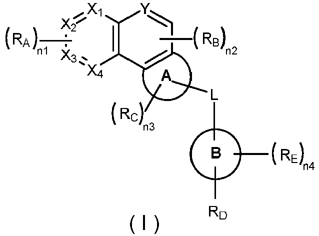
wherein X₁, X₂, X₃, X₄ and Y are each independently CH or N;
and when X₁, X₂, X₃ and X₄ are all CH, Y is not CH;
R_{A} is a substituent of the ring where X₁ is located, R_{B} is a substituent of the ring where Y is located, R_{A} and R_{B} are each independently hydrogen, deuterium, halogen, oxime, -CN, -OH, -SH, -NO₂, -NH₂, -Z-OR₁, -Z-SR₁, -Z-NR₂R₃, -Z-NR₂C(O)R₄, -Z-NR₂C(O)OR₁, -Z-C(O)R₄, -Z-C(O)OR₁, -Z-C(O)(CR₅R₆)ₙC(O)R₄, -Z-C(O)(CR₅R₆)ₙC(O)OR₁, - Z-C(O)NR₂R₃, -Z-S(O)₂R₄, -Z-S(O)₂NR₂R₃, C₁₋₆ alkyl, C₂-₆ alkenyl, C₂-₆ alkynyl, C₁₋₆ alkoxy, C₁₋₆ alkylthio, C₃-₈ cycloalkyl, C₃-₈ cycloalkenyl, 3-20 membered heterocyclyl, 5-16 membered heteroaryl; the alkyl, alkenyl, alkynyl, alkoxy, alkylthio, cycloalkyl, cycloalkenyl, heterocyclyl, or heteroaryl is optionally substituted by one or more substituents selected from deuterium, halogen, oxo, oxime, -CN, -OH, -NO₂, -NH₂, C₁₋₆ alkyl, phenyl, C₁₋₆ alkoxy, C₁₋₆ haloalkyl, C₁₋₆ haloalkoxy, and halophenyl;
n1 is 1, 2, 3 or 4;
n2 is 1 or 2;
ring A is 6-10 membered heterocyclyl or 6-12 membered heteroaryl;
R_{C} is a substituent of ring A, and R_{C} is each independently hydrogen, deuterium, halogen, oxo, oxime, -CN, -OH, - SH, -NO₂, -NH₂, -Z-OR₁, -Z-SR₁, -Z-NR₂R₃, -Z-NR₂C(O)R₄, -Z-NR₂C(O)OR₁, -Z-C(O)R₄, -Z-C(O)OR₁, -Z-C(O)(CR₅R₆)ₙC(O)R₄, -Z-C(O)(CR₅R₆)ₙC(O)OR₁, -Z-C(O)NR₂R₃, -Z-S(O)₂R₄, -Z-S(O)₂NR₂R₃, C₁₋₆ alkyl, C₂-₆ alkenyl, C₂-₆ alkynyl, C₁₋₆ alkoxy, C₁₋₆ alkylthio, C₃-₈ cycloalkyl, C₃-₈ cycloalkenyl, 3-20 membered heterocyclyl, 5-16 membered heteroaryl; the alkyl, alkenyl, alkynyl, alkoxy, alkylthio, cycloalkyl, cycloalkenyl, heterocyclyl, or heteroaryl is optionally substituted by one or more substituents selected from deuterium, halogen, oxo, oxime, -CN, -OH, -NO₂, -NH₂, C₁₋₆ alkyl, phenyl, C₁₋₆ alkoxy, C₁₋₆haloalkyl, C₁₋₆haloalkoxy, and halophenyl;
n3 is 1, 2, 3, 4, 5, or 6;
Z is selected from a bond, C₁₋₃ alkylene, C₁₋₃ alkyleneoxy, and C₁₋₃ alkylenethio, the alkylene, alkyleneoxy, or alkylenethio is optionally substituted by one or more substituents selected from deuterium, halogen, oxo, -CN, -OH, and -NH₂;
ring B is C₃-₁₀ cycloalkyl, 3-20 membered heterocyclyl, C₆-₁₄ aryl or 5-16 membered heteroaryl;
R_{D} is selected from -W-OC(O)OR₁, -W-C(O)NR₂R₃, -W-C(O)NR₂OR₁, -W-OC(O)NR₂R₃, -W-NR₂C(O)R₄, -W-NR₂C(O)OR₁, -W-NR₂C(O)NR₂R₃, -W-S(O)₂R₄, -W-SO₂NR₂R₃, -W-NR₂S(O)₂R₄, -W-OS(O)₂R₄, -W-NR₂S(O)₂NR₂R₃, -W-OS(O)₂NR₂R₃, -W-P(O)(OR₁)₂, -W-P(S)(OR₁)₂, -W-O-P(S)(OR₁)₂, and -W-B(OH)₂;
each R_{E} is independently hydrogen, deuterium, halogen, oxo, oxime, -CN, -OH, -SH, -NO₂, -NH₂, -W-OR₁, -W-SR₁, -W-C(O)R₄, -W-C(O)OR₁, -W-OC(O)R₁, -W-OC(O)OR₁, -W-C(O)NR₂R₃, -W-C(O)NR₂OR₁, -W-OC(O)NR₂R₃, - W-NR₂R₃, -W-NR₂C(O)R₄, -W-NR₂C(O)OR₁, -W-NR₂C(O)NR₂R₃, -W-S(O)₂R₄, -W-SO₂NR₂R₃, -W-NR₂S(O)₂R₄, -W-OS(O)₂R₄, -W-NR₂S(O)₂NR₂R₃, -W-OS(O)₂NR₂R₃, -W-P(O)(OR₁)₂, -W-P(S)(OR₁)₂, -W-O-P(S)(OR₁)₂, -W-B(OH)₂, C₁₋₆ alkyl, C₂-₆ alkenyl, C₂-₆ alkynyl, C₁₋₆ alkoxy, C₃-₈ cycloalkyl, C₃-₈ cycloalkenyl, 3-20 membered heterocyclyl, 5-16 membered heteroaryl; the alkyl, alkenyl, alkynyl, alkoxy, cycloalkyl, cycloalkenyl, heterocyclyl, or heteroaryl is optionally substituted by one or more substituents selected from deuterium, halogen, oxo, oxime, - CN, -OH, -NO₂, -NH₂, C₁₋₆ alkyl, phenyl, C₁₋₆ alkoxy, C₁₋₆ haloalkyl, C₁₋₆ haloalkoxy, and halophenyl;
n4 is 1, 2, 3, 4, 5, or 6;
W is selected from a bond, C₁₋₃ alkylene, C₁₋₃ alkyleneoxy, and C₁₋₃ alkylenethio, the alkylene, alkyleneoxy, or alkylenethio is optionally substituted by one or more substituents selected from deuterium, halogen, oxo, -CN, -OH, and -NH₂;
L is selected from a bond, -O-, -S-, C₁₋₆ alkylene, C₁₋₆ alkyleneoxy, C₃₋₆ cycloalkylene, C₁₋₆ alkylenethio, C₂-₆ alkenylene, and C₂-₆ alkynylene, the alkylene, alkyleneoxy, cycloalkylene, alkylenethio, alkenylene, or alkynylene is optionally substituted by one or more substituents selected from deuterium, halogen, C₁₋₃ alkyl, C₁₋₆ alkoxy, oxo, - CN, -OH, and -NH₂;
R₁ at each occurrence is independently hydrogen, C₁₋₆ alkyl, C₁₋₆ alkoxy, C₃-₈ cycloalkyl, phenyl, or 3-20 membered heterocyclyl, wherein the alkyl, alkoxy, cycloalkyl, phenyl, or heterocyclyl is optionally substituted by one or more substituents selected from halogen, cyano, hydroxy, amino, C₁₋₃ alkyl, C₁₋₄ alkoxy, phenyl, C₁₋₃ haloalkyl, C₁₋₄ haloalkoxy, and halophenyl;
R₂ and R₃ at each occurrence are independently hydrogen, C₁₋₆ alkyl, or C₁₋₆ alkoxy, wherein the alkyl, or alkoxy is optionally substituted by one or more substituents selected from halogen, cyano, hydroxy, amino, C₁₋₃ alkyl, C₁₋₄ alkoxy, phenyl, C₁₋₃ haloalkyl, C₁₋₃ haloalkoxy, and halophenyl;
R₄ at each occurrence is independently hydrogen, deuterium, C₁₋₆ alkyl, C₁₋₆ alkoxy, C₃-₈ cycloalkyl, phenyl, or 3-20 membered heterocyclyl, wherein the alkyl, alkoxy, cycloalkyl, phenyl, or heterocyclyl is optionally substituted by one or more substituents selected from halogen, cyano, hydroxy, amino, C₁₋₃ alkyl, C₁₋₄ alkoxy, phenyl, C₁₋₃ haloalkyl, C₁₋₃ haloalkoxy, and halophenyl;
R₅ and R₆ at each occurrence are independently hydrogen, C₁₋₃ alkyl, C₂-₆ alkenyl, C₂-₆ alkynyl, C₁₋₆ alkoxy, C₁₋₃ alkylthio, C₃₋₆ cycloalkyl, C₃₋₆ cycloalkenyl, 3-20 membered heterocyclyl, or 5-16 membered heteroaryl, wherein the alkyl, alkenyl, alkynyl, alkoxy, alkylthio, cycloalkyl, cycloalkenyl, heterocyclyl, or heteroaryl is optionally substituted by one or more substituents selected from deuterium, halogen, oxo, oxime, -CN, -OH, -NH₂, C₁₋₃ alkyl, and C₁₋₃ haloalkyl.
In a preferable embodiment according to the second aspect of this invention, X₁ is N, and X₂, X₃, and X₄ are all CH. In a preferable embodiment according to the second aspect of this invention, X₃ is N, and X₁, X₂, and X₄ are all CH. In a preferable embodiment according to the second aspect of this invention, X₁ and X₃ are both N, and X₂ and X₄ are both CH.
In a preferable embodiment according to the second aspect of this invention, X₁, X₂, X₃, and X₄ are all CH.

In a preferable embodiment according to the first aspect of this invention, in any of the preceding embodiments of the first aspect, Y is CH; or Y is N.

In a preferable embodiment according to the first aspect of this invention, in any of the preceding embodiments of the first aspect, each R_{A} is independently hydrogen, deuterium, halogen, oxime, -CN, -OH, -SH, -NO₂, -NH₂, C₁₋₆ alkyl, C₂-₆ alkenyl, C₂-₆ alkynyl, C₁₋₆ alkoxy, C₁₋₆ alkylthio, or C₃-₈ cycloalkyl, the alkyl, alkenyl, alkynyl, alkoxy, alkylthio, or cycloalkyl is optionally substituted by one or more substituents selected from deuterium, halogen, oxo, oxime, -CN, -OH, -NO₂, -NH₂, C₁₋₆ alkyl, phenyl, C₁₋₆ alkoxy, C₁₋₆haloalkyl, C₁₋₆ haloalkoxy, and halophenyl;
or, each R_{A} is independently hydrogen, deuterium, halogen, -CN, -OH, -SH, -NO₂, -NH₂, C₁₋₃ alkyl, C₂-₆ alkenyl, C₂-₆ alkynyl, C₁₋₆ alkoxy, C₁₋₆ alkylthio, or C₃₋₆ cycloalkyl, the alkyl, alkenyl, alkynyl, alkoxy, alkylthio, or cycloalkyl is optionally substituted by one or more substituents selected from deuterium, halogen, oxo, oxime, -CN, -OH, -NO₂, - NH₂, C₁₋₆ alkyl, phenyl, C₁₋₆ alkoxy, C₁₋₆ haloalkyl, C₁₋₆ haloalkoxy, and halophenyl;
or, each R_{A} is independently hydrogen, deuterium, halogen, -CN, -OH, -SH, -NO₂, -NH₂, C₁₋₃ alkyl, C₂-₄ alkenyl, C₂-₄ alkynyl, C₁₋₃ alkoxy, C₁₋₃ alkylthio, or C₃₋₆ cycloalkyl, the alkyl, alkenyl, alkynyl, alkoxy, alkylthio, or cycloalkyl is optionally substituted by one or more substituents selected from deuterium, halogen, oxo, -CN, -OH, -NO₂, -NH₂, C₁₋₆ alkyl, C₁₋₆ alkoxy, C₁₋₆ haloalkyl, and C₁₋₆ haloalkoxy;
or, each R_{A} is independently hydrogen, deuterium, F, Cl, Br, -CN, -OH, -SH, -NO₂, -NH₂, methyl, ethyl, propyl, isopropyl, ethenyl, ethynyl, methoxy, ethoxy, propoxy, isopropoxy, methylthio, ethylthio, propylthio, isopropylthio, cyclopropyl, cyclobutyl, trifluoromethyl, difluoromethyl, monofluoromethyl, trifluoroethyl, difluoroethyl, monofluoroethyl, hydroxypropyl, hydroxyethyl, hydroxymethyl, methoxypropyl, methoxyethyl, or methoxymethyl; or, each R_{A} is independently hydrogen, F, Cl, Br, -OH, methyl, ethyl, methoxy, ethoxy;
or, R_{A} is hydrogen, F, Cl, or methoxy.

In a preferable embodiment according to the second aspect of this invention, n1 is 1, 2, or 3; more preferably, n1 is 1 or 2.

In a preferable embodiment according to the second aspect of this invention, each R_{B} is independently hydrogen, deuterium, halogen, oxime, -CN, -OH, -SH, -NO₂, -NH₂, C₁₋₃ alkyl, C₂-₆ alkenyl, C₂-₆ alkynyl, C₁₋₆ alkoxy, C₁₋₆ alkylthio, or C₃₋₆ cycloalkyl, the alkyl, alkenyl, alkynyl, alkoxy, alkylthio, or cycloalkyl is optionally substituted by one or more substituents selected from deuterium, halogen, oxo, oxime, -CN, -OH, -NO₂, -NH₂, C₁₋₆ alkyl, phenyl, C₁₋₆ alkoxy, C₁₋₆ haloalkyl, C₁₋₆ haloalkoxy, and halophenyl;
further preferably, each R_{B} is independently hydrogen, deuterium, F, Cl, Br, -CN, -OH, -SH, -NO₂, -NH₂, methyl, ethyl, propyl, isopropyl, methoxy, ethoxy, propoxy, isopropoxy, methylthio, ethylthio, propylthio, isopropylthio, cyclopropyl, cyclobutyl, trifluoromethyl, difluoromethyl, monofluoromethyl, trifluoroethyl, difluoroethyl, monofluoroethyl, hydroxypropyl, hydroxyethyl, hydroxymethyl, methoxypropyl, methoxyethyl, or methoxymethyl; more preferably, each R_{B} is independently hydrogen, F, Cl, Br, -OH, methyl, ethyl, methoxy, ethoxy; most preferably, R_{B} is H.

In a preferable embodiment according to the second aspect of this invention, n2 is 1.

In a preferable embodiment according to the second aspect of this invention, ring A is 6-9 membered heterocyclyl or 6-12 membered heteroaryl;
preferably, ring A is 6-membered heterocyclyl or 6-membered heteroaryl;
preferably, ring A is piperidyl, hexahydropyrimidyl, piperazinyl, 1,3-oxazinanyl, morpholinyl, thiomorpholinyl, 1,3-thiazinyl, 1,2,3,6-tetrahydropyridyl, 1,2,3,6-tetrahydropyrazinyl, 1,4,5,6-tetrahydropyrimidyl, homopiperidyl, homopiperazinyl, homomorpholinyl, pyridyl, pyridazinyl, pyrimidyl;
preferably, ring A is 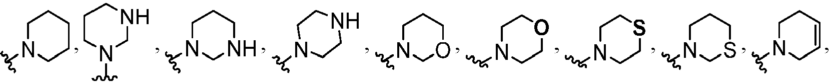 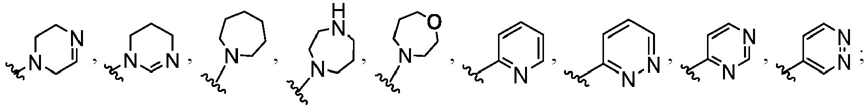 \ represents the attachment position of the L group.
still, further preferably, ring A is 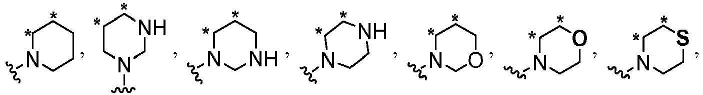 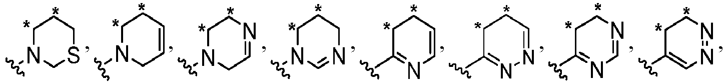 ** represents the site of fusing, 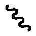 represents the attachment position of the L group.
preferably, when one or two R_{C}s therein are oxo, the above-mentioned ring A substituted by one or two R_{C}s is 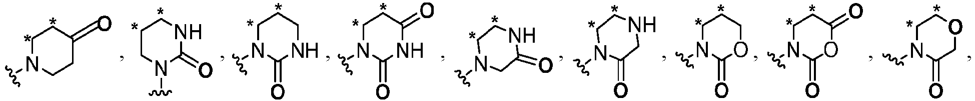 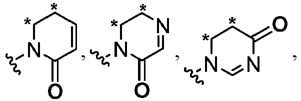 ** represents the site of fusing, \ represents the attachment position of the L group. more preferably, the ring A substituted by R_{C}(s) is

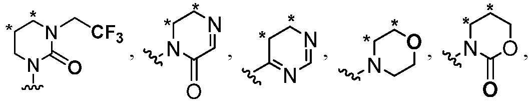

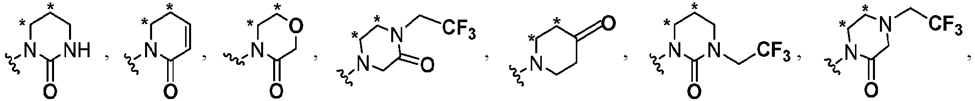

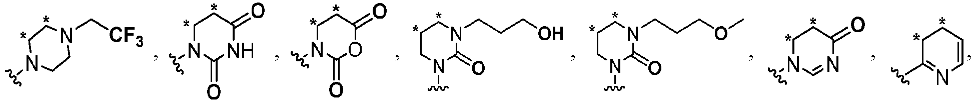

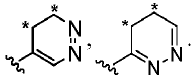

In a preferable embodiment according to the second aspect of this invention, in any of the preceding embodiments of the second aspect, each R_{C} is independently hydrogen, deuterium, halogen, oxo, oxime, -CN, -OH, -SH, -NO₂, - NH₂, C₁₋₆ alkyl, C₂-₆ alkenyl, C₂-₆ alkynyl, C₁₋₆ alkoxy, C₁₋₆ alkylthio, C₃₋₆ cycloalkyl; the alkyl, alkenyl, alkynyl, alkoxy, alkylthio, or cycloalkyl is optionally substituted by one or more substituents selected from hydrogen, deuterium, halogen, oxo, oxime, -CN, -OH, -NO₂, -NH₂, C₁₋₆ alkyl, phenyl, C₁₋₆ alkoxy, C₁₋₆ haloalkyl, C₁₋₆ haloalkoxy, and halophenyl;
or, each R_{C} is independently hydrogen, deuterium, halogen, oxo, oxime, -CN, -OH, -SH, -NO₂, -NH₂, C₁₋₃ alkyl, C₁₋₆ alkoxy, C₁₋₆ alkylthio, C₃₋₆ cycloalkyl; the alkyl, alkoxy, alkylthio, or cycloalkyl is optionally substituted by one or more substituents selected from hydrogen, deuterium, halogen, oxo, oxime, -CN, -OH, -NO₂, -NH₂, C₁₋₆ alkyl, C₁₋₆ alkoxy, C₁₋₆ haloalkyl, and C₁₋₆ haloalkoxy;
or, each R_{C} is independently hydrogen, halogen, oxo, C₁₋₃ alkyl, C₁₋₃ alkoxy, C₁₋₃ alkylthio, C₃₋₆ cycloalkyl; the alkyl, alkoxy, alkylthio, or cycloalkyl is optionally substituted by one or more substituents selected from halogen, oxo, oxime, -CN, -OH, -NO₂, -NH₂, C₁₋₃ alkyl, C₁₋₃ alkoxy, C₁₋₃ haloalkyl, and C₁₋₃ haloalkoxy;
or, each R_{C} is independently hydrogen, F, Cl, Br, oxo, methyl, ethyl, n-propyl, isopropyl, cyclopropyl, cyclobutyl, cyclopentyl, cyclohexyl, methoxy, methylthio, trifluoromethyl, difluoromethyl, monofluoromethyl, trifluoroethyl, difluoroethyl, monofluoroethyl, hydroxypropyl, hydroxyethyl, hydroxymethyl, methoxypropyl, methoxyethyl, or methoxymethyl;
or, each Rc is independently hydrogen, -CH₂CF₃, oxo, -CH₂CH₂CH₂OH, -CH₂CH₂CH₂OCH₃.

In a preferable embodiment according to the second aspect of this invention, in any of the preceding embodiments of the second aspect, n3 is 1, 2, or 3.

In a preferable embodiment according to the second aspect of this invention, in any of the preceding embodiments of the second aspect, 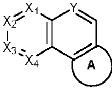 is selected from:

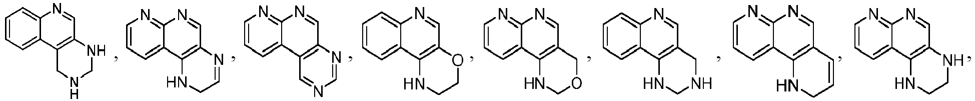

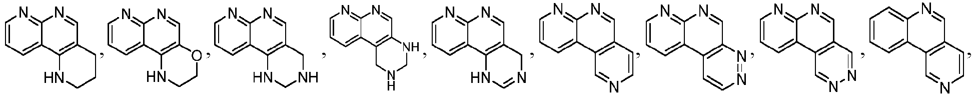

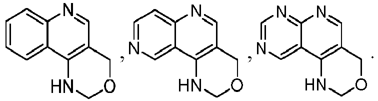

In a preferable embodiment according to the second aspect of this invention, in any of the preceding embodiments of the second aspect, ring B is C₅-₁₀cycloalkyl, 3-20 membered heterocyclyl, C₆-₁₄ aryl or 5-16 membered heteroaryl; or, ring B is C₅-₇ cycloalkyl, 5-7 membered monocyclic heterocyclyl, 5-14 membered spiro-heterocyclyl, 5-14 membered fused-heterocyclyl, C₆-₁₀ aryl, 5-6 membered monocyclic-heteroaryl or 5-14 membered fused-heteroaryl; or, ring B is C₆ cycloalkyl, C₇ cycloalkyl, 6-membered monocyclic heterocyclyl, 7-membered monocyclic heterocyclyl, 4-membered/4-membered spiro-heterocyclyl, 4-membered/5-membered spiro-heterocyclyl, 5-membered/4-membered spiro-heterocyclyl, 5-membered/5-membered spiro-heterocyclyl, 4-membered/6-membered spiro-heterocyclyl, 6-membered/4-membered spiro-heterocyclyl, 5-membered/6-membered spiro-heterocyclyl, 6-membered/5-membered spiro-heterocyclyl, 6-membered/6-membered spiro-heterocyclyl, 4-membered/4-membered fused-heterocyclyl, 4-membered/5-membered fused-heterocyclyl, 5-membered/4-membered fused-heterocyclyl, 5-membered/5-membered fused-heterocyclyl, 5-membered/6-membered fused-heterocyclyl, 6-membered/5-membered fused-heterocyclyl, 4-membered/6-membered fused-heterocyclyl, 6-membered/4-membered fused-heterocyclyl, 6-membered/6-membered fused-heterocyclyl, phenyl, naphthyl, 5-membered monocyclic-heteroaryl, 6-membered monocyclic-heteroaryl, 5-membered/5-membered fused-heteroaryl, 5-membered/6-membered fused-heteroaryl, 6-membered/5-membered fused-heteroaryl, 6-membered/6-membered fused-heteroaryl, the heteroatoms in the above-mentioned heterocyclyl, heteroaryl, fused-heterocyclyl, fused-heteroaryl, spiro-heterocyclyl are independently selected from O, N and S, the number of heteroatoms is 1, 2, or 3;
or, ring B is 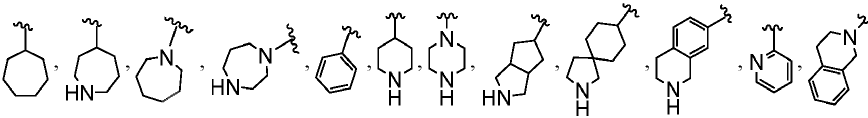 or 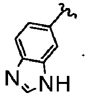
In a preferable embodiment according to the second aspect of this invention, in any of the preceding embodiments of the second aspect, R_{D} is selected from -W-C(O)NR₂OR₁, -W-OC(O)NR₂R₃, -W-NR₂C(O)R₄, -W-NR₂C(O)OR₁, - W-NR₂C(O)NR₂R₃, -W-S(O)₂R₄, -W-SO₂NR₂R₃, -W-NR₂S(O)₂R₄, -W-OS(O)₂R₄, -W-NR₂S(O)₂NR₂R₃, -W-OS(O)₂NR₂R₃, -W-P(O)(OR₁)₂, -W-P(S)(OR₁)₂, -W-O-P(S)(OR₁)₂, and -W-B(OH)₂;
or, R_{D} is selected from -methylene-C(O)NHOH, -methylene-OC(O)NH₂, -methylene-SO₂NH₂, -methylene-NHS(O)zH, -methylene-OS(O)₂H, -methylene-NHS(O)₂NH₂, -methylene-OS(O)₂NH₂, -methylene-P(O)(OH)₂, - methylene-P(S)(OH)₂, -methylene-O-P(S)(OH)₂, -methylene-B(OH)₂, -C(O)NHOH, -OC(O)NH₂, -SO₂NH₂, - NHS(O)zH, -OS(O)₂H, -NHS(O)₂NH₂, -OS(O)₂NH₂, -P(O)(OH)₂, -P(S)(OH)₂, -O-P(S)(OH)₂, and -B(OH)₂;
or, R_{D} is selected from -methylene-C(O)NHOH, -methylene-SO₂NH₂, -methylene-NHS(O)₂H, -methylene-NHS(O)₂NH₂, -methylene-P(O)(OH)₂, -methylene-P(S)(OH)₂, -methylene-O-P(S)(OH)₂, -methylene-B(OH)₂, - C(O)NHOH, -SO₂NH₂, -NHS(O)₂NH₂, -OS(O)₂NH₂, -P(O)(OH)₂, -P(S)(OH)₂, -O-P(S)(OH)₂, and -B(OH)₂;
or, R_{D} is selected from -C(O)NHOH, -SO₂NH₂, -methylene-NHS(O)₂NH₂, -NHS(O)₂NH₂, -B(OH)₂, -P(O)(OH)₂, - OP(S)(OH)₂, and -OS(O)₂NH₂.

In a preferable embodiment according to the second aspect of this invention, in any of the preceding embodiments of the second aspect, R_{E} is each independently hydrogen, deuterium, halogen, oxo, oxime, -CN, -OH, -SH, -NOz, - NH₂, C₁₋₆ alkyl, C₂-₆ alkenyl, C₂-₆ alkynyl, C₁₋₆ alkoxy, or C₃-₈ cycloalkyl, the alkyl, alkenyl, alkynyl, alkoxy, or cycloalkyl is optionally substituted by one or more substituents selected from hydrogen, deuterium, halogen, oxo, oxime, -CN, -OH, -NOz, -NH₂, C₁₋₆ alkyl, phenyl, C₁₋₆ alkoxy, C₁₋₆ haloalkyl, C₁₋₆ haloalkoxy, and halophenyl;
or, R_{E} is each independently hydrogen, deuterium, halogen, oxo, oxime, -CN, -OH, -SH, -NOz, -NH₂, C₁₋₃ alkyl, C₁₋₃ alkoxy, or C₃₋₆ cycloalkyl, the alkyl, alkoxy, or cycloalkyl is optionally substituted by one or more substituents selected from hydrogen, deuterium, halogen, oxo, oxime, -CN, -OH, -NOz, -NH₂, C₁₋₃ alkyl, C₁₋₃ alkoxy, C₁₋₃ haloalkyl, and C₁₋₃ haloalkoxy;
or, R_{E} is each independently hydrogen, halogen, -OH, -SH, C₁₋₃ alkyl, C₁₋₃ alkoxy, or C₃₋₆ cycloalkyl, the alkyl, alkoxy, or cycloalkyl is optionally substituted by one or more substituents selected from hydrogen, deuterium, halogen, oxo, -CN, -OH, -NOz, -NH₂, C₁₋₃ alkyl, C₁₋₃ alkoxy, C₁₋₃ haloalkyl, and C₁₋₃ haloalkoxy;
or, R_{E} is each independently hydrogen, F, Cl, Br, -OH, -SH, -methyl, ethyl, n-propyl, isopropyl, cyclopropyl, methoxy, methylthio, trifluoromethyl, difluoromethyl, monofluoromethyl, trifluoroethyl, difluoroethyl, monofluoroethyl, hydroxypropyl, hydroxyethyl, hydroxymethyl, methoxypropyl, methoxyethyl, or methoxymethyl; or, R_{E} is each independently hydrogen, F.

In a preferable embodiment according to the second aspect of this invention, in any of the preceding embodiments of the second aspect, n4 is 1, 2, 3 or 4; or, n4 is 1, 2, or 3.

In a preferable embodiment according to the second aspect of this invention, in any of the preceding embodiments of the second aspect, L is a bond, -O-, -S-, C₁₋₃ alkylene, C₁₋₃ alkyleneoxy, C₃₋₆ cycloalkylene, C₁₋₃ alkylenethio, C₂-₆ alkenylene, or C₂-₆ alkynylene, the alkylene, alkyleneoxy, cycloalkylene, alkylenethio, alkenylene, or alkynylene is optionally substituted by one or more substituents selected from deuterium, halogen, C₁₋₃ alkyl, C₁₋₃ alkoxy, oxo, - CN, -OH, and -NH₂;
or, L is a bond, -O-, -S-, methylene, ethylene, propylene, cyclopropylene, cyclobutylene, C₁₋₃ alkyleneoxy, C₁₋₃ alkylenethio, ethenylene, or ethynylene, the methylene, ethylene, propylene, cyclopropylene, cyclobutylene, alkyleneoxy, alkylenethio, ethenylene, or ethynylene is optionally substituted by one or more substituents selected from deuterium, halogen, methyl, ethyl, n-propyl, isopropyl, methoxy, ethoxy, propoxy, isopropoxy, oxo, -CN, -OH, and -NH₂;
or, L is a bond, -O-, -S-, cyclopropylene, methylenemethoxy, methylenemethyl, methylene-OH, methylene-NH₂, ethylene, or propylene;
or, L is a bond, -O-, -CH₂-,

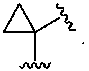

The third aspect of this invention further provides a compound represented by the following formula (I-a), a stereoisomer or tautomer of the compound or a mixture thereof, a pharmaceutically acceptable salt, co-crystal, polymorph or solvate of the compound, or a stable isotope derivative, metabolite or prodrug of the compound: 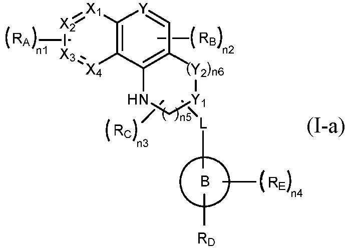
wherein Y₁ and Y₂ are independently selected from C, O or S, and Y₁ and Y₂ are different; n5 is 1 or 2; when Y₂ is not C, n6 is 1, when Y₂ is C, n6 is 1 or 2; X₁-X₄, Y, R_{A}, R_{B}, R_{C}, R_{D}, R_{E}, L, ring B, and n1-n4 are defined as in the compound of formula (I) according to any of the preceding embodiments of the first and second aspects of this invention;
In a preferable embodiment according to the third aspect of this invention, Y₁ is O, Y₂ is C;
In a preferable embodiment according to the third aspect of this invention, Y₁ is S, Y₂ is C;
In a preferable embodiment according to the third aspect of this invention, Y₂ is O, Y₁ is C;
In a preferable embodiment according to the third aspect of this invention, Y₂ is S, Y₁ is C;
In a preferable embodiment according to the third aspect of this invention, in any of the preceding embodiments of the third aspect, 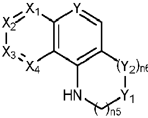 is selected from: 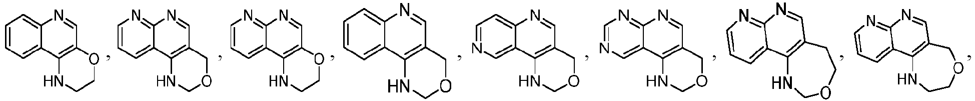 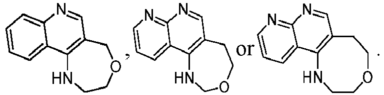
In a preferable embodiment according to the third aspect of this invention, in any of the preceding embodiments of the third aspect, at least one R_{C} is oxo.

The fourth aspect of this invention further provides a compound represented by the following formula (I-a1): 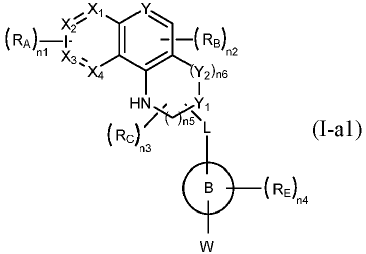 wherein W is halogen or 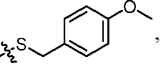 halogen is preferably Cl or Br; other substituents are defined as in formula (I-a) according to any of the embodiments in the preceding third aspect.

The fifth aspect of this invention further provides a compound represented by the following formula (I-b), a stereoisomer or tautomer of the compound or a mixture thereof, a pharmaceutically acceptable salt, co-crystal, polymorph or solvate of the compound, or a stable isotope derivative, metabolite or prodrug of the compound: 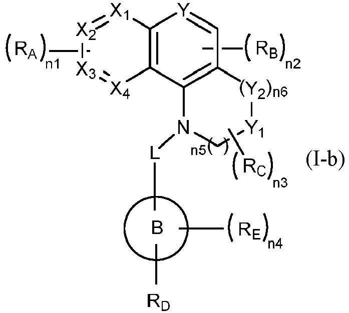
wherein Y₁ and Y₂ are independently selected from C, O and S, and Y₁ and Y₂ are different; n5 is 1 or 2; when Y₂ is not C, n6 is 1, when Y₂ is C, n6 is 1 or 2; X₁-X₄, Y, R_{A}, R_{B}, R_{C}, R_{D}, R_{E}, L, ring B, and n1-n4 are defined as in the compound of formula (I) according to any of the preceding embodiments of the first and second aspects of this invention;
In a preferable embodiment according to the fifth aspect of this invention, Y₁ is O, Y₂ is C;
In a preferable embodiment according to the fifth aspect of this invention, Y₁ is S, Y₂ is C;
In a preferable embodiment according to the fifth aspect of this invention, Y₂ is O, Y₁ is C;
In a preferable embodiment according to the fifth aspect of this invention, Y₂ is S, Y₁ is C;
In a preferable embodiment according to the fifth aspect of this invention, in any of the preceding embodiments of the fifth aspect, 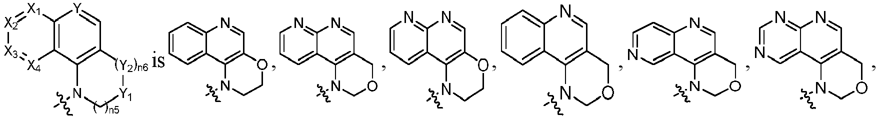 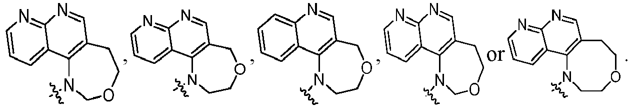
In a preferable embodiment according to the fifth aspect of this invention, in any of the preceding embodiments of the fifth aspect, at least one R_{C} is oxo.

The sixth aspect of this invention further provides a compound represented by the following formula (I-b1): 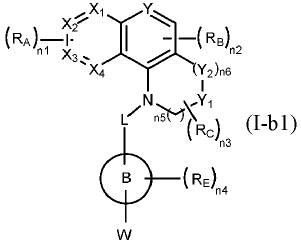 wherein W is halogen or 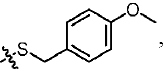 halogen is preferably Cl or Br; other substituents are defined as in formula (I-b) according to any of embodiments in the preceding fifth aspect.

The seventh aspect of this invention further provides a compound represented by the following formula (I-c), a stereoisomer or tautomer of the compound or a mixture thereof, a pharmaceutically acceptable salt, co-crystal, polymorph or solvate of the compound, or a stable isotope derivative, metabolite or prodrug of the compound: 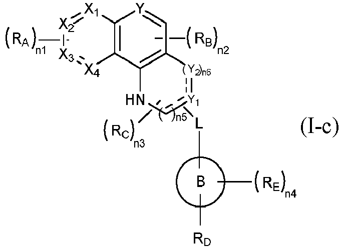
wherein Y₁ and Y₂ are independently selected from C and N; n5 is 1 or 2; when Y₂ is not C, n6 is 1, when Y₂ is C, n6 is 1 or 2; 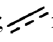 represents single bond or double bond, and at most one double bond exists, X₁-X₄, Y, R_{A}, R_{B}, R_{C}, R_{D}, R_{E}, L, ring B, and n1-n4 are defined as in the compound of formula (I) according to any of the preceding embodiments of the first and second aspects of this invention;
In a preferable embodiment according to the seventh aspect of this invention, Y₁ is N, Y₂ is C;
In a preferable embodiment according to the seventh aspect of this invention, Y₁ is C, Y₂ is N;
In a preferable embodiment according to the seventh aspect of this invention, Y₁ is C, Y₂ is C;
In a preferable embodiment according to the seventh aspect of this invention, in any of the preceding embodiments of the seventh aspect, 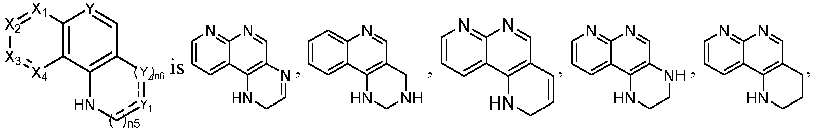 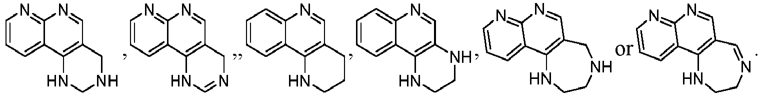
In a preferable embodiment according to the seventh aspect of this invention, in any of the preceding embodiments of the seventh aspect, at least one R_{C} is oxo.

The eighth aspect of this invention further provides a compound represented by the following formula (I-c1): 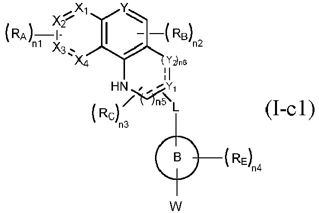 wherein W is halogen or 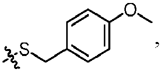 halogen is preferably Cl or Br; other substituents are defined as in formula (I-c) according to any of embodiments in the preceding seventh aspect.

The ninth aspect of this invention further provides a compound represented by the following formula (I-d), a stereoisomer or tautomer of the compound or a mixture thereof, a pharmaceutically acceptable salt, co-crystal, polymorph or solvate of the compound, or a stable isotope derivative, metabolite or prodrug of the compound: 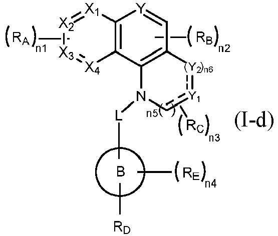
wherein Y₁ and Y₂ are independently selected from C and N; n5 is 1 or 2; when Y₂ is not C, n6 is 1, when Y₂ is C, n6 is 1 or 2; 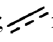 represents single bond or double bond, and at most one double bond exists, X₁-X₄, Y, R_{A}, R_{B}, R_{C}, R_{D}, R_{E}, L, ring B, and n1-n4 are defined as in the compound of formula (I) according to any of the preceding embodiments of the first and second aspects of this invention.
In a preferable embodiment according to the ninth aspect of this invention, Y₁ is N, Y₂ is C;
In a preferable embodiment according to the ninth aspect of this invention, Y₁ is C, Y₂ is N;
In a preferable embodiment according to the ninth aspect of this invention, in any of the preceding embodiments of the ninth aspect, 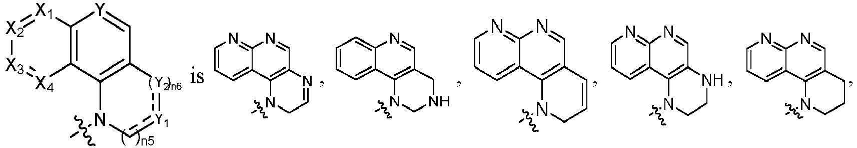 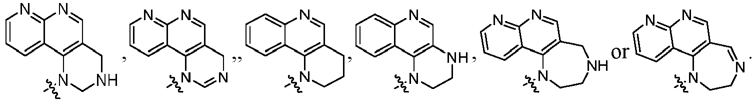
In a preferable embodiment according to the ninth aspect of this invention, in any of the preceding embodiments of the ninth aspect, at least one R_{C} is oxo.

The tenth aspect of this invention further provides a compound represented by the following formula (I-d1): 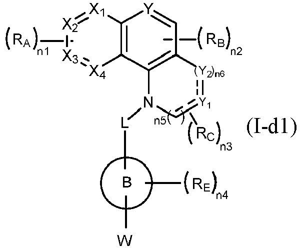 wherein W is halogen or 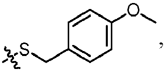 halogen is preferably Cl or Br; other substituents are defined as in formula (I-d) according to any of embodiments in the preceding ninth aspect.

The eleventh aspect of this invention further provides a compound represented by the following formula (I-e), a stereoisomer or tautomer of the compound or a mixture thereof, a pharmaceutically acceptable salt, co-crystal, polymorph or solvate of the compound, or a stable isotope derivative, metabolite or prodrug of the compound: 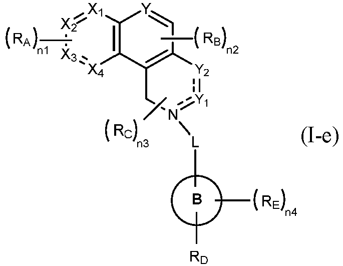
wherein Y₁ and Y₂ are independently selected from C and N, 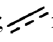 represents single bond or double bond, and at most one double bond exists, X₁-X₄, Y, R_{A}, R_{B}, R_{C}, R_{D}, R_{E}, L, ring B, and n1-n4 are defined as in the compound of formula (I) according to any of the preceding embodiments of the first and second aspects of this invention.
In a preferable embodiment according to the eleventh aspect of this invention, Y₁ is N, Y₂ is C;
In a preferable embodiment according to the eleventh aspect of this invention, Y₁ is C, Y₂ is N;
In a preferable embodiment according to the eleventh aspect of this invention, in any of the preceding embodiments of the eleventh aspect, 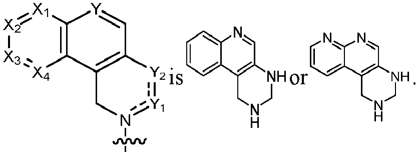
In a preferable embodiment according to the eleventh aspect of this invention, in any of the preceding embodiments of the eleventh aspect, at least one R_{C} is oxo.

The twelfth aspect of this invention further provides a compound represented by the following formula (I-e1): 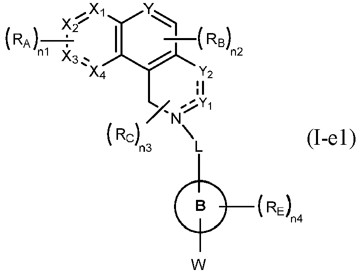 wherein W is halogen or 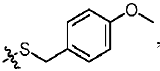 halogen is preferably Cl or Br; other substituents are defined as in formula (I-e) according to any of embodiments in the preceding eleventh aspect.

The thirteenth aspect of this invention further provides a compound represented by the following formula (I-f), a stereoisomer or tautomer of the compound or a mixture thereof, a pharmaceutically acceptable salt, co-crystal, polymorph or solvate of the compound, or a stable isotope derivative, metabolite or prodrug of the compound: 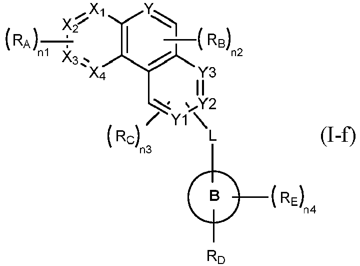
wherein Y₁, Y₂, and Y₃ are independently selected from C and N, and Y₁, Y₂ and Y₃ are not C or N at the same time, X₁-X₄, Y, R_{A}, R_{B}, R_{C}, R_{D}, R_{E}, L, ring B, and n1-n4 are defined as in the compound of formula (I) according to any of the preceding embodiments of the first and second aspects of this invention.
In a preferable embodiment according to the thirteenth aspect of this invention, one of Y₁, Y₂ and Y₃ is N, and the others are C;
In a preferable embodiment according to the thirteenth aspect of this invention, Y₁ and Y₃ are N, Y₂ is C;
In a preferable embodiment according to the thirteenth aspect of this invention, Y₁ and Y₂ are N, Y₃ is C;
In a preferable embodiment according to the thirteenth aspect of this invention, Y₂ and Y₃ are N, Y₁ is C;
In a preferable embodiment according to the thirteenth aspect of this invention, in any of the preceding embodiments of the thirteenth aspect,

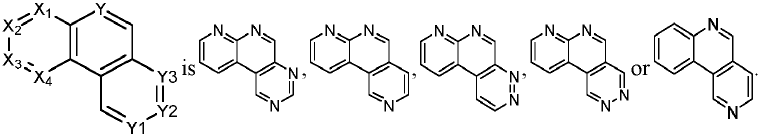

The fourteenth aspect of this invention further provides a compound represented by the following formula (I-f1): 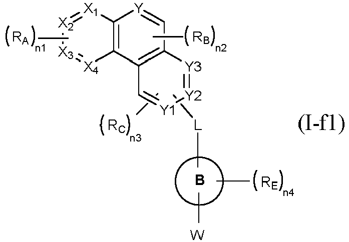 wherein W is halogen or 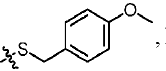 halogen is preferably Cl or Br; other substituents are defined as in formula (I-f) according to any of embodiments in the preceding thirteenth aspect.

The fifteenth aspect of this invention further provides a compound represented by the following formula (II), a stereoisomer or tautomer of the compound or a mixture thereof, a pharmaceutically acceptable salt, co-crystal, polymorph or solvate of the compound, or a stable isotope derivative, metabolite or prodrug of the compound: 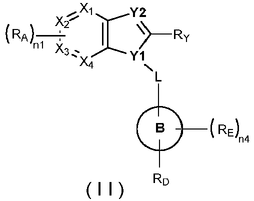
wherein, X₁, X₂, X₃, X₄, R_{A}, R_{D}, R_{E}, ring B, L, n1, and n4 are defined as in the compound of formula (I) according to any of the preceding embodiments of the first and second aspects of this invention;
Y₁ is N, Y₂ is CR_{B'} or N;
R_{B'} and RY are each independently hydrogen, deuterium, halogen, oxime, -CN, -OH, -SH, -NO₂, -NH₂, -Z-OR₁, -Z-SR₁, -Z-NR₂R₃, -Z-NR₂C(O)R₄, -Z-NR₂C(O)OR₁, -Z-C(O)R₄, -Z-C(O)OR₁, -Z-C(O)(CR₅R₆)ₙC(O)R₄, -Z-C(O)(CR₅R₆)ₙC(O)OR₁, -Z-C(O)NR₂R₃, -Z-S(O)₂R₄, -Z-S(O)₂NR₂R₃, C₁₋₆ alkyl, C₂-₆ alkenyl, C₂-₆ alkynyl, C₁₋₆ alkoxy, C₁₋₆ alkylthio, C₃-₈ cycloalkyl, C₃-₈ cycloalkenyl, 3-20 membered heterocyclyl, 5-16 membered heteroaryl; the alkyl, alkenyl, alkynyl, alkoxy, alkylthio, cycloalkyl, cycloalkenyl, heterocyclyl, or heteroaryl is optionally substituted by one or more substituents selected from deuterium, halogen, oxo, oxime, -CN, -OH, -NO₂, -NH₂, C₁₋₆ alkyl, phenyl, C₁₋₆ alkoxy, C₁₋₆ haloalkyl, C₁₋₆ haloalkoxy, and halophenyl;
n is 0, 1, 2, or 3.
In a preferable embodiment according to the fifteenth aspect of this invention, X₁ is N, and X₂, X₃, and X₄ are all CH.

In a preferable embodiment according to the fifteenth aspect of this invention, X₃ is N, and X₁, X₂, and X₄ are all CH.

In a preferable embodiment according to the fifteenth aspect of this invention, X₁ and X₃ are both N, and X₂ and X₄ are both CH.

In a preferable embodiment according to the fifteenth aspect of this invention, X₁, X₂, X₃, and X₄ are all CH.

In a preferable embodiment according to the fifteenth aspect of this invention, in any of the preceding embodiments of the fifteenth aspect, Y₂ is CH; or Y₂ is N.

In a preferable embodiment according to the fifteenth aspect of this invention, in any of the preceding embodiments of the fifteenth aspect, R_{B'} is hydrogen, deuterium, halogen, oxime, -CN, -OH, -SH, -NO₂, -NH₂, C₁₋₃ alkyl, C₂-₆ alkenyl, C₂-₆ alkynyl, C₁₋₆ alkoxy, C₁₋₆ alkylthio, or C₃₋₆ cycloalkyl, the alkyl, alkenyl, alkynyl, alkoxy, alkylthio, or cycloalkyl is optionally substituted by one or more substituents selected from deuterium, halogen, oxo, oxime, -CN, -OH, -NO₂, -NH₂, C₁₋₆ alkyl, phenyl, C₁₋₆ alkoxy, C₁₋₆haloalkyl, C₁₋₆haloalkoxy, and halophenyl;
or, R_{B'} is hydrogen, deuterium, F, Cl, Br, -CN, -OH, -SH, -NO₂, -NH₂, cyano, methyl, ethyl, propyl, isopropyl, methoxy, ethoxy, propoxy, isopropoxy, methylthio, ethylthio, propylthio, isopropylthio, cyclopropyl, cyclobutyl, trifluoromethyl, difluoromethyl, monofluoromethyl, trifluoroethyl, difluoroethyl, monofluoroethyl, hydroxypropyl, hydroxyethyl, hydroxymethyl, methoxypropyl, methoxyethyl, methoxymethyl, ethenyl, or ethynyl;
or, R_{B'} is hydrogen, F, Cl, Br, -OH, cyano, methyl, ethyl, methoxy, ethoxy, ethenyl, or ethynyl;
or, R_{B'} is hydrogen.

In a preferable embodiment according to the fifteenth aspect of this invention, in any of the preceding embodiments of the fifteenth aspect, each R_{Y} is independently hydrogen, deuterium, halogen, oxime, -CN, -OH, -SH, -NO₂, -NH₂, C₁₋₃ alkyl, C₂-₆ alkenyl, C₂-₆ alkynyl, C₁₋₆ alkoxy, C₁₋₆ alkylthio, or C₃₋₆ cycloalkyl, the alkyl, alkenyl, alkynyl, alkoxy, alkylthio, or cycloalkyl is optionally substituted by one or more substituents selected from deuterium, halogen, oxo, oxime, -CN, -OH, -NO₂, -NH₂, C₁₋₆ alkyl, phenyl, C₁₋₆ alkoxy, C₁₋₆haloalkyl, C₁₋₆haloalkoxy, and halophenyl;
or, each R_{Y} is independently hydrogen, deuterium, F, Cl, Br, -CN, -OH, -SH, -NO₂, -NH₂, cyano, methyl, ethyl, propyl, isopropyl, methoxy, ethoxy, propoxy, isopropoxy, methylthio, ethylthio, propylthio, isopropylthio, cyclopropyl, cyclobutyl, trifluoromethyl, difluoromethyl, monofluoromethyl, trifluoroethyl, difluoroethyl, monofluoroethyl, hydroxypropyl, hydroxyethyl, hydroxymethyl, methoxypropyl, methoxyethyl, methoxymethyl, ethenyl, ethynyl, propenyl, or propynyl;
or, each R_{Y} is independently hydrogen, deuterium, F, Cl, Br, -OH, cyano, amino, methyl, ethyl, methoxy, ethoxy, methylthio, ethylthio, ethenyl, ethynyl, propenyl, or propynyl;
or, R_{Y} is hydrogen, amino, methyl, ethenyl, or ethynyl.

In a preferable embodiment according to the fifteenth aspect of this invention, in any of the preceding embodiments of the fifteenth aspect,

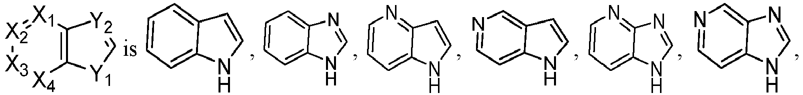

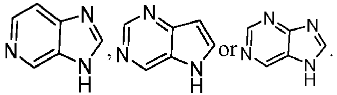

The sixteenth aspect of this invention further provides a compound represented by the following formula (III), a stereoisomer or tautomer of the compound or a mixture thereof, a pharmaceutically acceptable salt, co-crystal, polymorph or solvate of the compound, or a stable isotope derivative, metabolite or prodrug of the compound:

The seventeenth aspect of this invention further provides a compound represented by the following formula (IV-1), (IV-2), (IV-3), or (IV-4), a stereoisomer or tautomer of the compound or a mixture thereof, a pharmaceutically 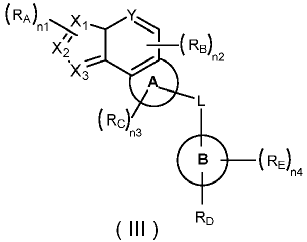
wherein, R_{A}, R_{B}, R_{C}, R_{D}, R_{E}, ring B, L, n2, n3, and n4 are defined as in the compound of formula (I) according to any of the preceding embodiments of the first and second aspects of this invention;
X₁, X₂, X₃ and Y are each independently CH or N;
and when X₁, X₂, and X₃ are all CH, Y is not CH;
n1 is 1, 2, 3 or 4;
ring A is 5-10 membered heterocyclyl or 5-12 membered heteroaryl.

In a preferable embodiment according to the sixteenth aspect of this invention, X₁ is N, and X₂ and X₃ are both CH. In a preferable embodiment according to the sixteenth aspect of this invention, X₃ is N, and X₁ and X₂ are both CH. In a preferable embodiment according to the sixteenth aspect of this invention, X₁ and X₃ are both N, and X₂ is CH. In a preferable embodiment according to the sixteenth aspect of this invention, X₁, X₂, and X₃ are all CH.

In a preferable embodiment according to the sixteenth aspect of this invention, in any of the preceding embodiments of the sixteenth aspect, Y is CH; or Y is N.

In a preferable embodiment according to the sixteenth aspect of this invention, in any of the preceding embodiments of the sixteenth aspect, 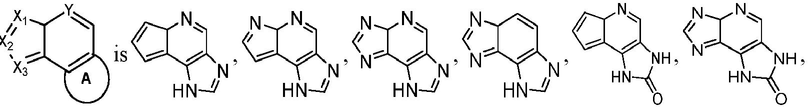 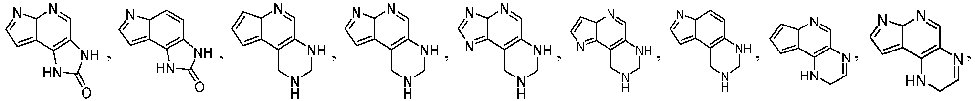 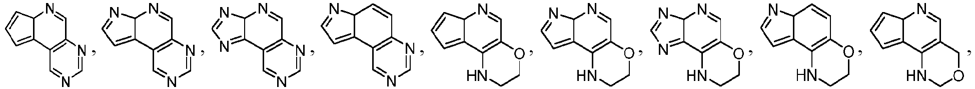 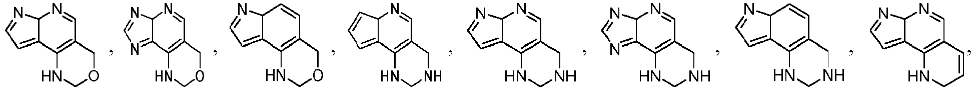 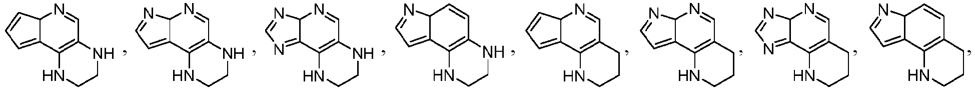 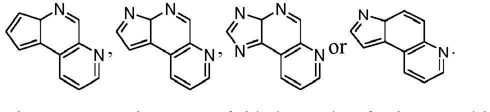 acceptable salt, co-crystal, polymorph or solvate of the compound, or a stable isotope derivative, metabolite or prodrug of the compound: 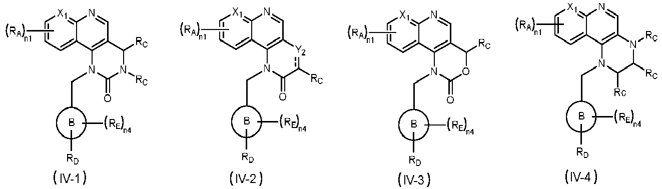
wherein, X₁, R_{A}, R_{D}, R_{E}, R_{C}, ring B, n1, and n4 are defined as in the compound of formula (I) according to any of the preceding embodiments of the first and second aspects of this invention;
Y₂ is C or N;
In a preferable embodiment according to formula (IV-1), (IV-2), (IV-3), or (IV-4) of this invention, in any of the preceding formulae (IV-1), (IV-2), (IV-3), and (IV-4), each Rc is independently hydrogen, oxo, -OH, -SH, -NH₂, - Boc, -C(O)CH₂OH, -NHC(O)CH₃, carboxyl, -S(O)₂CH₃, -S(O)₂NH₂, -methoxy, methylthio, -CH₂CF₃, cyclopropyl; In a preferable embodiment according to formula (IV-1), (IV-2), (IV-3), or (IV-4) of this invention, in any of the preceding formulae (IV-1), (IV-2), (IV-3), and (IV-4), each Rc is independently hydrogen, oxo, -Boc, -C(O)CHzOH, -CH₂CF₃, cyclopropyl;
In a preferable embodiment according to formula (IV-1), (IV-2), (IV-3), or (IV-4) of this invention, in any of the preceding formulae (IV-1), (IV-2), (IV-3), and (IV-4), each Rc is independently hydrogen, oxo;
In a preferable embodiment according to formula (IV-1), (IV-2), (IV-3), or (IV-4) of this invention, in any of the preceding formulae (IV-1), (IV-2), (IV-3), and (IV-4), ring B is 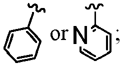 In a preferable embodiment according to formula (IV-1), (IV-2), (IV-3), or (IV-4) of this invention, in any of the preceding formulae (IV-1), (IV-2), (IV-3), and (IV-4), ring B is 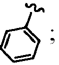 In a preferable embodiment according to formula (IV-1), (IV-2), (IV-3), or (IV-4) of this invention, in any of the preceding formulae (IV-1), (IV-2), (IV-3), and (IV-4), ring B is 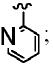
In a preferable embodiment according to formula (IV-1), (IV-2), (IV-3), or (IV-4) of this invention, in any of the preceding formulae (IV-1), (IV-2), (IV-3), and (IV-4), R_{D} is -SO₂NH₂.

The eighteenth aspect of this invention further provides a compound represented by the following formula (V-1), a stereoisomer or tautomer of the compound or a mixture thereof, a pharmaceutically acceptable salt, co-crystal, polymorph or solvate of the compound, or a stable isotope derivative, metabolite or prodrug of the compound: 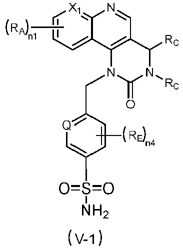
wherein, X₁, R_{A}, R_{E}, R_{C}, n1, and n4 are defined as in formula (I) according to any of the preceding embodiments of the first and second aspects of this invention or formula (IV-1), (IV-2), (IV-3), or (IV-4);
Q is C or N;
In a preferable embodiment according to formula (V-1) of this invention, in any of the preceding formulae (V-1), Q is C;
In a preferable embodiment according to formula (V-1) of this invention, in any of the preceding formulae (V-1), 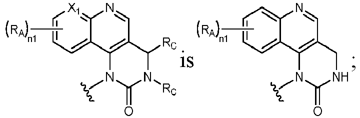 In a preferable embodiment according to formula (V-1) of this invention, in any of the preceding formulae (V-1), 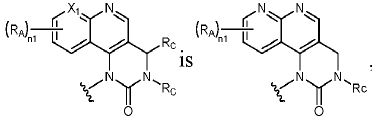 , wherein R_{C} is hydrogen, trifluoroethyl or cyclopropyl;
In a preferable embodiment according to formula (V-1) of this invention, in any of the preceding formulae (V-1), 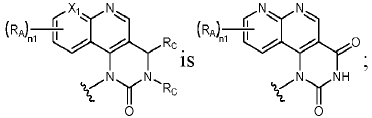
In a preferable embodiment according to formula (V-1) of this invention, in any of the preceding formulae (V-1), R_{A} is hydrogen, F, Cl, methoxy, or ethoxy;
In a preferable embodiment according to formula (V-1) of this invention, in any of the preceding formulae (V-1), R_{A} is hydrogen, F, or methoxy;
In a preferable embodiment according to formula (V-1) of this invention, in any of the preceding formulae (V-1), R_{E} is hydrogen, deuterium, F, or Cl;
In a preferable embodiment according to formula (V-1) of this invention, in any of the preceding formulae (V-1), R_{E} is hydrogen or F;
In a preferable embodiment according to formula (V-1) of this invention, in any of the preceding formulae (V-1), n1 is 1 or 2;
In a preferable embodiment according to formula (V-1) of this invention, in any of the preceding formulae (V-1), n4 is 1 or 2;
In a preferable embodiment according to formula (V-1) of this invention, in any of the preceding formulae (V-1), n4 is 2;
In a preferable embodiment according to formula (V-1) of this invention, in any of the preceding formulae (V-1), 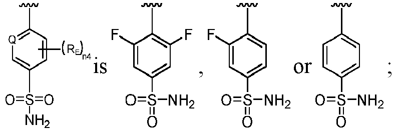
In a preferable embodiment according to formula (V-1) of this invention, in any of the preceding formulae (V-1), 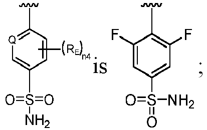
In a preferable embodiment according to formula (V-1) of this invention, in any of the preceding formulae (V-1),

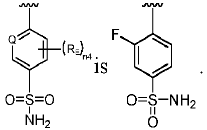

The nineteenth aspect of this invention further provides a compound represented by the following formula (V-2), a stereoisomer or tautomer of the compound or a mixture thereof, a pharmaceutically acceptable salt, co-crystal, polymorph or solvate of the compound, or a stable isotope derivative, metabolite or prodrug of the compound: 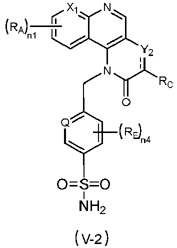
wherein, X₁, R_{A}, R_{E}, R_{C}, Q, n1, n4, and 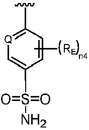 are defined as in formula (I) according to any of the preceding embodiments of the first and second aspects of this invention or formula (V-1);
In a preferable embodiment according to formula (V-2) of this invention, in any of the preceding formulae (V-2), 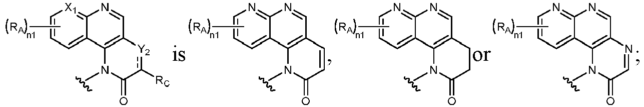
In a preferable embodiment according to formula (V-2) of this invention, in any of the preceding formulae (V-2), 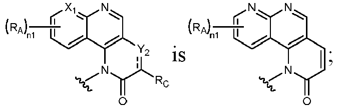
In a preferable embodiment according to formula (V-2) of this invention, in any of the preceding formulae (V-2), 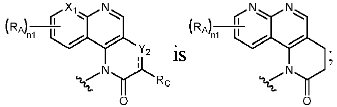
In a preferable embodiment according to formula (V-2) of this invention, in any of the preceding formulae (V-2),

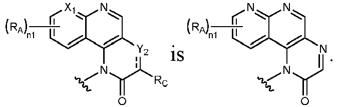

The twentieth aspect of this invention further provides a compound represented by the following formula (V-3), a stereoisomer or tautomer of the compound or a mixture thereof, a pharmaceutically acceptable salt, co-crystal, polymorph or solvate of the compound, or a stable isotope derivative, metabolite or prodrug of the compound: 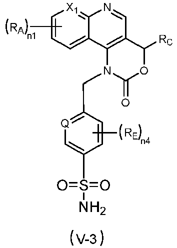
wherein, X₁, R_{A}, R_{E}, R_{C}, Q, n1, n4, and 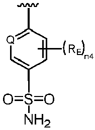 are defined as in formula (I) according to any of the preceding embodiments of the first and second aspects of this invention or formula (V-1);
In a preferable embodiment according to formula (V-3) of this invention, in any of the preceding formulae (V-3), R_{C} is H;
In a preferable embodiment according to formula (V-3) of this invention, in any of the preceding formulae (V-3), 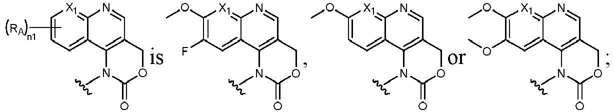
In a preferable embodiment according to formula (V-3) of this invention, in any of the preceding formulae (V-3), 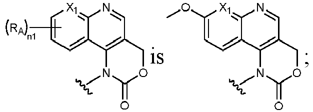
In a preferable embodiment according to formula (V-3) of this invention, in any of the preceding formulae (V-3),

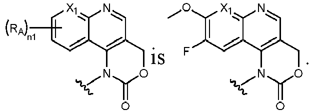

The twenty-first aspect of this invention further provides a compound represented by the following formula (V-4), a stereoisomer or tautomer of the compound or a mixture thereof, a pharmaceutically acceptable salt, co-crystal, polymorph or solvate of the compound, or a stable isotope derivative, metabolite or prodrug of the compound: 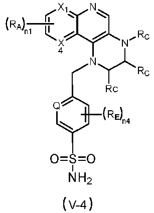
wherein, X₁, X₄, R_{A}, R_{E}, R_{C}, Q, n1, n4, and 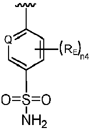 are defined as in formula (I) according to any of the preceding embodiments of the first and second aspects of this invention or formula (V-1);
In a preferable embodiment according to formula (V-4) of this invention, in any of the preceding formulae (V-4), 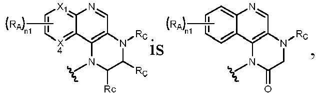 wherein R_{C} is hydrogen, -C(O)CH₂OH;
In a preferable embodiment according to formula (V-4) of this invention, in any of the preceding formulae (V-4), 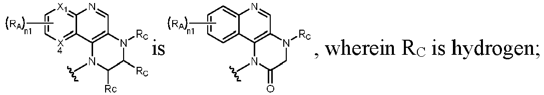
In a preferable embodiment according to formula (V-4) of this invention, in any of the preceding formulae (V-4), 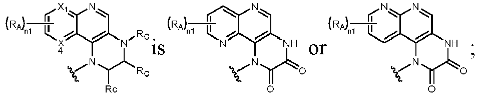
In a preferable embodiment according to formula (V-4) of this invention, in any of the preceding formulae (V-4), 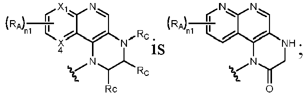
In a preferable embodiment according to formula (V-4) of this invention, in any of the preceding formulae (V-4), 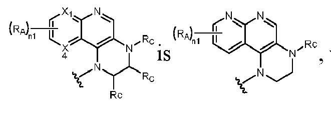 wherein R_{C} is hydrogen or Boc;
In a preferable embodiment according to formula (V-4) of this invention, in any of the preceding formulae (V-4), 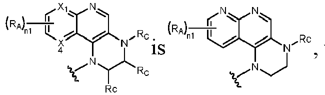 wherein R_{C} is hydrogen;
In a preferable embodiment according to formula (V-4) of this invention, in any of the preceding formulae (V-4), 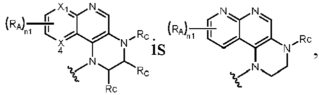 wherein R_{C} is Boc.
The twenty-second aspect of this invention relates to a preferable embodiment according to any of the first to twenty-first aspects of this invention, wherein X₂ and/or X₄ are substituted by R_{A}, and further preferably X₂ is substituted by R_{A}.

In a preferable embodiment of this invention, the compounds of this invention are selected from:

| No. | Structure | No. | Structure | No. | Structure |
|---|---|---|---|---|---|
| 1 | 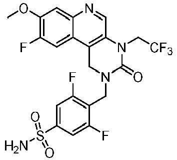 | 2 | 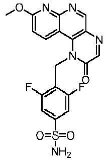 | 3 | 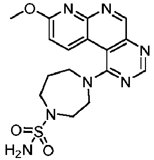 |
| 4 | 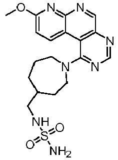 | 5 | 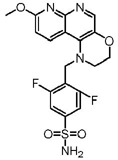 | 6 | 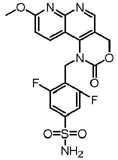 |
| 7 | 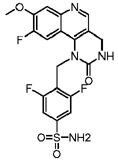 | 8 | 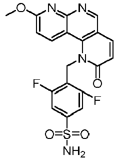 | 9 | 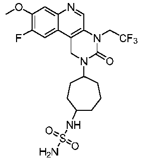 |
| 10 | 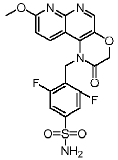 | 11 | 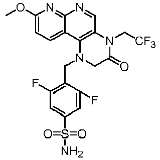 | 12 | 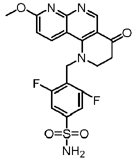 |
| 13 | 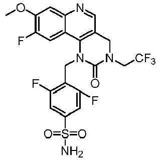 | 14 | 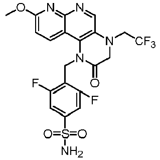 | 15 | 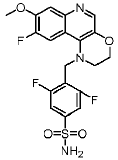 |
| 16 | 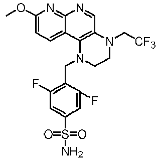 | 17 | 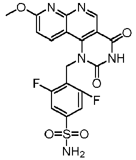 | 18 | 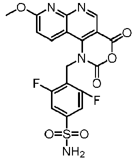 |
| 19 | 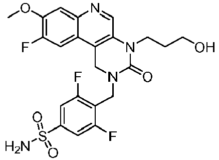 | 20 | 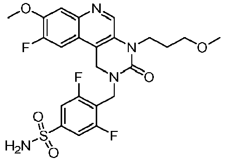 | 21 | 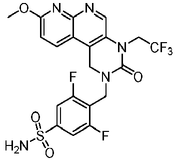 |
| 22 | 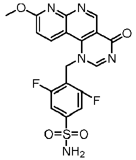 | 23 | 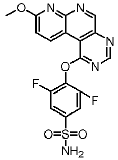 | 24 | 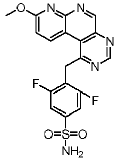 |
| 25 | 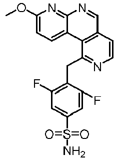 | 26 | 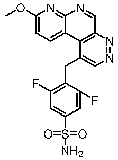 | 27 | 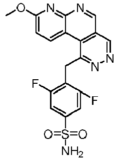 |
| 28 | 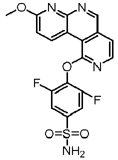 | 29 | 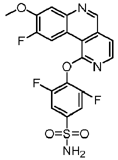 | 30 | 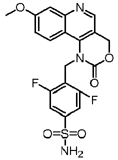 |
| 31 | 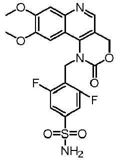 | 32 | 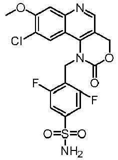 | 33 | 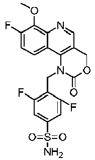 |
| 34 | 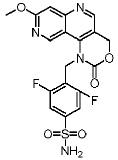 | 35 | 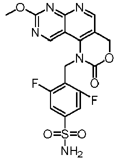 | 36 | 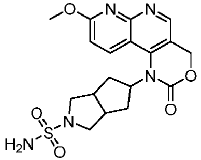 |
| 37 | 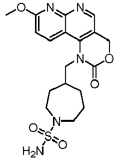 | 38 | 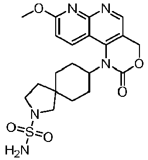 | 39 | 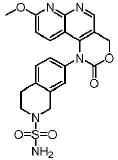 |
| 40 | 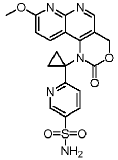 | 41 | 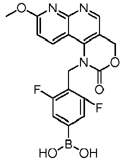 | 42 | 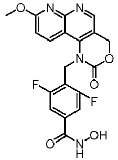 |
| 43 | 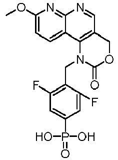 | 44 | 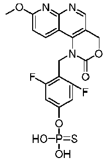 | 45 | 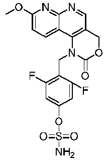 |
| 46 | 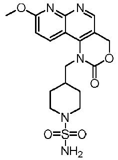 | 47 | 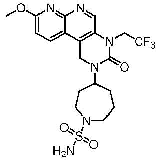 | 48 | 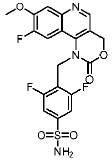 |
| 49 | 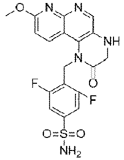 | 50 | 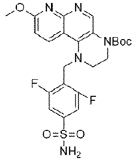 | 51 | 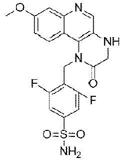 |
| 52 | 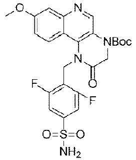 | 53 | 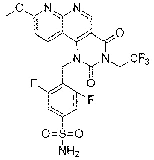 | 54 | 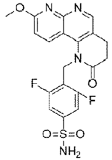 |
| 55 | 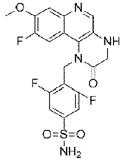 | 56 | 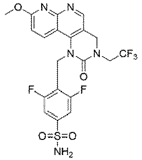 | 57 | 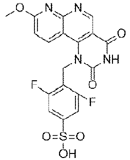 |
| 58 | 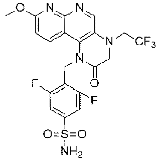 | 59 | 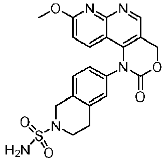 | 60 | 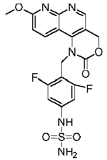 |
| 61 | 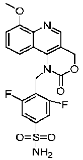 | 62 | 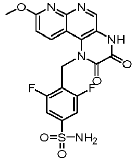 | 63 | 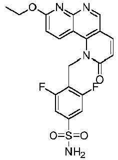 |
| 64 | 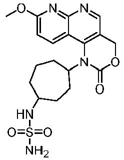 | 65 | 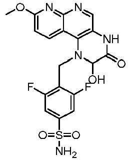 | 66 | 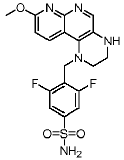 |
| 67 | 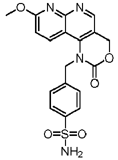 | 68 | 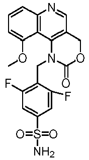 | 69 | 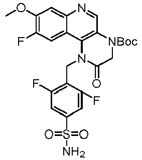 |
| 70 | 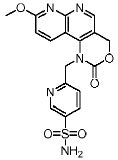 | 71 | 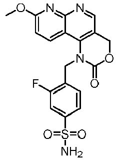 | 72 | 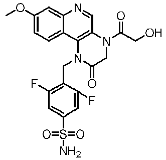 |
| 73 | 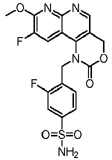 | 74 | 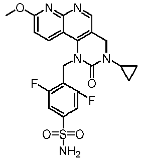 | 75 | 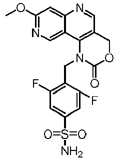 |
| 76 | 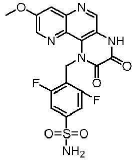 | 77 | 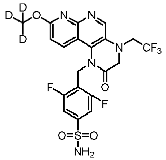 | 78 | 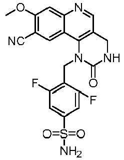 |
| 79 | 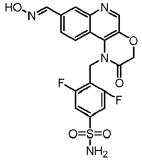 | 80 | 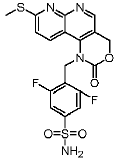 | 81 | 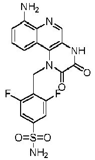 |
| 82 | 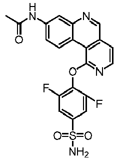 | 83 | 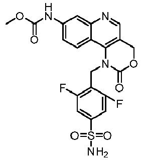 | 84 | 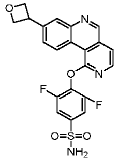 |
| 85 | 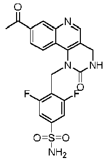 | 86 | 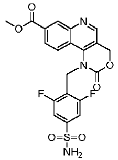 | 87 | 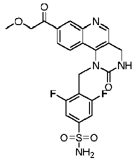 |
| 88 | 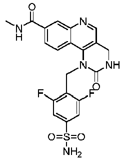 | 89 | 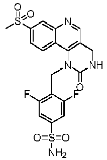 | 90 | 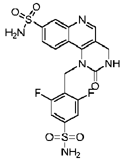 |
| 91 | 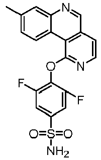 | 92 | 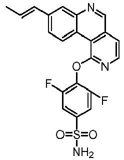 | 93 | 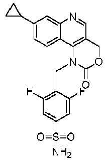 |
| 94 | 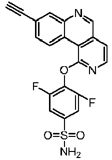 | 95 | 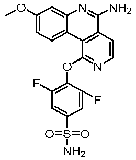 | 96 | 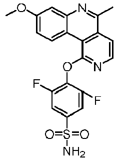 |
| 97 | 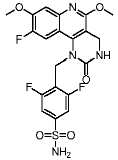 | 98 | 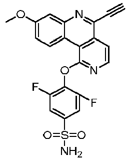 | 99 | 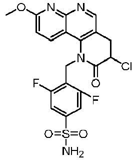 |
| 100 | 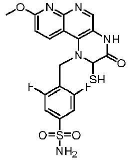 | 101 | 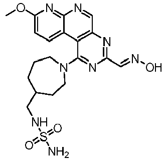 | 102 | 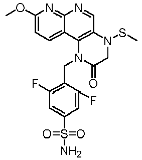 |
| 103 | 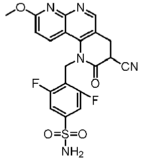 | 104 | 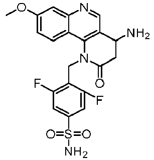 | 105 | 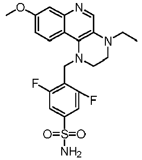 |
| 106 | 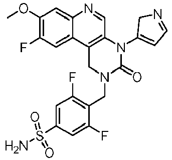 | 107 | 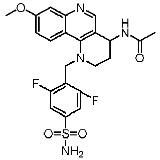 | 108 | 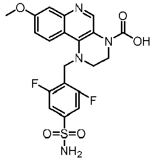 |
| 109 | 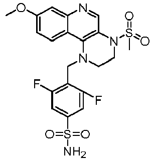 | 110 | 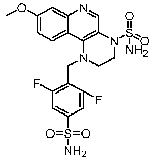 | 111 | 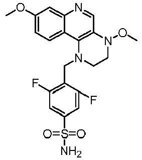 |
| 112 | 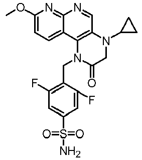 | 113 | 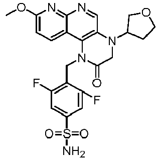 | 114 | 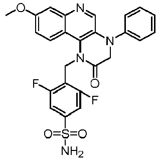 |
| 115 | 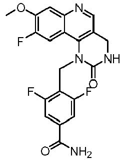 | 116 | 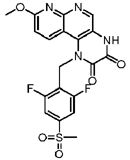 | 117 | 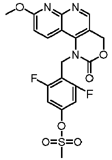 |
| 118 | 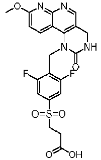 | 119 | 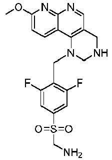 | 120 | 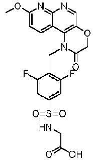 |
| 121 | 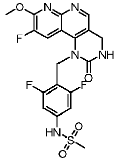 | 122 | 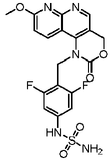 | 123 | 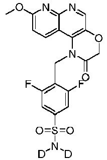 |
| 124 | 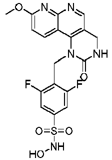 | 125 | 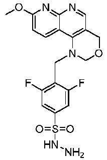 | 126 | 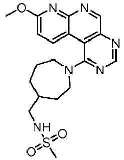 |
| 127 | 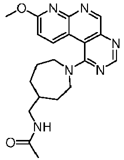 | 128 | 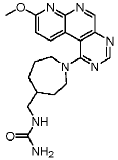 | 129 | 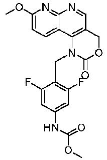 |
| 130 | 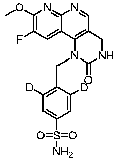 | 131 | 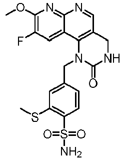 | 132 | 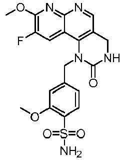 |
| 133 | 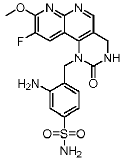 | 134 | 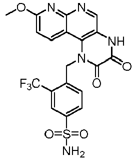 | 135 | 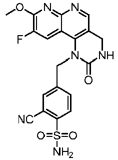 |
| 136 | 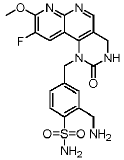 | 137 | 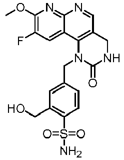 | 138 | 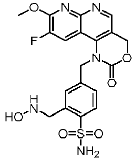 |
| 139 | 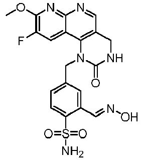 | 140 | 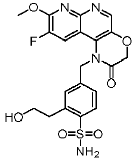 | 141 | 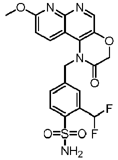 |
| 142 | 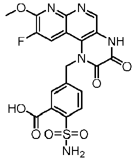 | 143 | 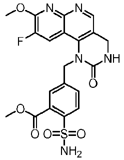 | 144 | 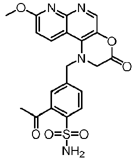 |
| 145 | 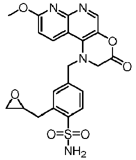 | 146 | 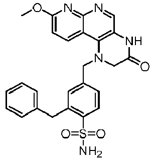 | 147 | 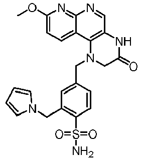 |
| 148 | 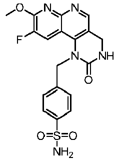 | 149 | 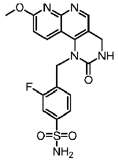 | 150 | 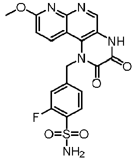 |
| 151 | 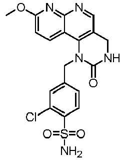 | 152 | 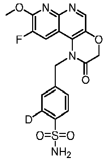 | 153 | 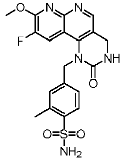 |
| 154 | 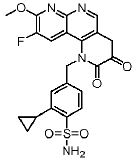 | 155 | 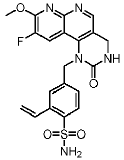 | 156 | 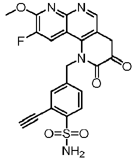 |
| 157 | 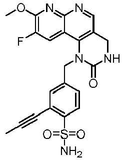 | 158 | 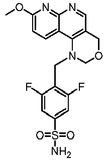 | 159 | 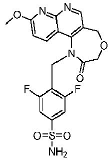 |
| 160 | 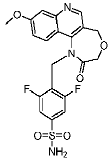 | 161 | 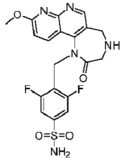 | 162 | 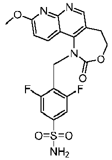 |
| 163 | 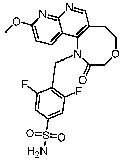 | 164 | 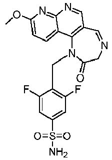 | 165 | 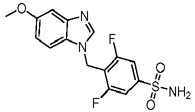 |
| 166 | 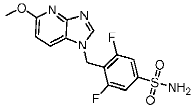 | 167 | 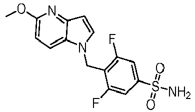 | 168 | 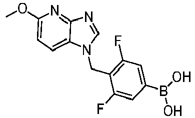 |
| 169 | 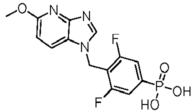 | 170 | 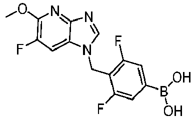 | 171 | 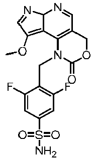 |
| 172 | 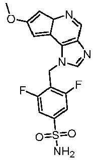 | 173 | 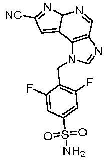 | 174 | 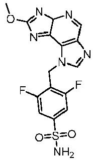 |
| 175 | 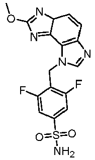 | 176 | 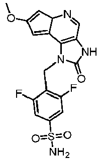 | 177 | 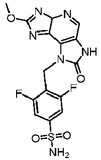 |
| 178 | 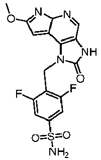 | 179 | 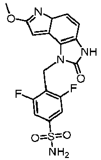 | 180 | 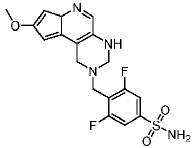 |
| 181 | 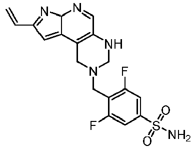 | 182 | 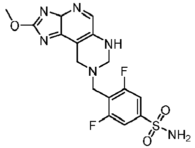 | 183 | 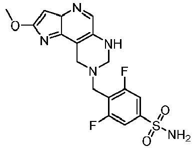 |
| 184 | 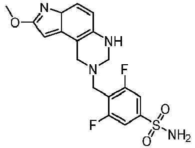 | 185 | 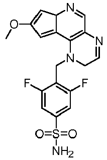 | 186 | 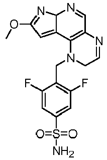 |
| 187 | 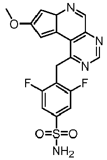 | 188 | 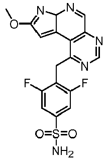 | 189 | 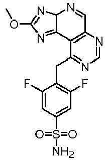 |
| 190 | 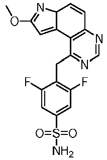 | 191 | 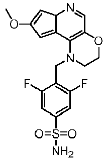 | 192 | 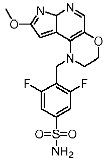 |
| 193 | 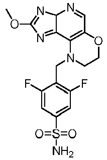 | 194 | 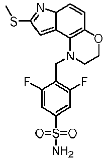 | 195 | 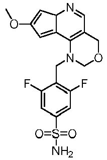 |
| 196 | 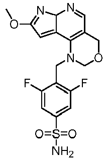 | 197 | 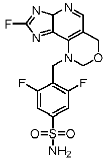 | 198 | 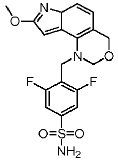 |
| 199 | 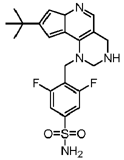 | 200 | 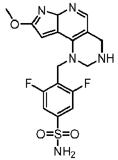 | 201 | 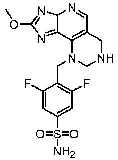 |
| 202 | 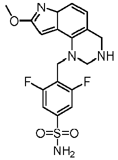 | 203 | 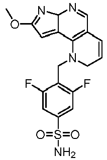 | 204 | 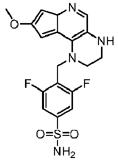 |
| 205 | 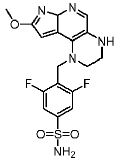 | 206 | 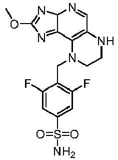 | 207 | 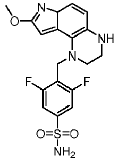 |
| 208 | 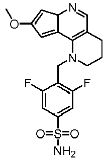 | 209 | 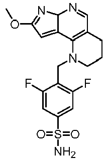 | 210 | 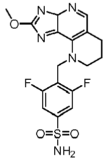 |
| 211 | 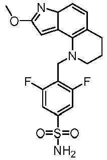 | 212 | 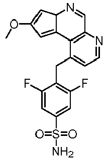 | 213 | 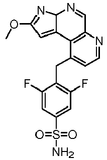 |
| 214 | 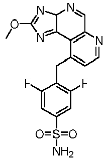 | 215 | 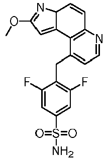 | 216 | 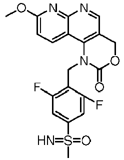 |

The objective of this invention further includes providing a process for preparing a compound represented by any of formulae (I) and (I-a) to (I-f), a stereoisomer or tautomer of the compound or a mixture thereof, a pharmaceutically acceptable salt, co-crystal, polymorph or solvate of the compound, or a stable isotope derivative, metabolite or prodrug of the compound.

The compound can be prepared by various processes, including but not limited to the following processes:

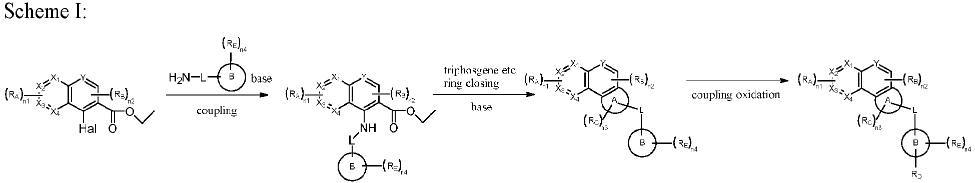

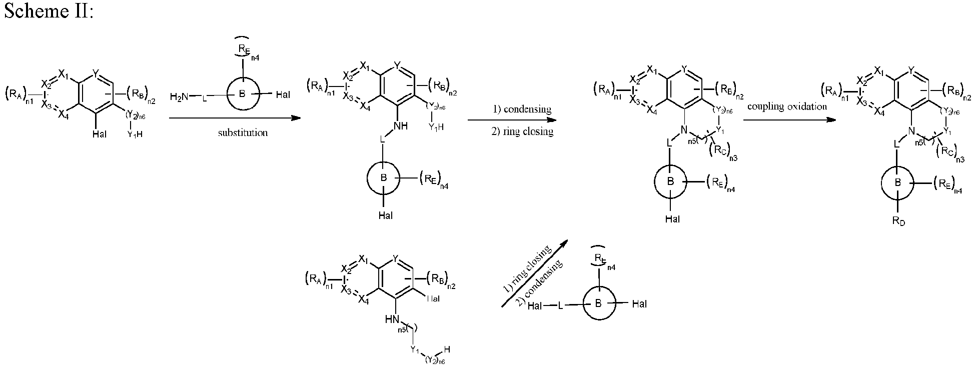

In a preferable embodiment of this invention,

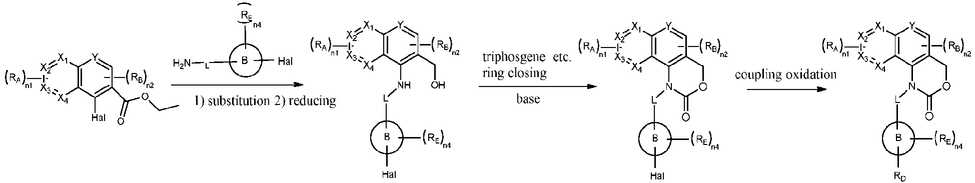

In a preferable embodiment of this invention,

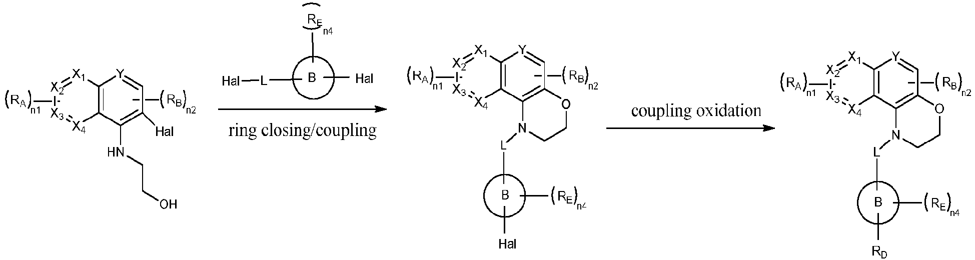

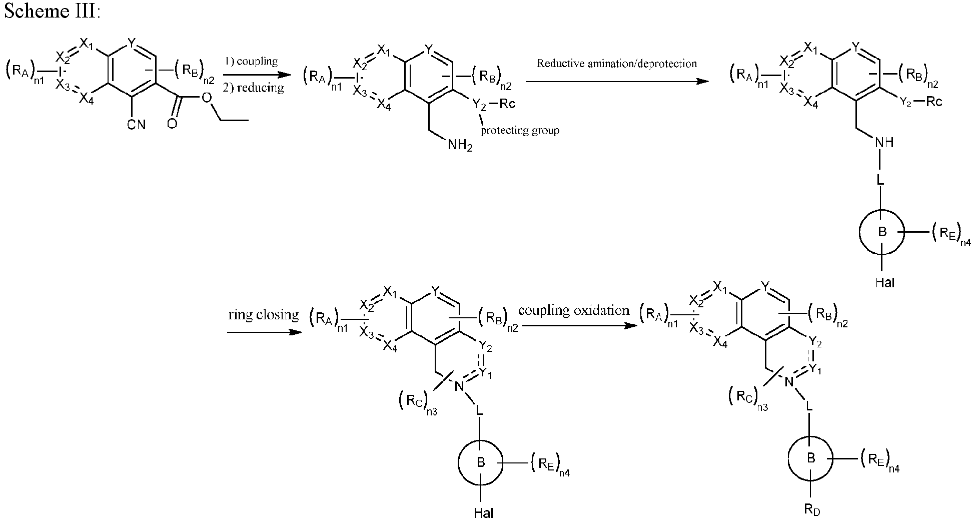

In a preferable embodiment of this invention,

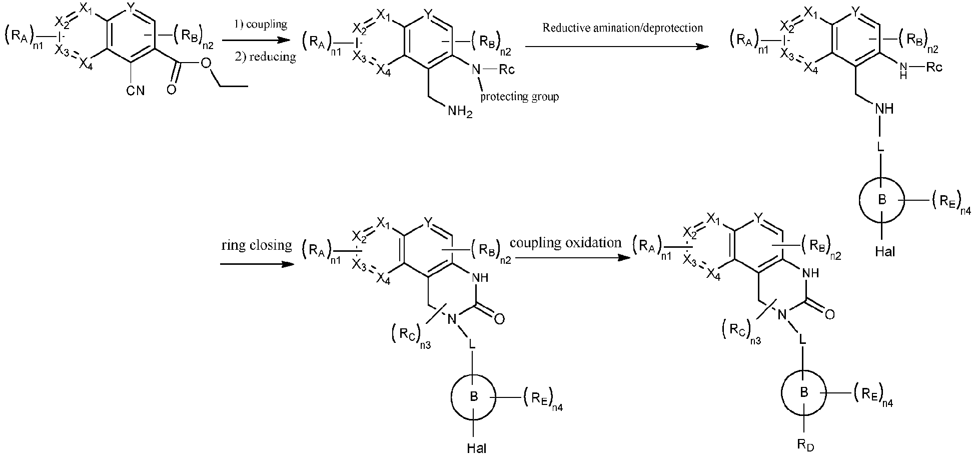

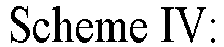

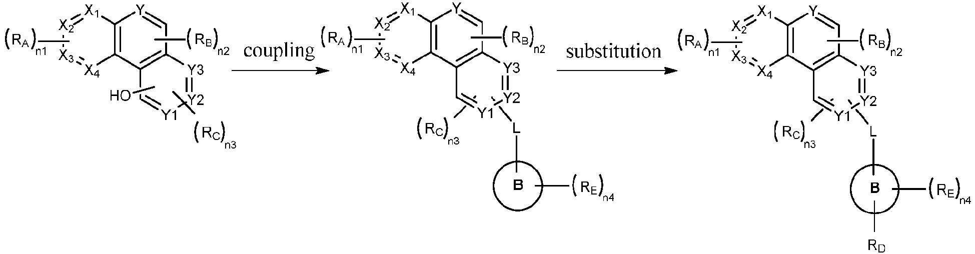

In a preferable embodiment of this invention,

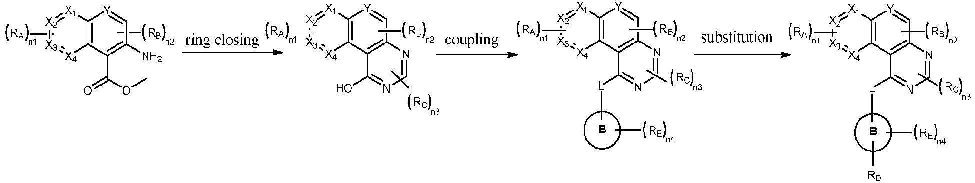

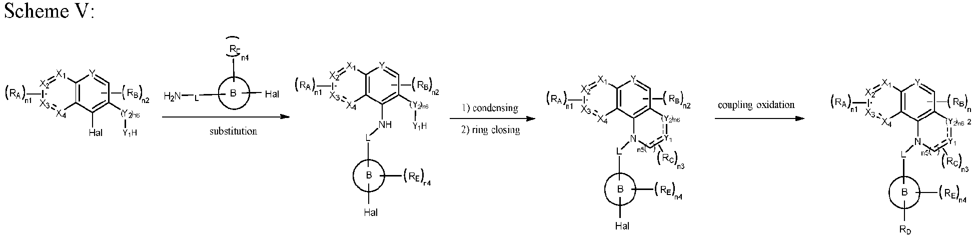

In a preferable embodiment of this invention,

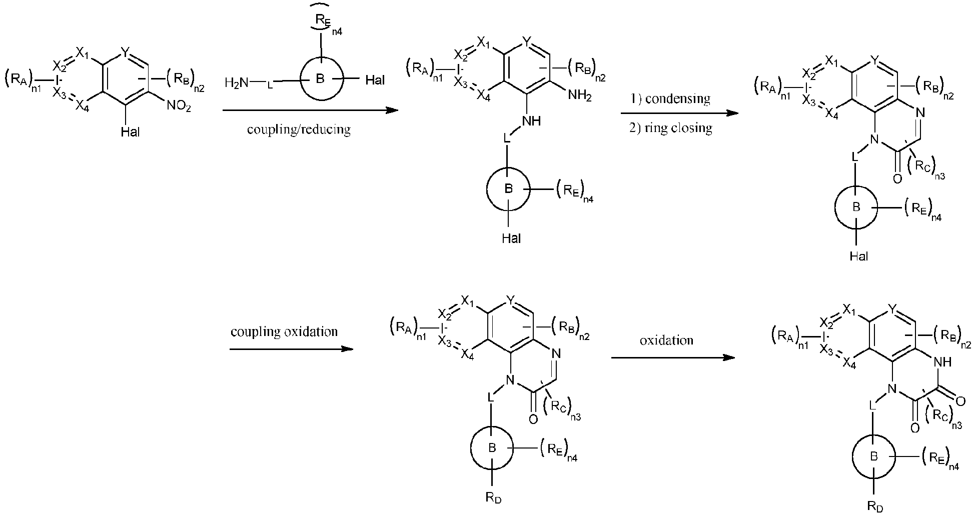

In a preferable embodiment of this invention,

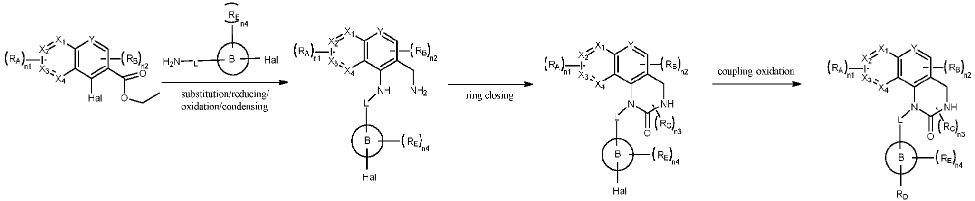

In a preferable embodiment of this invention,

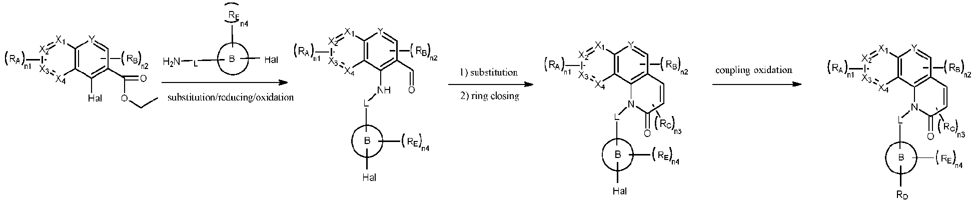

In a preferable embodiment of this invention,

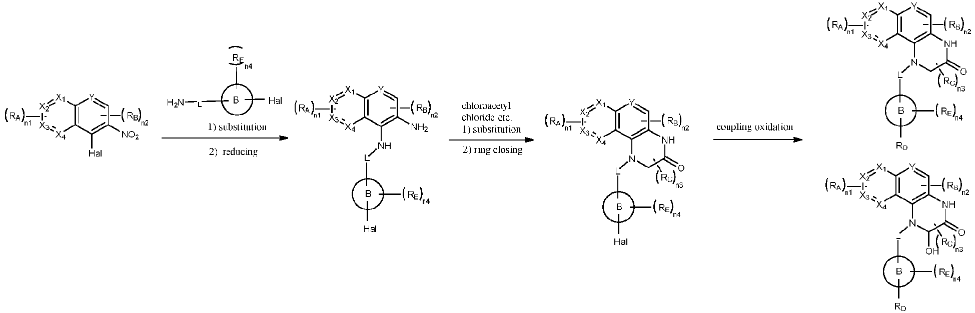

In a preferable embodiment of this invention,

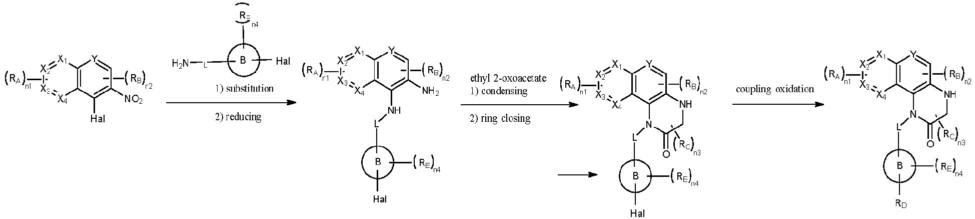

In a preferable embodiment of this invention, 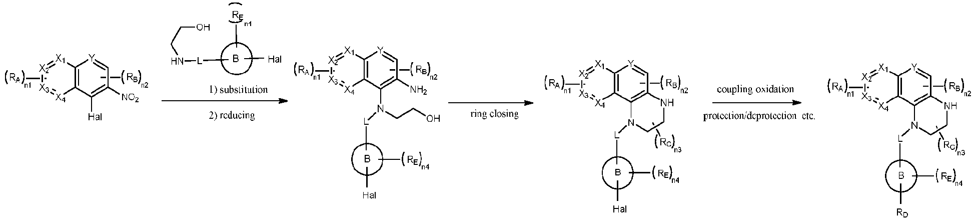 wherein in the above schemes, Hal represents halogen in the compound shown, and each substituent is defined as described earlier. After reading the disclosure of this application, those skilled in the art can easily determine the reaction conditions such as temperature, time, solvent, etc. suitable for the above schemes.

This invention further provides a pharmaceutical composition, containing the compound of this invention, a stereoisomer or tautomer of the compound or a mixture thereof, a pharmaceutically acceptable salt, co-crystal, polymorph or solvate of the compound, or a stable isotope derivative, metabolite or prodrug of the compound. This invention further provides a pharmaceutical composition, containing the compound of this invention, a stereoisomer or tautomer of the compound or a mixture thereof, a pharmaceutically acceptable salt, co-crystal, polymorph or solvate of the compound, or a stable isotope derivative, metabolite or prodrug of the compound, and pharmaceutically acceptable adjuvant.

The objective of this invention further comprises providing use of the compound of this invention, a stereoisomer or tautomer of the compound or a mixture thereof, a pharmaceutically acceptable salt, co-crystal, polymorph or solvate of the compound, or a stable isotope derivative, metabolite or prodrug of the compound in the manufacture of a medicament for preventing and/or treating ENPP1-mediated diseases.

In some embodiments, the ENPP1-mediated disease is cancer or tumour-related disease, e.g. pancreatic cancer.

In some embodiments, the ENPP1-mediated disease is cardiovascular disease, e.g. heart failure or myocardial infarction.

The objective of the present invention further includes providing a method for preventing and/or treating ENPP1-mediated diseases, which comprises administering a therapeutically effective dose of the compound of this invention, a stereoisomer or tautomer of the compound or a mixture thereof, a pharmaceutically acceptable salt, co-crystal, polymorph or solvate of the compound, or a stable isotope derivative, metabolite or prodrug of the compound, or the pharmaceutical composition of this invention to patient.

The compound of this invention, or prodrug, tautomer, stereoisomer, solvate, isotope derivative or pharmaceutically acceptable salt thereof can be administered in combination with another anticancer agent or immune checkpoint inhibitor for treating or preventing ENPP1-mediated diseases, such as cancer or tumour.

When the compound of this invention, or prodrug, tautomer, stereoisomer, solvate, isotope derivative or pharmaceutically acceptable salt thereof is administered in combination with another anticancer agent or immune checkpoint inhibitor for treating cancer or tumour, the compound of this invention or its pharmaceutically acceptable salt may provide an enhanced anticancer effect.

### Definition

Unless otherwise specified, the term "alkyl" refers to a monovalent saturated aliphatic hydrocarbon group, including a linear or branched group containing 1-20 carbon atoms, preferably containing 1-10 carbon atoms (namely C₁₋₁₀alkyl), further preferably containing 1-8 carbon atoms (C₁₋₈alkyl), more preferably containing 1-6 carbon atoms (namely C₁₋₆alkyl). For example "C₁₋₆ alkyl" means that the group is an alkyl group, and the number of carbon atoms in the carbon chain is between 1 and 6 (specifically 1, 2, 3, 4, 5 or 6). Its example includes but is not limited to methyl, ethyl, n-propyl, isopropyl, n-butyl, iso-butyl, tert-butyl, sec-butyl, n-pentyl, neo-pentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, n-octyl and the like.

Unless otherwise specified, the term "alkenyl" refers to a linear or branched unsaturated aliphatic hydrocarbon group consisting of carbon atoms and hydrogen atoms and having at least one double bond. The alkenyl can contain 2-20 carbon atoms, preferably contain 2-10 carbon atoms (namely C₂₋₁₀alkenyl), further preferably contain 2-8 carbon atoms (C₂₋₈alkenyl), more preferably contain 2-6 carbon atom (namely C₂-₆alkenyl), 2-5 carbon atom (namely C₂₋₅alkenyl), 2-4 carbon atoms (namely C₂-₄alkenyl), 2-3 carbon atoms (namely C₂₋₃alkenyl), 2 carbon atoms (namely C₂alkenyl). For example "C₂-₆alkenyl" means that the group is alkenyl, and the number of carbon atoms in the carbon chain is between 2 and 6 (specifically 2, 3, 4, 5 or 6). The non-limiting examples of alkenyl include but are not limited to ethenyl, 1-propenyl, 2-propenyl, 1-butenyl, isobutenyl and 1,3-butadienyl and the like.

Unless otherwise specified, the term "alkynyl" refers to a linear or branched unsaturated aliphatic hydrocarbon group consisting of carbon atoms and hydrogen atoms and having at least one triple bond. The alkynyl can contain 2-20 carbon atoms, preferably contain 2-10 carbon atoms (namely C₂₋₁₀alkynyl), further preferably contain 2-8 carbon atoms (namely C₂₋₈alkynyl), more preferably contain 2-6 carbon atoms (namely C₂-₆alkynyl), 2-5 carbon atoms (namely C₂₋₅alkynyl), 2-4 carbon atoms (namely C₂-₄alkynyl), 2-3 carbon atoms (namely C₂₋₃alkynyl), 2 carbon atoms (namely C₂alkynyl). For example "C₂-₆alkynyl" means that the group is alkynyl, and the number of carbon atoms in the carbon chain is between 2 and 6 (specifically 2, 3, 4, 5 or 6). The non-limiting examples of alkynyl include but are not limited to ethynyl, 1-propynyl, 2-propynyl, 1-butynyl and the like.

Unless otherwise specified, the term "cycloalkyl" refers to a monocyclic saturated aliphatic hydrocarbyl having a specified number of carbon atoms, preferably containing 3-12 carbon atoms (namely C₃₋₁₂cycloalkyl), more preferably 3-10 carbon atoms (C₃-₁₀cycloalkyl), further preferably 3-6 carbon atoms (C₃₋₆cycloalkyl), 4-6 carbon atoms (C4-6cycloalkyl), or 5-6 carbon atoms (C₅₋₆cycloalkyl). Examples include, but are not limited to cyclopropyl, cyclobutyl, cyclopentyl, cyclohexyl, methylcyclopropyl, 2-ethyl-cyclopentyl, dimethylcyclobutyl and the like. Unless otherwise specified, the term "alkoxy" refers to -O-alkyl, where the alkyl is defined as above, i.e., containing 1-20 carbon atoms, preferably 1-10 carbon atoms, more preferably 1-8 carbon atoms, and even more preferably 1-6 carbon atoms (specifically 1, 2, 3, 4, 5 or 6). Representative examples include but are not limited to methoxy, ethoxy, propoxy, isopropoxy, butoxy, 1-methylpropoxy, 2-methylpropoxy, tert-butoxy, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, etc.

Unless otherwise specified, the term "alkylthio" refers to the substitution of oxygen in the aforementioned "alkoxy" with sulfur.

Unless otherwise specified, the term "halogen" or "halo" refers to F, Cl, Br, or I.

The term "haloalkyl" refers to an alkyl group as defined above, in which one, two or more hydrogen atoms or all hydrogen atoms are substituted by halogen. Representative examples of haloalkyl include CCl₃, CF₃, CHCl₂, CH₂Cl, CH₂Br, CH₂I, CH₂CF₃, CF₂CF₃, etc.

Unless otherwise specified, the term "heterocyclyl" refers to a saturated or partially unsaturated monocyclic, bicyclic or polycyclic hydrocarbon substituent, which is in a non-aromatic structure and contains 3-20 ring atoms, of which 1, 2, 3 or more ring atoms are selected from N, O and S, and the remaining ring atoms are C. Preferably, it contains 3-12 ring atoms, more preferably 3-10 ring atoms, or 3-8 ring atoms, or 3-6 ring atoms, or 4-6 ring atoms, or 5-6 ring atoms. The number of heteroatoms is preferably 1-4, more preferably 1-3 (namely 1, 2, or 3). Examples of monocyclic heterocyclyl include pyrrolidinyl, imidazolinyl, tetrahydrofuryl, dihydropyrrolyl, piperidyl, piperazinyl, pyranyl, etc.

Bicyclic or polycyclic heterocyclyl include spiro, fused and bridged heterocyclyl.

Unless otherwise specified, the term "fused heterocyclyl" refers to a 5-20 membered polycyclic heterocyclic group, in which each ring in the system shares an adjacent pair of atoms with other rings in the system. One or more rings may contain one or more double bonds, but none of the rings have a fully conjugated π-electron system, where one or more ring atoms are heteroatoms selected from N, O, and S, and the remaining ring atoms are carbon. Preferably, it has 6-14, preferably 7-10 ring members. According to the number of constituent rings, it can be divided into bicyclic, tricyclic, tetracyclic, or polycyclic fused heterocyclyl, preferably bicyclic or tricyclic heterocyclyl, more preferably 4-membered/4-membered fused-heterocyclyl, 4-membered/5-membered fused-heterocyclyl, 5-membered/4-membered fused-heterocyclyl, 5-membered/5-membered fused-heterocyclyl, 5-membered/6-membered fused-heterocyclyl, 6-membered/5-membered fused-heterocyclyl, 4-membered/6-membered fused-heterocyclyl, 6-membered/4-membered fused-heterocyclyl, 6-membered/6-membered fused-heterocyclyl. The non-limiting examples of fused-heterocyclyl include

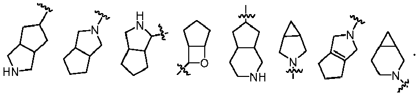

Unless otherwise specified, the term "spiro-heterocyclyl" refers to a 5-20 membered polycyclic heterocyclic group, in which each ring in the system shares one atom (called spiro-atom) with another ring in the system. One or more rings may contain one or more double bonds, but none of the rings have a fully conjugated π-electron system. Preferably, it has 6-14, preferably 7-10 ring members. According to the number of the spiro-atoms shared between rings, the spiro-heterocyclyl can be divided into mono-spiro-heterocyclyl, bis-spiro-heterocyclyl or poly-spiro-heterocyclyl, preferably mono-spiro-heterocyclyl and bis-spiro-heterocyclyl, more preferably 4-membered/4-membered spiro-heterocyclyl, 4-membered/5-membered spiro-heterocyclyl, 5-membered/4-membered spiro-heterocyclyl, 5-membered/5-membered spiro-heterocyclyl, 4-membered/6-membered spiro-heterocyclyl, 6-membered/4-membered spiro-heterocyclyl, 5-membered/6-membered spiro-heterocyclyl, 6-membered/5-membered spiro-heterocyclyl, 6-membered/6-membered spiro-heterocyclyl. The non-limiting examples of spiro-heterocyclyl include:

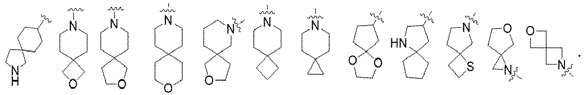

Unless otherwise specified, the term "aryl" refers to monocyclic, bicyclic and tricyclic aromatic carbocyclic ring systems containing 6-16, 6-14, 6-12 or 6-10 carbon atoms, preferably 6-10 carbon atoms. The term "aryl" may be used interchangeably with the term "aromatic ring". Examples of aryl may include but are not limited to phenyl, naphthyl, anthryl, phenanthryl or pyrenyl, etc.

Unless otherwise specified, the term "heteroaryl" refers to an aromatic monocyclic, bicyclic or polycyclic ring system having a 5-16, 5-14, 5-12, 5-10, 5-8, or 5-6 membered structure, in which 1, 2, 3 or more ring atoms are heteroatoms and the remaining atoms are carbon, the heteroatoms are independently selected from O, N and S, and the number of heteroatoms is preferably 1, 2 or 3. Examples of heteroaryl may include but are not limited to
furyl, thienyl, oxazolyl, thiazolyl, isoxazolyl, oxadiazolyl, thiadiazolyl, pyrrolyl, pyrazolyl, imidazolyl, triazolyl, tetrazolyl, pyridyl, pyrimidyl, pyrazinyl, pyridazinyl, thiadiazolyl, triazinyl, phthalazinyl, quinolinyl, isoquinolinyl, pteridinyl, purinyl, indolyl, isoindolyl, indazolyl, benzofuryl, benzothienyl, benzopyridyl, benzopyrimidyl, benzopyrazinyl, benzimidazolyl, benzophthalazinyl, pyrrolo[2,3-b]pyridyl, imidazo[1,2-a]pyridyl, pyrazolo[1,5-a]pyridyl, pyrazolo[1,5-a]pyrimidyl, imidazo[1,2-b]pyridazinyl, [1,2,4]triazolo [4,3 -b]pyridazinyl, [1,2,4]triazolo[1,5-a]pyrimidyl, [1,2,4]triazolo[1,5-a]pyridyl, etc.

Unless otherwise specified, the term "fused-heteroaryl" refers to an unsaturated aromatic fused ring structure containing 5-14 ring atoms (including at least one heteroatom) and formed by connecting two or more ring structures that share two adjacent atoms with each other, wherein 1, 2, 3 or more ring atoms are heteroatoms and the remaining atoms are carbon. The heteroatoms are independently selected from O, N, and S, and the number of heteroatoms is preferably 1, 2, or 3. Preferably, it is 5-12-membered fused-heteroaryl, 7-12-membered fused-heteroaryl, 9-12-membered fused-heteroaryl, etc., preferably 5-membered/5-membered fused-heteroaryl, 5-membered/6-membered fused-heteroaryl, 6-membered/5-membered fused-heteroaryl, 6-membered/6-membered bicyclic fused-heteroaryl. Examples of fused-heteroaryl may include but are not limited to benzofuryl, benzoisofuryl, benzothienyl, indolyl, isoindole, benzoxazolyl, benzimidazolyl, indazolyl, benzotriazolyl, quinolinyl, 2-quinolinone, 4-quinolinone, 1-isoquinolinone, isoquinolinyl, acridinyl, phenanthridinyl, benzopyridazinyl, phthalazinyl, quinazolinyl, quinoxalinyl, phenazinyl, pteridinyl, purinyl, naphthyridinyl, phenazine, phenothiazine, etc.

Unless otherwise specified, the term "pharmaceutically acceptable salt" or similar expressions refers to a salt that is suitable for use in contact with the tissues of mammals, especially humans, without undue toxicity, irritation, allergic response or the like and commensurates with a reasonable benefit/risk ratio within the range of reasonable medical judgment. For example, the pharmaceutically acceptable salts of amines, carboxylic acids and other types of compounds are well-known in the art. The salts can be prepared in situ during the final isolation and purification of the compounds of this invention, or separately by reacting the free base or acid with a suitable reagent.

Unless otherwise specified, the term "isotope derivative" means that the compounds of this invention can exist in isotope-tagged or enriched form containing one or more atoms having an atomic mass or mass number different from the atomic mass or mass number most abundantly found in nature. Isotopes can be radioactive or non-radioactive isotopes. Isotopes commonly used as isotopic labels are: hydrogen isotopes: ²H and ³H; carbon isotopes: ¹³C and ¹⁴C; chlorine isotopes: ³⁵Cl and ³⁷Cl; fluorine isotope: ¹⁸F; iodine isotopes: ¹²³I and ¹²⁵I; nitrogen isotopes: ¹³N and ¹⁵N; oxygen isotopes: ¹⁵O, ¹⁷O and ¹⁸O; and sulfur isotope: ³⁵S. These isotope-labelled compounds can be used to study the distribution of pharmaceutical molecules in tissues. In particular, ²H and ¹³C are more widely used due to their ease of labelling and ease of detection. The substitution with certain heavy isotopes, such as heavy hydrogen (²H), can enhance the stability of metabolism, and prolong the half-life, to achieve the purpose of reducing dosage and providing therapeutic advantages. Isotope-labelled compounds are generally synthesized starting from labelled starting materials in the same way as non-isotope-labelled compounds using known synthetic techniques.

Unless otherwise specified, the term "solvate" or similar expressions refer to the physical association of the present compound with one or more solvent molecules, regardless of whether organic or inorganic. This physical association includes hydrogen bonds. In some cases, for example, when one or more solvent molecules are incorporated into the crystal lattice of a crystalline solid, the solvates can be isolated. Solvent molecules in the solvates may exist in regular and/or disordered arrangements. The solvates may contain stoichiometric or non-stoichiometric amounts of solvent molecules. The "solvate" encompasses both solution-phase and isolatable solvate. Exemplary solvates include but are not limited to hydrates, ethanolates, methanolates and isopropanolates. Solvation methods are well-known in the art.

Unless otherwise specified, the term "stereoisomers" refers to compounds which have identical chemical constitutions, but differ about the arrangement of the atoms or groups in space. Stereoisomers include enantiomer, diastereomers, conformer (rotamer), geometric (cis/trans) isomers, atropisomers, etc. A mixture of any resulting stereoisomers can be separated into pure or substantially pure geometric isomers, enantiomers, and diastereomers, according to physicochemical differences of constituents, for example, by chromatography and/or fractional crystallization.

Unless otherwise specified, the term "tautomer" refers to structural isomers with different energies that can be converted to each other through low energy barriers. If tautomerism is possible (for example, in solution), a chemical equilibrium of tautomers can be reached. For example, protontautomer (also known as prototropic tautomer) includes interconversion through proton migration, e.g. ketone-enol isomerization and imine-enamine isomerization. Valence tautomer includes interconversion through the recombination of some bonding electrons.

Unless otherwise indicated, structure formulae depicted in this invention include all isomeric forms (e.g. enantiomers, diastereomers, and geometric isomers (or conformational isomers)): e.g., Rand S configurations with the asymmetric centre, (Z) and (E) isomers of double bond, and (Z) and (E) conformational isomers. Therefore, individual stereochemical isomers or mixtures of enantiomers, diastereomers, or geometric isomers (or conformational isomers) of the present compounds are within the scope of this invention.

Unless otherwise specified, the term "co-crystal" is used to describe the situation where neutral molecular components are present within a crystalline compound in a definite stoichiometric ratio. The preparation of pharmaceutical co-crystals enables modifications to be made to the crystalline form of an active pharmaceutical ingredient, which in turn can alter its physicochemical properties without compromising its intended biological activity (see Pharmaceutical Salts and Co-crystals, ed. J. Wouters & L. Quere, RSC Publishing, 2012).

Unless otherwise specified, the term "polymorph" refers to the different arrangement of chemical drug molecules, which is generally presented as the existing form of the drug's raw materials in the solid state. A drug may exist in a variety of crystal forms, and different crystal forms of the same drug may have different dissolution and absorption properties in vivo, thereby affecting the dissolution and release of the formulation.

Unless otherwise specified, the term "metabolite" refers to a product afforded through metabolism in the body of a specific compound or a salt thereof. Metabolite of a compound may be identified using routine techniques known in the art and its activity may be characterized using tests as those described herein. Such product may be obtained through routes such as oxidation, reduction, hydrolysis, amidation, deamidation, esterification, de-esterification, and enzymatic cleavage of the administered compound. Accordingly, this invention includes the metabolite of a compound, including a metabolite afforded by sufficiently contacting a compound of this invention with a mammal for some time.

Unless otherwise specified, the term "prodrug" refers to a drug that is converted into the parent drug in vivo. The prodrug is usually useful, which may improve certain undesirable physical or biological properties. The physical properties usually refer to related solubility (excessive or insufficient solubility in lipid or water) or stability, while problematic biological properties include overquick metabolism or poor bioavailability, which may itself be related to physical and chemical properties. For example, they are bioavailable via oral administration, whereas the parent drug cannot. Compared with the parent drug, the solubility of the prodrug in a pharmaceutical composition is also improved. An example of prodrug may be but is not limited to, any compound of this invention, which is administered as an ester ("prodrug") to facilitate transport through cell membranes, wherein the water solubility is detrimental to mobility but beneficial once it gets into the cell, and which is subsequently metabolically hydrolyzed to a carboxylic acid (i.e. active entity). Another example of the prodrug may be a short peptide (polyamino acid) bound to an acid group, wherein the peptide is metabolized to reveal the active moiety.

Unless otherwise specified, the term "optionally substituted" means that the hydrogen at the substitutable site of the group is unsubstituted, or substituted by one or more substituents, which are preferably selected from the group consisting of halogen, hydroxy, mercapto, cyano, nitro, amino, azido, oxo, carboxyl, C₂-₆alkenyl, C₂-₆alkynyl, C₁₋₆alkyl, C₁₋₆alkoxy, C₃-₁₀cycloalkyl, C₃-₁₀cycloalkylsulfonyl, 3-10 membered heterocycloalkyl, C₆-₁₄aryl, and 5-10 membered heteroaryl, wherein the C₂-₆alkenyl, C₂-₆alkynyl, C₁₋₆alkyl, C₁₋₆alkoxy, C₃-₁₀cycloalkyl, C₃-₁₀cycloalkylsulfonyl, 3-10 membered heterocycloalkyl, C₆-₁₄aryl or 5-10 membered heteroaryl may be optionally substituted by one or more substituents selected from halogen, hydroxy, amino, cyano, C₁₋₆alkyl, and C₁₋₆alkoxy, the oxo refers to the formation of a double bond by replacing two hydrogen atoms at the same substitution site with the same oxygen atom.

The beneficial effects of this invention lie in that:
This invention designs a class of compounds with novel structures and provides a new direction for the development of ENPP1 inhibitor drugs. Biochemical enzyme activity in vitro and MDA-MB-231 cell enzyme activity inhibition studies showed that these compounds had a strong inhibitory effect on both recombinant ENPP1 enzymes and endogenously expressed ENPP1 enzymes in cells, while exhibiting minimal inhibitory activity on ENPP2 enzymes, demonstrating good selectivity. In vitro, ADME test results indicated relatively high stability in liver microsomes, no risk of hERG inhibition, and good cell permeability. In vivo pharmacokinetic assays in mice also showed good bioavailability and exposure with excellent metabolic characteristics, making these compounds promising for the treatment of ENPP1-mediated diseases.

### Detailed description

This invention is further described below in connection with specific examples. It should be understood that these examples are intended to illustrate this invention only and are not intended to limit the scope of this invention. Experimental methods for which no specific conditions are indicated in the following examples are generally by conventional conditions or in accordance with the conditions recommended by the manufacturers. Unless otherwise defined, all professional and scientific terms used herein have the same meaning as those familiar to those skilled in the art. In addition, any methods and materials similar or equivalent to those described herein may be used in the methods of this invention. The preferred embodiments and materials shown in this disclosure are illustrative only. The structures of the compounds according to this invention are determined by nuclear magnetic resonance (NMR) or/and liquid chromatography-mass spectrometry (LC-MS) or/and liquid chromatography (HPLC). The NMR measurement instrument used is Bruker 400MHz or/and Varian 400MHz; the LC-MS instrument used is Agilent 1260 Infinity II-6120/6125MSD; the HPLC instrument used is Waters Acquity UPLC 2 or/and Shimadzu LC2030 or/and Agilent 1260 Infinity II.

The starting materials in the examples of this invention are known and commercially available, or can be synthesized using those methods known in the art or according to those methods known in the art.

This invention provides processes for preparing the compounds. The compounds can be prepared through the following steps.

In the following examples, abbreviations have the following meanings:

| | |
|---|---|
| Ac | acetyl |
| ACN | acetonitrile |
| AcOH | acetic acid |
| Bn | benzyl |
| Boc | tert-butoxycarbonyl |
| Boc₂O | di-tert-butyl dicarbonate |
| BTC | bis(trichloromethyl)carbonate (triphosgene) |
| DCM | dichloromethane |
| DIBAL-H | diisobutylaluminium hydride |
| DIEA | N,N-diisopropylethylamine |
| dioxane | dioxane/1,4-dioxacyclohexane |
| DMAP | 4-dimethylaminopyridine |
| DMF | N,N-dimethylformamide |
| Et | ethyl |
| HATU | O-(7-azabenzotriazol-1-yl)-N,N,N',N'-tetramethyluronium hexafluorophosphate |
| Me | methyl |
| Pd₂(dba)₂ | tris(dibenzylideneacetone)dipalladium |
| PMB | para-methoxybenzyl |
| pTsNHNH₂ | para-toluenesulfonyl |
| TFA | trifluoroacetic acid |
| THF | tetrahydrofuran |
| Xantphos | 4,5-bis(diphenylphosphino)-9,9-dimethylxanthene |
| Pd(dppf)Cl₂ | (1,1'-bis(diphenylphosphino)ferrocene)dichloropalladium |
| TEA | triethylamine |
| TMSCHN₂ | trimethylsilyldiazomethane |

PerkinElmer's Chemdraw^{®} (Version 20.0.0.41) was used to name the example compounds.

### Preparation Example 1

### Preparation of (4-bromo-2,6-difluorophenyl)methanamine:

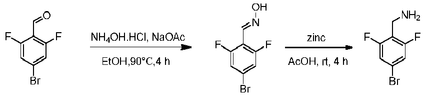

### Step 1: Preparation of (E)-4-bromo-2,6-difluorobenzaldehyde oxime

4-bromo-2,6-difluorobenzaldehyde (50g, 0.23 mol, 1.0eq.), hydroxylamine hydrochloride (78.61g, 1.13 mol, 5.0eq.) and sodium acetate (79.1g, 1.13 mol, 5.0eq.) were added to ethanol (2 L). The resulting mixture was stirred, and heated to 90°C under refluxing for 4hrs. The completion of the reaction was monitored with LCMS. The reaction was cooled and distilled in vacuum to remove the solvent ethanol. Water (1L) was added. The mixture was extracted with EA (ethyl acetate, 1L×2). The organic phases were washed with saturated brine, and then dried over anhydrous sodium sulfate. The solvent was removed by distillation under reduced pressure to afford the target compound (50g, yield 93.64%).

LCMS (ESI): [M+H]⁺=236.0.

### Step 2: Preparation of (4-bromo-2,6-difluorophenyl)methanamine

(E)-4-bromo-2,6-difluorobenzaldehyde oxime (12.0g, 93.22mmol, 1.0eq.), and zinc powder (60.9g, 932.2mmol, 10.0eq.) were dissolved in an aqueous acetic acid solution (300mL). The mixed solution was stirred at rt (rt=room temperature, 20°C) for 4hrs. The completion of the reaction was monitored with LCMS. The reaction mixture was filtered. The resulting filtrate was concentrated. The residue was dissolved in water (200mL). The solution was adjusted to alkalinity with saturated sodium hydroxide solution, and extracted with EA (3×200mL). The organic phases were combined, dried over anhydrous sodium sulfate, and concentrated. The residue was purified by flash chromatography (Silica gel, DCM/MeOH=20:1) to afford the target compound (11.8g, yield 57%).

LCMS (ESI): [M+H]⁺=222.0; ¹HNMR(400MHz, DMSO-d₆) δ 7.57-7.30 (m, 2H), 3.66 (s, 2H).

### Preparation Example 2

### Preparation of 7-methoxy-1,8-naphthyridin-4-ol:

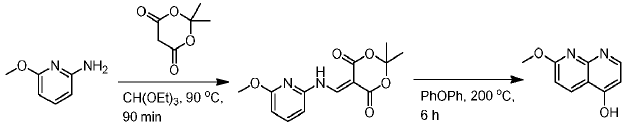

### Step 1: Preparation of 5-(((6-methoxypyridin-2-yl)amino)methylene)-2,2-dimethyl-1,3-dioxane-4,6-dione

2,2-dimethyl-1,3-dioxane-4,6-dione (23.82g, 165.3mmol, 0.9eq.) was dissolved in triethyl orthoformate (91mL, 615.27mmol, 3.35eq.). The mixture was reacted at 90°C for 1.5hrs, and then cooled to 70°C. 2-amino-6-methoxypyridine (22.8g, 183.66mmol, 1.0eq.) was slowly added in batch. The reaction continued at 70°C for 30 minutes. TLC and The completion of the reaction was monitored with LCMS. The mixture was cooled to rt, and filtered. The filter cake was washed with petroleum ether, and dried to afford the target compound (38.3g, yield 74.94%).

LCMS (ESI) [2M+Na]⁺⁼579.1; ¹H NMR (400MHz, DMSO-d₆) δ 11.33 (d, J=13.6 Hz, 1H), 9.18 (d, J=14.0 Hz, 1H), 7.78 (q, J=8.0 Hz, 1H), 7.21 (d, J=7.6 Hz, 1H), 6.70 (d, J=8.4 Hz, 1H), 3.91 (s, 3H), 1.69 (s, 6H).

### Step 2: Preparation of 7-methoxy-1,8-naphthyridin-4-ol

5-(((6-methoxypyridin-2-yl)atnino)methylene)-2,2-dimethyl-1,3-dioxane-4,6-dione (20g, 71.88mmol, 1.0eq.) was dissolved in diphenyl ether (95.3mL). The mixture was reacted at 200°C for 6hrs. After the completion of the reaction, the mixture was cooled to rt. A large amount of petroleum ether was added to precipitate a solid. The mixture was filtered. The filter cake was washed with petroleum ether, and dried to afford the target compound (10.3g, yield 81.34%).

LCMS (ESI) [M+H]⁺=177.1; ¹H NMR (400MHz, DMSO-d₆) δ 11.98 (s, 1H), 8.29 (d, J=8.8 Hz, 1H), 7.78-7.75 (m, 1H), 6.79 (d, J=8.4 Hz, 1H), 6.05-6.03 (m, 1H), 3.96 (s, 3H).

### Example 1

### Preparation of 3,5-difluoro-4-((9-fluoro-8-methoxy-3-oxo-4-(2,2,2-trifluoroethyl)-3,4-dihydropyrimido[4,5-c]quinolin-2(1H)-yl)methyl)benzenesulfonamide (compound 1):

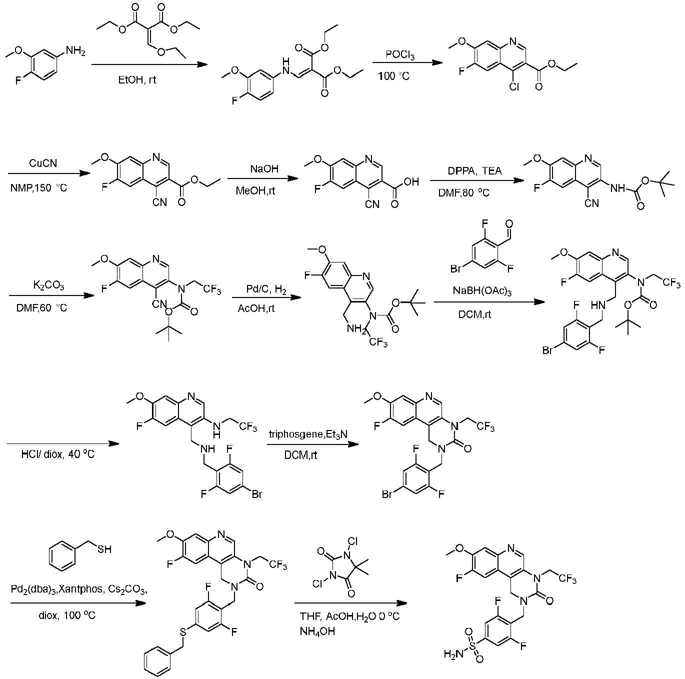

### Step 1: Preparation of diethyl 2-(((4-fluoro-3-methoxyphenyl)amino)methylene)malonate

4-fluoro-3-methoxyaniline (9.79g, 0.07 mol, 1.5eq.) and 1,3-diethyl 2-(ethoxymethylene)malonate (10g, 0.05 mol, 1eq.) were added to ethanol (200mL). The mixture was stirred at rt for 4hrs. After the completion of the reaction, the reaction mixture was purified by flash silica gel column chromatography (Silica gel, PE:EA=5:95) to afford the target compound (12.8g, yield 88.91%).

LCMS (ESI) [M+H]⁺=312.1; ¹H NMR (400MHz, DMSO-d₆) δ 10.70 (d, J=13.6 Hz, 1H), 8.36 (d, J=13.6 Hz, 1H), 7.24 (m, J=5.6 Hz, 2H), 6.92 (d, J=8.6 Hz, 1H), 4.21 (m, J=7.2 Hz, 2H), 4.13 (m, J=7.2 Hz, 2H), 3.88 (s, 3H), 1.26 (m, J=8.6 Hz, 6H).

### Step 2: Preparation of ethyl 4-chloro-6-fluoro-7-methoxyquinoline-3-carboxylate

Diethyl 2-(((4-fluoro-3-methoxyphenyl)amino)methylene)malonate (5g, 16.06mmol) and phosphorus oxychloride (8mL) were placed in a flask (100mL), and warmed to 100°C. The mixture was reacted under stirring for 5hrs. LCMS monitoring indicated the completion of the reaction, the mixture was cooled to rt and slowly added dropwise to a flask containing 200mL of water. After the completion of the addition, the mixture was cooled. An aqueous sodium bicarbonate solution was gradually added to adjust the mixture to neutrality. The mixture was extracted with EA (200mL×2). The organic phases were combined, washed with brine, and distilled under reduced pressure to remove the solvent to afford the target compound (4.2g, yield 92.19%).

LCMS (ESI) [M+H]⁺=284.0.

### Step 3: Preparation of ethyl 4-cyano-6-fluoro-7-methoxyquinoline-3-carboxylate

Ethyl 4-chloro-6-fluoro-7-methoxyquinoline-3-carboxylate (3.1g, 10.93mmol, 1.0eq.) and cyanocopper (3.9g, 43.72mmol, 4.0eq.) were added to NMP(20mL). The mixture was reacted at 150°C for 15hrs, and filtered while hot. Water (100mL) was added. The mixture was extracted with EA (200mL×2). The organic phases were washed with brine five times, dried over anhydrous sodium sulfate, and distilled under reduced pressure to remove the solvent. The residue was purified by flash silica gel column chromatography (Silica gel, PE:EA=8:92) to afford the target compound (950mg, yield 31.7%).

LCMS (ESI) [M+H]⁺=275.0; ¹H NMR (400MHz, DMSO-d₆) δ 9.38 (s, 1H), 7.89 (m, J=9.6 Hz, 2H), 4.47 (q, J=7.2 Hz, 2H), 4.11 (s, 3H), 1.42 (t, J=7.2 Hz, 3H).

### Step 4: Preparation of 4-cyano-6-fluoro-7-methoxyquinoline-3-carboxylic acid

Ethyl 4-cyano-6-fluoro-7-methoxyquinoline-3-carboxylate (950mg, 3.46mmol, 1.0eq.) was added to methanol (50mL). Then NaOH (2M, 3mL) solution was added. The mixture was stirred at rt for 1hr. After the completion of the reaction, methanol was removed by rotary-evaporation in vacuum. Water (20mL) was added. The mixture was adjusted to acidity, and extracted with EA (200mL×2). The organic phases were washed with saturated brine twice, then dried over anhydrous sodium sulfate, and filtered. The filtrate was distilled under reduced pressure to remove the solvent to afford the target compound (606mg, yield 75.27%).

LCMS (ESI) [M+H]⁺=247.1.

### Step 5: Preparation of tert-butyl (4-cyano-6-fluoro-7-methoxyquinolin-3-yl)carbamate

4-cyano-6-fluoro-7-methoxyquinoline-3-carboxylic acid (330mg, 1.34mmol, 1.0eq.), triethylamine (1353.4mg, 13.4mmol, 10eq.), diphenylphosphoryl azide (1105mg, 4.02mmol, 3.0eq.) and tert-butanol (5mL) were added to DMF (5mL). The mixture was stirred at 80°C for 2hrs. After the completion of the reaction, the mixture was distilled under reduced pressure to remove the solvent. Water (20mL) was added. The mixture was extracted with EA (1000mL×2). The organic phases were washed with saturated brine, dried over anhydrous sodium sulfate, and distilled under reduced pressure to remove the solvent. The residue was purified by silica gel column chromatography (Silica gel, PE:EA=85:15) to afford the target compound (180mg, yield 42.55%).

LCMS (ESI) [M+H]⁺=318.1.

### Step 6: Preparation of tert-butyl (4-cyano-6-fluoro-7-methoxyquinolin-3-yl)(2,2,2-trifluoroethyl)carbamate

Tert-butyl (4-cyano-6-fluoro-7-methoxyquinolin-3-yl)carbamate (20mg, 0.13mmol, 1.0eq.) and 2,2,2-trifluoroethyl trifluoromethanesulfonate (60.3mg, 0.26mmol, 2.0eq.) *were added to DMF (*2mL). K₂CO₃ (35.8mg, 0.26mmol, 2.0eq.) was added. The mixture was stirred at 60°C for 1hr. After the completion of the reaction, water (20mL) was added. The mixture was extracted with EA (50mL×2). The organic phases were washed with saturated brine five times, then dried over anhydrous sodium sulfate, and filtered. The filtrate was distilled under reduced pressure to remove the solvent to afford a crude product, which was purified by silica gel column chromatography (Silica gel, PE:EA=13:87) to afford the target compound (20mg, yield 39.73%).

LCMS (ESI) [M+H]⁺=400.0; ¹H NMR (400MHz, DMSO-d₆) δ 9.10 (s, 1H), 7.88 (d, J=10.8 Hz, 1H), 7.83 (d, J=8.2 Hz, 1H), 4.72 (s, 3H), 4.08 (s, 3H), 1.37 (d, J=35.8 Hz, 6H), 1.28-1.05 (m, 2H).

### Step 7: Preparation of tert-butyl (4-(aminomethyl)-6-fluoro-7-methoxyquinolin-3-yl)(2,2,2-trifluoroethyl)carbamate

Tert-butyl (4-cyano-6-fluoro-7-methoxyquinolin-3-yl)(2,2,2-trifluoroethyl)carbamate (190mg, 0.12mmol, 1.0eq.) was added to acetic acid (5mL). Pd/C (40mg) was added. The mixture was reacted at rt under the protection of hydrogen gas for 2hrs. After the completion of the reaction, Pd/C was filtered off. Water (20mL) was added. The mixture was extracted with EA (50mL×2). The organic phases were washed with saturated brine, dried over anhydrous sodium sulfate, and distilled under reduced pressure to remove the solvent to afford the target compound (190mg, yield 99%).

LCMS (ESI) [M+H]⁺=404.1.

### Step 8: Preparation of tert-butyl (4-(((4-bromo-2,6-difluorobenzyl)amino)methyl)-6-fluoro-7-methoxyquinolin-3-yl)(2,2,2-trifluoroethyl)carbamate

Tert-butyl (4-(aminomethyl)-6-fluoro-7-methoxyquinolin-3-yl)(2,2,2-trifluoroethyl)carbamate (160mg, 0.4mmol, 1.0eq.) and 4-bromo-2,6-difluorobenzaldehyde (32.8mg, 0.48mmol, 1.2eq.) were added to dichloromethane (10mL). A drop of was acetic acid added dropwise. sodium triacetoxyborohydride (420mg, 1.6mmol, 4.0eq.) and *a molecular sieve* were added. The mixture was stirred at rt for 2hrs. The completion of the reaction was monitored with LCMS. The reaction mixture was purified by flash silica gel column chromatography (Silica gel, PE:EA=75:25) to afford the target compound (170mg, yield 70.45%).

LCMS (ESI) [M+H]⁺=609.1; ¹H NMR (400MHz, DMSO-d₆) δ 8.58 (s, 1H), 8.11 (s, 1H), 7.60 (s, 1H), 7.50 (s, 2H), 4.46 (s, 2H), 4.10-3.99 (m,J=1.6 Hz 4H), 3.87 (s, 3H), 3.78 (s, 1H), 1.44 (s, 3H), 1.21 (d, J=16.2 Hz, 6H).

### Step 9: Preparation of 4-(((4-bromo-2,6-difluorobenzyl)amino)methyl)-6-fluoro-7-methoxy-N-(2,2,2-trifluoroethyl)-quinoline-3 -amine

Tert-butyl (4-(((4-bromo-2,6-difluorobenzyl)amino)methyl)-6-fluoro-7-methoxyquinolin-3-yl)(2,2,2-trifluoroethyl)carbamate (200mg, 0.32mmol, 1.0eq.) was added to HCl/dioxane (1M, 5mL). The mixture was stirred at room temperature for 2hrs, and distilled under reduced pressure to remove the solvent to afford a crude target compound (160mg), which was not further purified but directly used in the next step reaction.

LCMS (ESI) [M+H]⁺=509.1.

### Step 10: Preparation of 2-(4-bromo-2,6-difluorobenzyl)-9-fluoro-8-methoxy-4-(2,2,2-trifluoroethyl)-1,4-dihydropyrimidine[4,5-c]quinolin-3(2H)-one

4-(((4-bromo-2,6-difluorobenzyl)amino)methyl)-6-fluoro-7-methoxy-N-(2,2,2-trifluoroethyl)-quinoline-3-amine (160mg, crude) was added to tetrahydrofuran (10mL). Triphosgene (60mg) was added. The mixture was stirred at room temperature for 2hrs. After the completion of the reaction, the reaction mixture was extracted with EA (100mL×2). The organic phases were washed with saturated brine, dried over anhydrous sodium sulfate, and purified by flash silica gel column chromatography (Silica gel, PE:EA=70:30) to afford the target compound (140mg, yield 87.35%).

LCMS (ESI) [M+H]⁺=534.0.

### Step 11: Preparation of 2-(4-(benzylthio)-2,6-difluorobenzyl)-9-fluoro-8-methoxy-4-(2,2,2-trifluoroethyl)-1,4-dihydropyrimidine[4,5-c]quinolin-3(2H)-one

2-(4-bromo-2,6-difluorobenzyl)-9-fluoro-8-methoxy-4-(2,2,2-trifluoroethyl)-1,4-dihydropyrimidine [4,5-c] quinolin-3(2H)-one (140mg, 0.26mmol, 1eq.) and phenylmethanethiol (39mg, 0.31mmol, 1.2eq.) were added to dioxane (10mL). Tris(dibenzylideneacetone)dipalladium (24mg, 0.026mmol, 0.1eq.), and caesium carbonate (169mg, 0.52mmol, 2eq.) were added. Under the protection of nitrogen gas, the mixture was reacted at 100°C for 3hrs. After the completion of the reaction, the reaction mixture was extracted with EA (100mL×2). The organic phases were washed with saturated brine, dried over anhydrous sodium sulfate, and purified by flash silica gel column chromatography (Silica gel, PE:EA=65:35) to afford the target compound (85mg, yield 56.61%).

LCMS (ESI) [M+H]⁺=578.1.

### Step 12: Preparation of 3,5-difluoro-4-((9-fluoro-8-methoxy-3-oxo-4-(2,2,2-trifluoroethyl)-3,4-dihydropyrimido[4,5-c]quinolin-2(1H)-yl)methyl)benzenesulfonamide

2-(4-(benzylthio)-2,6-difluorobenzyl)-9-fluoro-4-methoxy-4-(2,2,2-trifluoroethyl)-1,4-dihydropyrimidine[4,5-c]quinolin-3(2H)-one (80mg, 0.14mmol, 1.0eq.) was added to tetrahydrofuran (2mL) and and acetonitrile (1mL). At 0°C, a drop of acetic acid, a drop of water and 1,3-dichloro-5,5-dimethylhydantoin (110mg, 0.56mmol, 4eq.) were added. The mixture was reacted at 0°C for 2hrs. After the completion of the reaction, ammonia solution (2mL) was added dropwise at 0°C. The mixture was extracted with EA (100mL×2). The organic phases were washed with saturated brine, dried over anhydrous sodium sulfate, and distilled under reduced pressure to remove the solvent to afford a crude product, which was purified to afford the title compound (12mg, yield 13.33%).

LCMS (ESI) [M+H]⁺=535.1; ¹HNMR (400MHz, DMSO-d₆) δ 8.90 (s, 1H), 7.69 (d, J=12.6 Hz, 1H), 7.58 (d, J=8.4 Hz, 1H), 7.45 (d, J=6.8 Hz, 2H), 7.18 (s, 2H), 5.04 (d, J=9.2 Hz, 2H), 4.96 (s, 2H), 4.79 (s, 2H), 3.99 (s, 3H).

### Example 2

### Preparation of 3,5-difluoro-4-((8-methoxy-2-oxopyrazino[2,3-c][1,8]naphthyridin-1(2H)-yl)methyl)benzenesulfonamide (compound 2):

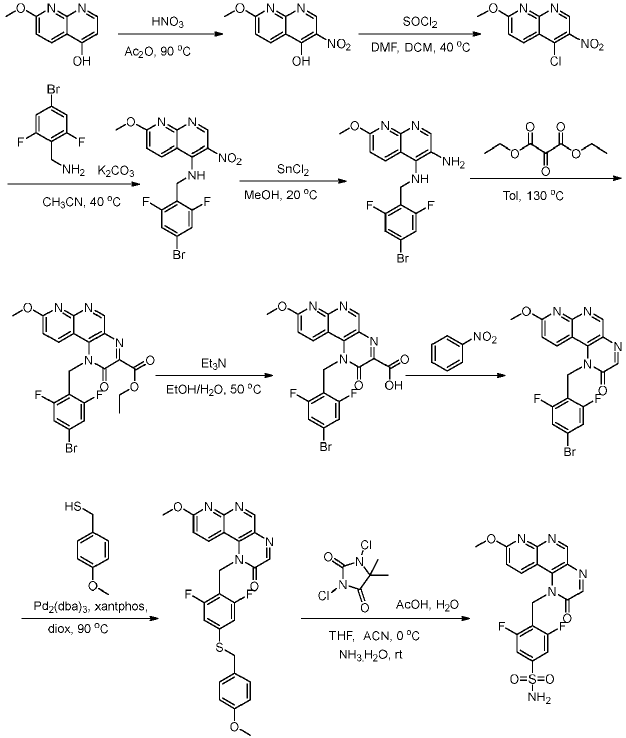

### Step 1: Preparation of 7-methoxy-3-nitro-1,8-naphthyridin-4-ol

7-methoxy-1,8-naphthyridin-4-ol (14g, 79.47mmol, 1.0eq.) was dissolved in acetic anhydride (100mL). In an ice bath, nitric acid (8.41mL, 133.51mmol, 1.68eq.) was slowly added dropwise. Then the mixture was heated to 90°C and reacted at 90°C for 2hrs. After the completion of the reaction, the reaction mixture was cooled to rt, and filtered. The filter cake was washed with petroleum ether, and dried to afford the target compound (5.8g, yield 33%).

LCMS (ESI) [M+H]⁺=222.1; ¹H NMR (400MHz, DMSO-d₆) δ 13.27 (s, 1H), 8.95 (s, 1H), 8.45 (d, J=8.8 Hz, 1H), 6.98 (d, J=8.8 Hz, 1H), 4.01 (s, 3H).

### Step 2: Preparation of 5-chloro-2-methoxy-6-nitro-1,8-naphthyridine

7-methoxy-3-nitro-1,8-naphthyridin-4-ol (8.8g, 39.79mmol, 1.0eq.) was dissolved in dichloromethane (50mL). A catalytic amount of N,N-dimethylformamide (1mL) was added. Then under an ice bath, sulfurous dichloride (9mL, 75.69mmol, 1.9eq.) was slowly added dropwise. The mixture was warmed to 40°C and reacted at 40°C for 2hrs. After the completion of the reaction, the reaction was quenched with ice water, and extracted with dichloromethane (100mL×3). The organic phases were collected, dried, filtered, concentrated, and purified by column chromatography (Silica gel, PE:EA=2:1) to afford the target compound (7.2g, yield 75.52%).

LCMS (ESI) [M+H]⁺=239.9; ¹H NMR (400MHz, DMSO-d₆) δ 8.95-8.94 (m, 1H), 8.44 (d, J=8.8 Hz, 1H), 6.98 (d, J=8.8 Hz, 1H), 4.01 (s, 3H).

### Step 3: Preparation of N-(4-bromo-2,6-difluorobenzyl)-7-methoxy-3-nitro-1,8-naphthyridin-4-amine

5-chloro-2-methoxy-6-nitro-1,8-naphthyridine (600mg, 2.5mmol, 1eq.) and 1-(4-bromo-2,6-difluorophenyl)methanamine (610.6mg, 2.75mmol, 1.1eq.) were dissolved in acetonitrile (100mL). K₂CO₃ (1.04g, 7.5mmol, 3eq.) was added. The mixture was reacted at 40°C for 2hrs. After the completion of the reaction. The reaction mixture was concentrated, washed with water and extracted with EA (20mL×3). The organic phases were collected, dried, filtered, concentrated, and purified by column chromatography (Silicagel, PE:EA=1:1) to afford the target compound (700mg, yield 65.9%).

LCMS (ESI) [M+H]⁺=427.0; ¹H NMR (400MHz, DMSO-d₆) δ 9.14 (s, 1H), 8.84-8.67 (m, 2H), 7.51 (d, J=6.8 Hz, 2H), 7.10 (d, J=8.6 Hz, 1H), 4.69 (s, 2H), 4.02 (s, 3H).

### Step 4: Preparation of N⁴-(4-bromo-2,6-difluorobenzyl)-7-methoxy-1,8-naphthyridine-3,4-diamine

N-(4-bromo-2,6-difluorobenzyl)-7-methoxy-3-nitro-1,8-naphthyridin-4-amine (660mg, 1.55mmol, 1.0eq.) was dissolved in methanol (50mL). Stannous chloride (2943mg, 15.52mmol, 10eq.) was added. The mixture was reacted at 20°C for 2hrs. After the completion of the reaction. The reaction mixture was concentrated to remove a part of the solvent. The residue was washed with an aqueous saturated sodium bicarbonate solution to weak alkalinity, and then washed with water and extracted with EA (50mL×3). The organic phases were combined, dried over anhydrous sodium sulfate, filtered, concentrated, and purified by column chromatography (Silica gel, DCM:MeOH=20:1) to afford the target compound (700mg, crude).

LCMS (ESI) [M+H]⁺=397.0; ¹H NMR (400MHz, DMSO-d₆) δ 8.70 (d, J=9.6 Hz, 1H), 8.08 (s, 1H), 7.51 (d, J=7.2 Hz, 2H), 7.06 (d, J=9.2 Hz, 1H), 5.00 (s, 2H), 4.07-3.94 (m, 6H).

### Step 5: Preparation of ethyl 1-(4-bromo-2,6-difluorobenzyl)-8-methoxy-2-oxo-1,2-dihydropyrazino[2,3-c][1,8]naphthyridine-3-carboxylate

To a single necked flask were added N⁴-(4-bromo-2,6-difluorobenzyl)-7-methoxy-1,8-naphthyridine-3,4-diamine (300mg, crude), diethyl 2-oxomalonate (264mg, 1.51mmol), and toluene (10mL). The reaction apparatus was equipped with a water separator. The mixture was reacted at 130°C for 16hrs. After the reaction product was monitored, the reaction mixture was concentrated to afford a crude product, which was purified by flash silica gel column chromatography (petroleum ether:EA=100:0-100:70) to afford the target compound (220mg).

LCMS (ESI) [M+H]⁺=505/507; ¹HNMR (400MHz, CDCl₃) δ 9.33 (s, 1H), 8.37 (d, J=12.0 Hz, 1H), 7.19-6.95(m, 3H), 5.69 (s, 2H), 4.44-4.42 (m, 2H), 4.06 (s, 3 H), 1.17 (t, J=6.4 Hz, 3 H).

### Step 6: Preparation of 1-(4-bromo-2,6-difluorobenzyl)-8-methoxy-2-oxo-1,2-dihydropyrazino[2,3-c][1,8]naphthyridine-3-carboxylic acid

To a single-necked round-bottom flask were added ethyl 1-(4-bromo-2,6-difluorobenzyl)-8-methoxy-2-oxo-1,2-dihydropyrazino[2,3-c][1,8]naphthyridine-3-carboxylate (220mg, 0.43mmol, 1.0eq.), ethanol (5mL), triethylamine (176mg, 1.74mmol, 4.0eq.), and water (2mL). The reaction mixture was warmed to 50°C and stirred for 16hrs. The reaction mixture was concentrated at rt to remove a part of the solvent. The residue was diluted with EA, and the resulting solution was adjusted with dilute hydrochloric acid (1mol/L) to a pH of 4. The EA layer was separated, dried over anhydrous sodium sulfate, and filtered. The filtrate was concentrated to afford the target compound (180mg, yield 90%).

LCMS (ESI) [M+H]⁺=477.0/479.0; ¹HNMR (400MHz, CDCl₃) δ 9.54 (s, 1H), 8.45 (d, J=6.4 Hz, 1H), 7.28 (d, J=6.4 Hz, 1H), 7.05-7.01 (m, 3 H), 5.75 (s, 2H), 4.06 (s, 3 H).

### Step 7: Preparation of 1-(4-bromo-2,6-difluorobenzyl)-8-methoxypyrazino[2,3-c][1,8]naphthyridin-2(1H)-one

To a wicrowave tube were added 1-(4-bromo-2,6-difluorobenzyl)-8-methoxy-2-oxo-1,2-dihydropyrazino[2,3-c][1,8]naphthyridine-3-carboxylic acid (150mg, 0.31mmol, 1.0eq.) and nitrobenzene (3mL). The reaction mixture was heated to 160°C and stirred for 2hrs. After the completion of the reaction, the reaction mixture was directly purified by column chromatography (petroleum ether:EA=100:0-0:100) to afford the target compound (80mg, yield 59%).

LCMS (ESI): [M+H]⁺=432.9/433.9; ¹HNMR (400MHz, CDCl₃) δ 9.33 (s, 1H), 8.40 (d, J=8.0 Hz, 1H), 8.32 (s, 1H), 7.19-6.98 (m, 3H), 5.69 (s, 2 H), 4.21 (s, 3H).

### Step 8: Preparation of 1-(2,6-difluoro-4-((4-methoxybenzyl)thio)benzyl)-8-methoxypyrazino[2,3-c] [1,8]naphthyridin-2(1H)-one

To a wicrowave tube were added 1-(4-bromo-2,6-difluorobenzyl)-8-methoxypyrazino[2,3-c][1,8]naphthyridin-2(1H)-one (52mg, 0.12mmol, 1.0eq.), para-methoxyphenylmethanethiol (55mg, 0.36mmol, 3.0eq.), caesium carbonate (84mg, 0.26mmol, 2.2eq.) and tris(dibenzylideneacetone)dipalladium (10mg, 0.01mmol, 0.1eq.), and 4,5-bis(diphenylphosphino)-9,9-dimethylxanthene (10mg, 0.02mmol, 0.2eq.). The reaction system was purged with nitrogen gas for 1 minute, heated to 90°C in a sealed manner, and reacted for 4hrs. After the completion of the reaction, the reaction mixture was washed with water and extracted with EA. The EA layer was dried and concentrated to afford a crude product, which was purified (petroleum ether:EA=1:1) to afford the target compound (20mg, yield 32%).

LCMS (ESI): [M+H]⁺=507.1; ¹HNMR (400MHz, CDCl₃) δ 9.33 (s, 1H), 8.44 (d, J=8.0 Hz, 1H), 8.32 (s, 1H), 7.19 (d, J=8.0 Hz, 2H), 6.78 (d, J=8.0 Hz, 1H), 6.69 (d, J=8.0 Hz, 2H), 6.55 (d, J=8.0 Hz, 2H), 5.69 (s, 2H), 4.17(s, 3H), 4.01(s, 2 H), 3.74(s, 3H).

### Step 9: Preparation of 3,5-difluoro-4-((8-methoxy-2-oxopyrazino[2,3-c][1,8]naphthyridin-1(2H)-yl)methyl)benzenesulfonamide

To a round-bottom flask were added 1-(2,6-difluoro-4-((4-methoxybenzyl)thio)benzyl)-8-methoxypyrazino[2,3-c][1,8]naphthyridin-2(1H)-one (15mg, 0.029mmol, 1.0eq.), tetrahydrofuran (2mL), acetonitrile (1mL), acetic acid (2mg, 0.033mmol, 1.1eq.), and water (2mg, 0.11mmol, 3.7eq.). To the ice-water cooled solution was added 1,3-dichloro-5,5-dimethylhydantoin (12mg, 0.058mmol, 2.0eq.). The reaction was performed under stirring for 60 minutes. Ammonia solution (1mL) was added. The stirring continued for 60 minutes. After the completion of the reaction, the reaction mixture was washed with water and extracted with EA. The EA layer was dried over anhydrous sodium sulfate, and filtered. The filtrate was concentrated to afford a crude product, which was purified by liquid chromatography to afford the target compound (4.5mg, yield 35%).

LCMS (ESI) [M+H]⁺=434.0; ¹HNMR (400MHz, DMSO-d₆) δ 9.24 (s, 1H), 8.77 (d, J=8.0 Hz, 1H), 8.35 (s, 1H), 7.63 (s, 2H), 7.47 (d, J=8.0 Hz, 2H), 7.17 (d, J=8.0 Hz, 1H), 5.83 (s, 2H), 4.06 (s, 3H).

### Example 3

### Preparation of 4-(8-methoxypyrimido[4,5-c][1,8]naphthyridin-1-yl)-1,4-diazepane-1-sulfonamide (compound 3):

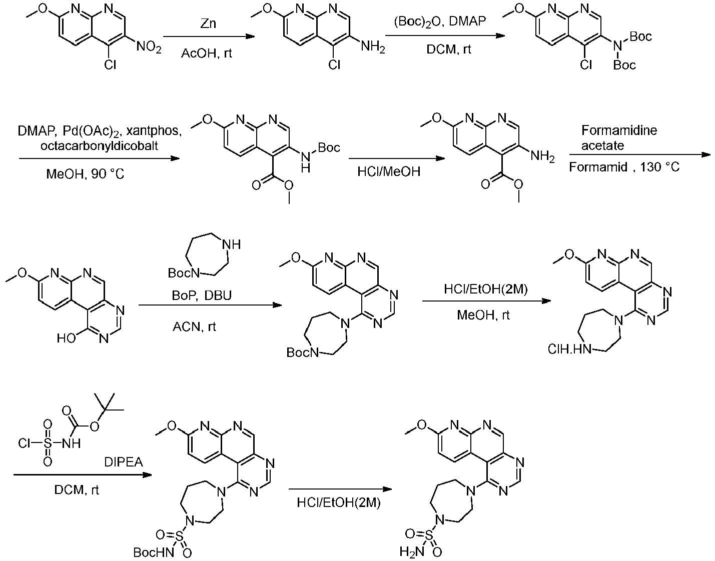

### Step 1: Preparation of 4-chloro-7-methoxy-1,8-naphthyridin-3-amine

To a single-neck flask was added 5-chloro-2-methoxy-6-nitro-1,8-naphthyridine (5.0g, 20.87mmol, 1.0eq.) dissolved in acetic acid (20mL). Zinc powder (2.7g, 41.74mmol, 2.0eq.) was added to the reaction mixture. The mixture was reacted at rt under stirring for 16hrs. After the reaction product was monitored, the reaction mixture was concentrated. The resulting residue was purified by silica gel column chromatography (petroleum ether:EA=1:1) to afford the target compound (400mg, yield 9%).

LCMS (ESI) [M+H]⁺=210.1; ¹H NMR (400MHz, DMSO-d₆) δ 8.57 (s, 1H), 8.24 (d, J=8.8 Hz, 1H), 7.11 (d, J=8.8 Hz, 1H), 4.01 (s, 2H), 3.96 (s, 3H).

### Step 2: Preparation of tert-butyl (tert-butoxycarbonyl)(4-chloro-7-methoxy-1,8-naphthyridin-3-yl)carbamate

In a single-neck round-bottom flask, 4-chloro-7-methoxy-1,8-naphthyridin-3-amine (800mg, 3.82mmol, 1.0eq.) and 4-dimethylaminopyridine (233mg, 1.91mmol, 0.5eq.) were dissolved in dichloromethane (20mL). Then di-tert-butyl dicarbonate (1.7g, 7.63mmol, 2.0eq.) was added. The mixture was reacted at rt under stirring for 4hrs. After the reaction product was monitored, the reaction mixture was concentrated. The resulting residue was purified by silica gel column chromatography (petroleum ether:EA=3:1) to afford the target compound (380mg, yield 23%).

LCMS (ESI) [M+H]⁺=410.2.

### Step 3: Preparation of methyl 3-((tert-butoxycarbonyl)amino)-7-methoxy-1,8-naphthyridin-4-carboxylate

To a single-neck round-bottom flask were added tert-butyl (tert-butoxycarbonyl)(4-chloro-7-methoxy-1,8-naphthyridin-3-yl)carbamate (800mg, 2.02mmol, 1.0eq.) and 4-dimethylaminopyridine (247mg, 2.02mmol, 1.0eq.), palladium acetate (23.0mg, 0.1mmol, 0.05eq.) and methanol (10mL). Then 4,5-bis(diphenylphosphino)-9,9-dimethylxanthene (1169mg, 2.02mmol, 1.0eq.), octacarbonyldicobalt (CAS:10210-68-1) (765mg, 2.22mmol, 1.1eq.) were added. The mixture was reacted at 90°C under refluxing for 5hrs. The completion of the reaction was monitored with LCMS. The reaction mixture was concentrated. The residue was purified by silica gel column chromatography (petroleum ether:EA=7:3) to afford the target compound (400mg, yield 46%).

LCMS (ESI): [M+H]⁺=334.1; ¹H NMR (400MHz, CD₃OD) δ 9.04 (s, 1H), 8.47 (d, J=9.2 Hz, 1H), 7.12 (d, J=9.2 Hz, 1H), 4.09 (s, 3H), 4.01 (s, 3H), 1.53 (s, 9H).

### Step 4: Preparation of methyl 3-amino-7-methoxy-1,8-naphthyridin-4-carboxylate

Methyl 3-((tert-butoxycarbonyl)amino)-7-methoxy-1,8-naphthyridin-4-carboxylate (400mg, 1.2mmol, 1.0eq.) was dissolved in HCl/methanol (4M, 10mL). The reaction system was stirred at rt for 16hrs. The completion of the reaction was monitored with LCMS. The reaction mixture was distilled under reduced pressure to remove the solvent to afford a crude target compound (250mg).

LCMS (ESI): [M+H]⁺=234.1; ¹H NMR (400MHz, DMSO-d₆) δ 8.81-8.66 (m, 2H), 7.16 (d, J=9.2 Hz, 1H), 6.28 (s, 3H), 4.00 (s, 3 H), 3.99 (s, 3 H).

### Step 5: Preparation of 8-methoxypyrimido[4,5-c][1,8]naphthyridin-1-ol

To a round-bottom flask were added methyl 3-amino-7-methoxy-1,8-naphthyridin-4-carboxylate (310mg, crude) and formamidine acetate (831mg, 7.98mmol) and formamide (5mL). The reaction system was stirred at 130°C for 16hrs. The completion of the reaction was monitored with LCMS. The reaction mixture was cooled to rt and then washed with water to precipitate a solid. to precipitate a solid. The mixture was filtered. The filter cake was dried to afford the target compound (200mg, yield 66%).

LCMS (ESI) [M+H]⁺=229.2; ¹H NMR (400MHz, DMSO-d₆) δ 12.71 (s, 1H), 9.84 (d, J=9.2 Hz, 1H), 9.31 (s, 1H), 8.31 (s, 1H), 7.30 (d, J=9.2 Hz, 1H), 4.08 (s, 3H).

### Step 6: Preparation of tert-butyl 4-(8-methoxypyrimido[4,5-c][1,8]naphthyridin-1-yl)-1,4-diazepane-1-carboxylate

To a single-neck round-bottom flask were added 8-methoxypyrimido[4,5-c][1,8]naphthyridin-1-ol (50mg, 0.22mmol, 1.0eq.), and acetonitrile (2mL). Under stirring, a drop of 1,8-diazabicyclo[5.4.0]undec-7-ene (100mg, 0.66mmol, 3.0eq) was added. The reaction system became clear. Then a drop of diphenyl ether was added, and Castro's reagent (290mg, 0.66mmol, 3eq) was added to the reaction system. The mixture was stirred for 10 minutes, and then tert-butyl 1,4-diazepane-1-carboxylate (130mg, 0.66mmol, 3eq) was added to the above stirred solution. The reaction continued under stirring for 16hrs. After the completion of the reaction. The reaction mixture was concentrated to afford a crude product, which was purified by preparative plate chromatography to afford the target compound (20mg, yield 22%).

LCMS (ESI) [M+H]⁺=411.2; ¹HNMR (400MHz, DMSO-d₆) δ 9.35 (s, 1H), 8.71 (s, 1H), 8.57 (t, J=8.0 Hz, 1H), 7.24 (d, J=8.0 Hz, 1H), 4.25-4.22 (m, 1H), 4.06 (s, 3H), 3.75-3.30 (m, 5H), 2.10-2.02 (m, 1H), 1.75-1.52 (m, 1H), 1.37 (s, 4.5H), 1.17 (s, 4.5H), 0.88-0.85 (m, 2H).

### Step 7: Preparation of 1-(1,4-diazepane-1-yl)-8-methoxypyrimido[4,5-c][1,8]naphthyridine hydrochloride

To a single-neck round-bottom flask were added tert-butyl 4-(8-methoxypyrimido[4,5-c][1,8]naphthyridin-1-yl)-1,4-diazepane-1-carboxylate (20mg, 0.05mmol, 1.0eq.) and methanol (2mL). To the resulting solution was added a solution of HCl/ethanol (2mol/L, 1.0mL, 40eq.). The reaction mixture was stirred at rt for 16hrs. After the completion of the reaction. The reaction mixture was concentrated to afford a crude target compound (20mg), which was directly used in the next step reaction.

LCMS (ESI) [M+H]⁺=311.2.

### Step 8: Preparation of tert-butyl ((4-(8-methoxypyrimido[4,5-c][1,8]naphthyridin-1-yl)-1,4-diazepane-1-yl)sulfonyl)carbamate

To a single-neck round-bottom flask were added tert-butyl (chlorosulfonyl)carbamate (40mg, 0.29mmol, 5eq.) and dichloromethane (2mL). Under cooling in an ice bath, tert-butanol (20mg, 0.29mmol, 5eq.) was added. The solution obtained after stirring for 30 minutes was added to another round-bottom single-neck flask under stirring, which flask contained a solution of 1-(1,4-diazepane-1-yl)-8-methoxypyrimido[4,5-c][1,8]naphthyridine hydrochloride (20mg, 0.06mmol, 40% purity, 1.0eq.) and diisopropylethylamine (39mg, 0.3mmol, 5eq) in dichloromethane (2mL). The reaction mixture was stirred at room temperature for 2hrs. After the completion of the reaction. The reaction mixture was concentrated to afford a crude target compound (80mg), which was directly used in the next step reaction.

LCMS (ESI): [M+H]⁺=490.0.

### Step 9: Preparation of 4-(8-methoxypyrimido[4,5-c][1,8]naphthyridin-1-yl)-1,4-diazepane-1-sulfonamide

To a single-neck flask was added the crude product obtained in the above step tert-butyl ((4-(8-methoxypyrimido[4,5-c][1,8]naphthyridin-1-yl)-1,4-diazepane-1-yl)sulfonyl)carbamate (80mg, crude), dichloromethane (1mL) and trifluoroacetic acid (1mL). The reaction mixture was stirred for 1hr. After the completion of the reaction. The reaction mixture was concentrated to afford a crude product, which was directly purified by preparative HPLC to afford the target compound (1.6mg, total yield of three steps: 8%).

LCMS (ESI): [M+H]⁺=390.1; ¹HNMR (400MHz, MeOD) δ 9.36 (s, 1H), 8.75 (s, 1H), 8.67 (d, J=8.0 Hz, 1H), 7.27 (d, J=8.0 Hz, 1H), 4.15-4.10 (m, 4H), 3.95-3.80 (m, 5H), 3.60-3.50 (m, 2H), 2.01-1.98 (m, 2H).

### Example 4

### Preparation of N-((1-(8-methoxypyrimido[4,5-c][1,8]naphthyridin-1-yl)azepan-4-yl)methyl)atninosulfonatnide (compound 4):

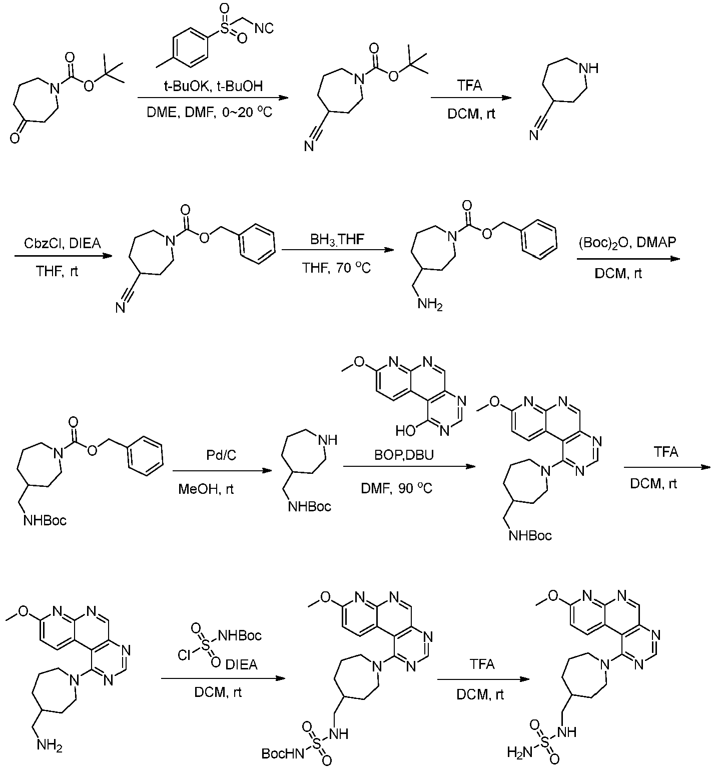

### Step 1: Preparation of tert-butyl 4-cyanoazepan-1-carboxylate

Tert-butyl 4-oxoazepan-1-carboxylate (6g, 28.13mmol, 1eq.) was dissolved in DMF(30mL). Then a solution of 1-((isocyanomethyl)sulfonyl)-4-methylbenzene (6.87g, 35.16mmol, 1.25eq.) and potassium tert-butoxide (6.31g, 56.26mmol, 2eq.) in tert-butanol (30mL) and 1,2-dimethoxyethane (24.05mL) was added at 0°C. The mixture was warmed to 20°C and reacted at 20°C for 3hrs. After TLC monitoring indicated the completion of the reaction, the mixture was washed with water and extracted with EA. The organic phases were combined, dried over anhydrous sodium sulfate, and filtered. The filtrate was concentrated, and purified by column chromatography (petroleum ether:EA=10:1) to afford the target compound (4.2g, yield 66.57%).

LCMS (ESI) [M+Na]⁺=247.1; ¹H NMR (400MHz, DMSO-d₆) δ 3.45-3.20 (m, 4H), 3.15-3.05 (m, 1H), 1.95-1.82 (m, 2H), 1.80-1.65 (m, 4H), 1.40 (s, 9H).

### Step 2: Preparation of azepan-4-carbonitrile

To a single-neck round-bottom flask were added tert-butyl 4-cyanoazepan-1-carboxylate (3.00g, 13.37mmol, 1eq.), trifluoroacetic acid (30mL) and dichloromethane (5mL). The reaction mixture was stirred at room temperature for 16hrs. After the completion of the reaction, the reaction was concentrated to afford a crude target compound (1.5g), which was directly used in the next step reaction.

LCMS (ESI) [M+H]⁺=125.2; ¹H NMR(400MHz, CDCl₃)δ 3.75-3.70 (m, 4H), 3.15-3.05 (m, 1H), 2.36-2.34 (m, 2H), 2.33-2.00 (m, 4H).

### Step 3: Preparation of benzyl 4-cyanoazepan-1-carboxylate

To a single-neck round-bottom flask were added azepan-4-carbonitrile (1500mg, crude), diisopropylethylamine (4.7g, 36.24mmol) and tetrahydrofuran (30mL). Under stirring, benzyl carbonochloridate (2060mg, 12.08mmol) was slowly added dropwise. The mixture was reacted at rt under stirring for 2hrs. After the completion of the reaction, the reaction was concentrated to afford a crude product, which was purified by column chromatography (petroleum ether:EA=10:1) to afford the target compound (2.3g, yield 74%).

LCMS (ESI): [M+Na]⁺=281.1; ¹H NMR (400MHz, CDCl₃) δ 7.39-7.23 (m, 5H), 5.13 (s, 2H), 3.75-3.25 (m, 4H), 2.87-2.82 (m, 1H), 1.99-1.65(m, 6H).

### Step 4: Preparation of benzyl 4-(aminomethyl)azepan-1-carboxylate

To a round-bottom single-neck flask was added a solution of benzyl 4-cyanoazepan-1-carboxylate (2200mg, 8.52mmol, 1eq.) and borane in tetrahydrofuran (1M, 30mL). The reaction mixture was stirred at 70°C for 16hrs. After the completion of the reaction. The reaction mixture was concentrated to afford a crude target compound (2.0g), which was directly used in the next step reaction.

LCMS (ESI): [M+H]⁺=263.1.

### Step 5: Preparation of benzyl 4-(((tert-butoxycarbonyl)amino)methyl)azepan-1-carboxylate

In a single-neck round-bottom flask, benzyl 4-(aminomethyl)azepan-1-carboxylate (2000mg, crude) was dissolved in dichloromethane (10mL), and 4-dimethylaminopyridine (465.68mg, 3.81mmol) was added. Under stirring, di-tert-butyl dicarbonate (3327.62mg, 15.25mmol) was slowly added. The mixture was reacted at rt under stirring for 2hrs. After the completion of the reaction. The reaction mixture was concentrated to afford a crude product, which was purified by column chromatography (petroleum ether:EA=100:20) to afford the target compound (900mg, total yield of two steps: 30%).

LCMS (ESI) [M+Na]⁺=385.2; ¹H NMR (400MHz, CDCl₃) δ 7.28-7.23 (m, 5H), 5.15 (s, 2H), 4.59-4.55 (m, 1H), 3.55-3.05 (m, 4H), 3.05-2.95 (m, 2H), 1.95-1.44 (m, 7H), 1.42 (s, 9H).

### Step 6: Preparation of tert-butyl (azepan-4-ylmethyl)carbamate

To a single-neck round-bottom flask, benzyl 4-(((tert-butoxycarbonyl)amino)methyl)azepan-1-carboxylate (900mg, 2.48mmol, 1eq.) was dissolved in methanol (10mL), and Pd/C(10% content, 50% humidity) and 2 drops of ammonia solution were added. The reaction system was replaced with a hydrogen gas balloon and reacted in an atmosphere of hydrogen gas at 20°C for 16hrs. After the completion of the reaction, the reaction mixture was filtered. The filtrate was concentrated to afford a crude target compound (400mg), which was directly used in the next step reaction. LCMS (ESI) [M+H]⁺=229.2; ¹HNMR (400MHz, DMSO-d₆) δ 6.88-6.83 (m, 1H), 2.70-2.59 (m, 4H), 2.67 (s, 2 H), 2.58-2.52 (m, 1H), 1.66-1.51 (m, 5H), 1.40 (s, 9H), 1.20-1.15 (m, 2H).

### Step 7: Preparation of tert-butyl ((1-(8-methoxypyrimido[4,5-c][1,8]naphthyridin-1-yl)azepan-4-yl)methyl)carbamate

To a single-neck round-bottom flask were added 8-methoxypyrimido[4,5-c][1,8]naphthyridin-1-ol (60mg, 0.026mmol, 1.0eq.) and N,N-dimethylformamide (4mL). Under stirring, a drop of DBU (160mg, 1.05mmol, 4.0eq.) was added. The reaction system became clear. Then benzotriazol-1-yloxytris(dimethylamino)phosphonium hexafluorophosphate (220mg, 0.5mmol, 2eq.) was added to the reaction system. After stirring for 10 minutes, tert-butyl (azepan-4-ylmethyl)carbamate (120mg, crude product, obtained from the above-step reaction) was added to the above-stirred solution. The mixture was reacted at 90°C under stirring for 16hrs. After the completion of the reaction, the reaction mixture was directly purified by reverse-phase preparative HPLC to afford the target compound (20mg, yield 22%).

LCMS (ESI) [M+H]⁺⁼439.3; ¹HNMR (400MHz, DMSO-d₆) δ 9.33 (s, 1H), 8.69 (s, 1H), 8.55-8.53 (m, 1H), 7.23 (d, J=12.0 Hz, 1H), 6.85 (s, 1H), 4.06 (s, 3H), 3.83-3.75 (m, 2H), 3.23-3.05 (m, 2H), 2.83-2.74 (m, 2H), 1.85-1.52 (m, 7H), 1.37 (s, 9H).

### Step 8: Preparation of (1-(8-methoxypyrimido[4,5-c][1,8]naphthyridin-1-yl)azepan-4-yl)methanatnine

To a single-neck round-bottom flask were added tert-butyl ((1-(8-methoxypyrimido[4,5-c][1,8]naphthyridin-1-yl)azepan-4-yl)methyl)carbamate (15mg, 0.05mmol, 1.0eq.) and dichloromethane (1mL). To the solution was added trifluoroacetic acid (2.0mL, 40eq.). The reaction mixture was stirred at rt for 2hrs. The reaction mixture was concentrated to afford a crude target compound (20mg), which was directly used in the next step reaction.

LCMS (ESI) [M+H]⁺=339.1.

### Step 9: Preparation of tert-butyl (N-((1-(8-methoxypyrimido[4,5-c][1,8]naphthyridin-1-yl)azepan-4-yl)methyl)aminosulfonyl)carbamate

To a single-neck round-bottom flask were added tert-butyl (chlorosulfonyl)carbamate (40mg, 0.29mmol, Seq.) and dichloromethane (2mL). Under cooling in an ice bath, tert-butanol (20mg, 0.29mmol) was added. After stirring for 30 minutes, the reaction mixture was added to another flask containing a solution of (1-(8-methoxypyrimido[4,5-c][1,8]naphthyridin-1-yl)azepan-4-yl)methanamine (20mg, crude, obtained from the above step reaction) and diisopropylethylamine (39mg, 0.3mmol) in dichloromethane (2mL). The reaction mixture was stirred at room temperature for 3hrs. After the completion of the reaction. The reaction mixture was concentrated to afford a crude target compound (20mg), which was directly used in the next step reaction.

LCMS (ESI) [M+H]⁺=518.3.

### Step 10: Preparation of N-((1-(8-methoxypyrimido[4,5-c][1,8]naphthyridin-1-yl)azepan-4-yl)methyl)aminosulfonamide

To a single-neck round-bottom flask were added tert-butyl (N-((1-(8-methoxypyrimido[4,5-c][1,8]naphthyridin-1-yl)azepan-4-yl)methyl)aminosulfonyl)carbamate (20 mg, the crude product obtained in the above step) dissolved in dichloromethane (2mL), and then trifluoroacetic acid (2mL). The reaction mixture was stirred at room temperature for 3hrs. After the completion of the reaction, the reaction was concentrated to afford a crude product, which was purified by preparative HPLC to afford the target compound (4.3mg, total yield of three steps: 25%).

LCMS (ESI) [M+H]⁺=418.3; ¹HNMR (400MHz, MeOD) δ 9.32 (s, 1H), 8.72 (s, 1H), 8.44-8.41(m, 1H), 7.30 (d, J=12 Hz, 1H), 4.30-3.95 (m, 5H), 3.78-3.70 (m, 2H), 2.90-2.81 (m, 2H), 2.01-1.88 (m, 4H), 1.55-1.23 (m, 2H), 0.99-0.95 (m, 1H).

### Example 5

### Preparation of 3,5-difluoro-4-((8-methoxy-2,3-dihydro-1H-[1,4]oxazino[2,3-c][1,8]naphthyridin-1-yl)methyl)benzenesulfonamide (compound 5):

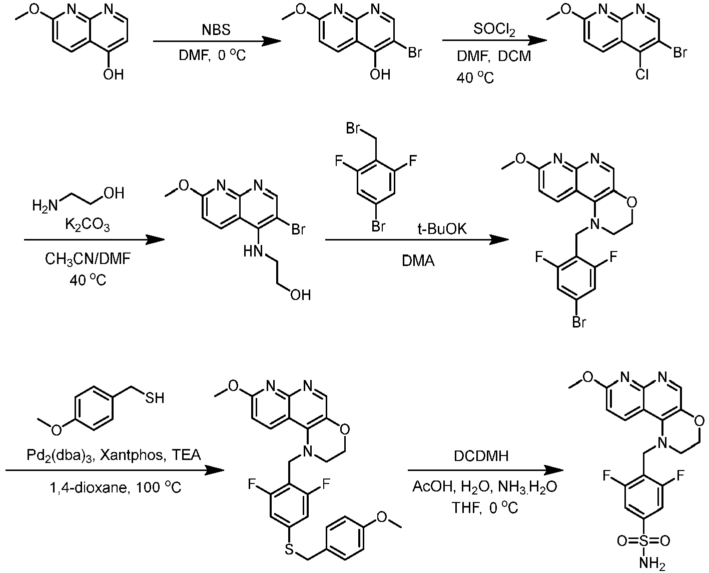

### Step 1: Preparation of 3-bromo-7-methoxy-1,8-naphthyridin-4-ol

7-methoxy-1,8-naphthyridin-4-ol (12g, 68.12mmol, 1eq.) was dissolved in N,N-dimethylformamide (50mL). Then N-bromosuccinimide (7.3g, 40.87mmol, 0.6eq.) was added at 0°C. The mixture was reacted at 0°C for 2hrs. The completion of the reaction was monitored with LCMS. The reaction mixture was washed with water, and extracted with EA (30mL×2). The organic phases were concentrated, and then purified by flash silica gel column chromatography (Silica gel, PE:EA=50:50) to afford the target compound (2.0g, yield 11.5%).

LCMS (ESI) [M+H]⁺=254.9.

### Step 2: Preparation of 6-bromo-5-chloro-2-methoxy-1,8-naphthyridine

3-bromo-7-methoxy-1,8-naphthyridin-4-ol (500mg, 1.96mmol, 1eq.) was dissolved in dichloromethane (50mL). A drop of N,N-dimethylformamide was added. Sulfurous dichloride (2332mg, 19.6mmol, 10eq.) was added. Then the mixture was reacted at 40°C for 16hrs. The completion of the reaction was monitored with LCMS. The reaction mixture was slowly added dropwise to ice-water, and divided into layers. The aqueous phase was extracted with dichloromethane (20mL×3). The organic phases were combined, dried over anhydrous sodium sulfate, and concentrated to afford the target compound (520mg, yield 97%).

LCMS (ESI) [M+H]⁺=272.9.

### Step 3: Preparation of 2-((3-bromo-7-methoxy-1,8-naphthyridin-4-yl)amino)ethyl-1-ol

6-bromo-5-chloro-2-methoxy-1,8-naphthyridine (500mg, 1.83mmol, 1eq.) was dissolved in N-methylpyrrolidinone (15mL). Ethanol amine (2.2g, 36.6mmol, 20eq.) was added. The mixture was reacted at 100°C for 2hrs. The completion of the reaction was monitored with LCMS. The reaction mixture was poured into water, and extracted with EA (20mL×3). The organic phases were combined, dried over anhydrous sodium sulfate, and filtered. The filtrate was concentrated to afford the target compound (260mg, yield 57%).

LCMS (ESI) [M+H]⁺=298.0; ¹H NMR (400MHz, DMSO-d₆) δ 8.61 (d, J=9.2 Hz, 1H), 8.56 (s, 1H), 6.97 (d, J=9.2 Hz, 1H), 6.22 (t, J=5.6 Hz, 1H), 4.97 (t, J=5.2 Hz, 1H), 3.97 (s, 3H), 3.76-3.67 (m, 2H), 3.65-3.57 (m, 2H).

### Step 4: Preparation of 1-(4-bromo-2,6-difluorobenzyl)-8-methoxy-2,3-dihydro-1H-[1,4]oxazino[2,3-c][1,8]naphthyridine

2-((3-bromo-7-methoxy-1,8-naphthyridin-4-yl)atnino)ethyl-1-ol (200mg, 0.67mmol, 1eq.) was dissolved in N,N-dimethylpyrrolidinone (5mL). Cuprous iodide (128mg, 0.67mmol, 1.0eq.), trans-1,2-cyclohexanediamine (77mg, 0.67mmol, 1.0eq.) and potassium tert-butoxide (226mg, 2.01mmol, 3eq.) were added. The mixture was reacted in the microwave at 140°C for 2hrs. The completion of the reaction was monitored with LCMS. 5-bromo-2-(bromomethyl)-1,3-difluorobenzene (284mg, 1.01mmol, 1.5eq.) was added to the reaction mixture. The mixture was reacted at 70°C for 1hr. The completion of the reaction was monitored with LCMS. The reaction mixture was poured into water, and extracted with EA. The organic phase was dried over anhydrous sodium sulfate, and filtered. The filtrate was concentrated, and purified by silica gel column to afford the target compound (100mg, yield 36%).

LCMS (ESI) [M+H]⁺=422.0; ¹H NMR (400MHz, DMSO-d₆) δ 8.91-8.82 (m, 1H), 8.75 (s, 1H), 7.54 (d, J=8.0 Hz, 2H), 7.22 (d, J=9.2 Hz, 1H), 5.78 (s, 2H), 4.32 (t, J=4.4 Hz, 2H), 3.93 (s, 3H), 3.74 (t, J=4.0 Hz, 2H).

### Step 5: Preparation of 1-(2,6-difluoro-4-((4-methoxybenzyl)thio)benzyl)-8-methoxy-2,3-dihydro-1H-[1,4]oxazino[2,3-c][1,8]naphthyridine

1-(4-bromo-2,6-difluorobenzyl)-8-methoxy-2,3-dihydro-1H-[1,4]oxazino[2,3-c][1,8]naphthyridine (100mg, 0.24mmol, 1eq.) was dissolved in dioxane (10mL). (4-methoxyphenyl)methanethiol (73mg, 0.47mmol, 2eq.), (5-(diphenylphosphino)-9,9-dimethyl-9H-xanthene-4-yl)diphenylphosphine (27mg, 0.05mmol, 0.2eq.), tris(1,5-diphenylpent-1,4-diene-3-one)dipalladium (43mg, 0.05mmol, 0.2eq.) and triethylamine (48mg, 47mmol, 2eq.) were added. The mixture was reacted at 100°C and under the protection of nitrogen gas for 2hrs. The completion of the reaction was monitored with LCMS. The reaction mixture was concentrated to dryness, and purified by silica gel column chromatography (Silica gel, DCM:MeOH=95:5) to afford the target compound (70mg, yield 60%).

LCMS (ESI) [M+H]⁺=496.1.

### Step 6: Preparation of 3,5-difluoro-4-((8-methoxy-2,3-dihydro-1H-[1,4]oxazino[2,3-c][1,8]naphthyridin-1-yl)methyl)benzenesulfonamide

1-(2,6-difluoro-4-((4-methoxybenzyl)thio)benzyl)-8-methoxy-2,3-dihydro-1H-[1,4]oxazino[2,3-c][1,8]naphthyridine (60mg, 0.12mmol, 1.0eq.), acetic acid (51mg, 0.85mmol, 7.0eq.), water (31mg, 1.7mmol, 14.0eq.) were dissolved in tetrahydrofuran (15mL). 1,3-dichloro-5,5-dimethylhydantoin (72mg, 0.36mmol, 3eq.) was added at 0°C. The mixture was reacted at 0°C for 2hrs, followed by adding ammonia solution (0.5mL), and then reacted at 0°C for 10 minutes. The completion of the reaction was monitored with LCMS. The reaction mixture was concentrated, and purified by preparative HPLC to afford the target compound (13.7mg, yield 27%).

LCMS (ESI) [M+H]⁺=423.1; ¹HNMR (400MHz, MeOD-d₄) δ 8.53 (s, 1H), 8.48 (d, J=9.2 Hz, 1H), 7.53 (d, J=7.2 Hz, 2H), 7.11 (d, J=8.8 Hz, 1H), 5.92 (s, 2H), 4.36 (t, J=4.4 Hz, 2H), 4.01 (s, 3H), 3.81 (t, J=4.4 Hz, 2H).

### Example 6

### Preparation of 3,5-difluoro-4-((8-methoxy-2-oxo-2H-[1,3]oxazino[5,4-c][1,8]naphthyridin-1(4H)-yl)methyl)benzenesulfonamide (compound 6):

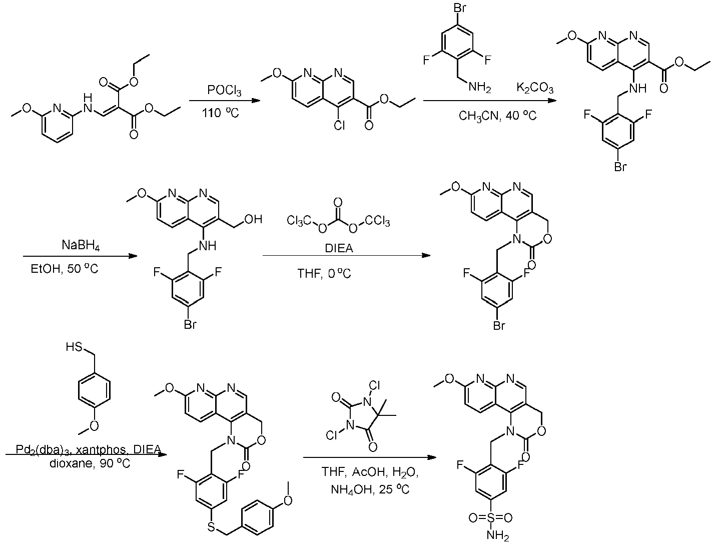

### Step 1: Preparation of ethyl 4-chloro-7-methoxy-1,8-naphthyridine-3-carboxylate

1,3-diethyl 2-(((6-methoxypyridin-2-yl)amino)methylene)malonate (12g, 22.7mmol, 1eq.) was dissolved in phosphorus oxychloride (60mL). The mixture was reacted under the protection of nitrogen gas at 110°C under stirring for 4hrs. The completion of the reaction was monitored with LCMS. The reaction mixture was distilled under reduced pressure to remove the solvent. The resulting residue was dissolved in EA (60mL) to become clear and quickly washed with water (20mL). The organic phase was dried over anhydrous sodium sulfate, and filtered. The filtrate was rotary-evaporated to dryness. The resulting crude product was purified by flash chromatography (Silica gel, DCM:EA=1:1) to afford the target compound (1.5g, yield 12%).

LCMS (ESI) [M+H]⁺=266.0.

### Step 2: Preparation of ethyl 4-((4-bromo-2,6-difluorobenzyl)amino)-7-methoxy-1,8-naphthyridine-3-carboxylate

Ethyl 4-chloro-7-methoxy-1,8-naphthyridine-3-carboxylate (3.0g, 18.75mmol, 1eq.) was dissolved in acetonitrile (80mL). Then K₂CO₃ (5.2g, 37.5mmol, 2.0eq.), and (4-bromo-2,6-difluorophenyl)methanamine (4.37g, 19.69mmol, 1.05eq.) *were added.* The reaction was performed under the protection of nitrogen gas at 40°C for 20hrs. The completion of the reaction was monitored with LCMS. The reaction mixture was concentrated, diluted with EA, and washed with water. The organic phase was dried over anhydrous sodium sulfate, and filtered. The filtrate was rotary-evaporated to dryness. The resulting crude product was purified by flash chromatography (Silica gel, DCM:EA=4:1) to afford the target compound (3.5g, yield 69.2%).

LCMS (ESI) [M+H]⁺=452.0.

### Step 3: Preparation of (4-((4-bromo-2,6-difluorobenzyl)amino)-7-methoxy-1,8-naphthyridin-3-yl)methanol

Ethyl 4-((4-bromo-2,6-difluorobenzyl)amino)-7-methoxy-1,8-naphthyridine-3-carboxylate (120mg, 22.7mmol, 1eq.) was dissolved in ethanol (10mL). Sodium borohydride (134mg, 5eq.) *was* slowly added. The mixture was reacted under the protection of nitrogen gas at 50°C under stirring for 16hrs. The completion of the reaction was monitored with LCMS. The mixture was rotary-evaporated to dryness to remove the solvent. The resulting residue was quenched with saturated NaCl solution (5mL), and extracted with EA (10mL). The organic phase was dried over anhydrous sodium sulfate, and filtered. The filtrate was rotary-evaporated to dryness. The resulting crude product was purified by flash chromatography (Silica gel, DCM:MeOH=2:1) to afford the target compound (120mg, yield 38%).

LCMS (ESI) [M+H]⁺⁼410.0; ¹H NMR (400MHz, CDCl₃) δ 8.43 (s, 1H), 8.35 (d, J=9.2 Hz, 1H), 7.09 (d, J=6.8 Hz, 2H), 6.90 (d, J=9.2 Hz, 1H), 4.75 (s, 2H), 4.69 (s, 2H), 4.12 (s, 3H).

### Step 4: Preparation of 1-(4-bromo-2,6-difluorobenzyl)-8-methoxy-1,4-dihydro-2H-[1,3]oxazino[5,4-c][1,8]naphthyridin-2-one

(4-((4-bromo-2,6-difluorobenzyl)amino)-7-methoxy-1,8-naphthyridin-3-yl)methanol (300mg, 0.73mmol, 1eq.) was dissolved in dichloromethane (15mL). N,N-diisopropylethylamine (473mg, 3.66mmol, 5eq.) and triphosgene (651mg, 2.19mmol, 3eq.) were added. The mixture was reacted at 0°C for 2hrs. The completion of the reaction was monitored with LCMS. The reaction mixture was washed with water, dried over anhydrous sodium sulfate, and filtered. The filtrate was rotary-evaporated to dryness. The resulting crude product was purified by flash silica gel column chromatography (Silica gel, MeOH:DCM=5:95) to afford the target compound (250mg, yield 78.51%).

LCMS (ESI) [M+H]⁺=436.0.

### Step 5: Preparation of 1-(2,6-difluoro-4-((4-methoxybenzyl)thio)benzyl)-8-methoxy-1,4-dihydro-2H-[1,3]oxazino[5,4-c] [1,8]naphthyridin-2-one

1-(4-bromo-2,6-difluorobenzyl)-8-methoxy-1,4-dihydro-2H-[1,3]oxazino[5,4-c][1,8]naphthyridin-2-one (200mg, 0.46mmol, 1eq.) was dissolved in dioxane (10mL). (4-methoxyphenyl)methanethiol (141mg, 0.92mmol, 2eq.), [5-(diphenylphosphino)-9,9-dimethyl-9H-xanthene-4-yl]diphenylphosphine(53mg, 0.09mmol, 0.2eq.), tris(1,5-diphenylpent-1,4-diene-3-one)dipalladium(84mg, 0.09mmol, 0.2eq.) and N,N-diisopropylethylamine (178mg, 1.38mmol, 3eq.) *were* added. The mixture was reacted at 90°C for 2hrs. The completion of the reaction was monitored with LCMS. The reaction mixture was concentrated, rotary-evaporated to dryness, and purified by flash silica gel column chromatography (Silica gel, MeOH:DCM=5:95) to afford the target compound (110mg, yield 47.09%) as yellow solid.

LCMS (ESI) [M+H]⁺=510.1.

### Step 6: Preparation of 3,5-difluoro-4-((8-methoxy-2-oxo-2H-[1,3]oxazino[5,4-c][1,8]naphthyridin-1(4H)-yl)methyl)benzenesulfonamide

1-(2,6-difluoro-4-((4-methoxybenzyl)thio)benzyl)-8-methoxy-1,4-dihydro-2H-[1,3]oxazino[5,4-c][1,8]naphthyridin-2-one (100mg, 0.2mmol, 1.0eq.), acetic acid (83mg, 1.37mmol, 7.0eq.), water (50mg, 2.75mmol, 14.0eq.) were dissolved in tetrahydrofuran (15mL). 1,3-dichloro-5,5-dimethylhydantoin (116mg, 0.59mmol, 3eq.) was added at 0°C. The mixture was reacted at 25°C for 1hr, followed by adding ammonia solution (0.5mL), then reacted at 25°C for 10 minutes. The completion of the reaction was monitored with LCMS. The reaction mixture was concentrated, and purified by preparative HPLC to afford the target compound (14.6mg, yield 17.05%).

LCMS (ESI) [M+H]⁺=437.1; ¹HNMR (400MHz, MeOD-d₄) δ 8.71 (s, 1H), 8.59 (d, J=9.2 Hz, 1H), 7.46 (d, J=7.6 Hz, 2H), 7.13 (d, J=9.6 Hz, 1H), 5.57 (s, 2H), 5.30 (s, 2H), 4.12 (s, 3H).

### Example 7

### Preparation of 3,5-difluoro-4-((9-fluoro-8-methoxy-2-oxo-3,4-dihydropyrimido[5,4-c]quinolin-1(2H)-yl)methyl)benzenesulfonamide (compound 7):

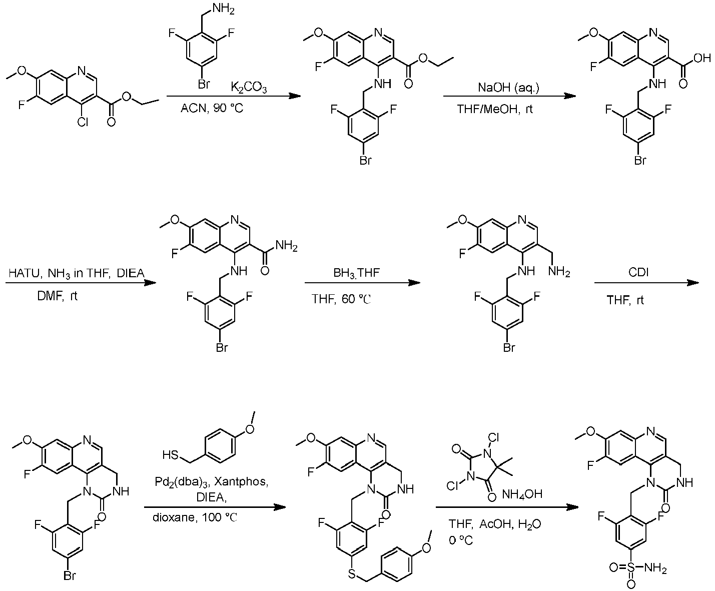

### Step 1: Preparation of ethyl 4-((4-bromo-2,6-difluorobenzyl)amino)-6-fluoro-7-methoxyquinoline-3-carboxylate

Ethyl 4-chloro-6-fluoro-7-methoxyquinoline-3-carboxylate (1g, 3.6mmol, 1eq.), (4-bromo-2,6-difluorophenyl)methanamine (1.2g, 5.4mmol, 1.5eq.) and K₂CO₃ (1.47g, 10.8mmol, 3eq.) were dissolved in acetonitrile (50mL). The mixture was heated to 90°C and reacted for 12hrs. After the completion of the reaction, the reaction mixture was purified by flash silica gel column chromatography (Silica gel, DCM:MEOH=95:5) to afford the target compound (900mg, yield 54.41%).

LCMS (ESI) [M+H]⁺=469.0; ¹H NMR (400MHz, DMSO-d₆) δ 8.82 (s, 1H), 8.24 (t, J=5.2 Hz, 1H), 8.19 (d, J=13.4 Hz, 1H), 7.49 (dd, J=8.0 Hz, 3H), 4.73 (d, J=5.2 Hz, 2H), 4.28 (d, J=7.2 Hz, 2H), 4.00 (s, 3H), 1.30 (t, J=7.2 Hz, 3H).

### Step 2: Preparation of 4-((4-bromo-2,6-difluorobenzyl)amino)-6-fluoro-7-methoxyquinoline-3-carboxylic acid

Ethyl 4-((4-bromo-2,6-difluorobenzyl)amino)-6-fluoro-7-methoxyquinoline-3-carboxylate (500mg, 1.07mmol, 1eq.) was added to methanol (5mL) and tetrahydrofuran (5mL). An aqueous NaOH solution (2M, 3mL) was added. The mixture was stirred at room temperature for 2hrs. After the completion of the reaction, the reaction mixture was distilled under reduced pressure to remove the solvent. Water (20 mL) was added, and the mixture was adjusted to acidity. A solid precipitated and the mixture was filtered. The filter cake was washed with petroleum ether, and the resulting solid was dried in vacuum to afford the target compound (400mg, yield 75.27%).

LCMS (ESI) [M+H]⁺=440.9.

### Step 3: Preparation of 4-((4-bromo-2,6-difluorobenzyl)amino)-6-fluoro-7-methoxyquinolin-3-formamide

4-((4-bromo-2,6-difluorobenzyl)amino)-6-fluoro-7-methoxyquinoline-3-carboxylic acid (380mg, 0.76mmol, 1eq.) and O-(7-azabenzotriazol-1-yl)-N,N,N',N'-tetramethyluronium hexafluorophosphate (2.8g, 7.6mmol, 10eq.) and ammonia/tetrahydrofuran (19mL, 7.6mmol, 10eq.) were added to N,N-dimethylformamide (10mL). N,N-diisopropylethylamine (98.0mg, 0.76mmol, 1eq.) was added dropwise. The mixture was reacted at rt for 40hrs. Water (50mL) was added. The reaction mixture was extracted with EA (100mL×2). The organic phases were washed with brine five times, dried over anhydrous sodium sulfate, and filtered. The filtrate was distilled under reduced pressure to remove the solvent. The residue was purified by flash silica gel column chromatography (Silica gel, DCM:MeOH=70:30) to afford the target compound (250mg, yield 65.94%).

LCMS (ESI) [M+H]⁺=440.0.

### Step 4: Preparation of 3-(aminomethyl)-N-(4-bromo-2,6-difluorobenzyl)-6-fluoro-7-methoxyquinolin-4-amine

4-((4-bromo-2,6-difluorobenzyl)amino)-6-fluoro-7-methoxyquinolin-3-formamide (380mg, 0.86mmol, 1eq.) was added to tetrahydrofuran (20mL). A solution of borane in tetrahydrofuran (1M, 8.6mL, 8.6mmol, 10eq.) was added. The mixture was reacted at 60°C for 15hrs. After the completion of the reaction, a methanol solution (10mL) was added. The mixture was rotary-evaporated to dryness to remove the solvent, and purified by flash silica gel column chromatography (Silica gel, DCM:MeOH=65:35) to afford the target compound (160mg, yield 43.5%).

LCMS (ESI) [M+H]⁺=426.1.

### Step 5: Preparation of 1-(4-bromo-2,6-difluorobenzyl)-9-fluoro-8-methoxy-3,4-dihydropyrimido[5,4-c]quinolin-2(1H)-one

3-(aminomethyl)-N-(4-bromo-2,6-difluorobenzyl)-6-fluoro-7-methoxyquinolin-4-amine (160mg, 0.38mmol, 1eq.) was added to tetrahydrofuran (10mL). N,N'-carbonyldiimidazole (123.1mg, 0.76mmol, 2eq.) was added. The mixture was reacted at rt for 15hrs. The completion of the reaction was monitored with LCMS. The reaction mixture was concentrated, and purified by flash silica gel column chromatography (Silica gel, DCM:MeOH=70:30) was purified by to afford the target compound (70mg, yield 41.24%).

LCMS (ESI) [M+H]⁺=452.1.

### Step 6: Preparation of 1-(2,6-difluoro-4-((4-methoxybenzyl)thio)benzyl)-9-fluoro-8-methoxy-3,4-dihydropyrimido[5,4-c]quinolin-2(1H)-one

1-(4-bromo-2,6-difluorobenzyl)-9-fluoro-8-methoxy-3,4-dihydropyrimido[5,4-c]quinolin-2(1H)-one (45mg, 0.1mmol, 1eq.) and (4-methoxyphenyl)methanethiol (18.5mg, 0.12mmol, 1.2eq.) were added to dioxane (10mL). Tris(dibenzylideneacetone)dipalladium (5mg, 0.03mmol, 0.3eq.), 4,5-bis(diphenylphosphino)-9,9-dimethylxanthene (39mg, 0.07mmol, 0.2eq.), N,N-diisopropylethylamine (25.8mg, 0.2mmol, 2eq.) were added. Under the protection of nitrogen gas, the mixture was reacted at 100°C for 1hr. After the completion of the reaction, the reaction mixture was purified by flash silica gel column chromatography (Silica gel, DCM:MeOH=95:5) to afford the target compound (20mg, yield 38.06%).

LCMS (ESI) [M+H]⁺=526.1.

### Step 7: Preparation of 3,5-difluoro-4-((9-fluoro-8-methoxy-2-oxo-3,4-dihydropyrimido[5,4-c]quinolin-1(2H)-yl)methyl)benzenesulfonamide

1-(2,6-difluoro-4-((4-methoxybenzyl)thio)benzyl)-9-fluoro-8-methoxy-3,4-dihydropyrimido[5,4-c]quinolin-2(1H)-one (18mg, 0.03mmol, 1eq.) was added to tetrahydrofuran (2mL). At 0°C, a drop of acetic acid, a drop of water and 1,3-dichloro-5,5-dimethylhydantoin (20.2mg, 0.12mmol, 3eq.) were added. The mixture was reacted at 0°C for 2hrs. After the completion of the reaction, ammonia solution (2mL) was added dropwise at 0°C. LCMS monitoring indicated the completion of the reaction, the mixture was distilled under reduced pressure to remove the solvent and purified to afford the target compound (1.6mg, yield 11.12%).

LCMS (ESI) [M+H]⁺=453.0; ¹H NMR (400MHz, CD₃OD) δ 8.73 (s, 1H), 8.14 (d, J=12.6 Hz, 1H), 7.71 (d, J=8.4 Hz, 1H), 7.62 (d, J=7.6 Hz, 2H), 5.72 (s, 2H), 4.60 (s, 2H), 4.26 (s, 3H).

### Example 8

### Preparation of 3,5-difluoro-4-((8-methoxy-2-oxopyrido[2,3-h][1,6]naphthyridine-1(2H)-yl)methyl)benzenesulfonamide (compound 8):

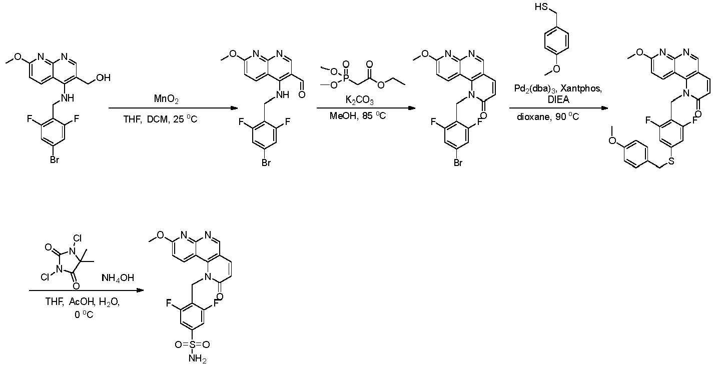

### Step 1: Preparation of 4-((4-bromo-2,6-difluorobenzyl)atnino)-7-methoxy-1,8-naphthyridine-3-carbaldehyde

(4-((4-bromo-2,6-difluorobenzyl)amino)-7-methoxy-1,8-naphthyridin-3-yl)methanol (300mg, 0.73mmol, 1eq.) were dissolved in dichloromethane (10mL) and tetrahydrofuran (10mL). MnO₂ (317mg, 3.65mmol, 5eq.) was added. The mixture was reacted at 25°C for 2hrs. The completion of the reaction was monitored with LCMS. The reaction mixture was washed with water, dried over anhydrous sodium sulfate, and filtered. The filtrate was concentrated and purified by flash silica gel column chromatography (Silica gel, MeOH:DCM=5:95) to afford the target compound (160mg, yield 53.42%).

LCMS (ESI) [M+H]⁺=408.0; ¹H NMR (400MHz, CDCl₃) *δ* 10.10 (s, 1H), 9.77 (s, 1H), 8.68 (s, 1H), 8.44 (d, J=9.2 Hz, 1H), 7.20 (d, J=7.2 Hz, 2H), 6.84 (d, J=8.8 Hz, 1H), 4.92 (d, J=4.8 Hz, 2H), 4.10 (s, 3H).

### Step 2: Preparation of 1-(4-bromo-2,6-difluorobenzyl)-8-methoxypyrido[2,3-h][1,6]naphthyridine-2(1H)-one

4-((4-bromo-2,6-difluorobenzyl)amino)-7-methoxy-1,8-naphthyridine-3-carbaldehyde (150mg, 0.37mmol, 1eq.) was dissolved in methanol (15mL). Ethyl 2-(dimethoxyphosphoryl)acetate (247mg, 1.1mmol, 3eq.) and K₂CO₃ (254mg, 1.84mmol, 5eq.) were added. The mixture was reacted at 85°C for 8hrs. The completion of the reaction was monitored with LCMS. The reaction mixture was concentrated, and purified by flash silica gel column chromatography (Silica gel, MeOH:DCM=2:98) to afford the target compound (110mg, yield 69.26%).

LCMS (ESI) [M+H]⁺=432.0; ¹H NMR (400MHz, DMSO-*d*₆) *δ* 9.15 (s, 1H), 8.70 (d, J=9.2 Hz, 1H), 8.19 (d, *J*=9.2 Hz, 1H), 7.42 (d, *J*=8.0 Hz, 2H), 7.11 (d, J=9.2 Hz, 1H), 6.71 (d, J=9.2 Hz, 1H), 5.69 (s, 2H), 4.04 (s, 3H).

### Step 3: Preparation of 1-(2,6-difluoro-4-((4-methoxybenzyl)thio)benzyl)-8-methoxypyrido[2,3-h] [1,6]naphthyridine-2(1H)-one

1-(4-bromo-2,6-difluorobenzyl)-8-methoxypyrido[2,3-h][1,6]naphthyridine-2(1H)-one (145mg, 0.34mmol, 1eq.) was dissolved in dioxane (10mL). (4-methoxyphenyl)methanethiol (103mg, 0.67mmol, 2eq.), 4,5-bis(diphenylphosphino)-9,9-dimethylxanthene (40mg, 0.07mmol, 0.2eq.), tris(1,5-diphenylpent-1,4-diene-3-one)dipalladium(61mg, 0.07mmol, 0.2eq.) and N,N-diisopropylethylamine (130mg, 1.01mmol, 3eq.) were added. The mixture was reacted at 90°C for 2hrs. The completion of the reaction was monitored with LCMS. The reaction mixture was concentrated, and purified by flash silica gel column chromatography (Silica gel, MeOH:DCM=5:95) to afford the target compound (110mg, yield 64.86%).

LCMS (ESI) [M+H]⁺=506.1.

### Step 4: Preparation of 3,5-difluoro-4-((8-methoxy-2-oxopyrido[2,3-h][1,6]naphthyridine-1(2H)-yl)methyl)benzenesulfonamide

1-(2,6-difluoro-4-((4-methoxybenzyl)thio)benzyl)-8-methoxypyrido[2,3-h][1,6]naphthyridine-2(1H)-one (110mg, 0.22mmol, 1.0eq.), acetic acid (91mg, 1.52mmol, 7.0eq.), water (55mg, 3.05mmol, 14.0eq.) were dissolved in tetrahydrofuran (15mL). 1,3-dichloro-5,5-dimethylhydantoin (129mg, 0.65mmol, 3eq.) was added at 0°C. The mixture was reacted at 0°C for 1hr, followed by adding ammonia solution (0.5mL), then reacted at 0°C for 10 minutes. The completion of the reaction was monitored with LCMS. The reaction mixture was concentrated, and purified by preparative HPLC to afford the target compound (40.9mg, yield 43.47%).

LCMS (ESI) [M+H]⁺=433.0; ¹H NMR (400MHz, DMSO-*d*₆) *δ* 9.16 (s, 1H), 8.72 (d, *J*=9.2 Hz, 1H), 8.21 (d, *J*=9.6 Hz, 1H), 7.62 (s, 2H), 7.43 (d, *J*=7.6 Hz, 2H), 7.12 (d, *J*=9.2 Hz, 1H), 6.70 (d, *J*=9.2 Hz, 1H), 5.78 (s, 2H), 4.05 (s, 3H).

### Example 9

### Preparation of 3,5-difluoro-4-((8-methoxy-3-oxo-4-(2,2,2-trifluoroethyl)-3,4-dihydropyrazino[2,3-c][1,8]naphthyridine-1(2H)-yl)methyl)benzenesulfonamide (compound 11)

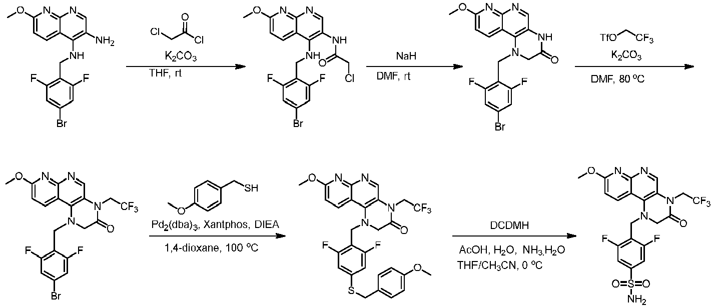

### Step 1: Preparation of N-(4-((4-bromo-2,6-difluorobenzyl)amino)-7-methoxy-1,8-naphthyridin-3-yl)-2-chloroacetamide

N⁴-(4-bromo-2,6-difluorobenzyl)-7-methoxy-1,8-naphthyridine-3,4-diamine (120mg, 0.3mmol, 1.0eq.), 2-chloroacetyl chloride (38mg, 0.34mmol, 1.1eq.), and K₂CO₃ (47mg, 0.34mmol, 1.1eq.) were dissolved in tetrahydrofuran (10mL). The mixture was reacted at normal temperature under stirring for 20 minutes. After the reaction product was monitored, the reaction mixture was concentrated to afford a crude product, which was purified by flash silica gel column chromatography (Silica gel, DCM:MeOH=20:1) to afford the target compound (68mg, yield 48%).

LCMS (ESI) [M+H]⁺=471.0.

### Step 2: Preparation of 1-(4-bromo-2,6-difluorobenzyl)-8-methoxy-1,4-dihydropyrazino[2,3-c][1,8]naphthyridine-3(2H)-one

N-(4-((4-bromo-2,6-difluorobenzyl)atnino)-7-methoxy-1,8-naphthyridin-3-yl)-2-chloroacetamide (48mg, 0.15mmol, 1.0eq.), sodium hydride (4mg, 0.17mmol, 1.1eq.) were dissolved in N,N-dimethylformamide (10 mL). The mixture was reacted at rt under stirring for 20 minutes. After the reaction product was monitored, the reaction mixture was concentrated to afford a crude product, which was purified by flash silica gel column chromatography (Silica gel, DCM:MeOH=20:1) to afford the target product (50mg, yield 77%).

LCMS (ESI) [M+H]⁺=435.0.

### Step 3: Preparation of 1-(4-bromo-2,6-difluorobenzyl)-8-methoxy-4-(2,2,2-trifluoroethyl)-1,4-dihydropyrazino[2,3-c][1,8]naphthyridine-3 (2H)-one

1-(4-bromo-2,6-difluorobenzyl)-8-methoxy-1,4-dihydropyrazino[2,3-c][1,8]naphthyridine-3(2H)-one (50mg, 0.12mmol, 1eq.) and K₂CO₃ (49mg, 0.35mmol, 3eq.) was dissolved in N,N-dimethylformamide (10mL). Then 2,2,2-trifluoroethyl trifluoromethanesulfonate (27mg, 0.12mmol, 1eq.) was added. The mixture was reacted at 80°C for 2hrs. The completion of the reaction was monitored with LCMS. The reaction mixture was diluted with water, and extracted with EA (20mL×3). The organic phases were combined, dried over anhydrous sodium sulfate, and filtered. The filtrate was concentrated, and purified by column chromatography (Silica gel, PE:EA=2:1) to afford the target compound (32mg, yield 53.93%).

LCMS (ESI) [M+H]⁺=517.0.

### Step 4: Preparation of 1-(2,6-difluoro-4-((4-methoxybenzyl)thio)benzyl)-8-methoxy-4-(2,2,2-trifluoroethyl)-1,4-dihydropyrazino[2,3-c][1,8]naphthyridine-3(2H)-one

1-(4-bromo-2,6-difluorobenzyl)-8-methoxy-4-(2,2,2-trifluoroethyl)-1,4-dihydropyrazino[2,3-c] [1,8]naphthyridine-3(2H)-one (32mg, 0.06mmol, 1eq.) was dissolved in dioxane (10mL). Then (4-methoxyphenyl)methanethiol (19mg, 0.12mmol, 2eq.), tris(dibenzylideneacetone)dipalladium (10mg, O.Olmmol, 0.17eq.), 4,5-bis(diphenylphosphino)-9,9-dimethylxanthene (10.4mg, 0.02mmol, 0.3eq.) and diisopropylethylamine (23mg, 0.18mmol, 3eq.) were successively added. Under the protection of nitrogen gas, the mixture was reacted at 100°C for 2hrs. The completion of the reaction was monitored with LCMS. Then the reaction mixture was washed with water and extracted with EA (15mL×3). The organic phases were combined, dried over anhydrous sodium sulfate, and filtered. The filtrate was concentrated, and purified by column chromatography (Silica gel, PE:EA=2:1) to afford the target compound (10mg, yield 28.25%).

LCMS (ESI) [M+H]⁺=591.1.

### Step 5: Preparation of 3,5-difluoro-4-((8-methoxy-3-oxo-4-(2,2,2-trifluoroethyl)-3,4-dihydropyrazino[2,3-c][1,8]naphthyridine-1(2H)-yl)methyl)benzenesulfonamide

1-(2,6-difluoro-4-((4-methoxybenzyl)thio)benzyl)-8-methoxy-4-(2,2,2-trifluoroethyl)-1,4-dihydropyrazino[2,3-c][1,8]naphthyridine-3(2H)-one (10mg, 0.017mmol, 1eq.) was dissolved in tetrahydrofuran (2mL) and acetonitrile (1mL). Acetic acid (7mg, 0.05mmol, 7eq.), water (1.8mg, 0.1mmol, 14eq.) and 1,3-dichloro-5,5-dimethylhydantoin (5.91mg, 0.03mmol, 1.8eq.) were added at 0°C. The mixture was reacted at 0°C for 2hrs, followed by adding ammonia solution (178mg, 5.1mmol, 300eq.), then reacted at 0°C for 1hr. After the completion of the reaction. Then the reaction mixture was washed with water and extracted with EA (15mL×3). The organic phases were combined, dried over anhydrous sodium sulfate, and filtered. The filtrate was concentrated, and purified to afford the target compound.

LCMS (ESI) [M+H]⁺=518.0; ¹H NMR (400MHz, CD₃OD) *δ* 8.80 (s, 1H), 8.57 (d, *J*=9.0 Hz, 1H), 8.52 (s, 2H), 7.39 (d, J=7.0 Hz, 2H), 7.13 (d, J=9.0 Hz, 1H), 4.78-4.66 (m, 4H), 4.11 (s, 3H), 4.04 (s, 2H).

### Example 10

### Preparation of 3,5-difluoro-4-((9-fluoro-8-methoxy-2-oxo-2H-[1,3]oxazino[5,4-c]quinolin-1(4H)-yl)methyl)benzenesulfonamide (compound 48)

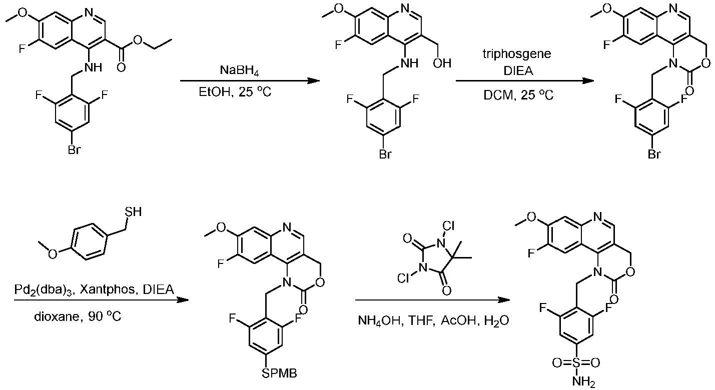

### Step 1: Preparation of (4-((4-bromo-2,6-difluorobenzyl)amino)-6-fluoro-7-methoxyquinolin-3-yl)methanol

Ethyl 4-((4-bromo-2,6-difluorobenzyl)amino)-6-fluoro-7-methoxyquinoline-3-carboxylate (500mg, 1.07mmol, 1eq.) was dissolved in ethanol (15mL). Sodium borohydride (202mg, 5.35mmol, 5eq.) was added. The mixture was reacted at 25°C for 16hrs. The completion of the reaction was monitored with LCMS. The reaction mixture was poured into water, extracted with EA, dried over anhydrous sodium sulfate, and filtered. The filtrate was concentrated, and purified by silica gel column chromatography (Silica gel, DCM:MeOH=97:3) to afford the target compound (300mg, yield 65.63%).

LCMS (ESI) [M+H]⁺=427.0.

### Step 2: Preparation of 1-(4-bromo-2,6-difluorobenzyl)-9-fluoro-8-methoxy-1,4-dihydro-2H-[1,3]oxazino[5,4-c]quinolin-2-one

(4-((4-bromo-2,6-difluorobenzyl)amino)-6-fluoro-7-methoxyquinolin-3-yl)methanol (150mg, 0.35mmol, 1eq.) was dissolved in dichloromethane (10mL). Triphosgene (313mg, 1.05mmol, 3eq.) and N,N-diisopropylethylamine (227mg, 1.76mmol, 5eq.) were added. The mixture was reacted at 25°C for 1hr. The completion of the reaction was monitored with LCMS. The reaction mixture was concentrated to dryness, and purified by silica gel column chromatography (Silica gel, DCM:MeOH=97:3) to afford the target compound (100mg, yield 62.66%).

LCMS (ESI) [M+H]⁺=453.0.

### Step 3: Preparation of 1-(2,6-difluoro-4-((4-methoxybenzyl)thio)benzyl)-9-fluoro-8-methoxy-1,4-dihydro-2H-[1,3]oxazino [5,4-c] quinolin-2-one

1-(4-bromo-2,6-difluorobenzyl)-9-fluoro-8-methoxy-1,4-dihydro-2H-[1,3]oxazino[5,4-c]quinolin-2-one (90mg, 0.2mmol, 1eq.) was dissolved in dioxane (10mL). (4-methoxyphenyl)methanethiol (61mg, 0.4mmol, 2eq.), tris(dibenzylideneacetone)dipalladium (18mg, 0.02mmol, 0.1eq.), 4,5-bis(diphenylphosphino)-9,9-dimethylxanthene (23mg, 0.04mmol, 0.2eq.), and N,N-diisopropylethylamine (77mg, 0.13mmol, 3eq.) were added. The mixture was reacted at 90°C for 2hrs. The completion of the reaction was monitored with LCMS. The reaction mixture was concentrated, and purified by silica gel column chromatography (Silica gel, DCM:MeOH=97:3) to afford the target compound (60mg, yield 57.38%).

LCMS (ESI) [M+H]⁺=527.0.

### Step 4: Preparation of 3,5-difluoro-4-((9-fluoro-8-methoxy-2-oxo-2H-[1,3]oxazino[5,4-c]quinolin-1(4H)-yl)methyl)benzenesulfonamide

1-(2,6-difluoro-4-((4-methoxybenzyl)thio)benzyl)-9-fluoro-8-methoxy-1,4-dihydro-2H-[1,3]oxazino[5,4-c]quinolin-2-one (5mg, 0.01mmol, 1.0eq.), acetic acid (4.0mg, 0.07mmol, 7.0eq.), water (2.4mg, 0.13mmol, 14.0eq.) were dissolved in tetrahydrofuran (15mL). 1,3-dichloro-5,5-dimethylhydantoin (5.6mg, 0.03mmol, 3eq.) was added at 0°C. The mixture was reacted at 0°C for 2hrs, followed by adding ammonia solution (0.5mL), then reacted at 0°C for 10 minutes. The completion of the reaction was monitored with LCMS. The reaction mixture was concentrated, and purified to afford the target compound (12.1mg, yield 25.55%).

LCMS (ESI) [M+H]⁺=454.0; ¹H NMR (400MHz, MeOD) *δ* 8.65 (s, 1H), 8.01 (d, *J*=12.8 Hz, 1H), 7.57 (d, *J*=8.4 Hz, 1H), 7.48 (d, J--7.2 Hz, 2H), 5.61 (s, 2H), 5.31 (s, 2H), 4.08 (s, 3H).

### Example 11

### Preparation of 3,5-difluoro-4-((8-methoxy-2,3-dioxo-3,4-dihydropyrazino[2,3-c][1,8]naphthyridine-1(2H)-yl)methyl)benzenesulfonamide (compound 62):

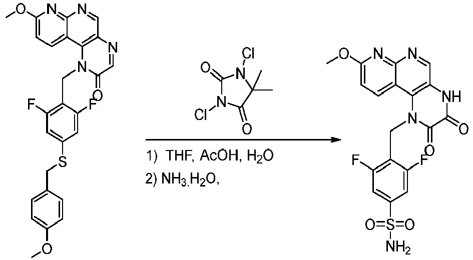

### Preparation of 3,5-difluoro-4-((8-methoxy-2,3-dioxo-3,4-dihydropyrazino[2,3-c][1,8]naphthyridine-1(2H)-yl)methyl)benzenesulfonamide

To a 50mL round-bottom flask were added 1-(2,6-difluoro-4-((4-methoxybenzyl)thio)benzyl)-8-methoxypyrazino[2,3-c][1,8]naphthyridine-2(1H)-one (100mg, 1.0eq.), tetrahydrofuran (10mL), acetic acid (10mg, 0.84eq.), water (10mg, 2.81eq.). While the mixture was cooled in ice water, 1,3-dichloro-5,5-dimethylhydantoin (118mg, 3.0eq.) was added. The resulting mixture was reacted under stirring for 1hr, followed by adding ammonia solution (3mL), then reacted under stirring for 1hr. LCMS monitoring indicated the completion of the reaction, the reaction mixture was extracted with EA. The organic phase was dried over anhydrous sodium sulfate, and filtered. The filtrate was concentrated to afford a crude product, which was purified to afford the target compound (19.3mg, yield: 21%).

LCMS (ESI) [M+H]⁺=450.1; ¹HNMR (400MHz, DMSO-*d₆*) *δ* 12.44 (s, 1H), 8.76 (s, 1H), 8.67 (d, *J*=9.6 Hz, 1H), 7.60 (s, 2H), 7.43 (d, *J*=7.2 Hz, 2H), 7.11 (d, *J*=9.2 Hz, 1H), 5.69 (s, 2H), 4.01 (s, 3H).

### Example 12

### Preparation of 7-(8-methoxy-2-oxo-2H-[1,3]oxazino[5,4-c][1,8]naphthyridin-1(4H)-yl)-3,4-dihydroisoquinolin-2(1H)-sulfonamide (compound 39):

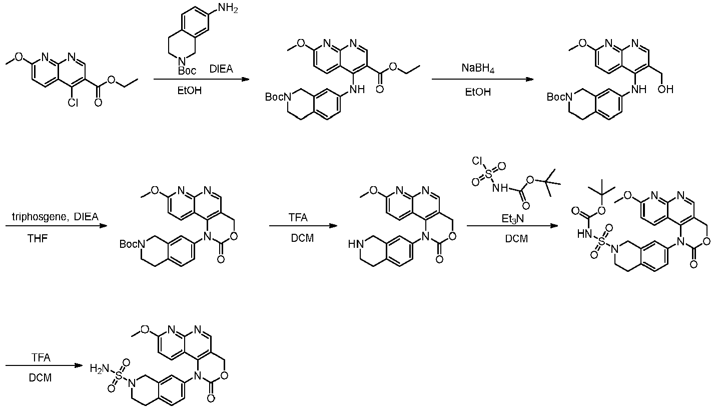

### Step 1: Preparation of ethyl 4-((2-(tert-butoxycarbonyl)-1,2,3,4-tetrahydroisoquinolin-7-yl)atnino)-7-methoxy-1,8-naphthyridine-3 -carboxylate

Ethyl 4-chloro-7-methoxy-1,8-naphthyridine-3-carboxylate (600mg, 1eq.), tert-butyl 7-amino-3,4-dihydroisoquinolin-2(1H)-carboxylate (838.4mg, 1.5eq.) and N,N-diisopropylethylamine (1.45g, 5eq.) were dissolved in ethanol (150mL). The mixture was heated to 90°C and reacted for 24hrs. The completion of the reaction was monitored with LCMS. The reaction mixture was purified by flash silica gel column chromatography to afford the target compound (800mg, yield: 74.3%).

LCMS (ESI) [M+H]⁺=479.0; ¹H NMR (400MHz, DMSO-*d₆*) *δ* 9.85 (s, 1H), 9.02 (s, 1H), 8.10 (d, *J*=9.6 Hz, 1H), 7.10 (d, *J*=8.2 Hz, 1H), 6.93 (d, *J*=9.6 Hz, 1H), 6.90 (d, *J*=9.6 Hz, 2H), 4.41 (s, 2H), 4.16 (q, *J*=7.2 Hz, 2H), 4.00 (s, 3H), 3.54 (t, *J*=5.6 Hz, 2H), 2.73 (t, *J*=5*.*6 Hz, 2H), 1.42 (s, 9H), 1.24 (t, *J*=7.2 Hz, 3H).

### Step 2: Preparation of tert-butyl 7-((3-(hydroxymethyl)-7-methoxy-1,8-naphthyridin-4-yl)amino)-3,4-dihydroisoquinolin-2(1H)-carboxylate

Ethyl 4-((2-(tert-butoxycarbonyl)-1,2,3,4-tetrahydroisoquinolin-7-yl)amino)-7-methoxy-1,8-naphthyridine-3-carboxylate (400mg, 1eq.) and sodium borohydride (307.8mg, 10eq.) were dissolved in ethanol (40mL). The mixture was warmed to 60°C and reacted under stirring for 4hrs. The completion of the reaction was monitored with LCMS. The reaction mixture was rotary-evaporated in vacuum to remove the solvent, diluted with water (100mL), extracted with EA (200mL×2). The organic phases were dried over anhydrous sodium sulfate, and filtered. The filtrate was concentrated, and purified by flash chromatography to afford the target compound (230mg, yield 62%).

LCMS (ESI) [M+H]⁺=437.3; ¹H NMR (400MHz, DMSO-*d₆*) *δ* 8.86 (s, 1H), 8.35 (s, 1H), 8.18 (d, *J*=9.0 Hz, 1H), 6.97 (d, *J*=9.0 Hz, 2H), 6.55 (d, *J*=8.8 Hz, 2H), 5.33 (t, *J*=5.4 Hz, 1H), 4.46 (d, *J*=5.4 Hz, 2H), 4.36 (s, 2H), 4.00 (s, 3H), 3.51 (t, *J*=5.8 Hz, 2H), 2.67 (t, *J*=5.6 Hz, 2H), 1.41 (s, 9H).

### Step 3: Preparation of tert-butyl 7-(8-methoxy-2-oxo-2H-[1,3]oxazino[5,4-c][1,8]naphthyridin-1(4H)-yl)-3,4-dihydroisoquinolin-2(1H)-carboxylate

Tert-butyl 7-((3-(hydroxymethyl)-7-methoxy-1,8-naphthyridin-4-yl)amino)-3,4-dihydroisoquinolin-2(1H)-carboxylate (230mg, 1eq.) was added to tetrahydrofuran (20mL). N,N-diisopropylethylamine (683.7mg, 10eq.) was added. Then triphosgene (312.7mg, 2eq.) was added. The resulting mixture was reacted at rt for 1hr. LCMS monitoring indicated the completion of the reaction, the reaction mixture was purified by flash chromatography to afford the target compound (200mg, yield: 81.61%).

LCMS (ESI) [M+H]⁺=463.0; ¹H NMR (400MHz, DMSO-*d₆*) *δ* 8.84 (s, 1H), 8.57-8.43 (m, 1H), 7.33 (s, 2H), 7.18 (d, *J*=9.6 Hz, 1H), 6.78 (d, *J*=9.2 Hz, 1H), 5.60 (s, 2H), 4.45 (s, 2H), 3.58 (s, 3H), 3.14 (s, 2H), 2.85 (s, 2H), 1.41 (s, 9H).

### Step 4: Preparation of 8-methoxy-1-(1,2,3,4-tetrahydroisoquinolin-7-yl)-1,4-dihydro-2H-[1,3]oxazino[5,4-c][1,8]naphthyridin-2-one

Tert-butyl 7-(8-methoxy-2-oxo-2H-[1,3]oxazino[5,4-c][1,8]naphthyridin-1(4H)-yl)-3,4-dihydroisoquinolin-2(1H)-carboxylate (200mg, 1eq.) was added to dichloromethane (10mL). Then trifluoroacetic acid (3mL) was added. The mixture was reacted at rt for 2hrs. The completion of the reaction was monitored with LCMS. The reaction mixture was rotary-evaporated in vacuum to remove the solvent to afford a crude target compound (140mg), which was not further purified but directly used in the next step reaction.

LCMS (ESI) [M+H]⁺=363.1.

### Step 5: Preparation of tert-butyl ((7-(8-methoxy-2-oxo-2H-[1,3]oxazino[5,4-c][1,8]naphthyridin-1(4H)-yl)-3,4-dihydroisoquinolin-2(1H)-yl)sulfonyl)carbamate

8-methoxy-1-(1,2,3,4-tetrahydroisoquinolin-7-yl)-1,4-dihydro-2H-[1,3]oxazino[5,4-c][1,8]naphthyridin-2-one (130mg, crude) and triethylamine (54.6mg) were added to dichloromethane (20mL). Tert-butyl N-(chlorosulfonyl)carbamate (154.7mg) was added dropwise at 0°C. The mixture was reacted at rt for 4hrs. The completion of the reaction was monitored with LCMS. The reaction mixture was diluted with water (50mL), and extracted with dichloromethane (100mL×2). The organic phases were dried over anhydrous sodium sulfate, and filtered. The filtrate was concentrated in vacuum to afford a crude target compound (150mg), which was not further purified but directly used in the next step reaction.

LCMS (ESI) [M+H]⁺=542.1.

### Step 6: Preparation of 7-(8-methoxy-2-oxo-2H-[1,3]oxazino[5,4-c][1,8]naphthyridin-1(4H)-yl)-3,4-dihydroisoquinolin-2(1H)-sulfonamide

Tert-butyl ((7-(8-methoxy-2-oxo-2H-[1,3]oxazino[5,4-c][1,8]naphthyridin-1(4H)-yl)-3,4-dihydroisoquinolin-2(1H)-yl)sulfonyl)carbamate (150mg, crude) was added to dichloromethane (10mL). Then trifluoroacetic acid (2mL) was added. The mixture was reacted at rt for 4hrs. The completion of the reaction was monitored with LCMS. The reaction mixture was rotary-evaporated in vacuum to remove the solvent, dissolved in N,N-dimethylformamide (3mL), and purified by preparative HPLC to afford the target compound (27mg, yield: 22%).

LCMS (ESI) [M+H]⁺=442.1; ¹H NMR (400MHz, DMSO-*d₆*) *δ* 8.83 (s, 1H), 7.38 (s, 1H), 7.30 (s, 2H), 7.18 (d, J=9.2, 1H), 6.95 (s, 2H), 6.79 (d, *J*=9.2, 1H), 5.60 (s, 2H), 4.17(s, 2H), 3.95 (s, 3H), 3.28 (t, *J*=5.6, 2H), 2.98 (t, *J*=5.6, 2H).

### Example 13

### Preparation of 3,5-difluoro-4-((2-hydroxy-8-methoxy-3-oxo-3,4-dihydropyrazino[2,3-c][1,8]naphthyridine-1(2H)-yl)methyl)benzenesulfonamide (compound 65)

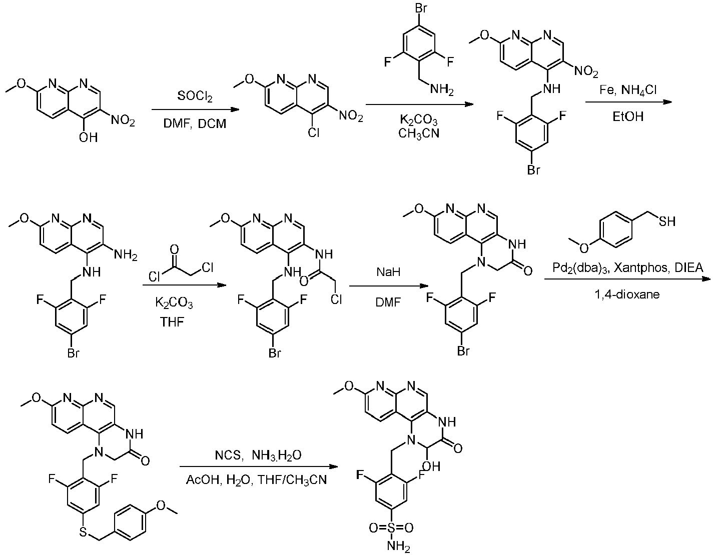

### Step 1: Preparation of 5-chloro-2-methoxy-6-nitro-1,8-naphthyridine

7-methoxy-3-nitro-1,8-naphthyridine-4-ol (3g, 1eq.) was dissolved in dichloromethane (30mL). Then N,N-dimethylformamide (1mL) was added. In an ice bath, sulfurous dichloride (3mL, 1.9eq.) was slowly added dropwise. Then the mixture was warmed to 40°C and reacted for 2hrs. The completion of the reaction was monitored with LCMS. The reaction was quenched with ice water, and extracted with dichloromethane (100mL×3). The organic phases were collected, dried over anhydrous sodium sulfate, and filtered. The filtrate was concentrated, and purified by column chromatography to afford the target compound (2.8g, yield: 88%).

LCMS (ESI) [M+H]⁺=240.0.

### Step 2: Preparation of N-(4-bromo-2,6-difluorobenzyl)-7-methoxy-3-nitro-1,8-naphthyridine-4-atnine

5-chloro-2-methoxy-6-nitro-1,8-naphthyridine (2g, 1eq.) and 1-(4-bromo-2,6-difluorophenyl)methanatnine (5.07g, 1.1eq.) were dissolved in acetonitrile (100mL). Then K₂CO₃ (8.63g, 3eq.) was added. Then the mixture was reacted at 40°C for 2hrs. The completion of the reaction was monitored with LCMS. The reaction mixture was concentrated, diluted with water, and extracted with EA (20mL×3). The organic phases were collected, dried over anhydrous ammonium sulfate, and filtered. The filtrate was concentrated, and purified by column chromatography to afford the target compound (3g, yield: 85%).

LCMS (ESI) [M+H]⁺=427.0.

### Step 3: Preparation of N⁴-(4-bromo-2,6-difluorobenzyl)-7-methoxy-1,8-naphthyridine-3,4-diamine

N-(4-bromo-2,6-difluorobenzyl)-7-methoxy-3-nitro-1,8-naphthyridine-4-atnine (3g, 1eq.) was dissolved in ethanol (50mL). Then Fe powder (4g, 10eq.) and a saturated NH4Cl solution (2mL) were added. The resulting mixture was reacted at 80°C for 2hrs. The completion of the reaction was monitored with LCMS. The reaction mixture was concentrated to remove a part of the solvent, then diluted with water, and extracted with EA (50mL×3). The organic phases were collected, dried over anhydrous ammonium sulfate, and filtered. The filtrate was concentrated, and purified by column chromatography to afford N⁴-(4-bromo-2,6-difluorobenzyl)-7-methoxy-1,8-naphthyridine-3,4-diamine (1.5g, yield: 54%).

LCMS (ESI) [M+H]⁺=397.0.

### Step 4: Preparation of N-(4-((4-bromo-2,6-difluorobenzyl)amino)-7-methoxy-1,8-naphthyridin-3-yl)-2-chloroacetamide

N⁴-(4-bromo-2,6-difluorobenzyl)-7-methoxy-1,8-naphthyridine-3,4-diamine (500mg, 1.0eq.), 2-chloroacetyl chloride (154mg, 1.1eq.), and K₂CO₃ (197mg, 1.1eq.) were dissolved in tetrahydrofuran (30mL). The mixture was reacted at rt for 20 minutes. The completion of the reaction was monitored with LCMS. The reaction mixture was concentrated to afford a crude product, which was purified by flash silica gel column chromatography to afford the target compound (330mg, yield: 56%).

LCMS (ESI) [M+H]⁺=471.0; ¹H NMR (400MHz, dmso-*d₆*) *δ* 10.49 (s, 1H), 8.88 (d, J=9.2 Hz, 1H), 8.42 (s, 1H), 7.51 (d, *J*=7.4 Hz, 2H), 7.19 (d, *J*=9.2 Hz, 1H), 4.94 (d, *J*=4.4 Hz, 2H), 4.38 (s, 2H), 4.05 (s, 3H), 3.17 (s, 1H).

### Step 5: Preparation of 1-(4-bromo-2,6-difluorobenzyl)-8-methoxy-1,4-dihydropyrazino[2,3-c][1,8]naphthyridine-3 (2H)-one

N-(4-((4-bromo-2,6-difluorobenzyl)atnino)-7-methoxy-1,8-naphthyridin-3-yl)-2-chloroacetamide (320mg, 1.0eq.), and sodium hydride (18mg, 1.1eq.) were dissolved in N,N-dimethylformamide (20mL). The mixture was reacted at rt for 20 minutes. The completion of the reaction was monitored with LCMS. The reaction mixture was concentrated to afford a crude product, which was purified by flash silica gel column chromatography to afford the target compound (120mg, yield: 42%).

LCMS (ESI) [M+H]⁺=435.0; ¹H NMR (400MHz, dmso-*d₆*) *δ* 10.56 (s, 1H), 8.48 (d, *J*=9.0 Hz, 1H), 8.37 (s, 1H), 7.41 (d, *J*=7.2 Hz, 2H), 7.12 (d, *J*=9.0 Hz, 1H), 4.49 (s, 2H), 3.99 (s, 3H), 3.77 (s, 2H).

### Step 6: Preparation of 1-(2,6-difluoro-4-((4-methoxybenzyl)thio)benzyl)-8-methoxy-1,4-dihydropyrazino[2,3-c][1,8]naphthyridine-3 (2H)-one

1-(4-bromo-2,6-difluorobenzyl)-8-methoxy-1,4-dihydropyrazino[2,3-c][1,8]naphthyridine-3(2H)-one (120mg, 1eq.) was dissolved in dioxane (10mL). Then (4-methoxyphenyl)methanethiol (73mg, 2eq.), tris(dibenzylideneacetone)dipalladium (32mg), 4,5-bis(diphenylphosphino)-9,9-dimethylxanthene (72mg) and diisopropylethylamine (58mg, 3eq.) were successively added. Under the protection of nitrogen gas, the mixture was reacted at 100°C for 2hrs. The completion of the reaction was monitored with LCMS. Then the reaction mixture was washed with water and extracted with EA (15mL×3). The organic phases were collected, dried over anhydrous sodium sulfate, and filtered. The filtrate was concentrated, and purified by column chromatography to afford the target product (76mg, yield: 64%).

LCMS (ESI) [M+H]⁺=509.1.

### Step 7: Preparation of 3,5-difluoro-4-((2-hydroxy-8-methoxy-3-oxo-3,4-dihydropyrazino[2,3-c][1,8]naphthyridine-1(2H)-yl)methyl)benzenesulfonamide)

1-(2,6-difluoro-4-((4-methoxybenzyl)thio)benzyl)-8-methoxy-1,4-dihydropyrazino[2,3-c][1,8]naphthyridine-3(2H)-one (40mg, 1eq.) was dissolved in tetrahydrofuran (2mL) and acetonitrile (1mL). Acetic acid (41mg, 7eq.), water (11.5mg, 14eq.) and N-chlorosuccinimide (25mg, 1.8eq.) were added at 0°C. The mixture was reacted at 0°C for 2hrs, followed by adding ammonia solution (1g, 300eq.), then reacted at 0°C for 1hr. The completion of the reaction was monitored with LCMS. The reaction mixture was diluted with water, and extracted with EA (15mL×3).

The organic phases were collected, dried over anhydrous sodium sulfate, and filtered. The filtrate was concentrated and purified by preparative chromatography to afford the target compound (5.1mg, yield: 17%).

LCMS (ESI) [M+H]⁺=452.0; ¹H NMR (400MHz, MeOD) *δ* 8.63 (d, *J*=9.2 Hz, 1H), 8.44 (s, 1H), 7.41 (d, *J*=7.0 Hz, 2H), 7.14 (d, *J*=9.0 Hz, 1H), 5.21 (s, 1H), 5.03 (s, 2H), 4.12 (s, 3H).

### Example 14

### Preparation of 4-((8-methoxy-2-oxo-2H-[1,3]oxazino[5,4-c][1,8]naphthyridin-1(4H)-yl)methyl)piperidine-1-sulfonamide (compound 46)

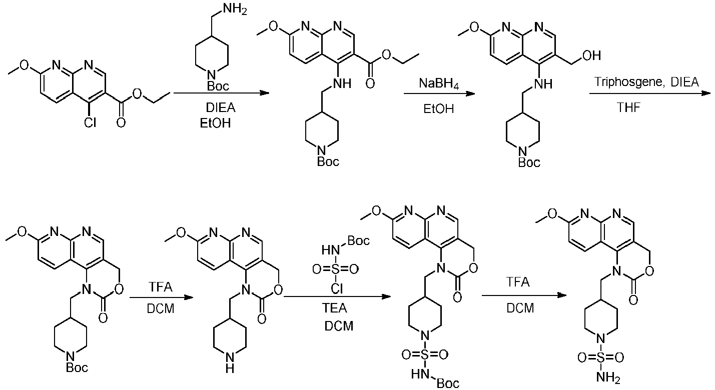

### Step 1: Preparation of ethyl 4-(((1-(tert-butoxycarbonyl)piperidine-4-yl)methyl)amino)-7-methoxy-1,8-naphthyridine-3 -carboxylate

Ethyl 4-chloro-7-methoxy-1,8-naphthyridine-3-carboxylate (300mg, 1eq.), tert-butyl 4-(aminomethyl)piperidine-1-carboxylate (241mg, 1eq.) and N,N-diisopropylethylamine (726mg, 5eq.) were dissolved in ethanol (100mL). The mixture was heated to 90°C and reacted for 24hrs. The completion of the reaction was monitored with LCMS. The reaction mixture was purified by flash silica gel column chromatography to afford the target compound (300mg, yield: 24%).

LCMS (ESI) [M+H]⁺=445.2.

### Step 2: Preparation of tert-butyl 4-(((3-(hydroxymethyl)-7-methoxy-1,8-naphthyridin-4-yl)amino)methyl)piperidine-1-carboxylate

Ethyl 4-(((1-(tert-butoxycarbonyl)piperidine-4-yl)methyl)atnino)-7-methoxy-1,8-naphthyridine-3-carboxylate (300mg, 1eq.) and sodium borohydride (248mg, 10eq.) were dissolved in ethanol (40mL). The mixture was warmed to 60°C and reacted for 16hrs. The completion of the reaction was monitored with LCMS. The reaction mixture was rotary-evaporated in vacuum to remove the solvent, diluted with water (50mL), and extracted with EA (100mL×2). The organic phases were dried over anhydrous sodium sulfate, and filtered. The filtrate was concentrated and purified by flash chromatography to afford the target compound (50mg, yield: 18%).

LCMS (ESI) [M+H]⁺=403.3.

### Step 3: Preparation of tert-butyl 4-((8-methoxy-2-oxo-2H-[1,3]oxazino[5,4-c][1,8]naphthyridin-1(4H)-yl)methyl)piperidine-1-carboxylate

Tert-butyl 4-(((3-(hydroxymethyl)-7-methoxy-1,8-naphthyridin-4-yl)atnino)methyl)piperidine-1-carboxylate (50mg, 1eq.) was added to tetrahydrofuran (5mL). Then N,N-diisopropylethylamine (160mg, 10eq.) was added. Triphosgene (73mg, 2eq.) was added. The mixture was reacted at rt for 1hr. LCMS monitoring indicated the completion of the reaction, the reaction mixture was diluted with water (10mL), and extracted with EA (20mL×3). The organic phases were dried over anhydrous sodium sulfate, and filtered. The filtrate was concentrated to afford a crude target compound (50mg), which was not further purified but directly used in the next step reaction.

LCMS (ESI) [M+H]⁺=429.2.

### Step 4: Preparation of 8-methoxy-1-(piperidine-4-ylmethyl)-1,4-dihydro-2H-[1,3]oxazino[5,4-c][1,8]naphthyridin-2-one

Tert-butyl 4-((8-methoxy-2-oxo-2H-[1,3]oxazino[5,4-c][1,8]naphthyridin-1(4H)-yl)methyl)piperidine-1-carboxylate (50mg, crude) was added to dichloromethane(5mL). Then trifluoroacetic acid (1mL) was added. The mixture was reacted at rt for 2hrs. The completion of the reaction was monitored with LCMS. The reaction mixture was rotary-evaporated in vacuum to remove the solvent to afford a crude target compound (30mg), which was not further purified but directly used in the next step reaction.

LCMS (ESI) [M+H]⁺=329.2.

### Step 5: Preparation of tert-butyl ((4-((8-methoxy-2-oxo-2H-[1,3]oxazino[5,4-c][1,8]naphthyridin-1(4H)-yl)methyl)piperidine-1-yl)sulfonyl)carbamate

8-methoxy-1-(piperidine-4-ylmethyl)-1,4-dihydro-2H-[1,3]oxazino[5,4-c][1,8]naphthyridin-2-one (30mg, crude) and triethylamine (46mg) were added to dichloromethane (5mL). At 0°C, Tert-butyl N-(chlorosulfonyl)carbamate (39mg) was added dropwise. The mixture was reacted at rt for 5hrs. The completion of the reaction was monitored with LCMS. The reaction mixture was diluted with water (10mL), and extracted with dichloromethane (20mL×2). The organic phases were dried over anhydrous sodium sulfate, and filtered. The filtrate was concentrated to afford a crude target compound (30mg), which was not further purified but directly used in the next step reaction.

LCMS (ESI) [M+H]⁺=508.2.

### Step 6: Preparation of 4-((8-methoxy-2-oxo-2H-[1,3]oxazino[5,4-c][1,8]naphthyridin-1(4H)-yl)methyl)piperidine-1-sulfonamide

Tert-butyl ((4-((8-methoxy-2-oxo-2H-[1,3]oxazino[5,4-c][1,8]naphthyridin-1(4H)-yl)methyl)piperidine-1-yl)sulfonyl)carbamate (28mg, crude) was added to dichloromethane (5mL). Then trifluoroacetic acid (1mL) was added. The mixture was reacted at rt for 4hrs. The completion of the reaction was monitored with LCMS. The reaction mixture was rotary-evaporated in vacuum to remove the solvent to afford a crude product, which was purified by liquid-phase preparative HPLC to afford the target compound (5.2mg, yield: 18%).

LCMS (ESI) [M+H]⁺=408.1; ¹H NMR (400MHz, DMSO-*d₆*) *δ* 8.77 (s, 1H), 8.53 (d, *J*=9.2, 1H), 7.11 (d, *J*=9.2, 1H), 6.62 (s, 2H), 5.41 (s, 2H), 4.12 (d, *J*=6.8, 2H), 4.02 (s, 3H), 2.39-2.33 (m, 2 H), 1.73-1.61 (m, 3H), 1.13-1.06 (m, 2H).

### Example 15

### Preparation of tert-butyl 1-(2,6-difluoro-4-sulfamoylbenzyl)-8-methoxy-2,3-dihydropyrazino[2,3-c][1,8]naphthyridine-4(1H)-carboxylate (compound 50):

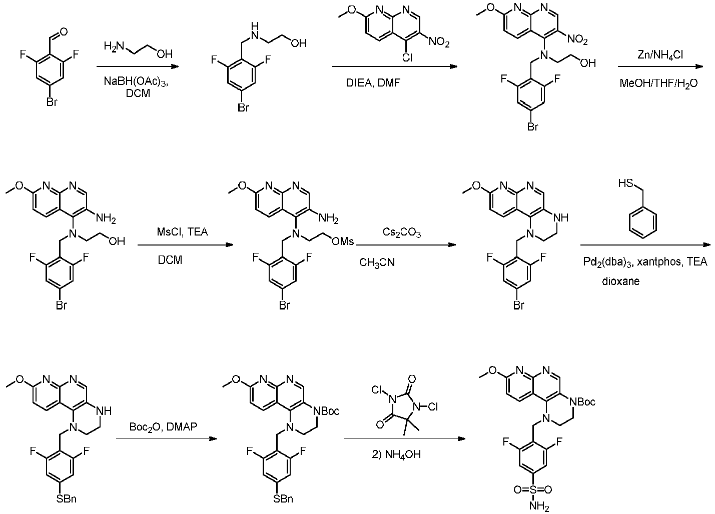

### Step 1: Preparation of 2-((4-bromo-2,6-difluorobenzyl)amino)ethan-1-ol

4-bromo-2,6-difluorobenzaldehyde (10g, 1eq.) and 2-aminoethan-1-ol (8.29g, 3eq.) were dissolved in dichloromethane (200mL). Acetic acid (5.43g, 2eq.) was added. The mixture was reacted at rt for 30 minutes. Sodium triacetoxyborohydride (19.18g, 2eq.) was added. The mixture was reacted at rt overnight. The completion of the reaction was monitored with LCMS. The reaction mixture was filtered. The filtrate was concentrated under reduced pressure to afford a crude product, which was purified by column chromatography (DCM/MeOH=10:1) to afford the target compound (8.5g, yield 70%).

LCMS (ESI) [M+H]⁺=266.0; ¹H NMR (400MHz, CDCl₃) δ 7.13-7.06 (m, 2H), 3.87 (s, 2H), 3.68-3.62 (m, 2H), 2.77-2.69 (m, 2H).

### Step 2: Preparation of 2-((4-bromo-2,6-difluorobenzyl)(7-methoxy-3-nitro-1,8-naphthyridin-4-yl)amino)ethan-1-ol

5-chloro-2-methoxy-6-nitro-1,8-naphthyridine (5.00g, 1eq.) and 2-((4-bromo-2,6-difluorobenzyl)amino)ethan-1-ol (8.32g, 1.5eq.) were dissolved in dimethylformamide (100mL). Ethyldi(propane-2-yl)amine (10.78g, 4eq.) was added. The mixture was reacted at rt overnight. The completion of the reaction was monitored with LCMS. Water was added to the reaction mixture. A large amount of solid precipitated. The mixture was filtered. The filter cake was dried to afford the target compound (4.5g, yield 45%).

LCMS (ESI) [M+H]⁺=468.9; ¹H NMR (400MHz, CDCl₃) δ 9.23 (s, 1H), 8.10 (d, J=9.0 Hz, 1H), 7.09 (d, J=6.8 Hz, 2H), 6.99 (d, J=9.1 Hz, 1H), 4.45 (s, 2H), 4.17 (s, 3H), 3.96-3.81 (m, 2H), 3.59-3.46 (m, 2H), 2.66 (s, 1H).

### Step 3: Preparation of 2-((3-amino-7-methoxy-1,8-naphthyridin-4-yl)(4-bromo-2,6-difluorobenzyl)amino)ethan-1-ol

2-((4-bromo-2,6-difluorobenzyl)(7-methoxy-3-nitro-1,8-naphthyridin-4-yl)amino)ethan-1-ol (4.5g, 1eq.) was dissolved in methanol (20mL), tetrahydrofuran (20mL) and water (10mL). Zn (3.13g, 5eq.) and NH₄Cl (5.13g, 10eq.) were added. The mixture was reacted at rt overnight. The completion of the reaction was monitored with LCMS. The reaction mixture was filtered. The filtrate was concentrated under reduced pressure to afford a crude product, which was dissolved in dichloromethane (100mL), washed with water (100mL) and saturated brine (80mL), dried over anhydrous sodium sulfate, filtered, and concentrated to afford the target compound (3.0g, crude), which was not further purified but directly used in the next step reaction.

LCMS (ESI) [M+H]⁺=440.9.

### Step 4: Preparation of 2-((3-amino-7-methoxy-1,8-naphthyridin-4-yl)(4-bromo-2,6-difluorobenzyl)atnino)ethyl methanesulfonate

2-((3-amino-7-methoxy-1,8-naphthyridin-4-yl)(4-bromo-2,6-difluorobenzyl)amino)ethan-1-ol (1.2g, crude) was dissolved in dichloromethane (30mL). Triethylamine (829.3mg) was added. In an ice bath, methanesulfonyl chloride (469.4mg) was added. The mixture was reacted in an ice bath for 30 minutes. The completion of the reaction was monitored with LCMS. The reaction mixture was diluted with water (30mL), extracted with dichloromethane (30mL×2). The organic phases were combined, washed with saturated brine (50mL), dried over anhydrous sodium sulfate, filtered, and concentrated to afford the target compound (1.2g, crude), which was not further purified but directly used in the next step reaction.

LCMS (ESI) [M+H]⁺=519.0.

### Step 5: Preparation of 1-(4-bromo-2,6-difluorobenzyl)-8-methoxy-1,2,3,4-tetrahydropyrazino[2,3-c][1,8]naphthyridine

2-((3-amino-7-methoxy-1,8-naphthyridin-4-yl)(4-bromo-2,6-difluorobenzyl)amino)ethyl methanesulfonate (1.5g, crude) was dissolved in acetonitrile (50mL). Caesium carbonate (2.83g) was added. The mixture was reacted at 50°C for 16hrs. The completion of the reaction was monitored with LCMS. The reaction mixture was filtered. The filtrate was concentrated under reduced pressure to afford a crude product, which was purified by column chromatography (DCM/EA=1:1) to afford the target compound (500mg, yield 41%).

LCMS (ESI) [M+H]⁺=423.1; ¹H NMR (400MHz, CDCl₃) δ 8.50 (d, J=8.9 Hz, 1H), 8.39 (s, 1H), 7.17 (d, J=7.0 Hz, 2H), 6.94 (d, J=9.0 Hz, 1H), 4.32 (s, 2H), 4.11 (s, 3H), 3.37-3.30 (m, 2H), 3.09-3.02 (m, 2H).

### Step 6: Preparation of 1-(4-(benzylthio)-2,6-difluorobenzyl)-8-methoxy-1,2,3,4-tetrahydropyrazino[2,3-c][1,8]naphthyridine

1-(4-bromo-2,6-difluorobenzyl)-8-methoxy-1,2,3,4-tetrahydropyrazino[2,3-c][1,8]naphthyridine (300mg, 1eq.) and phenylmethanethiol (2.13g, 1.5eq.) were dissolved in dioxane (15mL). Tris((1E,4E)-1,5-diphenylpenta-1,4-diene-3-one) dipalladium (31.8mg, 0.1eq.), (5-(diphenylphosphino)-9,9-dimethyl-9H-xanthene-4-yl)diphenylphosphine (82.4mg, 0.2eq.) and triethylamine (216.2mg, 3eq.) were successively added. The atmosphere was replaced with argon three times. The mixture was reacted at 90°C for 16hrs. The completion of the reaction was monitored with LCMS. The reaction mixture was concentrated under reduced pressure to afford a crude product, which was purified by column chromatography to afford the target compound (300mg, yield 91%).

LCMS (ESI) [M+H]⁺=465.2; ¹H NMR (400MHz, CDCl₃) δ 8.48 (d, J=8.9 Hz, 1H), 8.43 (s, 1H), 7.38-7.30 (m, 5H), 6.92 (d, J=9.0 Hz, 1H), 6.85 (d, J=8.2 Hz, 2H), 4.36 (s, 2H), 4.17 (s, 2H), 4.11 (s, 3H), 3.32-3.27 (m, 2H), 3.11-3.06 (m, 2H).

### Step 7: Preparation of tert-butyl 1-(4-(benzylthio)-2,6-difluorobenzyl)-8-methoxy-2,3-dihydropyrazino[2,3-c] [1,8]naphthyridine-4(1H)-carboxylate

1-(4-(benzylthio)-2,6-difluorobenzyl)-8-methoxy-1,2,3,4-tetrahydropyrazino[2,3-c][1,8]naphthyridine (100mg, 1eq.) was dissolved in di-tert-butyl dicarbonate (5mL, 106.38eq.). 4-dimethylaminopyridine (100mg, 3.8eq.) was added. The mixture was reacted at 40°C for 2hrs. The completion of the reaction was monitored with LCMS. The reaction mixture was concentrated under reduced pressure to afford a crude product, which was purified by column chromatography to afford the target compound (100mg, yield 82%).

LCMS (ESI) [M+H]⁺=565.3.

### Step 8: Preparation of tert-butyl 1-(2,6-difluoro-4-sulfamoylbenzyl)-8-methoxy-2,3-dihydropyrazino[2,3-c] [1,8]naphthyridine-4(1H)-carboxylate

Tert-butyl 1-(4-(benzylthio)-2,6-difluorobenzyl)-8-methoxy-2,3-dihydropyrazino[2,3-c][1,8]naphthyridine-4(1H)-carboxylate (230mg, 1eq.) was dissolved in tetrahydrofuran (5mL). Water (102.8mg, 14eq.) and acetic acid (171.2mg, 7eq.) were added. In an ice bath, 1,3-dichloro-5,5-dimethylhydantoin (240.8mg, 3eq.) was added. The mixture was reacted in an ice bath for 10 minutes. In an ice bath, the reaction mixture was slowly added dropwise to the ammonia solution (5mL). The mixture was reacted in an ice bath for 30 minutes. The completion of the reaction was monitored with LCMS. The reaction mixture was extracted with EA (20mL). The organic phase was concentrated under reduced pressure to afford a crude product, which was purified by preparative HPLC to afford the target compound (13.0mg, yield 6%).

LCMS (ESI) [M+H]⁺=522.5; ¹H NMR (400MHz, MeOD-d₄) δ 8.85 (s, 1H), 8.54 (d, J=9.1 Hz, 1H), 7.48 (d, J=7.0 Hz, 2H), 7.00 (d, J=9.1 Hz, 1H), 4.98 (s, 2H), 4.09 (s, 3H), 3.71-3.64 (m, 2H), 3.46-3.38 (m, 2H), 1.48 (s, 9H).

### Example 16

### Preparation of 3,5-difluoro-4-((8-methoxy-3,4-dihydropyrazino[2,3-c][1,8]naphthyridine-1(2H)-yl)methyl)benzenesulfonamide (compound 66)

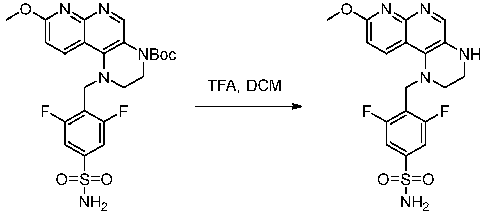

Tert-butyl 1-(2,6-difluoro-4-sulfamoylbenzyl)-8-methoxy-2,3 -dihydropyrazino [2,3-c][1,8]naphthyridine-4(1H)-carboxylate (10mg, 1eq.) was dissolved in dichloromethane (1mL). Trifluoroacetic acid (0.2mL) was added. The mixture was reacted at rt for 2hrs. The completion of the reaction was monitored with LCMS. The reaction mixture was concentrated under reduced pressure to afford a crude product, which was purified by preparative chromatography to afford the target compound 3,5-difluoro-4-((8-methoxy-3,4-dihydropyrazino[2,3-c][1,8]naphthyridine-1(2H)-yl)methyl)benzenesulfonamide (4.4mg, yield 52%).

LCMS (ESI) [M+H]⁺=422.1; ¹H NMR (400MHz, MeOD-d₄) δ 8.54 (d, J=9.1 Hz, 1H), 8.11 (s, 1H), 7.54 (t, J=5.3 Hz, 2H), 6.99 (d, J=9.1 Hz, 1H), 4.83 (s, 2H), 4.07 (s, 3H), 3.30-3.27 (m, 2H), 3.24-3.20 (m, 2H).

### Example 17

### Preparation of 3,5-difluoro-4-((8-methoxy-2-oxo-3,4-dihydropyrido[2,3-h][1,6]naphthyridine-1(2H)-yl)methyl)benzenesulfonamide (compound 54):

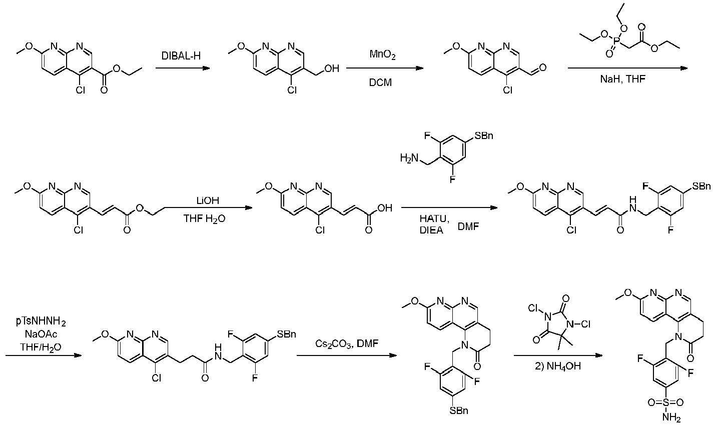

### Step 1: Preparation of (4-chloro-7-methoxy-1,8-naphthyridin-3-yl)methanol

Ethyl 4-chloro-7-methoxy-1,8-naphthyridine-3-carboxylate (10g, 1eq.) was dissolved in dichloromethane (200mL). The atmosphere was replaced with nitrogen gas. The temperature was lowered to -68°C. A solution of diisobutylaluminium hydride in cyclohexane (86mL, 1M) was slowly added dropwise to the reaction mixture. The mixture was stirred at -68°C for 2hrs. The completion of the reaction was monitored with LCMS. The reaction mixture was quenched with saturated NH4Cl solution. The aqueous phase was extracted with EA (100mL×5). The organic phases were combined, dried over anhydrous sodium sulfate, filtered, and concentrated to afford a crude product, which was purified by column chromatography to afford the target product (5.5g, yield 83%).

LCMS (ESI) [M+H]⁺=225.0.

### Step 2: Preparation of 4-chloro-7-methoxy-1,8-naphthyridine-3-carbaldehyde

(4-chloro-7-methoxy-1,8-naphthyridin-3-yl)methanol (5.5g, 1eq.) was dissolved in dichloromethane (20mL). An active MnOz was added (10.64g, 5eq.). The reaction mixture was stirred at 40°C overnight. The completion of the reaction was monitored with LCMS. The mixture was filtered through celite. The filtrate was washed with dichloromethane, and concentrated to afford a crude product, which was purified by column chromatography to afford the target compound (4.5g, yield 99%).

LCMS (ESI) [M+H]⁺=223.0; ¹H NMR (400MHz, DMSO-d₆) δ 10.47 (s, 1H), 9.20 (s, 1H), 8.67 (d, *J*=9.0 Hz, 1H), 7.37 (d, *J*=9.0 Hz, 1H), 4.10 (s, 3H).

### Step 3: Preparation of ethyl (E)-3-(4-chloro-7-methoxy-1,8-naphthyridin-3-yl)acrylate

Sodium hydride (808.5mg, 3eq.) was dissolved in tetrahydrofuran (20mL). In an ice bath, a solution of ethyl 2-(diethoxyphosphoryl)acetate (1.81g, 1.2eq.) in tetrahydrofuran (3mL) was slowly added. The mixture was stirred for 30 minutes. Then 4-chloro-7-methoxy-1,8-naphthyridine-3-carbaldehyde (1.5g, 1eq.) dissolved in tetrahydrofuran (30mL) was added. The mixture was reacted at rt for 30 minutes. The completion of the reaction was monitored with LCMS. The reaction mixture was quenched with water (20mL), extracted with EA (30mL×2). The organic phases were combined, washed with saturated brine (30mL), dried over anhydrous sodium sulfate, filtered, and concentrated to afford the target compound (1.5g, yield 76%).

LCMS (ESI) [M+H]⁺=293.0; ¹H NMR (400MHz, CDCl₃) δ 9.14 (s, 1H), 8.47 (d, *J*=9.0 Hz, 1H), 8.15 (d, *J*=16.2 Hz, 1H), 7.11 (d, *J*=9.0 Hz, 1H), 6.68 (d, *J*=16.2 Hz, 1H), 4.32 (q, *J*=7.1 Hz, 2H), 4.19 (s, 3H), 1.37 (t, *J*=7.1 Hz, 3H).

### Step 4: Preparation of (E)-3-(4-chloro-7-methoxy-1,8-naphthyridin-3-yl)acrylic acid

Ethyl (E)-3-(4-chloro-7-methoxy-1,8-naphthyridin-3-yl)acrylate (1.5g, 1eq.) was dissolved in tetrahydrofuran (30mL) and water (10mL). LiOH (613.6mg, 5eq.) was added. The mixture was reacted at rt overnight. The completion of the reaction was monitored with LCMS. The reaction mixture was concentrated under reduced pressure to remove tetrahydrofuran. The aqueous phase was adjusted with 1N HCl to a pH of 4. A large amount of solid precipitated. The mixture was filtered. The filter cake was dried to afford the target compound (1.3g, yield 96%). LCMS (ESI) [M+H]⁺=265.3; ¹H NMR (400MHz, DMSO-d₆) δ 12.78 (s, 1H), 9.39 (s, 1H), 8.56 (d, *J*=9.0 Hz, 1H), 7.94 (d, *J*=16.1 Hz, 1H), 7.30 (d, *J*=9.0 Hz, 1H), 6.94 (d, *J*=16.1 Hz, 1H), 4.07 (s, 3H).

### Step 5: Preparation of (E)-N-(4-(benzylthio)-2,6-difluorobenzyl)-3-(4-chloro-7-methoxy-1,8-naphthyridin-3-yl)acrylamide

(E)-3-(4-chloro-7-methoxy-1,8-naphthyridin-3-yl)acrylic acid (1.3g, 1eq.) was dissolved in dimethylformamide (30mL). O-(7-azabenzotriazol-1-yl)-N,N,N',N'-tetramethyluronium hexafluorophosphate (2.24g, 1.2eq.) and ethyldi(propane-2-yl)amine (2.54g, 4eq.) were added. The mixture was reacted at rt for 30 minutes. (4-(benzylthio)-2,6-difluorophenyl)methanamine (1.3g, 1eq.) was added to the reaction mixture and reacted at rt for 1hr. The completion of the reaction was monitored with LCMS. Water (50mL) was added to the reaction mixture. A large amount of solid precipitated. The mixture was filtered. The filter cake was dried to afford the target compound (1.5g, yield 59%).

LCMS (ESI) [M+H]⁺=512.2.

### Step 6: Preparation of N-(4-(benzylthio)-2,6-difluorobenzyl)-3-(4-chloro-7-methoxy-1,8-naphthyridin-3-yl)propanamide

(E)-N-(4-(benzylthio)-2,6-difluorobenzyl)-3-(4-chloro-7-methoxy-1,8-naphthyridin-3-yl)acrylamide (1.5g, 1eq.) was dissolved in tetrahydrofuran (60mL) and water (20mL). 4-toluenesulfonyl hydrazide (5.45g, 10eq.) and sodium

acetate trihydrate (3.99g, 10eq.) were added. The reaction mixture reacted at 80°C for 4hrs. The completion of the reaction was monitored with LCMS. The reaction mixture was cooled to rt. An aqueous sodium bicarbonate solution (60mL) was added. The resulting mixture was extracted with EA (80mL×2). The organic phases were combined, washed with saturated brine, dried over anhydrous sodium sulfate, filtered, and concentrated to afford a crude product, which was purified by column chromatography to afford the target compound (800mg, yield 53%).

LCMS (ESI) [M+H]⁺=514.2.

### Step 7: Preparation of 1-(4-(benzylthio)-2,6-difluorobenzyl)-8-methoxy-3,4-dihydropyrido[2,3-h] [1,6]naphthyridine-2(1H)-one

N-(4-(benzylthio)-2,6-difluorobenzyl)-3-(4-chloro-7-methoxy-1,8-naphthyridin-3-yl)propanamide (750mg, 1eq.) was dissolved in dimethylformamide (10mL). Caesium carbonate (1.43g, 3eq.) was added. The mixture was reacted at 100°C for 4hrs. The completion of the reaction was monitored with LCMS. The reaction mixture was diluted with water (20mL), and extracted with EA (30mL×2). The organic phases were combined, washed with saturated brine (50mL), dried over anhydrous sodium sulfate, filtered, and concentrated to afford the target compound (300mg, yield 43%).

LCMS (ESI) [M+H]⁺=478.2; ¹H NMR (400MHz, CDCl₃) δ 8.70 (s, 1H), 8.19 (d, *J*=9.1 Hz, 1H), 7.33-7.27 (m, 4H), 7.02-6.91 (m, 2H), 6.61 (t, *J*=6.0 Hz, 2H), 5.36 (s, 2H), 4.15 (s, 3H), 4.05 (s, 2H), 2.94-2.91 (m, 2H), 2.71-2.68 (m, 2H).

### Step 8: Preparation of 3,5-difluoro-4-((8-methoxy-2-oxo-3,4-dihydropyrido[2,3-h][1,6]naphthyridine-1(2H)-yl)methyl)benzenesulfonamide

1-(4-(benzylthio)-2,6-difluorobenzyl)-8-methoxy-3,4-dihydropyrido[2,3-h][1,6]naphthyridine-2(1H)-one (250mg, 1eq.) was dissolved in tetrahydrofuran (5mL). Water (132.1mg, 14eq.) and acetic acid (220.1mg, 7eq.) were added. In an ice bath, 1,3-dichloro-5,5-dimethylhydantoin (309.4mg, 3eq.) was added. The mixture was reacted in an ice bath for 10 minutes. In an ice bath, the reaction mixture was slowly added dropwise to the ammonia solution (5mL). The mixture was reacted in an ice bath for 30 minutes. The completion of the reaction was monitored with LCMS. The reaction mixture was extracted with EA (20mL). The organic phase was concentrated under reduced pressure to afford a crude product, which was purified by preparative chromatography to afford the target compound (93mg, yield 41%).

LCMS (ESI) [M+H]⁺=435.1; ¹H NMR (400MHz, MeOD-d₄) δ 8.68 (s, 1H), 8.48 (d, *J*=9.2 Hz, 1H), 7.40-7.33 (m, 2H), 7.09 (d, *J*=9.2 Hz, 1H), 5.50 (s, 2H), 4.10 (s, 3H), 2.99-2.92 (m, 2H), 2.72-2.64 (m, 2H).

### Example 18

### Preparation of tert-butyl 1-(2,6-difluoro-4-sulfamoylbenzyl)-8-methoxy-2-oxo-2,3-dihydropyrazino[2,3-c]quinoline-4(1H)-carboxylate (compound 52):

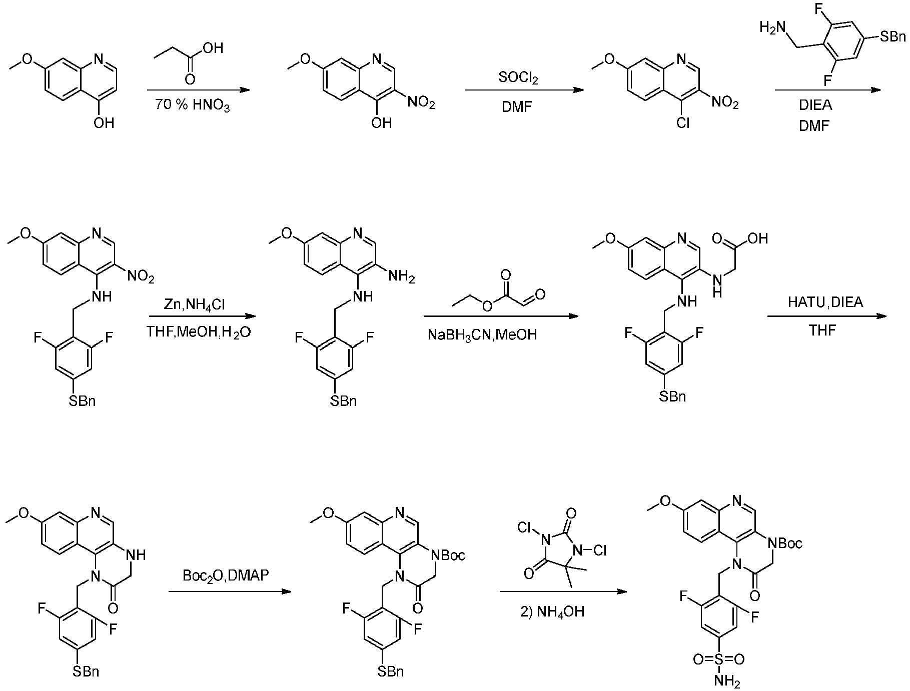

### Step 1: Preparation of 7-methoxy-3-nitroquinolin-4-ol 7-methoxyquinolin-4-ol (1g, 1eq.) was dissolved in propionic acid (10mL). Nitric acid (70%, 1mL) was added. The reaction mixture was stirred at 140°C for 12hrs. The completion of the reaction was monitored with LCMS. The reaction mixture was slowly poured into ice water to precipitate a solid. The mixture was filtered, and the resulting solid was washed with methanol three times, and rotary-evaporated to dryness to afford the target product (800mg, yield 63%).

LCMS (ESI) [M+H]⁺=221.3; ¹H NMR (400MHz, DMSO-d₆) δ 12.77 (s, 1H), 9.14 (s, 1H), 8.29-8.02 (m, 1H), 7.26-7.01 (m, 2H), 3.89 (s, 3H).

### Step 2: Preparation of 4-chloro-7-methoxy-3-nitroquinoline

7-methoxy-3-nitroquinolin-4-ol (800mg, 1eq.) was dissolved in N,N-dimethylformamide (10mL). Sulfurous dichloride (0.66mL, 2.51eq.) was added to the reaction mixture. The resulting mixture was stirred at 25°C for 1hr. The completion of the reaction was monitored with LCMS. The reaction mixture was poured into ice water. The mixture was adjusted with Na2CO3 solution to alkalinity, extracted with EA (20mL), and washed with saturated brine (20mL×3). The organic phases were dried and concentrated to afford the target product (600mg, yield 69%).

LCMS (ESI) [M+H]⁺=239.2; ¹H NMR (400MHz, DMSO-d₆) δ 9.34 (s, 1H), 8.34 (d, *J*=9.1 Hz, 1H), 7.60 (dd, *J*=7.2, 5.7 Hz, 2H), 4.02 (s, 3H).

### Step 3: Preparation of N-(4-(benzylthio)-2,6-difluorobenzyl)-7-methoxy-3-nitroquinolin-4-amine

4-chloro-7-methoxy-3-nitroquinoline (2.6g, 1eq.) was dissolved in N,N-dimethylformamide (30mL). (4-(benzylthio)-2,6-difluorophenyl)methanamine (4.34g, 1.5eq.) and N,N-diisopropylethylamine (4.22g, 3eq.) were successively added. The mixture was reacted at 25°C under stirring for 12hrs. The completion of the reaction was monitored with LCMS. The reaction mixture was filtered. The filter cake was washed with acetonitrile. The resulting solid was dried to afford the target product (2g, yield 39%).

LCMS (ESI) [M+H]⁺=468.2.

### Step 4: Preparation of N⁴-(4-(benzylthio)-2,6-difluorobenzyl)-7-methoxyquinoline-3,4-diamine

N-(4-(benzylthio)-2,6-difluorobenzyl)-7-methoxy-3-nitroquinolin-4-amine (2g, 1eq.) was dissolved in tetrahydrofuran (10mL), methanol (10mL), and water (5mL). NH4Cl (2.29g, 10eq.) and zinc powder (1.4g, 5eq.) was added. The mixture was stirred at 25°C for 1hr. The completion of the reaction was monitored with LCMS. The reaction mixture was filtered. The filtrate was rotary-evaporated to dryness, washed with water, extracted with EA. The organic phase was dried and concentrated to afford the target product (1.5g, crude), which was not further purified but directly used in the next step reaction.

LCMS (ESI) [M+H]⁺=438.2.

### Step 5: Preparation of (4-((4-(benzylthio)-2,6-difluorobenzyl)amino)-7-methoxyquinolin-3-yl)glycine

N⁴-(4-(benzylthio)-2,6-difluorobenzyl)-7-methoxyquinoline-3,4-diamine (1g, crude, obtained from the above step reaction) and ethyl 2-oxoacetate (466.7mg) were dissolved in methanol (10mL). The mixture was stirred for 1hr. Sodium cyanoborohydride (424.0mg) was added. The mixture was reacted at 25°C under stirring for 1hr. The completion of the reaction was monitored with LCMS. The reaction mixture was quenched with water, extracted with dichloromethane (20mL×3). The organic phases were dried and concentrated to afford the target product (1g, crude), which was not further purified but directly used in the next step reaction.

LCMS (ESI) [M+H]⁺=496.3.

### Step 6: Preparation of 1-(4-(benzylthio)-2,6-difluorobenzyl)-8-methoxy-3,4-dihydropyrazino[2,3-c]quinolin-2(1H)-one

(4-((4-(benzylthio)-2,6-difluorobenzyl)amino)-7-methoxyquinolin-3-yl)glycine (1g, crude, obtained from the above step reaction) was dissolved in tetrahydrofuran (10mL). O-(7-azabenzotriazol-1-yl)-N,N,N',N'-tetramethyluronium hexafluorophosphate (1.45g) was added. The mixture was stirred at room temperature for 1hr. N,N-diisopropylethylamine (740.6mg) was added. The resulting mixture was reacted at 25°C under stirring for 1hr. The completion of the reaction was monitored with LCMS. The reaction mixture was washed with water (10mL), and extracted with EA (20mL). The organic phase was dried and concentrated to afford a crude product, which was purified by column chromatography to afford the target compound (600mg).

LCMS (ESI) [M+H]⁺=478.2.

### Step 7: Preparation of tert-butyl 1-(4-(benzylthio)-2,6-difluorobenzyl)-8-methoxy-2-oxo-2,3-dihydropyrazino[2,3-c]quinoline-4(1H)-carboxylate

1-(4-(benzylthio)-2,6-difluorobenzyl)-8-methoxy-3,4-dihydropyrazino[2,3-c]quinolin-2(1H)-one (300mg, 1eq.), di-tert-butyl dicarbonate(5mL) and 4-dimethylaminopyridine (230.25mg, 3eq.) were stirred at 40°C for 1hr. The completion of the reaction was monitored with LCMS. The reaction mixture was concentrated to afford a crude product, which was purified by flash chromatography to afford the target product (270mg, yield 74%).

LCMS (ESI) [M+H]⁺=578.1.

### Step 8: Preparation of tert-butyl 1-(2,6-difluoro-4-sulfamoylbenzyl)-8-methoxy-2-oxo-2,3-dihydropyrazino[2,3-c]quinoline-4(1H)-carboxylate

Tert-butyl 1-(4-(benzylthio)-2,6-difluorobenzyl)-8-methoxy-2-oxo-2,3-dihydropyrazino[2,3-c]quinoline-4(1H)-carboxylate (270mg, 1eq.) was dissolved in tetrahydrofuran (5mL). Glacial acetic acid (196.5mg, 7eq.) and *water* (117.9mg, 14eq.) were added. In an ice bath, 1,3-dichloro-5,5-dimethylhydantoin (276.3mg, 3eq.) was added. The mixture was stirred at 0°C for 6 minutes. The completion of the reaction was monitored with LCMS. The reaction mixture was added to the ammonia solution (6mL). The resulting mixture was stirred for 6 minutes. The completion of the reaction was monitored with LCMS. The reaction mixture was adjusted with 1M HCl to neutral pH, and extracted with EA (5mL×2). The aqueous phase was concentrated to precipitate a solid. The solid was separated by filtering. The filtrate was purified by preparative HPLC to afford the target compound (22.4mg, yield 9%).

LCMS (ESI) [M+H]⁺=534.9; ¹H NMR (400MHz, MeOD-d₄) δ 8.92 (s, 1H), 8.13 (d, *J*=9.3 Hz, 1H), 7.37 (d, *J*=2.3 Hz, 1H), 7.34 (d, *J*=9.3 Hz, 1H), 7.27 (d, *J*=7.2 Hz, 2H), 5.59 (s, 2H), 4.36 (s, 2H), 3.96 (s, 3H), 1.54 (s, 9H).

### Example 19

### Preparation of 3,5 -difluoro-4-((8-methoxy-2-oxo-3,4-dihydropyrazino [2,3-c]quinolin-1(2H)-yl)methyl)benzenesulfonamide (compound 51):

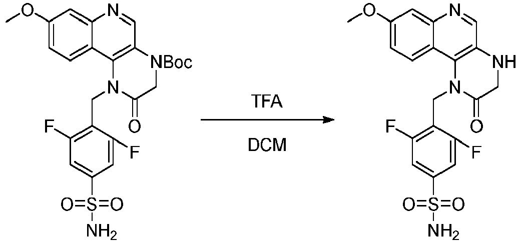

Tert-butyl 1-(2,6-difluoro-4-sulfamoylbenzyl)-8-methoxy-2-oxo-2,3-dihydropyrazino [2,3-c] quinoline-4(1H)-carboxylate (8mg, 1eq.) was dissolved in dichloromethane (1mL). Trifluoroacetic acid (1mL) was added. The reaction mixture was stirred at rt for 1hr. The completion of the reaction was monitored with LCMS. reaction mixture was concentrated to afford a crude product, which was purified by preparative HPLC to afford the target compound (1.4mg, yield 21%).

LCMS (ESI) [M+H]⁺=434.9; ¹H NMR (400MHz, MeOD-d₄) δ 8.54 (s, 1H), 8.12 (d, *J*=9.5 Hz, 1H), 7.39-7.32 (m, 4H), 5.55 (s, 2H), 3.98 (s, 3H), 3.85 (s, 2H).

### Example 20

### Preparation of 4-((8-ethoxy-2-oxopyrido[2,3-h][1,6]naphthyridine-1(2H)-yl)methyl)-3,5-difluorobenzenesulfonamide (compound 63):

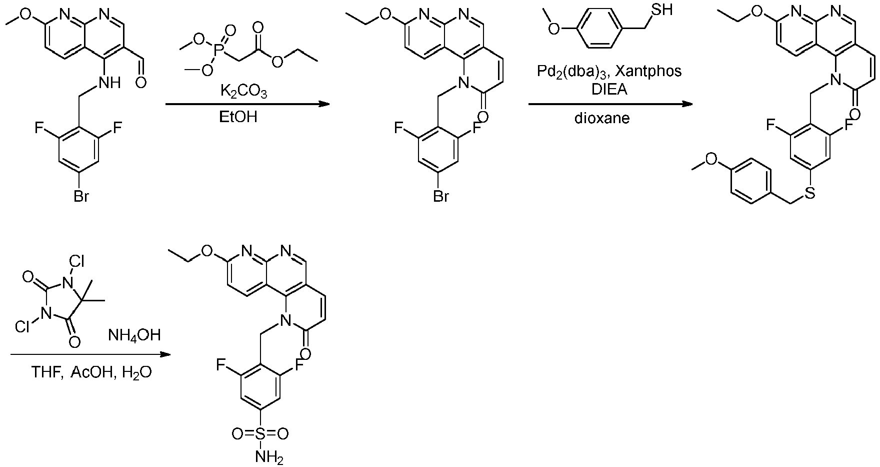

### Step 1: Preparation of 1-(4-bromo-2,6-difluorobenzyl)-8-ethoxypyrido[2,3-h][1,6]naphthyridine-2(1H)-one

4-((4-bromo-2,6-difluorobenzyl)amino)-7-methoxy-1,8-naphthyridine-3-carbaldehyde (120mg, 1eq.) was dissolved in ethanol (15mL). 2-(dimethoxyphosphoryl)ethyl acetate (198mg, 3.4eq.) and K₂CO₃ (203mg, 5eq.) were added. The mixture was reacted at 85°C for 36hrs. The completion of the reaction was monitored with LCMS. The reaction mixture was concentrated to dryness, and purified by flash silica gel column chromatography to afford the target product (100mg, yield: 76%).

LCMS (ESI) [M+H]⁺=446.0.

### Step 2: Preparation of 1-(2,6-difluoro-4-((4-methoxybenzyl)thio)benzyl)-8-ethoxypyrido[2,3-h][1,6]naphthyridine-2(1H)-one

1-(4-bromo-2,6-difluorobenzyl)-8-ethoxypyrido[2,3-h][1,6]naphthyridine-2(1H)-one (90mg, 1eq.) was dissolved in dioxane (5mL). (4-methoxyphenyl)methanethiol (62mg, 2eq.), 4,5-bis(diphenylphosphino)-9,9-dimethylxanthene (23mg, 0.2eq.), tris(dibenzylideneacetone)dipalladium (37mg, 0.2eq.) and N,N-diisopropylethylamine (78mg, 3eq.) were added. The mixture was reacted under the protection of nitrogen gas at 90°C for 2hrs. The completion of the reaction was monitored with LCMS. The reaction mixture was concentrated to dryness, and purified by flash silica gel column chromatography to afford the target product (70mg, yield: 66%).

LCMS (ESI) [M+H]⁺=520.1.

### Step 3: Preparation of 4-((8-ethoxy-2-oxopyrido[2,3-h][1,6]naphthyridine-1(2H)-yl)methyl)-3,5-difluorobenzenesulfonamide

1-(2,6-difluoro-4-((4-methoxybenzyl)thio)benzyl)-8-ethoxypyrido[2,3-h][1,6]naphthyridine-2(1H)-one (65mg, 1eq.), acetic acid (53mg, 7eq.), water (32mg, 14eq.) were dissolved in tetrahydrofuran (5mL). 1,3-dichloro-5,5-dimethylhydantoin (74mg, 3eq.) was added at 0°C. The mixture was reacted at 0°C for 1hr, followed by adding ammonia solution (0.5mL), then reacted at 0°C for 10 minutes. The completion of the reaction was monitored with LCMS. The reaction mixture was concentrated to dryness, and purified by preparative HPLC to afford the target compound (2.6mg, yield: 4.7%).

LCMS (ESI) [M+H]⁺=447.1; ¹H NMR (400MHz, DMSO-*d₆*) *δ* 9.15 (s, 1H), 8.71 (d, *J*=9.6 Hz, 1H), 8.20 (d, *J*=9.2 Hz, 1H), 7.62 (s, 2H), 7.43 (d, *J*=7.2 Hz, 2H), 7.09 (d, *J*=9.6 Hz, 1H), 6.69 (d, *J*=9.6 Hz, 1H), 5.77 (s, 2H), 4.53 (q, *J*=7.1 Hz, 2H), 1.41 (t, *J*=7.2 Hz, 3H).

### Example 21

### Preparation of 3,5-difluoro-4-((8-methoxy-2-oxo-3,4-dihydropyrazino[2,3-c][1,8]naphthyridine-1(2H)-yl)methyl)benzenesulfonamide (compound 49):

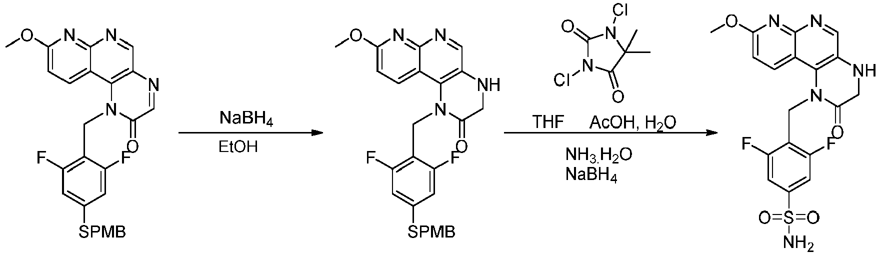

### Step 1: Preparation of 1-(2,6-difluoro-4-((4-methoxybenzyl)thio)benzyl)-8-methoxy-3,4-dihydropyrazino[2,3-c][1,8]naphthyridine-2(1H)-one

1-(2,6-difluoro-4-((4-methoxybenzyl)thio)benzyl)-8-methoxypyrazino[2,3-c][1,8]naphthyridine-2(1H)-one (50mg, 1eq.) was dissolved in ethanol (5mL). Then sodium borohydride (11mg, 3eq.) was added. The mixture was reacted at 25°C under stirring for 2hrs. The completion of the reaction was monitored with LCMS. The reaction mixture was diluted with water (10mL), and extracted with EA (50mL×3). The organic phases were washed with saturated brine (10mL), dried over anhydrous sodium sulfate, filtered, and concentrated to afford a crude product (50mg), which was not further purified but directly used in the next step reaction.

LCMS (ESI) [M+H]⁺=509.1.

### Step 2: Preparation of 3,5-difluoro-4-((8-methoxy-2-oxo-3,4-dihydropyrazino[2,3-c][1,8]naphthyridine-1(2H)-yl)methyl)benzenesulfonamide

To a 25mL round-bottom flask were added 1-(2,6-difluoro-4-((4-methoxybenzyl)thio)benzyl)-8-methoxy-3,4-dihydropyrazino[2,3-c][1,8]naphthyridine-2(1H)-one (20mg, crude, obtained from the above step reaction), tetrahydrofuran (10mL), acetic acid (10mg), water (10mL). While the solution was cooled in ice water, 1,3-dichloro-5,5-dimethylhydantoin (23mg) was added. The resulting mixture was reacted under stirring for 60 minutes, followed by adding ammonia solution (1mL). The stirring continued for 60 minutes. Sodium borohydride (15mg, 10eq.) was added. After the completion of the reaction, the reaction mixture was washed with water and extracted with EA. The EA layer was dried, and concentrated to afford a crude product, which was purified by liquid chromatography to afford the target compound (2.1mg).

LCMS (ESI) [M+H]⁺=436.1; ¹HNMR (400MHz, DMSO-*d₆*) *δ* 8.55 (s, 1H), 8.44 (d, *J*=9.2 Hz, 1H), 7.32 (d, *J*=6.8 Hz, 2H), 7.02 (d, *J*=9.2 Hz, 1H), 6.44 (s, 1H), 5.32 (s, 2H), 3.96 (s, 3H), 3.70 (s, 2H).

### Example 22

### Preparation of 4-((8,9-dimethoxy-2-oxo-2H-[1,3]oxazino[5,4-c]quinolin-1(4H)-yl)methyl)-3,5-difluorobenzenesulfonamide (compound 31):

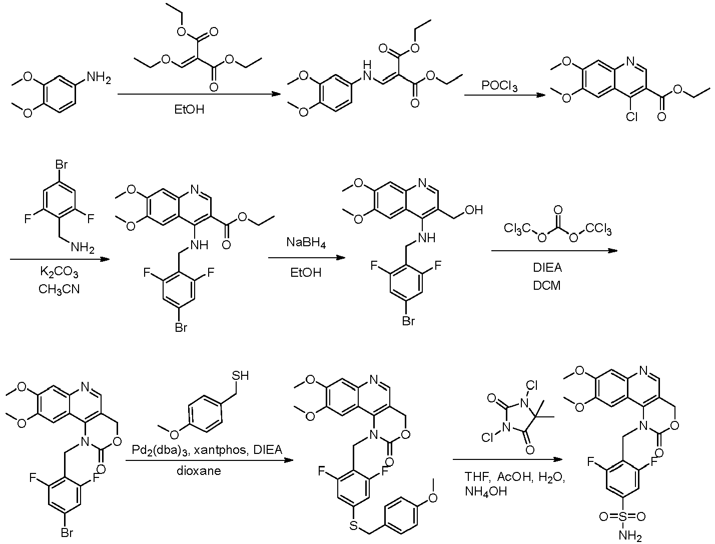

### Step 1: Preparation of 2-(((3,4-dimethoxyphenyl)amino)methylene)malonate diethyl

3,4-dimethoxyaniline (7g, 1eq.) and *1,3-diethyl 2-(ethoxymethylene)malonate* (10g, 1.01eq.) *were added to* ethanol (200mL). The mixture was stirred at rt for 4hrs. After the completion of the reaction, the reaction mixture was directly distilled under reduced pressure to remove the solvent to afford a crude target compound (18g), which was not further purified but directly used in the next step reaction.

LCMS (ESI) [M+H]⁺=324.1.

### Step 2: Preparation of ethyl 4-chloro-6,7-dimethoxyquinoline-3-carboxylate

2-(((3,4-dimethoxyphenyl)amino)methylene)malonate diethyl (18g, crude, obtained from the above step reaction) and phosphorus oxychloride (8mL) were placed in a flask (100mL) and warmed to 100°C. The mixture was reacted under stirring for 5hrs. LCMS monitoring indicated the completion of the reaction, the mixture was cooled to rt and slowly added dropwise to a flask containing 200mL of water. After the completion of the addition, the mixture was cooled, gradually adjusted with an aqueous sodium bicarbonate solution to neutrality, and extracted with EA (200mL×2). The organic phases were combined, washed with brine, was concentrated in vacuum to afford a crude target compound (16g), which was not further purified but directly used in the next step reaction.

LCMS (ESI) [M+H]⁺=296.1.

### Step 3: Preparation of ethyl 4-((4-bromo-2,6-difluorobenzyl)amino)-6,7-dimethoxyquinoline-3-carboxylate

Ethyl 4-chloro-6,7-dimethoxyquinoline-3-carboxylate (1g, crude, obtained from the above step reaction) was dissolved in acetonitrile (20mL). Then K₂CO₃ (1g) and (4-bromo-2,6-difluorophenyl)methanamine (0.79g) were added. The mixture was reacted under the protection of nitrogen gas at 100°C under refluxing for 20hrs. LCMS monitoring indicated the completion of the reaction, the reaction mixture was rotary-evaporated to dryness, and diluted with EA. The organic phase was washed with water, dried over anhydrous sodium sulfate, and rotary-evaporated to dryness to afford a crude product, which was purified by flash chromatography to afford the target compound (0.68g).

LCMS (ESI) [M+H]⁺=481.0.

### Step 4: Preparation of (4-((4-bromo-2,6-difluorobenzyl)amino)-6,7-dimethoxyquinolin-3-yl)methanol

Ethyl 4-((4-bromo-2,6-difluorobenzyl)amino)-6,7-dimethoxyquinoline-3-carboxylate (150mg, 1eq.) was dissolved in ethanol (15mL). Sodium borohydride (60mg, 5eq.) was added. The mixture was reacted at 25°C for 16hrs. The completion of the reaction was monitored with LCMS. The reaction mixture was poured into water, extracted with EA. The organic phase was dried over anhydrous sodium sulfate, concentrated to dryness, and purified by silica gel column chromatography to afford the target compound (100mg, yield: 72%).

LCMS (ESI) [M+H]⁺=439.0.

### Step 5: Preparation of 1-(4-bromo-2,6-difluorobenzyl)-8,9-dimethoxy-1,4-dihydro-2H-[1,3]oxazino[5,4-c]quinolin-2-one

(4-((4-bromo-2,6-difluorobenzyl)amino)-6,7-dimethoxyquinolin-3-yl)methanol (100mg, 1eq.) was dissolved in dichloromethane (10mL). Triphosgene (219mg, 3.2eq.) and N,N-diisopropylethylamine (159mg, 5.4eq.) were added. The mixture was reacted at 25°C for 1hr. The completion of the reaction was monitored with LCMS. The reaction mixture was concentrated to dryness, and purified by silica gel column chromatography to afford the target compound (85mg, yield: 80%).

LCMS (ESI) [M+H]⁺=465.0.

### Step 6: Preparation of 1-(2,6-difluoro-4-((4-methoxybenzyl)thio)benzyl)-8,9-dimethoxy-1,4-dihydro-2H-[1,3]oxazino [5,4-c] quinolin-2-one

1-(4-bromo-2,6-difluorobenzyl)-8,9-dimethoxy-1,4-dihydro-2H-[1,3]oxazino[5,4-c]quinolin-2-one (85mg, 1eq.) was dissolved in dioxane (10mL). (4-methoxyphenyl)methanethiol (55mg, 2eq.), tris(dibenzylideneacetone)dipalladium (18mg, 0.1eq.), 4,5-bis(diphenylphosphino)-9,9-dimethylxanthene (23mg, 0.2eq.) and N,N-diisopropylethylamine (71mg, 3eq.) were added. The mixture was reacted at 90°C for 2hrs. The completion of the reaction was monitored with LCMS. The reaction mixture was concentrated to dryness, and purified by silica gel column chromatography to afford the target compound (50mg, yield: 51%).

LCMS (ESI) [M+H]⁺=539.2.

### Step 7: Preparation of 4-((8,9-dimethoxy-2-oxo-2H-[1,3]oxazino[5,4-c]quinolin-1(4H)-yl)methyl)-3,5-difluorobenzenesulfonamide

1-(2,6-difluoro-4-((4-methoxybenzyl)thio)benzyl)-8,9-dimethoxy-1,4-dihydro-2H-[1,3]oxazino[5,4-c]quinolin-2-one (50mg, 1.0eq.), acetic acid (28.0mg, 5eq.), water (24mL) were dissolved in tetrahydrofuran (15mL). 1,3-dichloro-5,5-dimethylhydantoin (19mg, 1eq.) was added at 0°C. The mixture was reacted at 0°C for 2hrs, followed by adding ammonia solution (0.5mL), then reacted at 0°C for 10 minutes. The completion of the reaction was monitored with LCMS. The reaction mixture was concentrated to dryness, and purified by preparative HPLC to afford the target compound (2.7mg, yield: 6%).

LCMS (ESI) [M+H] ⁺=466.0; ¹H NMR (400MHz, CD₃OD) *δ* 8.69 (s, 1H), 7.52 (s, 1H), 7.50-7.43 (m, 2H), 7.25 (d, *J*=8.8 Hz, 1H), 6.87 (d, *J*=8.8 Hz, 1H), 5.71 (s, 2H), 5.34 (s, 2H), 4.07 (s, 3H), 4.02 (s, 3H).

### Example 23

### Preparation of 3,5-difluoro-4-((8-methoxy-2-oxo-2H-[1,3]oxazino[5,4-c]quinolin-1(4H)-yl)methyl)benzenesulfonamide (compound 30):

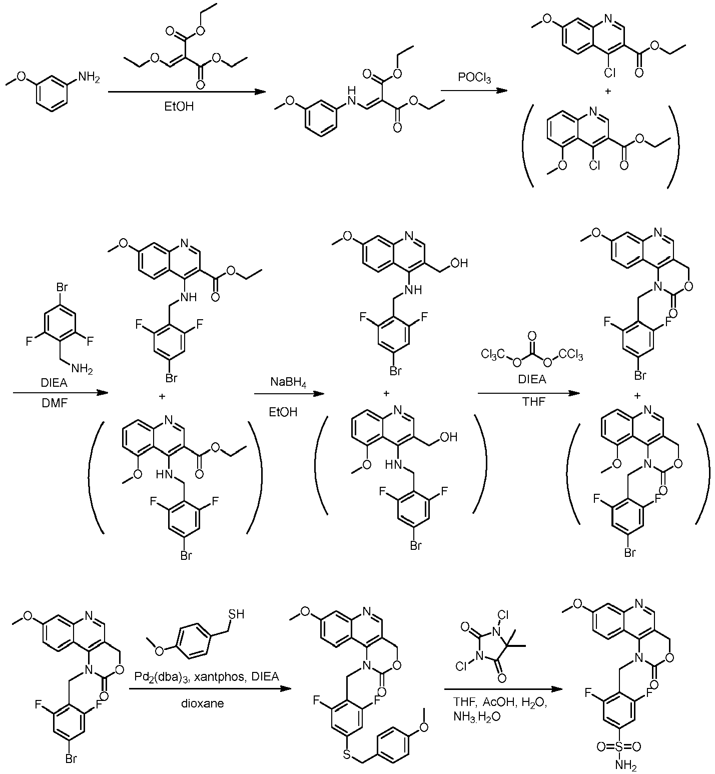

### Step 1: Preparation of diethyl 2-((3-methoxyphenyl)amino)methylene)malonate

3-methoxyaniline (10.0g, 1eq.) was dissolved in ethanol (100mL). Diethyl 2-(ethoxymethylene)malonate (21g, 1.2eq.) was added. The mixture was reacted at 90°C for 16hrs. The completion of the reaction was monitored with LCMS. The reaction mixture was concentrated under reduced pressure to dryness to afford a crude target compound (20g), which was not further purified but directly used in the next step reaction.

LCMS (ESI) [M+H]⁺=294.1.

### Step 2: Preparation of ethyl 4-chloro-7-methoxyquinoline-3-carboxylate :

diethyl 2-((3-methoxyphenyl)amino)methylene)malonate (5g, crude, obtained from the above step reaction) was dissolved in phosphorus oxychloride (15mL). The mixture was reacted at 110°C for 4hrs. The completion of the reaction was monitored with LCMS. The reaction mixture was concentrated under reduced pressure to dryness to afford a mixture of ethyl 4-chloro-7-methoxyquinoline-3-carboxylate and ethyl 4-chloro-5-methoxyquinoline-3-carboxylate (3.8g), which was not further purified but directly used in the next step reaction.

LCMS (ESI) [M+H]⁺=266.0.

### Step 3: Preparation of ethyl 4-((4-bromo-2,6-difluorobenzyl)amino)-7-methoxyquinoline-3-carboxylate

The mixture of ethyl 4-chloro-7-methoxyquinoline-3-carboxylate and ethyl 4-chloro-5-methoxyquinoline-3-carboxylate (2g, the mixture obtained from the above step reaction) was dissolved in N,N-dimethylformamide (10mL). 1-(4-bromo-2,6-difluorophenyl)methanamine (1.84g) and N,N-diisopropylethylamine (2.92g, 3eq.) were added. The mixture was reacted at 50°C for 16hrs. The completion of the reaction was monitored with LCMS. The reaction mixture was cooled to rt, poured into water, and extracted with EA. The organic phase was combined, dried over anhydrous sodium sulfate, and filtered. The filtrate was concentrated, and purified by silica gel column chromatography to afford a mixture of ethyl 4-((4-bromo-2,6-difluorobenzyl)amino)-7-methoxyquinoline-3-carboxylate and ethyl 4-((4-bromo-2,6-difluorobenzyl)amino)-5-methoxyquinoline-3-carboxylate (1.6g).

LCMS (ESI) [M+H]⁺=451.0.

### Step 4: Preparation of (4-((4-bromo-2,6-difluorobenzyl)amino)-7-methoxyquinolin-3-yl)methanol

The mixture of ethyl 4-((4-bromo-2,6-difluorobenzyl)amino)-7-methoxyquinoline-3-carboxylate and ethyl 4-((4-bromo-2,6-difluorobenzyl)amino)-S-methoxyquinoline-3-carboxylate (1.6g, the mixture obtained from the above step reaction) was dissolved in ethanol (50mL). Sodium borohydride (671mg, 5.0eq.) was added. The mixture was reacted at 50°C for 16hrs. The completion of the reaction was monitored with LCMS. The reaction mixture was cooled to rt, quenched with water, extracted with EA. The organic phase was dried over anhydrous sodium sulfate, and filtered. The filtrate was concentrated, and purified by silica gel column chromatography to afford a mixture of (4-((4-bromo-2,6-difluorobenzyl)amino)-7-methoxyquinolin-3-yl)methanol and (4-((4-bromo-2,6-difluorobenzyl)amino)-5-methoxyquinolin-3-yl)methanol (400mg).

LCMS (ESI) [M+H]⁺=409.1.

### Step 5: Preparation of 1-(4-bromo-2,6-difluorobenzyl)-8-methoxy-1,4-dihydro-2H-[1,3]oxazino[5,4-c]quinolin-2-one

The mixture of ((4-((4-bromo-2,6-difluorobenzyl)amino)-7-methoxyquinolin-3-yl)methanol and (4-((4-bromo-2,6-difluorobenzyl)amino)-5-methoxyquinolin-3-yl)methanol (400mg, the mixture obtained from the above step reaction) was dissolved in dichloromethane (10mL). Triphosgene (870mg, 3eq.) and N,N-diisopropylethylamine (632mg, 5eq.) were added. The mixture was reacted at 25°C for 2hrs. The completion of the reaction was monitored with LCMS. The reaction mixture was quenched with water, extracted with EA. The organic phase was dried over anhydrous sodium sulfate and filtered. The filtrate was concentrated, purified by silica gel column chromatography to afford 1-(4-bromo-2,6-difluorobenzyl)-8-methoxy-1,4-dihydro-2H-[1,3]oxazino[5,4-c]quinolin-2-one (70mg) and 1-(4-bromo-2,6-difluorobenzyl)-10-methoxy-1,4-dihydro-2H-[1,3]oxazino[5,4-c]quinolin-2-one (60mg).

LCMS (ESI) [M+H]⁺=435.1.

### Step 6: Preparation of 1-(2,6-difluoro-4-((4-methoxybenzyl)thio)benzyl)-8-methoxy-1,4-dihydro-2H-[1,3]oxazino [5,4-c] quinolin-2-one

1-(4-bromo-2,6-difluorobenzyl)-8-methoxy-1,4-dihydro-2H-[1,3]oxazino[5,4-c]quinolin-2-one (65mg, 1eq.) was dissolved in dioxane (5mL). (4-methoxyphenyl)methanethiol (46mg, 2eq.), 4,5-bis(diphenylphosphino)-9,9-dimethylxanthene (17mg, 0.2eq.), tris(dibenzylideneacetone)dipalladium (27mg, 0.2eq.) and N,N-diisopropylethylamine (58mg, 3eq.) were added. The mixture was reacted at 90°C for 2hrs. The completion of the reaction was monitored with LCMS. The reaction mixture was concentrated under reduced pressure, and purified by silica gel column chromatography to afford the target compound (60mg, yield 79%).

LCMS (ESI) [M+H]⁺=509.1.

### Step 7: Preparation of 3,5-difluoro-4-((8-methoxy-2-oxo-2H-[1,3]oxazino[5,4-c]quinolin-1(4H)-yl)methyl)benzenesulfonamide

A mixture of 1-(2,6-difluoro-4-((4-methoxybenzyl)thio)benzyl)-8-methoxy-1,4-dihydro-2H-[1,3]oxazino[5,4-c]quinolin-2-one (30mg, 1.0eq.), acetic acid (25mg, 7.0eq.), and water (15mL) was dissolved in tetrahydrofuran (5mL). 1,3-dichloro-5,5-dimethylhydantoin (35mg, 3eq.) was added at 0°C. The mixture was reacted at 0°C for 1hr, followed by adding ammonia solution (0.5mL), then reacted at 0°C for 10 minutes. The completion of the reaction was monitored with LCMS. The reaction mixture was concentrated under reduced pressure, and purified by preparative HPLC to afford the target compound (5.8mg, yield 22%).

LCMS (ESI) [M+H]⁺=436.0; ¹H NMR (400MHz, DMSO) δ 8.72 (s, 1H), 8.22 (d, J=9.6 Hz, 1H), 7.62 (s, 2H), 7.49-7.43 (m, 3H), 7.29 (dd, J=9.2, 2.4 Hz, 1H), 5.50 (s, 2H), 5.27 (s, 2H), 3.94 (s, 3H).

### Example 24

### Preparation of 3,5-difluoro-4-((10-methoxy-2-oxo-2H-[1,3]oxazino[5,4-c]quinolin-1(4H)-yl)methyl)benzenesulfonamide (compound 68):

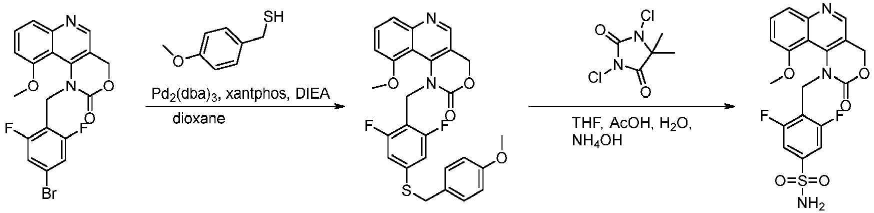

### Step 1: Preparation of 1-(2,6-difluoro-4-((4-methoxybenzyl)thio)benzyl)-10-methoxy-1,4-dihydro-2H-[1,3]oxazino[5,4-c]quinolin-2-one

1-(4-bromo-2,6-difluorobenzyl)-10-methoxy-1,4-dihydro-2H-[1,3]oxazino[5,4-c]quinolin-2-one (60mg, 1eq.) was dissolved in dioxane (5mL). (4-methoxyphenyl)methanethiol (43mg, 2eq.), 4,5-bis(diphenylphosphino)-9,9-dimethylxanthene (16mg, 0.2eq.), tris(dibenzylideneacetone)dipalladium (25mg, 0.2eq.) and N,N-diisopropylethylamine (53mg, 3eq.) were added. The mixture was reacted at 90°C for 2hrs. The completion of the reaction was monitored with LCMS. The reaction mixture was cooled to rt, concentrated under reduced pressure, and purified by silica gel column chromatography to afford the target compound (50mg), which was not further purified but directly used in the next step reaction.

LCMS (ESI) [M+H]⁺=509.1.

### Step 2: Preparation of 3,5-difluoro-4-((10-methoxy-2-oxo-2H-[1,3]oxazino[5,4-c]quinolin-1(4H)-yl)methyl)benzenesulfonamide

A mixture of 1-(2,6-difluoro-4-((4-methoxybenzyl)thio)benzyl)-10-methoxy-1,4-dihydro-2H-[1,3]oxazino[5,4-c]quinolin-2-one (30mg, 1.0eq.), acetic acid (25mg, 7.1eq.), and water (15mg, 14.1eq.) was dissolved in tetrahydrofuran (5mL). 1,3-dichloro-5,5-dimethylhydantoin (35mg, 3eq.) was added at 0°C. The mixture was reacted at 0°C for 1hr, followed by adding ammonia solution (0.2mL), then reacted at 0°C for 10 minutes. The completion of the reaction was monitored with LCMS. The reaction mixture was concentrated under reduced pressure, and purified by preparative HPLC to afford the target compound (2.0mg, yield: 8%).

LCMS (ESI) [M+H]⁺=436.0; ¹H NMR (400MHz, DMSO) δ 8.74 (s, 1H), 7.73 (t, J=8.0 Hz, 1H), 7.64 (d, J=8.0 Hz, 1H), 7.60 (s, 2H), 7.41 (d, J=6.4 Hz, 2H), 7.20 (d, J=8.0 Hz, 1H), 5.21 (s, 2H), 5.08 (s, 2H), 4.05 (s, 3H).

### Example 25

### Preparation of 6-((8-methoxy-2-oxo-2H-[1,3]oxazino[5,4-c][1,8]naphthyridin-1(4H)-yl)methyl)pyridine-3-sulfonamide (compound 70):

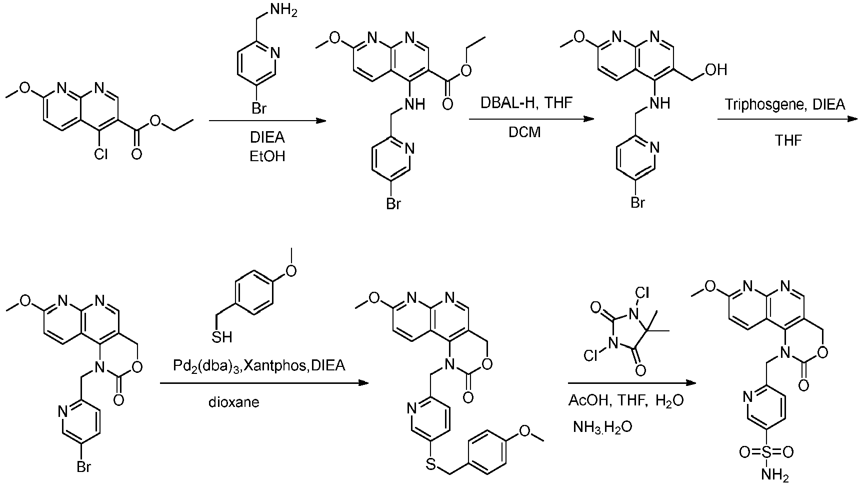

### Step 1: Preparation of ethyl 4-(((5-bromopyridin-2-yl)methyl)amino)-7-methoxy-1,8-naphthyridine-3-carboxylate

Ethyl 4-chloro-7-methoxy-1,8-naphthyridine-3-carboxylate (200mg, 1eq.) and (5-bromopyridin-2-yl)methanamine (210.4mg, 1.5e.q) and N,N-diisopropylethylamine (289.0mg, 3eq.) were dissolved in ethanol (50mL). The mixture was heated to 90°C and reacted for 16hrs. The completion of the reaction was monitored with LCMS. The reaction mixture was concentrated under reduced pressure, and purified by flash silica gel column chromatography to afford the target compound (150mg, yield: 48%).

LCMS (ESI) [M+H]⁺=417.1.

### Step 2: Preparation of (4-(((5-bromopyridin-2-yl)methyl)amino)-7-methoxy-1,8-naphthyridin-3-yl)methanol

Ethyl 4-(((5-bromopyridin-2-yl)methyl)amino)-7-methoxy-1,8-naphthyridine-3-carboxylate (150mg, 1eq.) was added to dichloromethane (20mL). A solution of diisobutylaluminium hydride in tetrahydrofuran (1mol/L, 7.2mL, 20eq.) was added at -78°C. The mixture was reacted at -78°C for 2hrs. The completion of the reaction was monitored with LCMS. The reaction mixture was quenched with water, extracted with dichloromethane (100mL×2). The organic phases were combined, dried over anhydrous sodium sulfate, and filtered. The filtrate was concentrated, and purified by flash chromatography to afford the target compound (100mg, yield: 74%).

LCMS (ESI) [M+H]⁺=377.1.

### Step 3: Preparation of 1-((5-bromopyridin-2-yl)methyl)-8-methoxy-1,4-dihydro-2H-[1,3]oxazino[5,4-c][1,8]naphthyridin-2-one

(4-(((5-bromopyridin-2-yl)methyl)amino)-7-methoxy-1,8-naphthyridin-3-yl)methanol (100mg, 1eq.) was added to tetrahydrofuran (20mL). N,N-diisopropylethylamine (335.4mg, 9.74eq.) was added. Triphosgene (154.3mg, 1.95eq.) was added. The mixture was reacted at rt for 1hr. The completion of the reaction was monitored with LCMS. The reaction mixture was quenched with water (20mL), extracted with EA (50mL×3). The organic phases were combined, dried over anhydrous sodium sulfate, and filtered. The filtrate was concentrated, and purified by column chromatography to afford the target compound (60mg, yield: 56%).

LCMS (ESI) [M+H]⁺=401.2.

### Step 4: Preparation of 8-methoxy-1-((5-((4-methoxybenzyl)thio)pyridin-2-yl)methyl)-1,4-dihydro-2H-[1,3]oxazino[5,4-c][1,8]naphthyridin-2-one

1-((5-bromopyridin-2-yl)methyl)-8-methoxy-1,4-dihydro-2H-[1,3]oxazino[5,4-c][1,8]naphthyridin-2-one (60mg, 1eq.), (4-methoxyphenyl)methanethiol (46.2mg, 2eq.), tris(dibenzylideneacetone)dipalladium (6mg, 0.04eq.), 4,5-bis(diphenylphosphino)-9,9-dimethylxanthene (17.4mg, 0.2eq.) andN,N-diisopropylethylamine (97.5mg, 5eq.) were added to 1,4-dioxane (10mL). The atmosphere was replaced with nitrogen gas three times. The mixture was reacted at 90°C for 3hrs. The completion of the reaction was monitored with LCMS. The reaction mixture was cooled to rt, then diluted with water (20mL), and extracted with EA. The organic phase was combined, dried over anhydrous sodium sulfate, and filtered. The filtrate was concentrated, and purified by column chromatography to afford the target compound (50mg, yield: 70%).

LCMS (ESI) [M+H]⁺=475.1.

### Step 5: Preparation of 6-((8-methoxy-2-oxo-2H-[1,3]oxazino[5,4-c][1,8]naphthyridin-1(4H)-yl)methyl)pyridine-3-sulfonamide

To a 25mL round-bottom flask were added 8-methoxy-1-((5-((4-methoxybenzyl)thio)pyridin-2-yl)methyl)-1,4-dihydro-2H-[1,3]oxazino[5,4-c][1,8]naphthyridin-2-one (50mg, 1.0eq.), tetrahydrofuran (10mL), acetic acid (8.0mg, 1.3eq.), and water (4.0mg, 2.1eq.). 1,3-dichloro-5,5-dimethylhydantoin (43.1mg, 2.1eq.) was added at 0°C. The mixture was reacted for 1hr. The completion of the reaction was monitored with LCMS. Ammonia solution (1mL) was added. The mixture was reacted at rt for 1hr. After the completion of the reaction, the reaction mixture was concentrated under reduced pressure, and purified by analytic preparative HPLC to afford the target compound (3.8mg, yield: 9%).

LCMS (ESI) [M+H]⁺=402.1; ¹H NMR (400MHz, DMSO-d6) δ 8.88 (d, J=2.2 Hz, 1H), 8.79 (s, 1H), 8.42 (d, J=9.2 Hz, 1H), 8.15 (m, J=8.2,1H), 7.66 (d, J=8.4 Hz, 1H), 7.59 (s, 2H), 7.07 (d, J=9.2 Hz, 1H), 5.55 (s, 2H), 5.51 (s, 2H), 4.00 (s, 3H).

### Example 26

### Preparation of 3-fluoro-4-((8-methoxy-2-oxo-2H-[1,3]oxazino[5,4-c][1,8]naphthyridin-1(4H)-yl)methyl)benzenesulfonamide (compound 71):

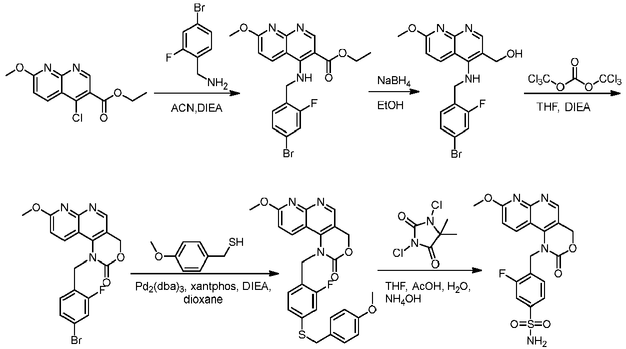

### Step 1: Preparation of ethyl 4-((4-bromo-2-fluorobenzyl)amino)-7-methoxy-1,8-naphthyridine-3-carboxylate

Ethyl 4-chloro-7-methoxy-1,8-naphthyridine-3-carboxylate (500mg, 1eq.), 1-(4-bromo-2-fluorophenyl)methanamine (381mg, 1eq.) and N,N-diisopropylethylamine (2.45g, 10.1eq.) were dissolved in acetonitrile (20mL). The mixture was warmed to 70°C and reacted for 16hrs. The completion of the reaction was monitored with LCMS. The reaction mixture was cooled to rt, diluted with water (20mL), extracted with EA (50mL×3). The organic phases were combined, concentrated under reduced pressure, and purified by column chromatography to afford the target compound (500mg, yield: 61%).

LCMS (ESI) [M+H]⁺=434.0.

### Step 2: Preparation of (4-((4-bromo-2-fluorobenzyl)amino)-7-methoxy-1,8-naphthyridin-3-yl)methanol

Ethyl 4-((4-bromo-2-fluorobenzyl)amino)-7-methoxy-1,8-naphthyridine-3-carboxylate (250mg, 1.0eq.) was dissolved in ethanol (20mL). Then sodium borohydride (211mg, 9.7eq.) was added. The mixture was reacted at 60°C for 16hrs. The completion of the reaction was monitored with LCMS. The reaction mixture was cooled to rt, quenched with water, and distilled under reduced pressure to remove ethanol. Water (50mL) was added to the residue. The mixture was extracted with EA (100mL×2). The organic phases were combined, and washed with brine five times, dried over anhydrous sodium sulfate, and filtered. The filtrate was concentrated in vacuum, and purified by flash silica gel purified by column chromatography to afford the target compound (100mg, yield: 44%).

LCMS (ESI) [M+H]⁺=392.0.

### Step 3: Preparation of 1-(4-bromo-2-fluorobenzyl)-8-methoxy-1,4-dihydro-2H-[1,3]oxazino[5,4-c][1,8]naphthyridin-2-one

(4-((4-bromo-2-fluorobenzyl)amino)-7-methoxy-l,8-naphthyridin-3-yl)methanol (100mg, 1eq.) was added to tetrahydrofuran (10mL). N,N-diisopropylethylamine (329mg, 10eq.) was added. Triphosgene (378mg, 5eq.) was added. The mixture was reacted at rt for 1hr. The completion of the reaction was monitored with LCMS. The reaction mixture was quenched with water (10mL), and extracted with EA (20mL×3). The organic phases were combined, concentrated under reduced pressure, and purified by column chromatography to afford the target compound (35mg, yield: 32%).

LCMS (ESI) [M+H]⁺=418.0.

### Step 4: Preparation of 1-(2-fluoro-4-((4-methoxybenzyl)thio)benzyl)-8-methoxy-1,4-dihydro-2H-[1,3]oxazino[5,4-c][1,8]naphthyridin-2-one

1-(4-bromo-2-fluorobenzyl)-8-methoxy-1,4-dihydro-2H-[1,3]oxazino[5,4-c][1,8]naphthyridin-2-one (33mg, 1eq.) was dissolved in dioxane (10mL). (4-methoxyphenyl)methanethiol (24mg, 2eq.), tris(dibenzylideneacetone)dipalladium (10mg, 0.1eq.), 4,5-bis(diphenylphosphino)-9,9-dimethylxanthene (9mg, 0.2eq.) and N,N-diisopropylethylamine (30mg, 3eq.) were added. The mixture was reacted at 90°C for 2hrs. The completion of the reaction was monitored with LCMS. The reaction mixture was concentrated under reduced pressure to dryness, and purified by thin-layer chromatography to afford the target compound (22mg, yield: 56%).

LCMS (ESI) [M+H]⁺=492.1.

### Step 5: Preparation of 3-fluoro-4-((8-methoxy-2-oxo-2H-[1,3]oxazino[5,4-c][1,8]naphthyridin-1(4H)-yl)methyl)benzenesulfonamide

1-(2-fluoro-4-((4-methoxybenzyl)thio)benzyl)-8-methoxy-1,4-dihydro-2H-[1,3]oxazino[5,4-c][1,8]naphthyridin-2-one (20mg, 1.0eq.), acetic acid (16mg, 6.6eq.), water (10mg, 13.6eq.) were dissolved in tetrahydrofuran (5mL). 1,3-dichloro-5,5-dimethylhydantoin (16mg, 2eq.) was added at 0°C. The mixture was reacted at 0°C for 2hrs, followed by adding ammonia solution (0.5mL), then reacted at 0°C for 10 minutes. The completion of the reaction was monitored with LCMS. The reaction mixture was concentrated under reduced pressure to dryness, and purified by preparative HPLC to afford the target compound (2.5mg, yield: 15%).

LCMS (ESI) [M+H]⁺=419.0; ¹H NMR (400MHz, DMSO) δ 8.79 (s, 1H), 8.41 (d, J=9.2 Hz, 1H), 7.65-7.56 (m, 3H), 7.48 (s, 2H), 7.08 (d, J=9.2 Hz, 1H), 5.43 (s, 4H), 4.01 (s, 3H).

### Example 27

### Preparation of 4-((8-methoxy-2-oxo-2H-[1,3]oxazino[5,4-c][1,8]naphthyridin-1(4H)-yl)methyl)benzenesulfonamide (compound 67):

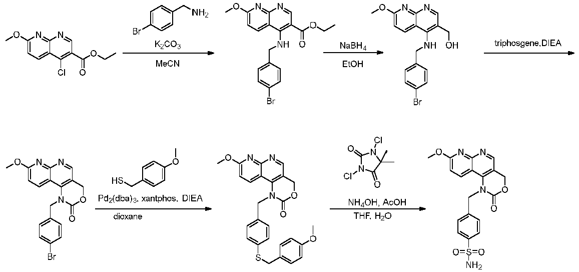

### Step 1: Preparation of ethyl 4-((4-bromobenzyl)amino)-7-methoxy-1,8-naphthyridine-3-carboxylate

Ethyl 4-chloro-7-methoxy-1,8-naphthyridine-3-carboxylate (500mg, 1.0eq.) was dissolved in acetonitrile (30mL). Then K₂CO₃ (1.3g, 5.0eq.), and (4-bromophenyl)methanamine (523.2mg, 1.5eq.) were added. The reaction was performed under the protection of nitrogen gas at 70°C for 5hrs. The completion of the reaction was monitored with LCMS. The reaction mixture was cooled to rt, diluted with water and extracted with EA. The organic phase was dried over anhydrous sodium sulfate, and concentrated to afford a crude product, which was purified by flash chromatography to afford the target compound (0.5g, yield: 64%).

LCMS (ESI) [M+H]⁺=416.1.

### Step 2: Preparation of (4-((4-bromobenzyl)amino)-7-methoxy-1,8-naphthyridin-3-yl)methanol

Ethyl 4-((4-bromobenzyl)atnino)-7-methoxy-1,8-naphthyridine-3-carboxylate (300mg, 1.0eq.) was dissolved in ethanol (10mL). Sodium borohydride (245.4mg, 9.0eq.) was added. The atmosphere was replaced with nitrogen gas, and the reaction system was stirred at 70°C for 10hrs. The completion of the reaction was monitored with LCMS.

The reaction mixture was cooled to rt, quenched with saturated aqueous NH₄Cl solution, and extracted with EA. The organic phase was combined, washed with saturated NaHCOs, and concentrated to afford a crude product, which was purified by flash chromatography to afford the target compound (200mg, yield: 74%).

LCMS (ESI) [M+H]⁺=376.0.

### Step 3: Preparation of 1-(4-bromobenzyl)-8-methoxy-1,4-dihydro-2H-[1,3]oxazino[5,4-c][1,8]naphthyridin-2-one

(4-((4-bromobenzyl)amino)-7-methoxy-1,8-naphthyridin-3-yl)methanol (300mg, 1.0eq.) was dissolved in dichloromethane (10mL). Then N,N-diisopropylethylamine (243mg, 2.35eq.), triphosgene (237mg, 1.0eq.) were successively added at 0°C. The mixture was reacted at 0°C under stirring for 2hrs. The completion of the reaction was monitored with LCMS. The reaction mixture was quenched with saturated brine (20mL), extracted with dichloromethane (10mL×2). The organic phases were combined, dried over anhydrous sodium sulfate, filtered, and distilled under reduced pressure to remove the solvent to afford a crude product, which was purified by flash chromatography to afford the target compound (200mg, yield 62%).

LCMS (ESI) [M+H]⁺=402.0.

### Step 4: Preparation of 8-methoxy-1-(4-((4-methoxybenzyl)thio)benzyl)-1,4-dihydro-2H-[1,3]oxazino[5,4-c][1,8]naphthyridin-2-one

1-(4-bromobenzyl)-8-methoxy-1,4-dihydro-2H-[1,3]oxazino[5,4-c][1,8]naphthyridin-2-one (200mg, 1.0eq.) was dissolved in dioxane (10mL). (4-methoxyphenyl)methanethiol (154mg, 2.0eq.), 4,5-bis(diphenylphosphino)-9,9-dimethylxanthene (57mg, 0.2eq.), tris(dibenzylideneacetone)dipalladium (44mg, 0.1eq.) and N,N-diisopropylethylamine (193mg, 3eq.) were added. The mixture was reacted at 90°C for 2hrs. The completion of the reaction was monitored with LCMS. The reaction mixture was cooled to rt, concentrated, and purified by flash silica gel column chromatography to afford the target compound (100mg, yield: 42%).

LCMS (ESI) [M+H]⁺=474.1.

### Step 5: Preparation of 4-((8-methoxy-2-oxo-2H-[1,3]oxazino[5,4-c][1,8]naphthyridin-1(4H)-yl)methyl)benzenesulfonamide

8-methoxy-1-(4-((4-methoxybenzyl)thio)benzyl)-1,4-dihydro-2H-[1,3]oxazino[5,4-c][1,8]naphthyridin-2-one (90mg, 1.0eq.), acetic acid (80mg, 7.0eq.), water (50mg, 14.6eq.) were dissolved in tetrahydrofuran (15mL). 1,3-dichloro-5,5-dimethylhydantoin (112mg, 3.0eq.) was added at 0°C. The mixture was reacted at 25°C for 1hr, followed by adding ammonia solution (0.5mL), then reacted at 25°C for 10 minutes. The completion of the reaction was monitored with LCMS. The reaction mixture was concentrated, and purified by preparative HPLC to afford the target compound (13.9mg, yield: 18%).

LCMS (ESI) [M+H]⁺=401.1; ¹HNMR (400MHz, MMSO-d₆) δ 8.79 (s, 1H), 8.37 (d, J=8.0 Hz, 1H), 7.74 (d, J=8.0 Hz, 2H), 7.51 (d, J=8.0 Hz, 2H), 7.33 (s, 2H), 7.02 (d, J=8.0 Hz, 1H), 5.51 (s, 2H), 5.43 (s, 2H), 3.99 (s, 3H).

### Example 28

### Preparation of (3,5-difluoro-4-((8-methoxy-2-oxo-2H-[1,3]oxazino[5,4-c][1,8]naphthyridin-1(4H)-yl)methyl)phenyl)boronic acid (compound 41):

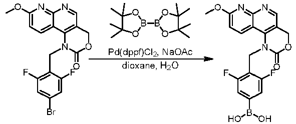

1-(4-bromo-2,6-difluorobenzyl)-8-methoxy-1,4-dihydro-2H-[1,3]oxazino[5,4-c][1,8]naphthyridin-2-one (100mg, 1.0eq., prepared in step 4, Example 6) was dissolved in 1,4-dioxane (10mL). Bis(pinacolato)diboron (76mg, 1.3eq.), (1,1'-bis(diphenylphosphino)ferrocene)dichloropalladium(34mg, 0.2eq.), sodium acetate (38mg, 2.0eq.) were added. The mixture was reacted at 90°C under the protection of nitrogen gas for 2hrs. The completion of the reaction was monitored with LCMS. The reaction mixture was concentrated to dryness, and purified by preparative HPLC to afford the target compound (15.8mg, yield 17%).

LCMS (ESI) [M+H]⁺=402.1; ¹H NMR (400MHz, MeOD) δ 8.71 (s, 1H), 8.62 (d, J=9.2 Hz, 1H), 7.15-7.04 (m, 3H), 5.57 (s, 2H), 5.31 (s, 2H), 4.13 (s, 3H).

### Example 29

### Preparation of (3,5-difluoro-4-((8-methoxy-2-oxo-2H-[1,3]oxazino[5,4-c][1,8]naphthyridin-1(4H)-yl)methyl)phenyl)phosphonic acid (compound 43):

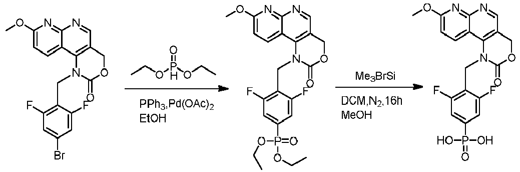

### Step 1: Preparation of diethyl (3,5-difluoro-4-((8-methoxy-2-oxo-2H-[1,3]oxazino[5,4-c][1,8]naphthyridin-1(4H)-yl)methyl)phenyl)phosphonate

1-(4-bromo-2,6-difluorobenzyl)-8-methoxy-1,4-dihydro-2H-[1,3]oxazino[5,4-c][1,8]naphthyridin-2-one (25mg, 1.0eq., prepared in step 4, Example 6) *was added to* ethanol (5mL). Diethyl phosphonate (12mg, 1.5eq.), N-methyldicyclohexylamine (18mg, 1.5eq.), triphenylphosphine (24mg, 1.6eq.) and palladium acetate (6mg, 0.5eq.) were added. Under nitrogen, the mixture was reacted at 80°C for 16hrs. The completion of the reaction was monitored with LCMS. The reaction mixture was cooled to rt, diluted with water (5mL), extracted with EA (20mL×2). The organic phases were combined, washed with saturated brine, dried over anhydrous sodium sulfate, and filtered. The filtrate was concentrated, and purified by preparative chromatography to afford the target compound (15mg, yield: 53%).

LCMS (ESI) [M+H]⁺=494.1.

### Step 2: Preparation of (3,5-difluoro-4-((8-methoxy-2-oxo-2H-[1,3]oxazino[5,4-c][1,8]naphthyridin-1(4H)-yl)methyl)phenyl)phosphonic acid

Diethyl (3,5-difluoro-4-((8-methoxy-2-oxo-2H-[1,3]oxazino[5,4-c][1,8]naphthyridin-1(4H)-yl)methyl)phenyl)phosphonate (15mg, 1.0eq.) was added to anhydrous dichloromethane (5mL). Trimethylbromosilane (0.46mg, 0.1eq.) was added. Under the protection of nitrogen gas, the mixture was reacted at rt for 16hrs. The completion of the reaction was monitored with LCMS. The reaction mixture was diluted with methanol (3mL), and stirred at rt for 1hr. LCMS monitoring indicated the completion of the reaction, the reaction mixture was purified by preparative HPLC to afford the target compound (1.5mg, yield 11%).

LCMS (ESI) [M+H]⁺=438.1; ¹H NMR (400MHz, MeOD) δ 8.72 (s, 1H), 8.63 (d, J=9.2 Hz, 1H), 7.28-7.25 (m, 2H), 7.15 (d, J=9.2Hz, 1H), 5.57 (s, 2H), 5.30 (s, 2H), 4.12 (s, 3H).

### Example 30

Preparation of tert-butyl 1-(2,6-difluoro-4-sulfamoylbenzyl)-9-fluoro-8-methoxy-2-oxo-2,3-dihydropyrazino[2,3-c]quinoline-4(1H)-carboxylate (compound 69)

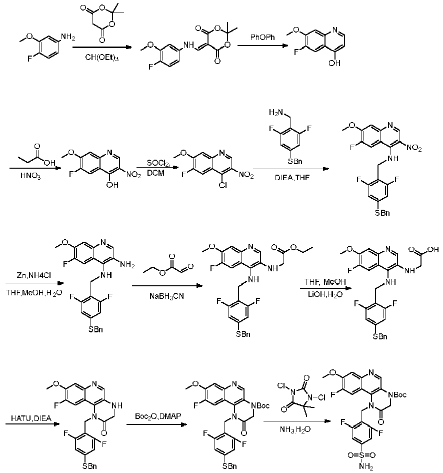

### Step 1: Preparation of 5-(((4-fluoro-3-methoxyphenyl)amino)methylene)-2,2-dimethyl-1,3-dioxane-4,6-dione

2,2-dimethyl-1,3-dioxane-4,6-dione (5.11g, 1eq.) was dissolved in triethoxymethane (15.75g, 3eq.). The mixture was stirred at 90°C for 1hr and cooled down to70°C. 4-fluoro-3-methoxyaniline (5g, 1eq.) was added. The mixture was reacted at 70°C for 0.5hrs. The completion of the reaction was monitored with LCMS. The reaction mixture was cooled to rt, and filtered. The filter cake was washed with petroleum ether, and dried to afford the target compound (10g, yield: 95%).

¹H NMR (400MHz, DMSO-d₆) δ 11.24 (d, *J*=14.5 Hz, 1H), 8.59 (d, *J*=14.5 Hz, 1H), 7.50 (dd, *J*=7.6, 2.6 Hz, 1H), 7.27 (dd, *J*=11.1, 8.8 Hz, 1H), 7.19-7.03 (m, 1H), 3.90 (s, 3H), 1.68 (s, 6H).

### Step 2: Preparation of 6-fluoro-7-methoxyquinolin-4-ol

5-(((4-fluoro-3-methoxyphenyl)amino)methylene)-2,2-dimethyl-1,3-dioxane-4,6-dione (10g, 1eq.) was dissolved in diphenylether (40mL). The mixture was stirred at 200°C for 6hrs. The completion of the reaction was monitored with LCMS. The reaction system was recovered to rt, and filtered. The filter cake was washed with petroleum ether to afford the target product (6.3g, yield: 96%).

LCMS (ESI) [M+H]⁺=194.1; ¹H NMR (400MHz, DMSO-d₆) δ 11.65 (s, 1H), 7.92-7.81 (m, 1H), 7.71 (d, *J*=11.8 Hz, 1H), 7.13 (d, *J*=7.4 Hz, 1H), 5.97 (d, *J*=7.4 Hz, 1H), 3.94 (s, 3H).

### Step 3: Preparation of 6-fluoro-7-methoxy-3-nitroquinolin-4-ol

6-fluoro-7-methoxyquinolin-4-ol (3g, 1eq.) was dissolved in propionic acid (50mL). Nitric acid (70%, 5mL) was added. The mixture was reacted at 140°C for 12hrs. The completion of the reaction was monitored with LCMS. The reaction mixture was cooled to rt, diluted with water, filtered. The filter cake was washed with water and EA to afford the target compound (2.4g, yield: 65%).

LCMS (ESI) [M+H]⁺=239.3; ¹H NMR (400MHz, DMSO-d₆) δ 12.89 (s, 1H), 9.17 (s, 1H), 7.90 (d, *J*=11.5 Hz, 1H), 7.32 (d, *J*=7.3 Hz, 1H), 3.98 (s, 3H).

### Step 4: Preparation of 4-chloro-6-fluoro-7-methoxy-3-nitroquinoline

6-fluoro-7-methoxy-3-nitroquinolin-4-ol (2.4g, 1eq.) was dissolved in dichloromethane (70mL) and N,N-dimethylformamide (20mL). Sulfonyl dichloride (3g, 2.2eq.) was added. The mixture was stirred at 40°C for 3hrs.

The completion of the reaction was monitored with LCMS. The reaction mixture was concentrated under reduced pressure to afford a crude product, which was washed with water to precipitate a solid. The mixture was filtered. The filter cake was washed with water and EA to afford the target compound (2.5g, yield: 96%).

LCMS (ESI) [M+H]⁺=257.3.

### Step 5: Preparation of N-(4-(benzylthio)-2,6-difluorobenzyl)-6-fluoro-7-methoxy-3-nitroquinolin-4-amine

4-chloro-6-fluoro-7-methoxy-3-nitroquinoline (2.8g, 1eq.) and (4-(benzylthio)-2,6-difluorophenyl)methanamine (2.89g, 1eq.) were dissolved in tetrahydrofuran (40mL). N,N-diisopropylethylamine (4.23g, 3eq.) was added. The mixture was stirred at rt for 1.5hrs. The completion of the reaction was monitored with LCMS. The reaction mixture was concentrated under reduced pressure, filtered. The filter cake was washed with methanol to afford the target compound (3.2g, yield: 60%).

LCMS (ESI) [M+H]⁺=486.1.

### Step 6: Preparation of N⁴-(4-(benzylthio)-2,6-difluorobenzyl)-6-fluoro-7-methoxyquinofine-3,4-diamine

N-(4-(benzylthio)-2,6-difluorobenzyl)-6-fluoro-7-methoxy-3-nitroquinolin-4-amine (3.1g, 1eq.) was dissolved in a mixture of methanol (50mL) and tetrahydrofuran (50mL). Zinc powder (2.09g, 5eq.) and *aqueous* NH4CI solution (3.4mg, 10eq., 25mL) were added. The mixture was reacted at rt for 1hr. The completion of the reaction was monitored with LCMS. The reaction mixture was filtered to remove zinc powder, diluted with water (20mL), extracted with EA (30mL×3). The organic phases were combined, distilled under reduced pressure rotary-evaporated to dryness to afford a crude target compound (2.2g), which was not further purified but directly used in the next step reaction.

LCMS (ESI) [M+H]⁺=456.0.

### Step 7: Preparation of ethyl (4-((4-(benzylthio)-2,6-difluorobenzyl)amino)-6-fluoro-7-methoxyquinolin-3-yl)glycinate

N⁴-(4-(benzylthio)-2,6-difluorobenzyl)-6-fluoro-7-methoxyquinoline-3,4-diamine (1.9g, crude) and ethyl glyoxylate/toluene solution (50wt%, 20mL) were dissolved in methanol (10mL). The mixture was stirred at rt for 1.5hrs, followed by adding sodium cyanoborohydride (1.29g), then stirred at rt for 0.5hrs. The completion of the reaction was monitored with LCMS. The reaction mixture was rotary-evaporated to dryness, diluted with water (30mL), extracted with EA (20mL×3). The organic phases were combined, rotary-evaporated to dryness to afford the target compound (4g, crude), which was not further purified but directly used in the next step reaction.

LCMS (ESI) [M+H]⁺=542.2.

### Step 8: Preparation of (4-((4-(benzylthio)-2,6-difluorobenzyl)amino)-6-fluoro-7-methoxyquinolin-3-yl)glycine

Ethyl (4-((4-(benzylthio)-2,6-difluorobenzyl)amino)-6-fluoro-7-methoxyquinolin-3-yl)glycinate (2g, crude) was dissolved in a mixture of tetrahydrofuran (50mL) and methanol (15mL). Aqueous LiOH solution (353.8mg, 10mL) was added. The mixture was stirred at rt for 2hrs. The completion of the reaction was monitored with LCMS. The reaction mixture was adjusted to pH=2, rotary-evaporated to dryness, diluted with water (30mL), extracted with EA (30mL×3). The organic phases were combined and rotary-evaporated to dryness to afford the target product (1.1g, yield: 58%).

LCMS (ESI) [M+H]⁺=514.1.

### Step 9: Preparation of 1-(4-(benzylthio)-2,6-difluorobenzyl)-9-fluoro-8-methoxy-3,4-dihydropyrazino[2,3-c]quinolin-2(1H)-one

(4-((4-(benzylthio)-2,6-difluorobenzyl)amino)-6-fluoro-7-methoxyquinolin-3-yl)glycine (1.1g, 1eq.) was dissolved in N,N-dimethylformamide (10mL). HATU (2443.4mg, 3eq.) and N,N-diisopropylethylamine (830.5mg, 3eq.) was added. The mixture was stirred at rt for 2hrs. The completion of the reaction was monitored with LCMS. The reaction mixture was diluted with water (20mL), extracted with EA (20mL×3). The organic phases were combined, washed with saturated brine, and filtered. The filtrate was rotary-evaporated to dryness to afford a crude product, which was purified by column chromatography to afford the target product (800mg, yield: 75%).

LCMS (ESI) [M+H]⁺=496.1.

### Step 10: Preparation of tert-butyl 1-(4-(benzylthio)-2,6-difluorobenzyl)-9-fluoro-8-methoxy-2-oxo-2,3-dihydropyrazino[2,3-c]quinoline-4(1H)-carboxylate

1-(4-(benzylthio)-2,6-difluorobenzyl)-9-fluoro-8-methoxy-3,4-dihydropyrazino[2,3-c]quinolin-2(1H)-one (300mg, 1eq.) was dissolved in di-tert-butyl dicarbonate (3mL). 4-dimethylaminopyridine (221.9mg, 3eq.) was added and the mixture was stirred at 40°C for 1hr. The completion of the reaction was monitored with LCMS. The reaction mixture was diluted with water (10mL), extracted with EA (20mL×3). The organic phases were combined, and rotary-evaporated to dryness to afford a crude product, which was purified by column chromatography to afford the target compound (100mg, yield: 28%).

LCMS (ESI) [M+H]⁺=596.4.

### Step 11: Preparation of tert-butyl 1-(2,6-difluoro-4-sulfamoylbenzyl)-9-fluoro-8-methoxy-2-oxo-2,3-dihydropyrazino[2,3-c]quinoline-4(1H)-carboxylate

Tert-butyl 1-(4-(benzylthio)-2,6-difluorobenzyl)-9-fluoro-8-methoxy-2-oxo-2,3-dihydropyrazino[2,3-c]quinoline-4(1H)-carboxylate (80mg, 1eq.) was dissolved in tetrahydrofuran (2mL). In an ice bath, water (33.9mg, 14eq.), acetic acid (56.5mg, 7eq.), 1,3-dichloro-5,5-dimethylhydantoin (52.9mg, 2eq.) were added. The mixture was stirred in an ice bath for 5 minutes. The completion of the reaction was monitored with LCMS. The reaction mixture was added to the ammonia solution (3mL) under stirring. The completion of the reaction was monitored with LCMS. The mixture was extracted with EA (5mL×3). The organic phases were combined, and distilled under reduced pressure to afford a crude product, which was purified by preparative HPLC to afford the target compound (6mg, yield: 8%). LCMS (ESI) [M+H]⁺=553.1; ¹H NMR (400MHz, MeOD-d₄) δ 8.91 (s, 1H), 7.94 (d, *J*=12.4 Hz, 1H), 7.50 (d, *J*=8.2 Hz, 1H), 7.27 (d, *J*=7.2 Hz, 2H), 5.59 (s, 2H), 4.36 (s, 2H), 4.03 (s, 3H), 1.54 (s, 9H).

### Example 31

### Preparation of 3,5 -difluoro-4-((9-fluoro-8-methoxy-2-oxo-3,4-dihydropyrazino [2,3-c]quinolin-1(2H)-yl)methyl)benzenesulfonamide (compound 55)

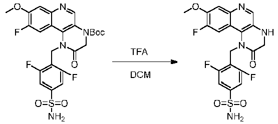

Tert-butyl 1-(2,6-difluoro-4-sulfamoylbenzyl)-9-fluoro-8-methoxy-2-oxo-2,3-dihydropyrazino[2,3-c]quinoline-4(1H)-carboxylate (50mg, 1eq.) prepared in Example 30 was dissolved in dichloromethane (2mL). Trifluoroacetic acid (0.5mL) was added. The mixture was stirred at rt stirred for 1hr. The completion of the reaction was monitored with LCMS. The reaction mixture was concentrated under reduced pressure, and purified by preparative HPLC to afford the target compound (1.23mg, yield: 3%).

LCMS (ESI) [M+H]⁺=453.1.

### Example 32

### Preparation of 3,5 -difluoro-4-((4-(2-hydroxyacetyl)-8-methoxy-2-oxo-3,4-dihydropyrazino [2,3-c]quinolin-1(2H)-yl)methyl)benzenesulfonamide (compound 72)

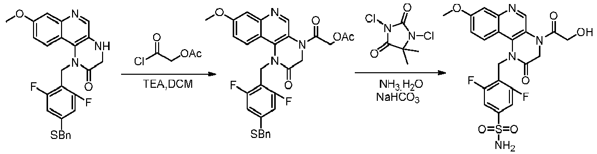

### Step 1: Preparation of 2-(1-(4-(benzylthio)-2,6-difluorobenzyl)-8-methoxy-2-oxo-2,3-dihydropyrazino[2,3-c]quinolin-4(1H)-yl)-2-oxoethyl acetate

1-(4-(benzylthio)-2,6-difluorobenzyl)-8-methoxy-3,4-dihydropyrazino[2,3-c]quinolin-2(1H)-one (400mg, 1eq., prepared in step 6, Example 18) was dissolved in dichloromethane (4mL). 2-chloro-2-oxoethyl acetate (171.6mg, 1.5eq.) and triethylamine (254.3mg, 3eq.) were added. The mixture was reacted at rt for 1hr. The completion of the reaction was monitored with LCMS. The reaction mixture was diluted with water, and extracted. The organic phase was dried over anhydrous sodium sulfate, and filtered. The filtrate was concentrated to afford a crude product, which was purified by column chromatography to afford the target compound (120mg, yield: 25%).

LCMS (ESI) [M+H]⁺=578.2.

### Step 2: Preparation of 3,5-difluoro-4-((4-(2-hydroxyacetyl)-8-methoxy-2-oxo-3,4-dihydropyrazino[2,3-c]quinolin-1(2H)-yl)methyl)benzenesulfonamide

2-(1-(4-(benzylthio)-2,6-difluorobenzyl)-8-methoxy-2-oxo-2,3-dihydropyrazino[2,3-c]quinolin-4(1H)-yl)-2-oxoethyl acetate (50mg, 1eq.) was dissolved in superdry tetrahydrofuran (2mL). In an ice bath, glacial acetic acid (39.3mg, 7.6eq.), water (23.6mg, 15.1eq.) and 1,3-dichloro-5,5-dimethylhydantoin (55.3mg, 3.2eq.) were added. The mixture was stirred at 0°C for 5 minutes. The completion of the reaction was monitored with LCMS. The reaction mixture was added to the ammonia solution (5mL), stirred for 30 minutes, followed by adding NaHCO3 (39.3mg, 5.4eq.), then stirred for 1hr. The completion of the reaction was monitored with LCMS. The reaction mixture was filtered. The filtrate was adjusted with 1M HCl to neutral pH, and concentrated. The residue was purified by preparative HPLC to afford the target compound (3.2mg, yield: 7.5%).

LCMS (ESI) [M+H]⁺=493.0; ¹H NMR (400MHz, MeOD-d₄) δ 9.10 (s, 1H), 8.25 (d, *J*=10.3 Hz, 1H), 7.43 (s, 2H), 7.33 (d, *J*=6.4 Hz, 2H), 5.63 (s, 2H), 4.46 (d, *J*=20.1 Hz, 4H), 4.00 (s, 3H).

### Example 33

### Preparation of 3,5-difluoro-4-((7-methoxy-2-oxo-2H-[1,3]oxazino[5,4-c]quinolin-1(4H)-yl)methyl)benzenesulfonamide (compound 61)

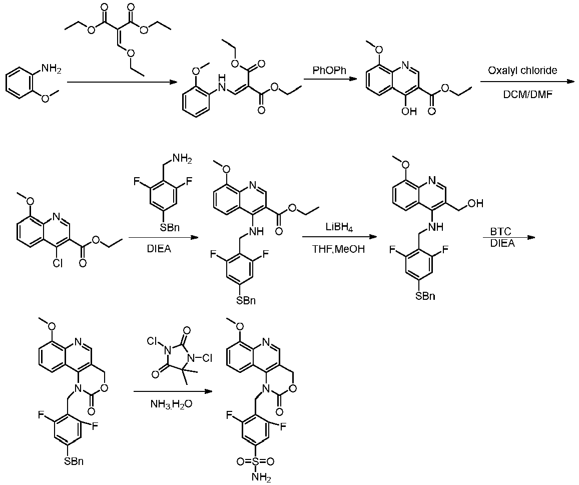

### Step 1: Preparation of diethyl 2-(((2-methoxyphenyl)amino)methylene)malonate

2-methoxyaniline (20g, 1eq.) was dissolved in diethyl 2-(ethoxymethylene)malonate (35.12g, 1eq.). The mixture was stirred at 140°C for 45 minutes. The completion of the reaction was monitored with LCMS. The reaction mixture was cooled to rt, and diluted with petroleum ether to precipitate a white solid. The mixture was filtered. The filter cake was washed with petroleum ether to afford the target compound (39.6g, yield: 83%).

LCMS (ESI) [M+H]⁺=294.1; ¹H NMR (400MHz, CDCl₃) δ 11.12 (d, *J*=13.4 Hz, 1H), 8.57 (d, *J*=14.1 Hz, 1H), 7.24 (s, 1H), 6.99 (dd, *J*=42.4, 26.6 Hz, 3H), 4.33 (q, *J*=7.1 Hz, 2H), 4.25 (q, *J*=7.1 Hz, 2H), 3.94 (s, 3H), 1.39 (t, *J*=7.1 Hz, 3H), 1.33 (t, *J*=7.1 Hz, 3H).

### Step 2: Preparation of ethyl 4-hydroxy-8-methoxyquinoline-3-carboxylate

To diphenyl ether (75mL) was added diethyl 2-(((2-methoxyphenyl)amino)methylene)malonate (15g, 1eq.). The mixture was reacted at 260°C for 45 minutes. The completion of the reaction was monitored with LCMS. The reaction mixture was cooled to rt, and diluted with petroleum ether to precipitate a solid. The mixture was filtered. The filter cake was washed with petroleum ether, and dried to afford the target compound (8.5g, yield: 67%).

LCMS (ESI) [M+H]⁺=248.0.

### Step 3: Preparation of ethyl 4-chloro-8-methoxyquinoline-3-carboxylate

Ethyl 4-hydroxy-8-methoxyquinoline-3-carboxylate (4g, 1eq.) was dissolved in dichloromethane (40mL). N,N-dimethylformamide (4mL) was added. Oxalyl chloride (3.08g, 1.5eq.) was slowly added dropwise. The mixture was stirred at rt stirred for 1hr. The completion of the reaction was monitored with LCMS. The reaction mixture was rotary-evaporated to dryness. Ethyl acetate was added. The mixture was filtered. The filter cake was washed with EA, and dried to afford the target product (4g, yield: 93%).

LCMS (ESI) [M+H]⁺=266.1; ¹H NMR (400MHz, CDCl₃) δ 9.67 (s, 1H), 8.15 (d, *J*=8.6 Hz, 1H), 7.94 (t, *J*=8.3 Hz, 1H), 7.49 (d, *J*=7.9 Hz, 1H), 4.55 (d, *J*=7.1 Hz, 2H), 4.26 (s, 3H), 1.48 (t, *J*=7.1 Hz, 3H).

### Step 4: Preparation of ethyl 4-((4-(benzylthio)-2,6-difluorobenzyl)amino)-8-methoxyquinoline-3-carboxylate

Ethyl 4-chloro-8-methoxyquinoline-3-carboxylate (2g, 1eq.) and (4-(benzylthio)-2,6-difluorophenyl)methanamine (2g, 1eq.) were dissolved in DMF (40mL). N,N-diisopropylethylamine (3.89g, 4eq.) was added. The mixture was reacted at 50°C for 16hrs. The completion of the reaction was monitored with LCMS. The reaction mixture was diluted with water (40mL), extracted with EA (30mL×3). The organic phases were combined, concentrated, and purified by column chromatography to afford the target compound (2.3g, yield: 62%).

LCMS (ESI) [M+H]⁺=495.0.

### Step 5: Preparation of 4-((4-(benzylthio)-2,6-difluorobenzyl)amino)-8-methoxyquinolin-3-yl)methanol

Ethyl 4-((4-(benzylthio)-2,6-difluorobenzyl)amino)-8-methoxyquinoline-3-carboxylate (2.1g, 1eq.) was dissolved in a mixed solution of tetrahydrofuran (30mL) and methanol (8mL). Lithium borohydride (462.4mg, 5eq.) was added. The mixture was reacted at rt for 2hrs. The completion of the reaction was monitored with LCMS. The reaction mixture was quenched with aqueous saturated NH4CI solution, concentrated under reduced pressure to precipitate a solid. The mixture was filtered. The filter cake was washed with EA, and dried to afford the target compound (1.2g, yield: 62%).

LCMS (ESI) [M+H]⁺=452.9.

### Step 6: Preparation of 1-(4-(benzylthio)-2,6-difluorobenzyl)-7-methoxy-1,4-dihydro-2H-[1,3]oxazino[5,4-c]quinolin-2-one

4-((4-(benzylthio)-2,6-difluorobenzyl)amino)-8-methoxyquinolin-3-yl)methanol (400mg, 1eq.) was dissolved in superdry tetrahydrofuran (40mL). N,N-diisopropylethylamine (342.7mg, 3eq.) was added. In an ice bath, triphosgene (262.3mg, 2eq.) was added. The mixture was stirred at room temperature for 3hrs. The completion of the reaction was monitored with LCMS. The reaction mixture was concentrated under reduced pressure, and purified by reverse column to afford the target compound (100mg, yield: 24%).

LCMS (ESI) [M+H]⁺=479.1.

### Step 7: Preparation of 3,5-difluoro-4-((7-methoxy-2-oxo-2H-[1,3]oxazino[5,4-c]quinolin-1(4H)-yl)methyl)benzenesulfonamide

1-(4-(benzylthio)-2,6-difluorobenzyl)-7-methoxy-1,4-dihydro-2H-[1,3]oxazino[5,4-c]quinolin-2-one (50mg, 1eq.) was dissolved in tetrahydrofuran (2mL). In an ice bath, water (26.4mg, 14eq.) and acetic acid (43.9mg, 7eq.), 1,3-dichloro-5,5-dimethylhydantoin (41.2mg, 2eq.) were added. The mixture was stirred for 5 minutes in an ice bath. Ammonia solution (1mL) was added. The stirring continued. The completion of the reaction was monitored with LCMS. The reaction mixture was distilled under reduced pressure to remove tetrahydrofuran, extracted with EA (10mL). The organic phase was concentrated, and purified by preparative HPLC to afford the target compound (2.0mg, yield: 4.4%).

LCMS (ESI) [M+H]⁺=436.0; ¹H NMR (400MHz, MeOD-d₄) δ 8.67 (s, 1H), 8.50 (s, 2H), 7.79 (d, *J*=8.5 Hz, 1H), 7.63 (t, *J*=8.3 Hz, 1H), 7.44 (d, *J*=7.4 Hz, 2H), 7.27 (d, *J*=8.2 Hz, 1H), 5.59 (s, 2H), 5.32 (s, 2H), 4.07 (s, 3H).

### Example 34

### Preparation of 6-(8-methoxy-2-oxo-2H-[1,3]oxazino[5,4-c][1,8]naphthyridin-1(4H)-yl)-3,4-dihydroisoquinolin-2(1H)-sulfonamide (compound 59)

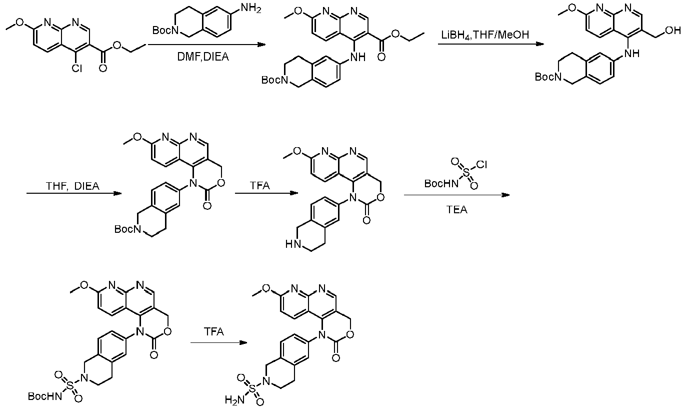

### Step 1: Preparation of ethyl 4-((2-(tert-butoxycarbonyl)-1,2,3,4-tetrahydroisoquinolin-6-yl)amino)-7-methoxy-1,8-naphthyridine-3 -carboxylate

Ethyl 4-chloro-7-methoxy-1,8-naphthyridine-3-carboxylate (2g, 1eq.) was dissolved in N,N-dimethylformamide (20mL). N,N-diisopropylethylamine (2.91g, 3eq.) and tert-butyl 6-amino-3,4-dihydroisoquinolin-2(1H)-carboxylate (2.79g, 1.5eq.) were added. The mixture was stirred at 50°C for 10hrs. The completion of the reaction was monitored with LCMS. The reaction mixture was diluted with water (100mL), extracted with EA (30mL×3). The organic phases were combined, washed with saturated brine (50mL×2), dried over anhydrous sodium sulfate, and filtered. The filtrate was concentrated, and purified by column chromatography to afford the target compound (2.3g, yield: 64%). LCMS (ESI) [M+H]⁺=479.1; ¹H NMR (400MHz, CDCl₃) δ 10.37 (s, 1H), 9.30 (s, 1H), 7.81 (d, *J*=9.1 Hz, 1H), 7.04 (d, *J*=8.0 Hz, 1H), 6.93-6.81 (m, 2H), 6.58 (d, *J*=9.1 Hz, 1H), 4.56 (s, 2H), 4.43 (q, *J*=7.1 Hz, 2H), 4.13 (s, 3H), 3.63 (t, *J*=5.0 Hz, 2H), 2.74 (s, 2H), 1.50 (s, 9H), 1.45 (t, *J*=7.1 Hz, 3H).

### Step 2: Preparation of tert-butyl 6-((3-(hydroxymethyl)-7-methoxy-1,8-naphthyridin-4-yl)amino)-3,4-dihydroisoquinolin-2(1H)-carboxylate

Ethyl 4-((2-(tert-butoxycarbonyl)-1,2,3,4-tetrahydroisoquinolin-6-yl)amino)-7-methoxy-1,8-naphthyridine-3-carboxylate (2g, 1eq.) was dissolved in tetrahydrofuran (20mL). In an ice bath, lithium borohydride (0.27g, 3eq.) and methanol (2mL) were added. The reaction mixture was stirred at 25°C for 2hrs. The completion of the reaction was monitored with LCMS. In an ice bath, a saturated NH4CI solution (10mL) was slowly added dropwise. The mixture was extracted with EA (30mLx3). The organic phases were combined, washed with saturated brine (50mLx2), dried over anhydrous sodium sulfate, and filtered. The filtrate was concentrated to afford the target compound (1.5g, crude), which was not further purified but directly used in the next step reaction.

LCMS (ESI) [M+H]⁺=437.3.

### Step 3: Preparation of tert-butyl 6-(8-methoxy-2-oxo-2H-[1,3]oxazino[5,4-c][1,8]naphthyridin-1(4H)-yl)-3,4-dihydroisoquinolin-2(1H)-carboxylate

Tert-butyl 6-((3-(hydroxymethyl)-7-methoxy-1,8-naphthyridin-4-yl)amino)-3,4-dihydroisoquinolin-2(1H)-carboxylate (1.9g, crude) was dissolved in tetrahydrofuran (20mL). N,N-diisopropylethylamine (1.69g) and triphosgene (1.29g) were added. The reaction mixture was stirred at 25°C for 4hrs. The completion of the reaction was monitored with LCMS. The reaction mixture was diluted with water (10mL), extracted with EA (10mL×3), washed with saturated brine (20mL×2), dried over anhydrous sodium sulfate, and filtered. The filtrate was concentrated to afford a crude product, which was purified by column chromatography to afford the target compound (300mg).

LCMS (ESI) [M+H]⁺=463.1; ¹H NMR (400MHz, CDCl₃) δ 8.75 (s, 1H), 7.24 (dd, *J*=18.5, 9.1 Hz, 3H), 7.16 (d, *J*=9.3 Hz, 1H), 6.63 (d, *J*=9.3 Hz, 1H), 5.47 (s, 2H), 4.64 (s, 2H), 4.11 (s, 3H), 3.67 (s, 2H), 2.83 (t, *J*=5.7 Hz, 2H), 1.51 (s, 9H).

### Step 4: Preparation of 8-methoxy-1-(1,2,3,4-tetrahydroisoquinolin-6-yl)-1,4-dihydro-2H-[1,3]oxazino[5,4-c][1,8]naphthyridin-2-one

Tert-butyl 6-(8-methoxy-2-oxo-2H-[1,3]oxazino[5,4-c][1,8]naphthyridin-1(4H)-yl)-3,4-dihydroisoquinolin-2(1H)-carboxylate (200mg) was dissolved in trifluoroacetic acid (2mL). The reaction mixture was stirred at 40°C for 4hrs. The completion of the reaction was monitored with LCMS. The reaction mixture was concentrated to remove trifluoroacetic acid to afford the target compound (170mg, crude), which was not further purified but directly used in the next step reaction.

LCMS (ESI) [M+H]⁺=363.3.

### Step 5: Preparation of tert-butyl ((6-(8-methoxy-2-oxo-2H-[1,3]oxazino[5,4-c][1,8]naphthyridin-1(4H)-yl)-3,4-dihydroisoquinolin-2(1H)-yl)sulfonyl)carbamate

Tert-butyl chlorosulfonylcarbamate (128.9mg) was dissolved in dichloromethane (2mL). In an ice bath, tert-butanol (67.5mg) was added. The reaction mixture was stirred in an ice bath for 0.5hrs. 8-methoxy-1-(1,2,3,4-tetrahydroisoquinolin-6-yl)-1,4-dihydro-2H-[1,3]oxazino[5,4-c][1,8]naphthyridin-2-one (300mg, crude) was dissolved in dichloromethane (8mL). In an ice bath, triethylamine (100.5mg) was added. The former was slowly added dropwise to this reaction mixture. In an ice bath, the resulting mixture was stirred for 1.5hrs. The completion of the reaction was monitored with LCMS. The reaction mixture was concentrated to remove the solvent, diluted with water (10mL), extracted with EA (5mL×3). The organic phases were combined, washed with saturated brine (20mL×2), dried over anhydrous sodium sulfate, and filtered. The filtrate was concentrated to dryness to afford the target compound (203mg, crude), which was not further purified but directly used in the next step reaction.

LCMS (ESI) [M+H]⁺=542.2.

### Step 6: Preparation of 6-(8-methoxy-2-oxo-2H-[1,3]oxazino[5,4-c][1,8]naphthyridin-1(4H)-yl)-3,4-dihydroisoquinofin-2(1H)-sulfonamide

Tert-butyl ((6-(8-methoxy-2-oxo-2H-[1,3]oxazino[5,4-c][1,8]naphthyridin-1(4H)-yl)-3,4-dihydroisoquinolin-2(1H)-yl)sulfonyl)carbamate (203mg, crude) was dissolved in trifluoroacetic acid (3mL). The reaction mixture was stirred at 40°C for 4hrs. The completion of the reaction was monitored with LCMS. The reaction mixture was concentrated to remove trifluoroacetic acid to afford a crude product, which was purified by preparative HPLC to afford the target compound (1.06mg).

LCMS (ESI) [M+H]⁺=442.1;¹H NMR (400MHz, MeOD-d₄) δ 8.88 (s, 1H), 7.43 (d, *J*=6.4 Hz, 2H), 7.38 (d, *J*=8.9 Hz, 1H), 7.19 (d, *J*=9.5 Hz, 1H), 6.88 (d, *J*=9.5 Hz, 1H), 5.66 (s, 2H), 4.44 (s, 2H), 4.12 (s, 3H), 3.47 (t, *J*=5.8 Hz, 2H), 3.02 (t, *J*=5.8 Hz, 2H).

### Example 35

### Preparation of (N-(4-(8-methoxy-2-oxo-2H-[1,3]oxazino[5,4-c][1,8]naphthyridin-1(4H)-yl)cycloheptyl)sulfamoyl)amine (compound 64):

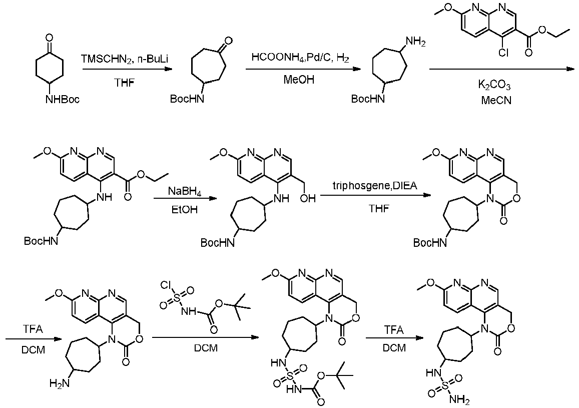

### Step 1: Preparation of tert-butyl (4-oxocycloheptyl)carbamate

n-butyl lithium (2.5M, in hexane)(4.2mL) was dissolved in tetrahydrofuran (100mL). The reaction system was replaced with nitrogen gas. Then trimethylsilyldiazomethane TMSCHN₂ (2M, in hexane)(6.96mL) was added at - 78°C. The mixture was stirred for 30 minutes. Then tert-butyl (4-oxocyclohexyl)carbamate (10.0g, 1eq.) was added at -78°C. The reaction system was replaced with nitrogen gas and stirred at -78°C for 1hr. The completion of the reaction was monitored with LCMS. The reaction system was quenched with aqueous saturated NH4CI solution, and extracted with EA. The organic phase was concentrated, and purified by flash chromatography to afford the target compound (9.0g, yield: 84.4%).

LCMS (ESI) [M+Na]⁺=250.1.

### Step 2: Preparation of tert-butyl (4-aminocycloheptyl)carbamate

Tert-butyl (4-oxocycloheptyl)carbamate (2.0g, 1eq.) and ammonium formate (3.33g, 6eq.) were dissolved in methanol (30mL). Pd/C (936mg, 1eq.) was added. The reaction system was replaced with hydrogen gas and stirred under the atmosphere of hydrogen gas at 20°C for 5hrs. The completion of the reaction was monitored with LCMS. The reaction mixture was filtered. The filtrate was rotary-evaporated to dryness and dissolved in HCl (5M, 5mL). Ethyl acetate was added, and the mixture was divided into layers. The phases were separated. The aqueous phase was alkalized, and extracted with EA. The organic phase was washed with brine, dried over sodium sulfate, and filtered. The filtrate was rotary-evaporated under reduced pressure to dryness to afford the target compound (1.0g, yield: 50%).

LCMS (ESI) [M+H]⁺=229.2.

### Step 3: Preparation of ethyl 4-((4-((tert-butoxycarbonyl)amino)cycloheptyl)amino)-7-methoxy-1,8-naphthyridine-3-carboxylate

Ethyl 4-chloro-7-methoxy-1,8-naphthyridine-3-carboxylate (50mg, 1eq.) was dissolved in acetonitrile (10mL). Then K₂CO₃ (77mg, 3.0eq.), tert-butyl (4-aminocycloheptyl)carbamate (128mg, 3eq.) were added. The reaction was performed under the protection of nitrogen gas at 40°C for 16hrs. The completion of the reaction was monitored with LCMS. The reaction mixture was rotary-evaporated under reduced pressure to dryness, dissolved in ethyl acetate, and washed with water. The organic phase was dried over anhydrous sodium sulfate, and filtered. The filtrate was concentrated, and purified by flash chromatography to afford the target compound (50mg, yield: 58%).

LCMS (ESI) [M+H]⁺=459.3.

### Step 4: Preparation of tert-butyl (4-((3-(hydroxymethyl)-7-methoxy-1,8-naphthyridin-4-yl)amino)cycloheptyl)carbamate

Ethyl 4-((4-((tert-butoxycarbonyl)amino)cycloheptyl)amino)-7-methoxy-1,8-naphthyridine-3-carboxylate (30.0mg, 1eq.) was suspended in ethanol (2mL) and methanol (0.5mL). Then sodium borohydride (15.1mg, 6.1eq.) was added. The reaction was performed under the protection of nitrogen gas at 80°C for 10hrs. The completion of the reaction was monitored with LCMS. The reaction mixture was quenched with saturated aqueous NH4CI solution (5mL), and extracted with EA (5mL×3). The organic phases were combined, washed with saturated brine (10mL), dried over anhydrous sodium sulfate, and filtered. The filtrate was concentrated, and purified by flash chromatography to afford the target compound (10mg, yield: 37%).

LCMS (ESI) [M+H]⁺=417.3.

### Step 5: Preparation of tert-butyl (4-(8-methoxy-2-oxo-2H-[1,3]oxazino[5,4-c][1,8]naphthyridin-1(4H)-yl)cycloheptyl)carbamate

Tert-butyl (4-((3-(hydroxymethyl)-7-methoxy-1,8-naphthyridin-4-yl)amino)cycloheptyl)carbamate (10mg, 1eq.) was dissolved in dichloromethane (1mL). Then triethylamine (7.3mg, 3eq.), triphosgene (7.1mg, 1eq.) were successively added at 0°C. The mixture was reacted at 0°C under stirring for 2hrs. The completion of the reaction was monitored with LCMS. The reaction mixture was quenched with saturated brine (10mL), extracted with EA (5mL×3). The organic phases were combined, dried over anhydrous sodium sulfate, and filtered. The filtrate was concentrated, and purified by flash chromatography to afford the target compound (10mg, yield: 94%).

LCMS (ESI) [M+H]⁺=443.2.

### Step 6: Preparation of 1-(4-aminocycloheptyl)-8-methoxy-1,4-dihydro-2H-[1,3]oxazino[5,4-c][1,8]naphthyridin-2-one

Tert-butyl (4-(8-methoxy-2-oxo-2H-[1,3]oxazino[5,4-c][1,8]naphthyridin-1(4H)-yl)cycloheptyl)carbamate (10mg) was dissolved in dichloromethane (1mL). Then a solution of trifluoroacetic acid (0.5mL) in dichloromethane (0.5mL) was added at 0°C. The mixture was reacted at 20°C stirred for 1hr. The completion of the reaction was monitored with LCMS. The reaction mixture was rotary-evaporated under reduced pressure to remove the solvent to afford the target compound (10mg, crude), which was not further purified but directly used in the next step reaction.

LCMS (ESI) [M+H]⁺=343.2.

### Step 7: Preparation of tert-butyl (N-(4-(8-methoxy-2-oxo-2H-[1,3]oxazino[5,4-c][1,8]naphthyridin-1(4H)-yl)cycloheptyl)sulfamoyl)carbamate

To a 50 mL single-neck round-bottom flask were added tert-butyl chlorosulfonylcarbamate (2mL), and dichloromethane (2mL). Then tert-butanol (2mL) was slowly added at 0°C. The reaction mixture was stirred at 0°C for 1hr and added to a solution of 1-(4-aminocycloheptyl)-8-methoxy-1,4-dihydro-2H-[1,3]oxazino[5,4-c][1,8]naphthyridin-2-one (10mg, crude, obtained from the above step reaction) in dichloromethane (2mL) at 0°C. The mixture was stirred at 0°C for 1hr. The completion of the reaction was monitored with LCMS. The reaction mixture was under reduced pressure rotary-evaporated to dryness to afford the target compound (10mg, crude), which was not further purified but directly used in the next step reaction.

### Step 8: Preparation of (N-(4-(8-methoxy-2-oxo-2H-[1,3]oxazino[5,4-c][1,8]naphthyridin-1(4H)-yl)cycloheptyl)sulfamoyl)amine

Tert-butyl (N-(4-(8-methoxy-2-oxo-2H-[1,3]oxazino[5,4-c][1,8]naphthyridin-1(4H)-yl)cycloheptyl)sulfamoyl)carbamate (9mg, crude) was dissolved in dichloromethane (1mL). Then a solution of trifluoroacetic acid (0.5mL) in dichloromethane (0.5mL) was added at 20°C. The mixture was reacted at 20°C under stirring for 1hr. The completion of the reaction was monitored with LCMS. The reaction mixture was concentrated under reduced pressure, and purified by preparative chromatography to afford the target compound (1.2mg). LCMS (ESI) [M+H]⁺=422.2; ¹HNMR (400MHz, MeOD-*d₄*) *δ*8.18 (d, *J*=8.0 Hz, 1H), 7.07 (d, *J*=4.0 Hz, 1H), 5.21 (s, 2H), 5.10 (s, 2H), 4.03 (s, 3H), 3.35 (s, 1H), 3.03 (s, 1H), 2.93 (m, 1H), 2.56 (s, 1H), 2.5 (s, 1H), 2.4 (m, 1H), 2.10 (m, 2H), 1.94 (m, 2H), 1.51 (m, 1H), 1.30 (m, 1H), 1.25 (m, 3H).

### Example 36

### Preparation of 5-(8-methoxy-2-oxo-2H-[1,3]oxazino[5,4-c][1,8]naphthyridin-1(4H)-yl)hexahydrocyclopenta[c]pyrrole-2(1H)-sulfonamide (compound 36)

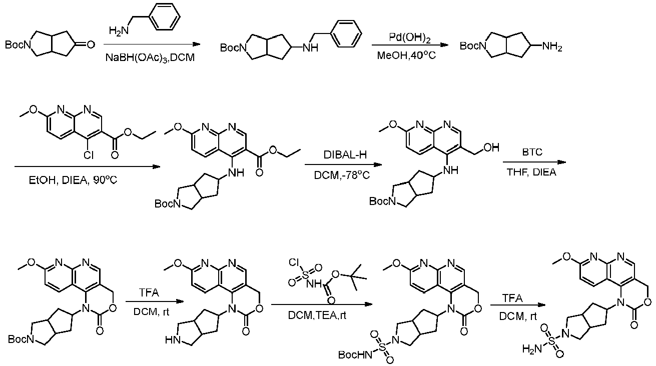

### Step 1: Preparation of tert-butyl 5-(benzylamino)hexahydrocyclopenta[c]pyrrole-2(1H)-carboxylate

Tert-butyl 5-oxohexahydrocyclopenta[c]pyrrole-2(1H)-carboxylate (1.0g) was dissolved in dichloromethane (100mL). Phenylmethanamine (475mg) and acetic acid (266mg) were added at 0°C. The mixture was reacted at 0°C under stirring for 30 minutes. Then sodium triacetoxyborohydride (1.88g) was added in batches. The mixture was warmed to rt and the reaction continued for 16hrs. The completion of the reaction was monitored with LCMS. The reaction mixture was diluted with water (20mL), extracted with dichloromethane (50mL×3). The organic phases were combined, dried over anhydrous sodium sulfate, filtered, and concentrated to afford the target compound (1.2g, crude).

LCMS (ESI) [M+H]⁺=317.3.

### Step 2: Preparation of tert-butyl 5-aminohexahydrocyclopenta[c]pyrrole-2(1H)-carboxylate

Tert-butyl 5-(benzylamino)hexahydrocyclopenta[c]pyrrole-2(1H)-carboxylate (1.2g, crude) was dissolved in methanol (30mL). Dihydroxypalladium (10%, 400mg) was added. The atmosphere was replaced with hydrogen gas three times, and the mixture was reacted under the atmosphere of hydrogen gas at 40°C under stirring for 16hrs. The completion of the reaction was monitored with LCMS. The reaction mixture was filtered. The filtrate was concentrated under reduced pressure to afford the target compound (700mg, crude).

LCMS (ESI) [M+H]⁺=227.2.

### Step 3: Preparation of ethyl 4-((2-(tert-butoxycarbonyl)octahydrocyclopenta[c]pyrrol-5-yl)amino)-7-methoxy-1,8-naphthyridine-3 -carboxylate

Ethyl 4-chloro-7-methoxy-1,8-naphthyridine-3-carboxylate (707mg), tert-butyl 5-aminohexahydrocyclopenta[c]pyrrole-2(1H)-carboxylate (600mg, crude) and N,N-diisopropylethylamine (3.43g) were dissolved in ethanol (20mL). The mixture was heated to 90°C and reacted for 16hrs. The completion of the reaction was monitored with LCMS. The reaction mixture was concentrated under reduced pressure to remove ethanol, diluted with water (50mL), extracted with EA (50mL×3). The organic phases were combined, dried, filtered, concentrated, and purified by column chromatography (DCM:MeOH=20:1) to afford the target compound (1.0g, yield: 82%).

LCMS (ESI) [M+H]⁺=457.2.

### Step 4: Preparation of tert-butyl 5-((3-(hydroxymethyl)-7-methoxy-1,8-naphthyridin-4-yl)amino)hexahydrocyclopenta[c]pyrrole-2(1H)-carboxylate

Ethyl 4-((2-(tert-butoxycarbonyl)octahydrocyclopenta[c]pyrrol-5-yl)amino)-7-methoxy-1,8-naphthyridine-3-carboxylate (100mg, 1.0eq.) was dissolved in dichloromethane (20mL). The mixture was cooled to -78°C. Then diisobutylaluminium hydride (2M, 0.55mL) was slowly added. The mixture was reacted at -78°C for 2hrs. The reaction mixture was quenched with water, extracted with dichloromethane (20mL×3). The organic phases were combined, dried, filtered, concentrated, and purified by column chromatography (DCM:MeOH=10:1) to afford the target compound (40mg, yield: 44%).

LCMS (ESI) [M+H]⁺=415.2.

### Step 5: Preparation of tert-butyl 5-(8-methoxy-2-oxo-2H-[1,3]oxazino[5,4-c][1,8]naphthyridin-1(4H)-yl)hexahydrocyclopenta[c]pyrrole-2(1H)-carboxylate

Tert-butyl 5-((3-(hydroxymethyl)-7-methoxy-1,8-naphthyridin-4-yl)amino)hexahydrocyclopenta[c]pyrrole-2(1H)-carboxylate (40mg, 1.0eq.) was added to tetrahydrofuran (5mL). N,N-diisopropylethylamine (124mg, 10.0eq.) was added. Triphosgene (86mg, 3.0eq.) was added. The mixture was reacted at rt for 1hr. The completion of the reaction was monitored with LCMS. The reaction mixture was diluted with water (10mL), extracted with EA (20mL×3). The organic phases were combined, dried, filtered, concentrated, and purified by column chromatography (DCM:MeOH=10:1) to afford the target compound (30mg, yield: 70%).

LCMS (ESI) [M+H]⁺=441.2.

### Step 6: Preparation of 8-methoxy-1-(octahydrocyclopenta[c]pyrrol-5-yl)-1,4-dihydro-2H-[1,3]oxazino[5,4-c][1,8]naphthyridin-2-one

Tert-butyl 5-(8-methoxy-2-oxo-2H-[1,3]oxazino[5,4-c][1,8]naphthyridin-1(4H)-yl)hexahydrocyclopenta[c]pyrrole-2(1H)-carboxylate (30mg) was added to dichloromethane (10mL). Trifluoroacetic acid (1mL) was added. The mixture was reacted at rt for 2hrs. The completion of the reaction was monitored with LCMS. The reaction mixture was rotary-evaporated to dryness in vacuum to afford the target compound (20mg, crude).

LCMS (ESI) [M+H]⁺=341.2.

### Step 7: Preparation of tert-butyl ((5-(8-methoxy-2-oxo-2H-[1,3]oxazino[5,4-c][1,8]naphthyridin-1(4H)-yl)hexahydrocyclopenta[c]pyrrole-2(1H)-yl)sulfonyl)carbamate

8-methoxy-1-(octahydrocyclopenta[c]pyrrol-5-yl)-1,4-dihydro-2H-[1,3]oxazino[5,4-c][1,8]naphthyridin-2-one (20mg, crude) and triethylamine (30mg) were added to dichloromethane (10mL). Tert-butyl N-(chlorosulfonyl)carbamate (25mg) was added dropwise at 0°C. The mixture was reacted at rt for 2hrs. The reaction mixture was diluted with water (10mL), extracted with EA (20mL×3). The organic phases were combined, dried, filtered, and concentrated to afford the target compound (20mg, crude).

LCMS (ESI) [M+H]⁺=520.1.

### Step 8: Preparation of 5-(8-methoxy-2-oxo-2H-[1,3]oxazino[5,4-c][1,8]naphthyridin-1(4H)-yl)hexahydrocyclopenta[c]pyrrole-2(1H)-sulfonamide

Tert-butyl ((5-(8-methoxy-2-oxo-2H-[1,3]oxazino[5,4-c][1,8]naphthyridin-1(4H)-yl)hexahydrocyclopenta[c]pyrrole-2(1H)-yl)sulfonyl)carbamate (20mg, crude) was added to dichloromethane (5mL). Trifluoroacetic acid (0.5mL) was added. The mixture was reacted at rt for 4hrs. The completion of the reaction was monitored with LCMS. The reaction mixture was rotary-evaporated to dryness in vacuum to afford a crude product, which was purified by liquid-phase preparative HPLC to afford the target compound (2.8mg).

LCMS (ESI) [M+H]⁺=420.1; ¹H NMR (400MHz, DMSO-d₆) δ 8.79 (s, 1H), 8.33 (d, J=9.2 Hz, 1H), 7.17 (d, J=9.2 Hz, 1H), 6.78 (s, 2H), 5.33 (s, 2H), 4.34-4.25 (m, 1H), 4.03 (s, 3H), 3.10-3.06 (m, 2H), 2.99-2.97 (m, 2H), 2.62-2.54 (m, 2H), 2.40-2.36 (m, 2H), 2.27-2.19 (m, 2H).

### Example 37

### Preparation of 1-(2,6-difluoro-4-(S-methylsulfonimidoyl)benzyl)-8-methoxy-1,4-dihydro-2H-[1,3]oxazino[5,4-c][1,8]naphthyridin-2-one (compound 216)

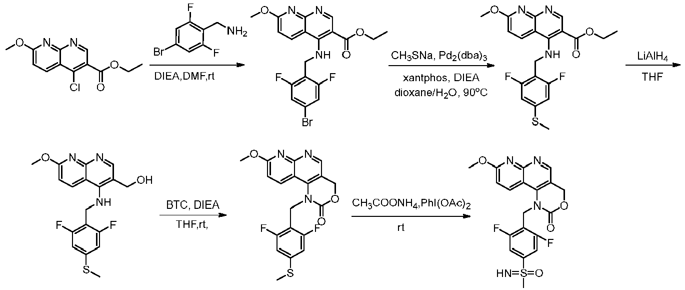

### Step 1: Preparation of ethyl 4-((4-bromo-2,6-difluorobenzyl)amino)-7-methoxy-1,8-naphthyridine-3-carboxylate

Ethyl 4-chloro-7-methoxy-1,8-naphthyridine-3-carboxylate (20g, 1.0eq.), (4-bromo-2,6-difluorophenyl)methanamine (16.65g, 1.0eq.) and N,N-diisopropylethylamine (29.08g, 3.0eq.) were added to dimethylformamide (200mL). The mixture was stirred at room temperature for 12hrs. The completion of the reaction was monitored with LCMS. The reaction mixture was added to petroleum ether (250mL), stirred, and cooled to rt. The stirring continued for 3hrs to fully precipitate a solid. The mixture was filtered, The filter cake was dried to afford the target compound (19g, yield: 56%).

LCMS (ESI) [M+H]⁺=453.9.

### Step 2: Preparation of ethyl 4-((2,6-difluoro-4-(methylthio)benzyl)amino)-7-methoxy-1,8-naphthyridine-3-carboxylate

Ethyl 4-((4-bromo-2,6-difluorobenzyl)amino)-7-methoxy-1,8-naphthyridine-3-carboxylate (450mg, 1.0eq.), sodium methyl mercaptide (69.74mg, 1.0eq.), 4,5-bis(diphenylphosphino)-9,9-dimethylxanthene (57.57mg, 0.1eq.), N,N-diisopropylethylamine (385.79mg, 3.0eq.) and bis(dibenzylideneacetone)palladium (91.12mg, 0.1eq.) were dissolved in dioxane (7mL) and water (1mL). The mixture was stirred at 90°C for 8hrs. The completion of the reaction was monitored with LCMS. The reaction mixture was diluted with water (10mL), extracted with EA (10mL×3). The organic phases were combined, dried, filtered, concentrated, and purified by a positive phase column (eluting with PE:EA=5:1) to afford the target compound (250mg, yield: 60%).

LCMS (ESI) [M+H]⁺=419.9.

### Step 3: Preparation of (4-((2,6-difluoro-4-(methylthio)benzyl)amino)-7-methoxy-1,8-naphthyridin-3-yl)methanol

Ethyl 4-((2,6-difluoro-4-(methylthio)benzyl)amino)-7-methoxy-1,8-naphthyridine-3-carboxylate (190mg, 1.0eq.) was dissolved in tetrahydrofuran (5mL). In an ice bath, lithium aluminium hydride (126.75mg, 7.4eq.) was added. The mixture was stirred for 2hrs in an ice bath. The completion of the reaction was monitored with LCMS. The reaction mixture was quenched with water (10mL), extracted with EA (10mL×3). The organic phases were combined, dried, filtered, concentrated, and purified by eluting with a reverse phase system to afford the target compound (120mg, yield: 70%).

LCMS (ESI) [M+H]⁺=378.0.

### Step 4: Preparation of 1-(2,6-difluoro-4-(methylthio)benzyl)-8-methoxy-1,4-dihydro-2H-[1,3]oxazino[5,4-c][1,8]naphthyridin-2-one

(4-((2,6-difluoro-4-(methylthio)benzyl)amino)-7-methoxy-1,8-naphthyridin-3-yl)methanol (100mg, 1.0eq.) was dissolved in tetrahydrofuran (2mL). Triphosgene (235.8mg, 3.0eq.) and N,N-diisopropylethylamine (136.98mg, 4.0eq.) were added. The mixture was reacted at 25°C for 1hr. The completion of the reaction was monitored with LCMS. The reaction mixture was diluted with water, extracted with EA. The organic phases were washed with saturated brine, dried over anhydrous sodium sulfate, and concentrated to afford the target compound (105mg, yield: 98.24%).

LCMS (ESI) (M+H)⁺=404.1.

### Step 5: Preparation of 1-(2,6-difluoro-4-(S-methylsulfonimidoyl)benzyl)-8-methoxy-1,4-dihydro-2H-[1,3]oxazino[5,4-c][1,8]naphthyridin-2-one

1-(2,6-difluoro-4-(methylthio)benzyl)-8-methoxy-1,4-dihydro-2H-[1,3]oxazino[5,4-c][1,8]naphthyridin-2-one (100mg, 1.0eq.) was dissolved in ethanol (4mL). Iodobenzene diacetate (239.54mg, 3.0eq.) and ammonium acetate (96.43mg, 5.0eq.) were added. The mixture was reacted at rt for 2hrs. The completion of the reaction was monitored with LCMS. The reaction mixture was concentrated to afford a crude product, which was purified by preparative HPLC to afford the target compound (24.05mg, yield: 22%).

LCMS (ESI) (M+H)⁺=435.1; ¹H NMR (400MHz, DMSO-d₆) δ 8.80 (s, 1H), 8.66 (d, J=9.2 Hz, 1H), 7.59 (d, J=7.2 Hz, 2H), 7.14 (d, J=9.2 Hz, 1H), 5.49 (s, 2H), 5.29 (s, 2H), 4.54 (s, 1H), 4.04 (s, 3H), 3.14 (s, 3H).

The following compounds were prepared with reference to the preparation methods of Example 1-37:

| No. | Structure | LCMS (ESI) [M+H]⁺ | No. | Structure | LCMS (ESI) [M+H] |
|---|---|---|---|---|---|
| 9 | 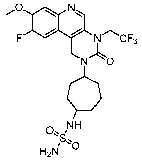 | 520.17 | 10 | 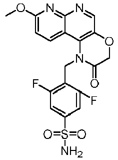 | 437.09 |
| 12 | 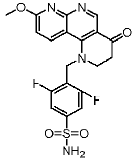 | 435.11 | 13 | 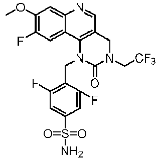 | 535.07 |
| 14 | 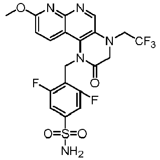 | 518.10 | 15 | 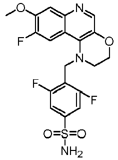 | 440.09 |
| 16 | 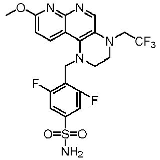 | 504.13 | 17 | 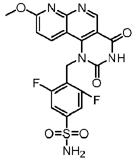 | 450.06 |
| 18 | 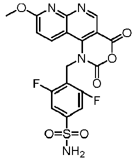 | 451.05 | 19 | 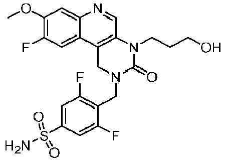 | 511.12 |
| 20 | 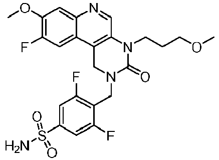 | 525.14 | 21 | 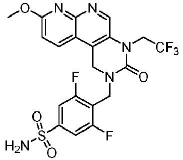 | 518.10 |
| 22 | 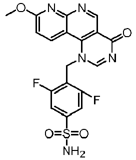 | 434.08 | 23 | 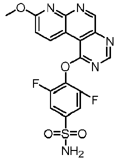 | 420.07 |
| 24 | 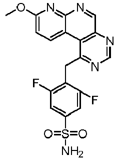 | 418.10 | 25 | 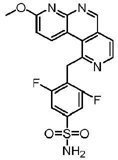 | 417.08 |
| 26 | 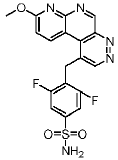 | 418.09 | 27 | 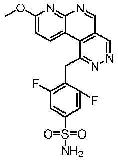 | 418.10 |
| 28 | 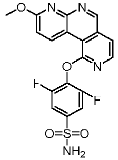 | 419.06 | 29 | 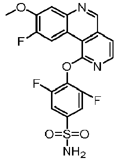 | 436.07 |
| 32 | 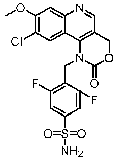 | 471.03 | 33 | 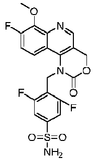 | 454.06 |
| 34 | 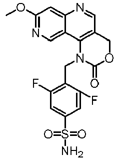 | 437.07 | 35 | 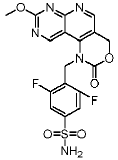 | 438.06 |
| 37 | 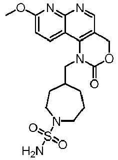 | 422.15 | 38 | 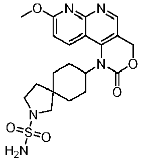 | 448.16 |
| 40 | 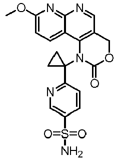 | 428.10 | 42 | 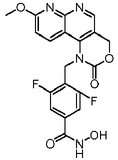 | 417.12 |
| 44 | 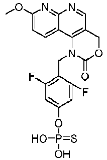 | 470.05 | 45 | 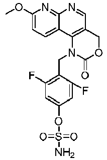 | 453.06 |
| 47 | 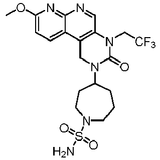 | 489.15 | 53 | 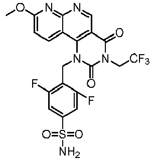 | 532.07 |
| 56 | 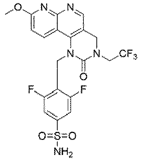 | 518.09 | 57 | 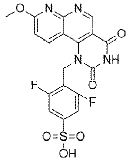 | 451.05 |
| 58 | 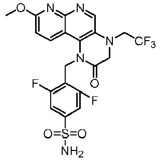 | 518.09 | 60 | 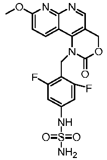 | 452.08 |
| 73 | 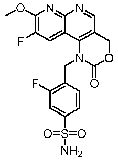 | 437.07 | 74 | 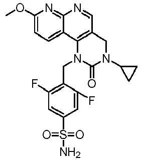 | 476.12 |
| 75 | 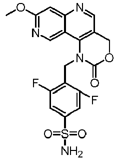 | 437.08 | 76 | 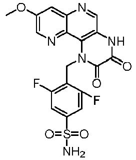 | 450.07 |
| 77 | 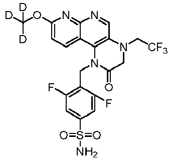 | 521.13 | 78 | 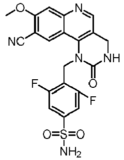 | 460.10 |
| 79 | 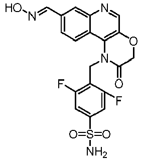 | 449.07 | 80 | 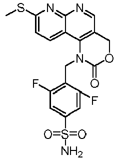 | 453.05 |
| 81 | 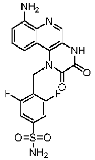 | 434.07 | 82 | 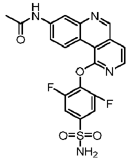 | 445.07 |
| 83 | 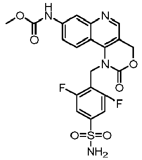 | 479.10 | 84 | 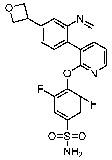 | 444.11 |
| 85 | 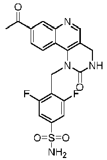 | 447.08 | 86 | 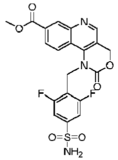 | 464.07 |
| 87 | 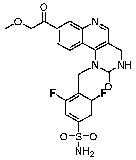 | 477.10 | 88 | 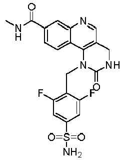 | 462.13 |
| 89 | 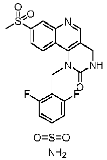 | 483.06 | 90 | 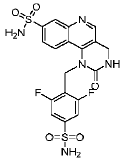 | 484.05 |
| 91 | 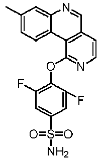 | 402.06 | 92 | 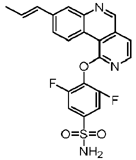 | 428.08 |
| 93 | 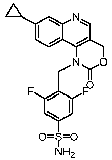 | 446.09 | 94 | 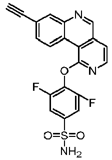 | 412.05 |
| 95 | 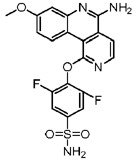 | 433.07 | 96 | 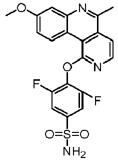 | 432.08 |
| 97 | 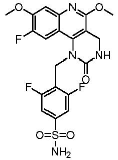 | 483.09 | 98 | 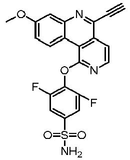 | 442.08 |
| 99 | 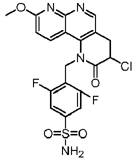 | 470.04 | 100 | 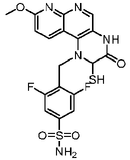 | 468.05 |
| 101 | 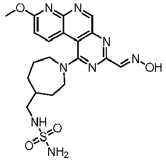 | 461.17 | 102 | 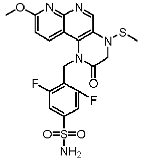 | 482.07 |
| 103 | 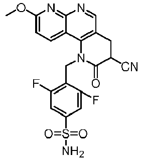 | 460.08 | 104 | 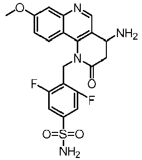 | 449.11 |
| 105 | 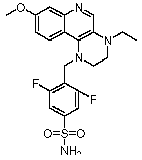 | 449.14 | 106 | 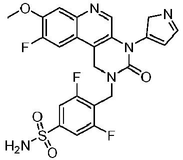 | 518.11 |
| 107 | 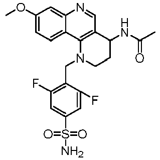 | 477.14 | 108 | 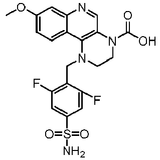 | 465.11 |
| 109 | 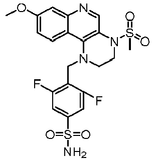 | 499.09 | 110 | 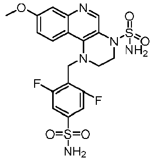 | 500.08 |
| 111 | 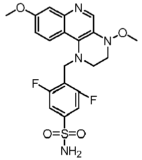 | 451.12 | 112 | 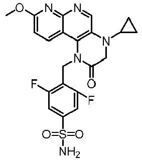 | 476.12 |
| 113 | 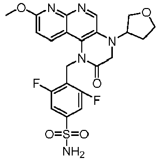 | 506.13 | 114 | 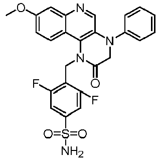 | 511.12 |
| 115 | 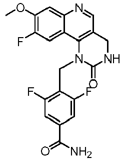 | 417.11 | 116 | 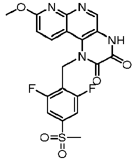 | 449.07 |
| 117 | 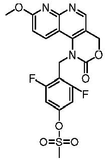 | 452.06 | 118 | 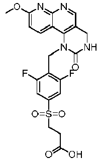 | 493.09 |
| 119 | 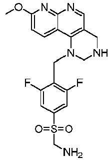 | 436.12 | 120 | 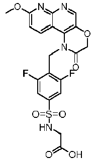 | 495.07 |
| 121 | 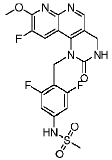 | 468.09 | 122 | 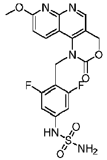 | 452.08 |
| 123 | 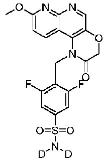 | 439.08 | 124 | 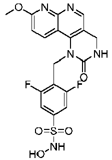 | 452.08 |
| 125 | 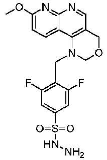 | 438.11 | 126 | 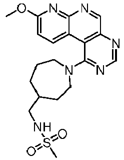 | 417.17 |
| 127 | 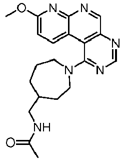 | 381.20 | 128 | 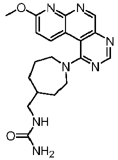 | 382.19 |
| 129 | 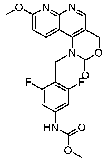 | 431.11 | 130 | 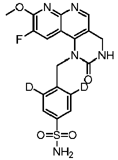 | 420.11 |
| 131 | 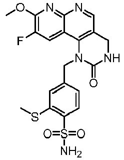 | 464.08 | 132 | 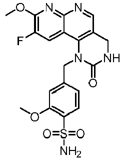 | 448.10 |
| 133 | 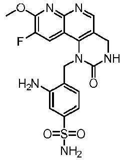 | 433.10 | 134 | 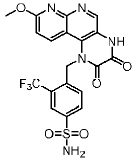 | 482.07 |
| 135 | 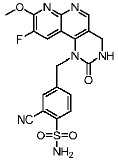 | 443.09 | 136 | 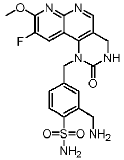 | 447.12 |
| 137 | 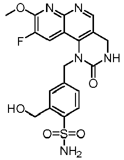 | 448.10 | 138 | 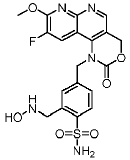 | 464.10 |
| 139 | 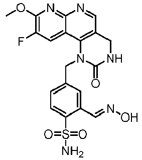 | 461.10 | 140 | 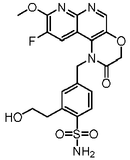 | 463.10 |
| 141 | 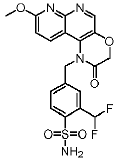 | 451.08 | 142 | 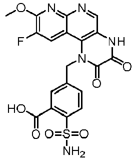 | 476.06 |
| 143 | 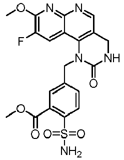 | 476.10 | 144 | 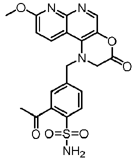 | 443.10 |
| 145 | 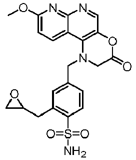 | 457.11 | 146 | 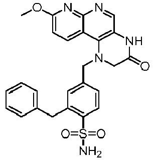 | 490.15 |
| 147 | 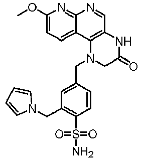 | 479.15 | 148 | 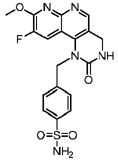 | 418.09 |
| 149 | 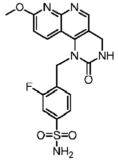 | 418.09 | 150 | 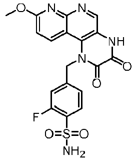 | 432.07 |
| 151 | 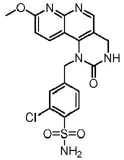 | 435.06 | 152 | 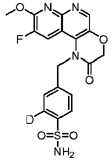 | 420.08 |
| 153 | 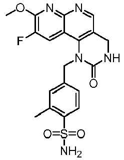 | 432.11 | 154 | 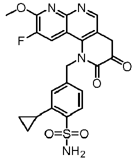 | 471.11 |
| 155 | 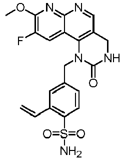 | 444.11 | 156 | 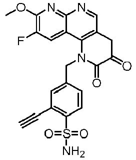 | 455.08 |
| 157 | 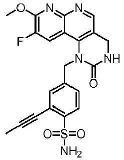 | 456.11 | 158 | 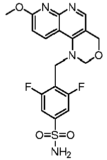 | 423.09 |
| 159 | 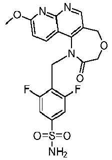 | 451.08 | 160 | 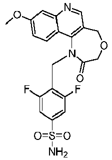 | 450.09 |
| 161 | 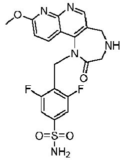 | 450.10 | 162 | 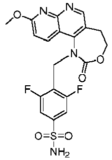 | 451.08 |
| 163 | 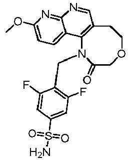 | 465.10 | 164 | 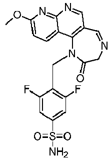 | 448.08 |
| 165 | 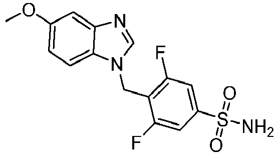 | 354.07 | 166 | 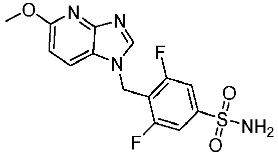 | 355.06 |
| 167 | 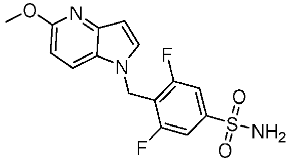 | 354.07 | 168 | 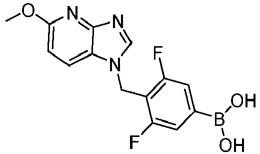 | 320.10 |
| 169 | 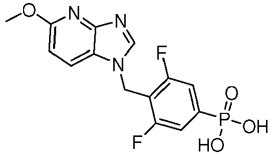 | 356.06 | 170 | 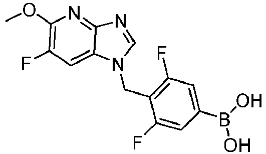 | 338.09 |
| 171 | 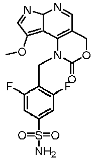 | 425.07 | 172 | 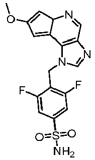 | 393.08 |
| 173 | 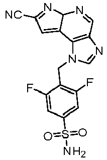 | 389.06 | 174 | 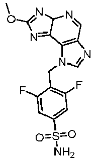 | 395.07 |
| 175 | 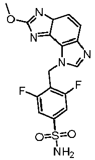 | 394.07 | 176 | 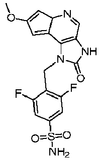 | 409.07 |
| 177 | 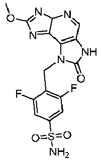 | 411.06 | 178 | 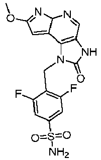 | 410.07 |
| 179 | 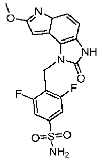 | 409.07 | 180 | 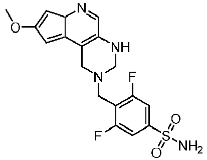 | 409.11 |
| 181 | 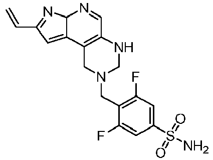 | 406.11 | 182 | 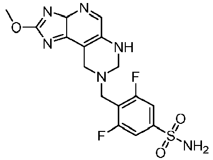 | 411.10 |
| 183 | 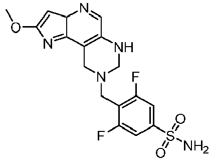 | 410.11 | 184 | 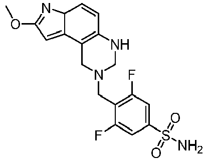 | 410.12 |
| 185 | 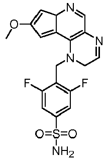 | 407.09 | 186 | 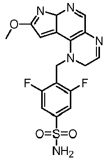 | 408.09 |
| 187 | 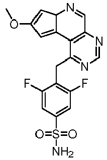 | 405.08 | 188 | 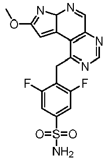 | 406.08 |
| 189 | 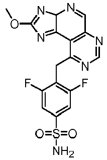 | 407.07 | 190 | 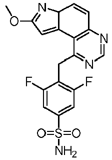 | 405.08 |
| 191 | 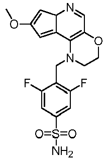 | 410.09 | 192 | 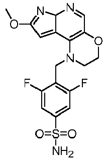 | 411.09 |
| 193 | 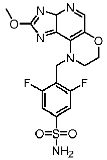 | 412.08 | 194 | 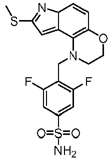 | 426.07 |
| 195 | 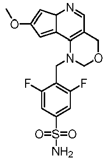 | 410.09 | 196 | 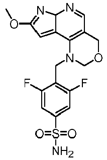 | 411.09 |
| 197 | 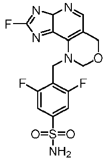 | 400.06 | 198 | 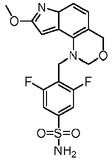 | 410.09 |
| 199 | 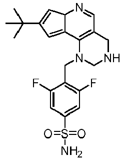 | 435.16 | 200 | 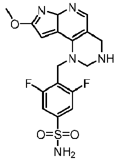 | 410.11 |
| 201 | 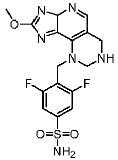 | 411.10 | 202 | 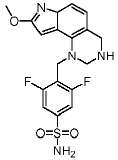 | 409.11 |
| 203 | 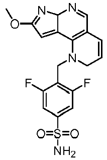 | 407.09 | 204 | 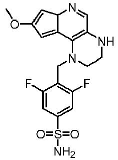 | 409.11 |
| 205 | 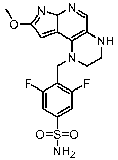 | 410.11 | 206 | 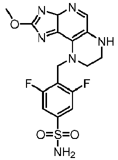 | 411.11 |
| 207 | 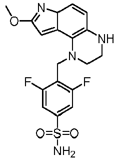 | 409.10 | 209 | 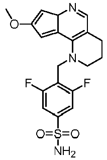 | 408.11 |
| 209 | 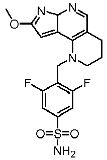 | 409.11 | 210 | 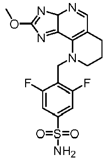 | 410.11 |
| 211 | 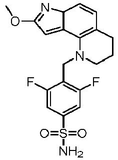 | 408.11 | 212 | 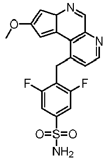 | 404.08 |
| 213 | 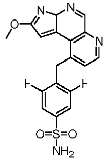 | 405.08 | 214 | 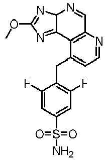 | 406.07 |
| 215 | 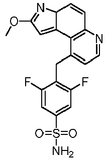 | 404.08 | | | |

### Biological assay and evaluation

### Example 1: ENPP1 enzymatic inhibition in biochemical assay

ENPP1 was a transmembrane glycoprotein capable of hydrolyzing nucleotides and derivatives with a nucleotide-5'-monophosphate structure. ENPP1 was capable of hydrolyzing artificial thymidine 5'-monophosphate p-nitrophenyl ester (TMP-pNP) into nucleotide 5'-monophosphate and p-nitrophenol which was a chromogenic product. The formation amount of p-nitrophenol product could be directly measured by its absorbance at 405nm, which was directly proportional to the enzyme activity.

### Experimental procedure

The compound was dissolved in DMSO, with stock concentration of 1 mM, and diluted in a 4-fold serial gradient to make 10 concentration points. Echo was used to transfer 300nL of gradient concentrations of compound to a 384-well assay plate (final concentration: starting at 10µM, 4-fold serial gradient dilution, 10 concentration points). First, 15µL of 0.2ng/µL hENPP1 enzyme (2x final concentration) prepared in assay buffer (250mM NaCl, 50mM Tris, pH=9.5) was added to each well. Then, 15µL of 400µM TMP-pNP (2x final concentration) diluted in assay buffer was added. After incubating at 37°C for 0.5hrs, the OD405nm value was read by the microplate reader. The percentage inhibition was calculated using the formula: %Inhibition Rate=(OD_{high signal control}-OD_{sample well})/(OD_{high signal control}-OD_{low signal control})* 100. The IC₅₀ value was calculated using four-parameter fit. The high signal control was the DMSO group without inhibitors, and the low signal control was the blank control group.

The data for the compounds including compound 131 (ZX-131) disclosed in WO2021061803A1 are shown in Table 1:

**Table 1: ENPP1 enzymatic inhibition activity**

| Compound No. | IC₅₀(nM) | Compound No. | IC₅₀(nM) | Compound No. | IC₅₀(nM) |
|---|---|---|---|---|---|
| ZX-131 | B | 048 | A | 063 | C |
| 002 | C | 049 | C | 066 | C |
| 006 | B | 050 | C | 067 | C |
| 007 | B | 051 | C | 070 | B |
| 008 | B | 054 | A | 071 | B |
| 017 | C | 055 | B | 072 | C |
| 030 | B | 056 | B | 074 | B |
| 031 | C | 062 | A | 076 | B |

| | | | | | |
|---|---|---|---|---|---|
| A represents IC₅₀<0.1nM, B represents 0.1nM<IC₅₀<0.2nM, C represents 0.2nM<IC₅₀<2nM | | | | | |

### Example 2: ENPP1 enzymatic inhibition assay incellular assay

ENPP1 was mainly expressed on the surface of cell membranes and could hydrolyze 2'3'-cGAMP into 5'-GMP and 5'-AMP. Similarly, ENPP1 could also hydrolyze the synthetic phosphate TMP-pNP, generating nucleotide-5'-monophosphate and the chromogenic product p-nitrophenol. Therefore, TMP-pNP was used as a substrate to reflect the enzyme activity of ENPP1 in cultured MDA-MB-231 cells. The amount of p-nitrophenol formed in the culture medium was determined by absorbance at 405 nm, which was directly proportional to the activity of ENPP1.

### Experimental procedure

MDA-MB-231 cells was seeded with a density of 6000 per well in 384-well plate and cultured overnight (about 18hrs) at 37°C. The compound was diluted into a 50×serial gradient dilution using an assay buffer (250mM NaCl, 50mM Tris, pH=9.5), starting at 500µM, and diluted in a 4-fold serial gradient to make 10 concentration points. The cell culture medium was removed, and 10µL of gradient concentrations of compounds were added to each well of the 384-well assay plate (final concentration: starting at 10 µM, 4-fold serial gradient dilution, with 10 concentration points). Then, 10µL of 1mM TMP-pNP (2x final concentration) dissolved in the assay buffer was added to the well. After incubating at 37°C for 3hrs, the OD405nm value was read by the microplate reader. The percentage inhibition was calculated using the formula: %Inhibition Rate=(ODhigh signal control-OD_{sample Well})/(ODhigh signal control-OD_{low signal control})* 100. The IC₅₀ value was calculated using four-parameter fit. The high signal control was the DMSO group without inhibitors, and the low signal control was the blank control group.

**Table 2: ENPP1 enzymatic inhibition activity at the cellular level**

| Compound No. | MDA-MB-231 EC₅₀(nM) | Compound No. | MDA-MB-231 EC₅₀(nM) |
|---|---|---|---|
| ZX-131 | B | 008 | A |
| 006 | A | 048 | A |
| 007 | A | 062 | A |

| | | | |
|---|---|---|---|
| A represents IC₅₀<0.5nM, B represents 0.5nM<IC₅₀<2nM | | | |

### Example 3: Pharmacokinetics using LC-MS/MS to determine compound concentration in mice

Assay principle: LC-MS/MS was used to determine the drug concentration of the target drug in plasma at different times, and to plot the pharmacokinetic curve of the target compound in vivo.

Assay method: The compound was dissolved in DMSO to prepare a stock solution of 20mg/mL. A solvent containing 5% DMSO (Sigma-Aldrich, SHBJ2847), 45% PEG400 (Sigma-Aldrich, BCCC0015), and 50% dd H₂O was used to dilute the above compound to 1mg/mL. The mice were sourced from CD-1 male mice (JH Laboratory Animal Co. LTD), with nine mice for each compound group. The mice were administered with 10mg/kg by oral gavage (PO). The blood was collected at 0.25hr, 0.5hr, 1hr, 2hr, 4hr, 8hr, and 24hr respectively with three samples taken at each time point, collecting 110µL of whole blood (K2EDTA anticoagulation). The samples were immediately centrifuged at 2000g for 5 minutes at 4°C, and the serum was collected and stored at -70°C. The drug concentration was determined using a Triple-quadrupole MS system (SCIEX), including standard curve and quality control preparation, and sample preparation. Standard curve and quality control preparation: formulating a working solution with MeOH:H₂O (1:1), and adding 3µL of the above standard curve and quality control working solution to 57µL of blank plasma. Sample preparation: 30 µL of plasma sample was added to 200µL of internal standard solution (Propranolol, 40ng/mL) and mixed for 1 minute, then centrifuged at 5800rpm for 10 minutes, and 100µL of supernatant was transferred to a new plate for sample analysis. The chromatographic conditions were optimized for each sample, including mobile phase composition, elution gradient conditions, flow rate, and retention time. The chromatographic column was Waters BEH C18 (2.1×50mm, 1.7µm), and the injection volume was 1µL. An electrospray ionization source (Tureo spray) was used in mass spectrometry. In the positive ion detection mode, the multi-channel reaction monitoring (MRM) mode was selected for secondary mass spectrometry analysis. Based on the drug concentration-time data, pharmacokinetic parameters were calculated using WinNonlin 8.2 software according to a non-compartmental model, including peak concentration Cmax, peak time Tmax, area under the drug-time curve AUC, and elimination half-life t1/2. The AUC was calculated using the linear trapezoidal method (linear up log down). The assay results showed that the compounds of this invention had good in vivo pharmacokinetics and had the potential to become drugs.

**Table 3: Pharmacokinetic parameters of compounds of this invention**

| | IV, 2 mpk | | | | PO, 10 mpk | | | | |
|---|---|---|---|---|---|---|---|---|---|
| Compound No. | T_{1/2} | AUC₀₋₂₄ₕ | CL | Vss | T_{1/2} | Cmax | Tmax | AUC₀₋₂₄ₕ | F% |
| | h | h*ng/mL | L/h/kg | L/kg | h | ng/mL | h | h*ng/mL | |
| ZX-131 | 6.01 | 4419 | 0.432 | 3.212 | 7.78 | 247 | 6 | 3274 | 15 |
| 006 | 5.35 | 10021 | 0.192 | 1.08 | 3.5 | 8080 | 2 | 47811 | 92.6 |
| 008 | 4.56 | 7450 | 0.287 | 1.518 | 3.87 | 798 | 3.17 | 7341 | 20 |
| 048 | 3.07 | 18311 | 0.115 | 0.46 | 2.79 | 4873 | 0.583 | 34842 | 38.1 |
| 054 | 3.51 | 8088 | 0.246 | 0.826 | 3.39 | 7900 | 0.5 | 20142 | 49.7 |

### Assay Example 4: Pharmacodynamic evaluation of compounds in mouse pancreatic cancer Pan02 model

1. Assay purpose: To evaluate the in vivo pharmacodynamics(efficacy) of the tested drug in a subcutaneous inoculated tumour model of mouse pancreatic cancer Pan02 cells.
2. Assay method:
   2.1 Cell culture: Mouse pancreatic cancer Pan02 cells were cultured in a monolayer in vitro in a DMEM culture medium supplemented with 10% fetal bovine serum, 0.01mg/ml insulin, 1% penicillin/streptomycin/amphotericin B in an incubator at 37°C with 5% CO₂. When cell saturation confluence 80%-90%, cells were collected, counted, and inoculated.
   2.2 Animals: Female C57BL/6 mice, aged 6-8 weeks, weighing 18-22 grams, were provided by Sino-British Sippr/BK Lab Animal Ltd or other suppliers.
   2.3 Tumor inoculation and animal grouping administration: 0.1mL Pan02 cells (5×10⁶ cells) were inoculated subcutaneously into the right hind back of each mouse, and the grouping administration began when the average tumour volume reached about 100-200mm³. Animals were weighed and tumour volumes were measured before administration. Mice were randomly divided into groups based on tumour volume, with 8 mice in each group. The control group received the vehicle: 5% DMSO+45% PEG400+50% H₂O (1% CMC-Na), while the treatment group received an oral gavage dose of 20mg/kg with a dosing volume of 10µL/g body weight twice daily. Animals were observed daily for health status, and if the tumour volume exceeded 3,000mm³, or if there were signs of severe illness, pain, weight loss greater than 20%, or continuous deterioration, euthanasia was performed.
   2.4 Tumor growth inhibition rate: tumour diameters were measured twice a week using a vernier calliper. Tumor volume was calculated using the formula: V=0.5a×b², where a and b represented the long and short diameters of the tumour, respectively. The tumour growth inhibition TGI rate (%) of the compound was calculated as follows: TGI(%)=[(1-(average tumour volume after administration in the assay group-average tumour volume before administration in the assay group))/(average tumour volume before administration in the vehicle control group-average tumour volume after administration in the vehicle control group)] × 100%. Data analysis was performed using a T-test for comparison between two groups, with p<0.05 considered a significant difference.
3. Assay result:
   Based on the mouse pancreatic cancer Pan02 model, the example compounds of this invention (e.g. those that performed well in the previous assay examples) could significantly inhibit tumour growth compared to the control group, with a statistically significant difference (p<0.05), and had no inhibitory effect on mouse body weight, indicating good safety.

### Assay example 5: Pharmacodynamic evaluation of compounds in a myocardial infarction model

1. Assay purpose: This study aimed to evaluate the pharmacodynamics(efficacy) of compounds in an SD rat myocardial infarction (MI) model.
2. Assay method
   2.1 Model establishment
      After one week of adaptation at the facility, animals were subj ected to ligation of the left anterior descending coronary artery (LAD) to create a myocardial infarction (MI) model. After surgery, penicillin (80000 units/rat i.m) was administered for 3 days and disinfected to prevent the infection.
   2.2 Grouping and drug administration
      One day after model establishment, the MI animals were randomly divided into four groups based on baseline weight and echocardiographic, with 4-6 animals in each group. The control group received the vehicle solution: 5% DMSO+45% PEG400+50% H₂O (1% CMC-Na), while the treatment group received oral gavage doses of 30mg/kg and 75mg/kg twice daily for 7 consecutive days. Animal weights were monitored daily after administration, and echocardiographic imaging were measured 7 days later. The primary echocardiographic evaluation were left ventricular fractional shortening (FS) and left ventricular ejection fraction (LVEF).
3. Assay result
   The compounds of this invention (e.g. those that performed well in the previous assay examples) exhibited activity in the rat myocardial infarction model. On the 7th day after administration, the compounds of this invention could significantly increase the left ventricular ejection fraction (LVEF) and the left ventricular fractional shortening (FS) in rats and exhibited a dose-dependent trend. At 30mpk, some compounds of this invention had LVEF>5%, FS>5%, some preferred compounds had LVEF>7%, FS>6%; some more preferred compounds had LVEF>10%, FS>8%. At 75mpk, some compounds of this invention had LVEF>8%, FS7%, some preferred compounds had LVEF>12%, FS>10%; some more preferred compounds had LVEF>15%, FS>12%. The statistical significance was analyzed using a t-test, with p<0.05 considered a significant difference.

## Claims

1. A compound represented by the following formula (I), a stereoisomer or tautomer of the compound or a mixture thereof, a pharmaceutically acceptable salt, co-crystal, polymorph or solvate of the compound, or a stable isotope derivative, metabolite or prodrug of the compound: 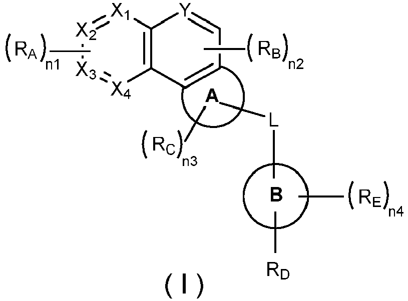
wherein X₁, X₂, X₃, X₄ and Y are each independently CH or N;
and when X₁, X₂, X₃ and X₄ are all CH, Y is not CH;
R_{A} is a substituent of the ring where X₁ is located, R_{B} is a substituent of the ring where Y is located, R_{A} and R_{B} are each independently hydrogen, deuterium, halogen, oxime, -CN, -OH, -SH, -NO₂, -NH₂, -Z-OR₁, -Z-SR₁, -Z-NR₂R₃, -Z-NR₂C(O)R₄, -Z-NR₂C(O)OR₁, -Z-C(O)R₄, -Z-C(O)OR₁, -Z-C(O)(CR₅R₆)ₙC(O)R₄, -Z-C(O)(CR₅R₆)ₙC(O)OR₁, - Z-C(O)NR₂R₃, -Z-S(O)₂R₄, -Z-S(O)₂NR₂R₃, C₁₋₆ alkyl, C₂-₆ alkenyl, C₂-₆ alkynyl, C₁₋₆ alkoxy, C₁₋₆ alkylthio, C₃-₈ cycloalkyl, C₃-₈ cycloalkenyl, 3-20 membered heterocyclyl, 5-16 membered heteroaryl; the alkyl, alkenyl, alkynyl, alkoxy, alkylthio, cycloalkyl, cycloalkenyl, heterocyclyl, or heteroaryl is optionally substituted by one or more substituents selected from deuterium, halogen, oxo, oxime, -CN, -OH, -NO₂, -NH₂, C₁₋₆ alkyl, phenyl, C₁₋₆ alkoxy,
C₁₋₆ haloalkyl, C₁₋₆ haloalkoxy, and halophenyl;
n1 is 1, 2, 3 or 4;
n2 is 1 or 2;
ring A is 6-10 membered heterocyclyl or 6-12 membered heteroaryl;
R_{C} is a substituent of ring A, and R_{C} is each independently hydrogen, deuterium, halogen, oxo, oxime, -CN, -OH, - SH, -NO₂, -NH₂, -Z-OR₁, -Z-SR₁, -Z-NR₂R₃, -Z-NR₂C(O)R₄, -Z-NR₂C(O)OR₁, -Z-C(O)R₄, -Z-C(O)OR₁, -Z-C(O)(CR₅R₆)ₙC(O)R₄, -Z-C(O)(CR₅R₆)ₙC(O)OR₁, -Z-C(O)NR₂R₃, -Z-S(O)₂R₄, -Z-S(O)₂NR₂R₃, C₁₋₆alkyl, C₂-₆alkenyl, C₂-₆alkynyl, C₁₋₆alkoxy, C₁₋₆alkylthio, C₃-₈cycloalkyl, C₃-₈cycloalkenyl, 3-20 membered heterocyclyl, C₆-₁₂aryl, 5-16 membered heteroaryl; the alkyl, alkenyl, alkynyl, alkoxy, alkylthio, cycloalkyl, cycloalkenyl, heterocyclyl, aryl, or heteroaryl is optionally substituted by one or more substituents selected from deuterium, halogen, oxo, oxime, -CN, -OH, -NO₂, -NH₂, C₁₋₆alkyl, phenyl, C₁₋₆alkoxy, C₁₋₆haloalkyl, C₁₋₆haloalkoxy, and halophenyl;
n3 is 1, 2, 3, 4, 5, or 6;
Z is selected from a bond, C₁₋₃alkylene, C₁₋₃alkyleneoxy, and C₁₋₃alkylenethio, the alkylene, alkyleneoxy, or alkylenethio is optionally substituted by one or more substituents selected from deuterium, halogen, oxo, -CN, -OH, and -NH₂;
ring B is C₃-₁₀cycloalkyl, 3-20 membered heterocyclyl, C₆-₁₄aryl or 5-16 membered heteroaryl;
R_{D} is selected from -W-OC(O)OR₁, -W-C(O)NR₂R₃, -W-C(O)NR₂OR₁, -W-OC(O)NR₂R₃, -W-NR₂C(O)R₄, -W-NR₂C(O)OR₁, -W-NR₂C(O)NR₂R₃, -W-S(O)₂R₄, -W-SO₂NR₂R₃, -W-S(O)=NR₂, -W-S(O)=NR₂NR₂R₃, -W-NR₂S(O)₂R₄, -W-OS(O)₂R₄, -W-NR₂S(O)₂NR₂R₃, -W-OS(O)₂NR₂R₃, -W-P(O)(OR₁)₂, -W-P(S)(OR₁)₂, -W-OP(S)(OR₁)₂, and -W-B(OH)₂;
R_{E} is each independently hydrogen, deuterium, halogen, oxo, oxime, carboxyl, -CN, -OH, -SH, -NO₂, -NH₂, -W-OR₁, -W-SR₁, -W-C(O)R₄, -W-C(O)OR₁, -W-OC(O)R₁, -W-OC(O)OR₁, -W-C(O)NR₂R₃, -W-C(O)NR₂OR₁, -W-OC(O)NR₂R₃, -W-NR₂R₃, -W-NR₂C(O)R₄, -W-NR₂C(O)OR₁, -W-NR₂C(O)NR₂R₃, -W-S(O)₂R₄, -W-SO₂NR₂R₃, - W-NR₂S(O)₂R₄, -W-OS(O)₂R₄, -W-NR₂S(O)₂NR₂R₃, -W-OS(O)₂NR₂R₃, -W-P(O)(OR₁)₂, -W-P(S)(OR₁)₂, -W-OP(S)(OR₁)₂, -W-B(OH)₂, C₁₋₆alkyl, C₂-₆alkenyl, C₂-₆alkynyl, C₁₋₆alkoxy, C₃-₈cycloalkyl, C₃-₈cycloalkenyl, 3-20 membered heterocyclyl, 5-16 membered heteroaryl; the alkyl, alkenyl, alkynyl, alkoxy, cycloalkyl, cycloalkenyl, heterocyclyl, or heteroaryl is optionally substituted by one or more substituents selected from deuterium, halogen, oxo, oxime, -CN, -OH, -NO₂, -NH₂, C₁₋₆alkyl, phenyl, C₁₋₆alkoxy, C₁₋₆haloalkyl, C₁₋₆haloalkoxy, and halophenyl;
n is 1, 2, or 3;
n4 is 1, 2, 3, 4, 5, or 6;
W is selected from a bond, C₁₋₃alkylene, C₁₋₃alkyleneoxy, and C₁₋₃alkylenethio, the alkylene, alkyleneoxy, or alkylenethio is optionally substituted by one or more substituents selected from deuterium, halogen, oxo, -CN, -OH, and -NH₂;
L is selected from a bond, -O-, -S-, C₁₋₆alkylene, C₁₋₆alkyleneoxy, C₃₋₆cycloalkylene, C₁₋₆alkylenethio, C₂-₆alkenylene, and C₂-₆alkynylene, the alkylene, alkyleneoxy, cycloalkylene, alkylenethio, alkenylene, or alkynylene is optionally substituted by one or more substituents selected from deuterium, halogen, C₁₋₃alkyl, C₁₋₆alkoxy, oxo, - CN, -OH, and -NH₂;
R₁ at each occurrence is independently hydrogen, C₁₋₆alkyl, C₁₋₆alkoxy, C₃-₈cycloalkyl, phenyl, or 3-20 membered heterocyclyl, wherein the alkyl, alkoxy, cycloalkyl, phenyl, or heterocyclyl is optionally substituted by one or more substituents selected from halogen, cyano, hydroxy, amino, C₁₋₃alkyl, C₁₋₄alkoxy, phenyl, C₁₋₃haloalkyl, C₁₋₄haloalkoxy, and halophenyl;
R₂ and R₃ at each occurrence are independently hydrogen, C₁₋₆alkyl, or C₁₋₆alkoxy, wherein the alkyl, or alkoxy is optionally substituted by one or more substituents selected from halogen, deuterium, cyano, hydroxy, amino, carboxyl, C₁₋₃alkyl, C₁₋₄alkoxy, C₁₋₄alkyl, C₃-₈cycloalkyl, phenyl, C₁₋₃haloalkyl, C₁₋₃haloalkoxy, and halophenyl; R₄ at each occurrence is independently hydrogen, deuterium, C₁₋₆alkyl, C₁₋₆alkoxy, C₃-₈cycloalkyl, phenyl, or 3-20 membered heterocyclyl, wherein the alkyl, alkoxy, cycloalkyl, phenyl, or heterocyclyl is optionally substituted by one or more substituents selected from halogen, cyano, hydroxy, amino, C₁₋₃alkyl, C₁₋₄alkoxy, phenyl, C₁₋₃haloalkyl, C₁₋₃haloalkoxy, and halophenyl.
R₅ and R₆ at each occurrence are independently hydrogen, C₁₋₃alkyl, C₂-₆alkenyl, C₂-₆alkynyl, C₁₋₆alkoxy, C₁₋₃alkylthio, C₃₋₆cycloalkyl, C₃₋₆cycloalkenyl, 3-20 membered heterocyclyl, or 5-16 membered heteroaryl, wherein the alkyl, alkenyl, alkynyl, alkoxy, alkylthio, cycloalkyl, cycloalkenyl, heterocyclyl, or heteroaryl is optionally substituted by one or more substituents selected from deuterium, halogen, oxo, oxime, -CN, -OH, -NH₂, C₁₋₃alkyl, and C₁₋₃haloalkyl;
in the case that multiple R_{A}s, R_{B}s, Res or R_{E}s appear at the same time, R_{A}s, R_{B}s, Res or R_{E}s can be identical or different from each other;
unless otherwise stated, the heteroatoms in the above-mentioned heterocyclyl and heteroaryl are independently selected from O, N and S, and the number of heteroatoms is 1, 2, 3, or 4.

2. The compound according to claim 1, a stereoisomer or tautomer of the compound or a mixture thereof, a pharmaceutically acceptable salt, co-crystal, polymorph or solvate of the compound, or a stable isotope derivative, metabolite or prodrug of the compound, wherein X₁ is N, and X₂, X₃, and X₄ are all CH; or X₃ is N, and X₁, X₂ and X₄ are all CH; or X₁ and X₃ are both N and X₂ and X₄ are both CH; or X₁, X₂, X₃, and X₄ are all CH.

3. The compound according to any of claims 1-2, a stereoisomer or tautomer of the compound or a mixture thereof, a pharmaceutically acceptable salt, co-crystal, polymorph or solvate of the compound, or a stable isotope derivative, metabolite or prodrug of the compound, wherein Y is CH; or Y is N.

4. The compound according to any of claims 1-3, a stereoisomer or tautomer of the compound or a mixture thereof, a pharmaceutically acceptable salt, co-crystal, polymorph or solvate of the compound, or a stable isotope derivative, metabolite or prodrug of the compound, wherein each R_{A} is independently hydrogen, deuterium, halogen, oxime, - CN, -OH, -SH, -NO₂, -NH₂, -Z-NR₂C(O)R₄, -Z-NR₂C(O)OR₁, 3-6 membered heterocyclyl, -Z-C(O)R₄, -Z-C(O)OR₁, -Z-C(O)NR₂R₃, -Z-S(O)₂R₄, -Z-S(O)₂NR₂R₃, C₁₋₆alkyl, C₂-₆alkenyl, C₂-₆alkynyl, C₁₋₆alkoxy, C₁₋₆alkylthio, or C₃-₈cycloalkyl, the alkyl, alkenyl, alkynyl, alkoxy, alkylthio, or cycloalkyl is optionally substituted by one or more substituents selected from deuterium, halogen, oxo, oxime, -CN, -OH, -NO₂, -NH₂, C₁₋₆alkyl, phenyl, C₁₋₆alkoxy, C₁₋₆haloalkyl, C₁₋₆haloalkoxy, and halophenyl;
preferably, each R_{A} is independently hydrogen, deuterium, halogen, oxime, -CN, -OH, -SH, -NO₂, -NH₂, -Z-NR₂C(O)R₄, -Z-NR₂C(O)OR₁, 3-6 membered heterocyclyl, -Z-C(O)R₄, -Z-C(O)OR₁, -Z-C(O)NR₂R₃, -Z-S(O)₂R₄, - Z-S(O)₂NR₂R₃, C₁₋₄alkyl, C₂-₆alkenyl, C₂-₆alkynyl, C₁₋₆alkoxy, C₁₋₆alkylthio, or C₃₋₆cycloalkyl, the alkyl, alkenyl, alkynyl, alkoxy, alkylthio, or cycloalkyl is optionally substituted by one or more substituents selected from deuterium, halogen, oxo, oxime, -CN, -OH, -NO₂, -NH₂, C₁₋₆alkyl, phenyl, C₁₋₆alkoxy, C₁₋₆haloalkyl, C₁₋₆haloalkoxy, and halophenyl;
further preferably, each R_{A} is independently hydrogen, deuterium, halogen, oxime, -CN, -OH, -SH, -NO₂, -NH₂, -Z-NR₂C(O)R₄, -Z-NR₂C(O)OR₁, 3-6 membered heterocyclyl, -Z-C(O)R₄, -Z-C(O)OR₁, -Z-C(O)NR₂R₃, -Z-S(O)₂R₄, - Z-S(O)₂NR₂R₃, C₁₋₄alkyl, C₂-₄alkenyl, C₂-₄alkynyl, C₁₋₃alkoxy, C₁₋₃alkylthio, or C₃₋₆cycloalkyl, the alkyl, alkenyl, alkynyl, alkoxy, alkylthio, or cycloalkyl is optionally substituted by one or more substituents selected from deuterium, halogen, oxo, -CN, -OH, -NO₂, -NH₂, C₁₋₆alkyl, C₁₋₆alkoxy, C₁₋₆haloalkyl, and C₁₋₆haloalkoxy;
still further preferably, each R_{A} is independently hydrogen, deuterium, F, Cl, Br, oxime, -CN, -OH, -SH, -NO₂, -NH₂, -NR₂C(O)R₄, -NR₂C(O)OR₁, 3-6 membered heterocyclyl, -C(O)R₄, -C(O)OR₁, -C(O)NR₂R₃, -S(O)₂R₄, - S(O)₂NR₂R₃, methyl, ethyl, propyl, isopropyl, tert-butyl, ethenyl, ethynyl, methoxy, ethoxy, propoxy, isopropoxy, methylthio, ethylthio, propylthio, isopropylthio, cyclopropyl, cyclobutyl, trifluoromethyl, difluoromethyl, monofluoromethyl, trifluoroethyl, difluoroethyl, monofluoroethyl, hydroxypropyl, hydroxyethyl, hydroxymethyl, methoxypropyl, methoxyethyl, methoxymethyl, ethenyl, ethynyl, propenyl, or propynyl;
more preferably, each R_{A} is independently hydrogen, F, Cl, Br, oxime, -CN, -OH, -NH₂, -NHC(O)C₁₋₃alkyl, - NHC(O)OC₁₋₃alkyl, 3-4 membered heterocyclyl, -C(O)C₁₋₃alkyl, -C(O)OC₁₋₃alkyl, -C(O)NHC₁₋₃alkyl, -S(O)₂C₁₋₃alkyl, -S(O)₂NH₂, -S(O)₂NHC₁₋₃alkyl, methyl, ethyl, methoxy, ethoxy, methylthio, ethenyl, propenyl, ethynyl, cyclopropyl, or tert-butyl;
most preferably, R_{A} is selected from hydrogen, F, Cl, -CN, -NH₂, oxime, methyl, methoxy, ethoxy, methylthio, - OCD₃, -NHCOCH₃, -NHCOOCH₃, 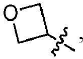 -COCH₃, -COOCH₃, -COCH₂OCH₃, -CONHCH₃, -SO₂CH₃, -SO₂NH₂, -CH=CH-CH₃, ethynyl, cyclopropyl, and tert-butyl.

5. The compound according to any of claims 1-4, a stereoisomer or tautomer of the compound or a mixture thereof, a pharmaceutically acceptable salt, co-crystal, polymorph or solvate of the compound, or a stable isotope derivative, metabolite or prodrug of the compound, wherein n1 is 1, 2, or 3; more preferably, n1 is 1 or 2.

6. The compound according to any of claims 1-5, a stereoisomer or tautomer of the compound or a mixture thereof, a pharmaceutically acceptable salt, co-crystal, polymorph or solvate of the compound, or a stable isotope derivative, metabolite or prodrug of the compound, wherein each R_{B} is independently hydrogen, deuterium, halogen, oxime, - CN, -OH, -SH, -NO₂, -NH₂, C₁₋₃alkyl, C₂-₆alkenyl, C₂-₆alkynyl, C₁₋₆alkoxy, C₁₋₆alkylthio, or C₃₋₆cycloalkyl, the alkyl, alkenyl, alkynyl, alkoxy, alkylthio, or cycloalkyl is optionally substituted by one or more substituents selected from deuterium, halogen, oxo, oxime, -CN, -OH, -NO₂, -NH₂, C₁₋₆alkyl, phenyl, C₁₋₆alkoxy, C₁₋₆haloalkyl, C₁₋₆haloalkoxy, and halophenyl;
further preferably, each R_{B} is independently hydrogen, deuterium, F, Cl, Br, -CN, -OH, -SH, -NO₂, -NH₂, methyl, ethyl, propyl, isopropyl, ethenyl, propenyl, ethynyl, propynyl, methoxy, ethoxy, propoxy, isopropoxy, methylthio, ethylthio, propylthio, isopropylthio, cyclopropyl, cyclobutyl, trifluoromethyl, difluoromethyl, monofluoromethyl, trifluoroethyl, difluoroethyl, monofluoroethyl, hydroxypropyl, hydroxyethyl, hydroxymethyl, methoxypropyl, methoxyethyl, or methoxymethyl;
more preferably, each R_{B} is independently hydrogen, F, Cl, Br, -OH, -NH₂, methyl, ethyl, methoxy, ethoxy, ethenyl, or ethynyl;
most preferably, R_{B} is hydrogen, -NH₂, methyl, methoxy, or ethynyl.

7. The compound according to any of claims 1-6, or a prodrug, tautomer, stereoisomer, metabolite, isotope derivative thereof or a mixture thereof, or a pharmaceutically acceptable salt thereof, co-crystal, polymorph or solvate thereof, wherein n2 is 1.

8. The compound according to any of claims 1-7, or a prodrug, tautomer, stereoisomer, metabolite, isotope derivative thereof or a mixture thereof, or a pharmaceutically acceptable salt thereof, co-crystal, polymorph or solvate thereof, wherein ring A is 6-9 membered heterocyclyl or 6-12 membered heteroaryl; preferably, ring A is 6-8 membered heterocyclyl or 6-8 membered heteroaryl;
preferably, ring A is 6-membered heterocyclyl or 6-membered heteroaryl;
preferably, ring A is piperidyl, hexahydropyrimidyl, piperazinyl, 1,3-oxazinanyl, morpholinyl, thiomorpholinyl, 1,3-thiazinyl, 1,2,3,6-tetrahydropyridyl, 1,2,3,6-tetrahydropyrazinyl, 1,4,5,6-tetrahydropyrimidyl, homopiperidyl, homopiperazinyl, homomorpholinyl, pyridyl, pyridazinyl, pyrimidyl, 1,4-diazepane, 1,5-diazocane, 1,4-oxazepane, 1,3-oxazepane, 1,4-oxazacyclooctane, 2,3,6,7-tetrahydro-1,4-diazepine;
more preferably, ring A is 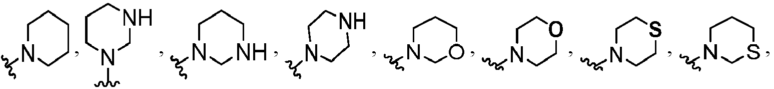 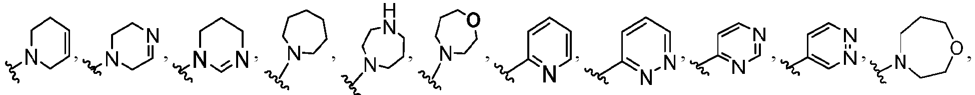 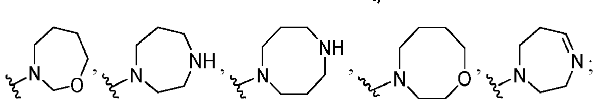 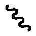 represents the attachment position of the L group;
still further preferably, ring A is 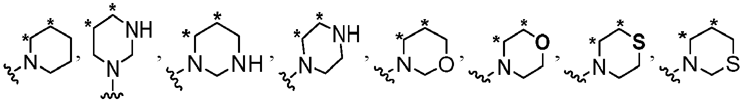 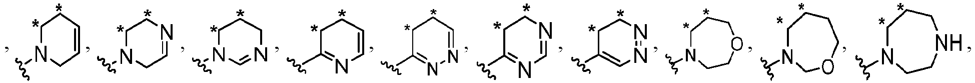 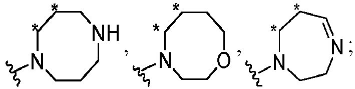 ** represents the site of fusing, 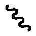 represents the attachment position of the L group.

9. The compound according to any of claims 1-8, or a prodrug, tautomer, stereoisomer, metabolite, isotope derivative thereof or a mixture thereof, or a pharmaceutically acceptable salt thereof, co-crystal, polymorph or solvate thereof, wherein each R_{C} is independently hydrogen, deuterium, halogen, oxo, oxime, -CN, -OH, -SH, -NO₂, -NH₂, - ZC(O)OR₁, -ZNR₂C(O)R₄, -Z-S(O)₂R₄, -Z-S(O)₂NR₂R₃, C₁₋₆alkyl, C₂-₆alkenyl, C₂-₆alkynyl, C₁₋₆alkoxy, C₁₋₆alkylthio, C₃₋₆cycloalkyl, 3-8 membered heterocyclyl, 5-8 membered heteroaryl, or C₆-₁₂aryl; the alkyl, alkenyl, alkynyl, alkoxy, alkylthio, cycloalkyl, heterocyclyl, heteroaryl or aryl is optionally substituted by one or more substituents selected from hydrogen, deuterium, halogen, oxo, oxime, -CN, -OH, -NO₂, -NH₂, C₁₋₆alkyl, phenyl, C₁₋₆alkoxy, C₁₋₆haloalkyl, C₁₋₆haloalkoxy, and halophenyl;
preferably, each R_{C} is independently hydrogen, deuterium, halogen, oxo, oxime, -CN, -OH, -SH, -NO₂, -NH₂, - ZC(O)OR₁, -ZNR₂C(O)R₄, -Z-S(O)₂R₄, -Z-S(O)₂NR₂R₃, C₁₋₃alkyl, C₂-₄alkenyl, C₂-₄alkynyl, C₁₋₆alkoxy, C₁₋₆alkylthio, C₃₋₆cycloalkyl, 3-6 membered heterocyclyl, 5-8 membered heteroaryl, or C₆-₁₂aryl; the alkyl, alkenyl, alkynyl, alkoxy, alkylthio, cycloalkyl, heterocyclyl, heteroaryl or aryl is optionally substituted by one or more substituents selected from hydrogen, deuterium, halogen, oxo, oxime, -CN, -OH, -NO₂, -NH₂, C₁₋₆alkyl, C₁₋₆alkoxy, C₁₋₆haloalkyl, and C₁₋₆haloalkoxy;
further preferably, each R_{C} is independently hydrogen, halogen, oxo, oxime, -CN, -OH, -SH, -NH₂, -C(O)OC₁₋₆alkyl, carboxyl, -NHC(O)C₁₋₆alkyl, -S(O)₂C₁₋₆alkyl, -S(O)₂NH₂, -CH₂S(O)₂C₁₋₆alkyl, C₁₋₃alkyl, C₂-₄alkenyl, C₂-₄alkynyl, C₁₋₃alkoxy, C₁₋₃alkylthio, C₃₋₆cycloalkyl, 3-6 membered heterocyclyl, 5-8 membered heteroaryl, or C₆-₁₂aryl; the alkyl, alkenyl, alkynyl, alkoxy, alkylthio, cycloalkyl, heterocyclyl, heteroaryl or aryl is optionally substituted by one or more substituents selected from halogen, oxo, oxime, -CN, -OH, -NO₂, -NH₂, C₁₋₃alkyl, C₁₋₃alkoxy, C₁₋₃haloalkyl, and C₁₋₃haloalkoxy;
still further preferably, each Rc is independently hydrogen, F, Cl, Br, oxo, oxime, -CN, -OH, -SH, -NH₂, -C(O)OC₁₋₄alkyl, carboxyl, -NHC(O)C₁₋₄alkyl, -S(O)₂C₁₋₄alkyl, -S(O)₂NH₂, -CH₂S(O)₂C₁₋₄alkyl, tetrahydrofuryl, pyrrolyl, phenyl, methyl, ethyl, n-propyl, isopropyl, cyclopropyl, cyclobutyl, cyclopentyl, cyclohexyl, ethenyl, propenyl, ethynyl, propynyl, methoxy, methylthio, trifluoromethyl, difluoromethyl, monofluoromethyl, trifluoroethyl, difluoroethyl, monofluoroethyl, hydroxypropyl, hydroxyethyl, hydroxymethyl, methoxypropyl, methoxyethyl, or methoxymethyl;
most preferably, each Rc is independently hydrogen, Cl, oxo, oxime, -CN, -OH, -SH, -NH₂, -Boc, -COCH₂OH, - NHC(O)CH₃, carboxyl, -S(O)₂CH₃, -S(O)₂NH₂, -CH₂S(O)₂CH₃, methoxy, methylthio, -CH₂CH₂CH₂OH, - CH₂CH₂CH₂OCH₃, ethyl, 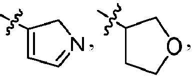 -CH₂CF₃, cyclopropyl, phenyl, or ethenyl.

10. The compound according to any of claims 1-9, or a prodrug, tautomer, stereoisomer, metabolite, isotope derivative thereof or a mixture thereof, or a pharmaceutically acceptable salt thereof, co-crystal, polymorph or solvate thereof, wherein n3 is 1, 2, or 3.

11. The compound according to any of claims 1-10, or a prodrug, tautomer, stereoisomer, metabolite, isotope derivative thereof or a mixture thereof, or a pharmaceutically acceptable salt thereof, co-crystal, polymorph or solvate thereof, wherein preferably, when one or two R_{C}s therein are oxo, the above-mentioned ring A substituted by one or two R_{C}s is 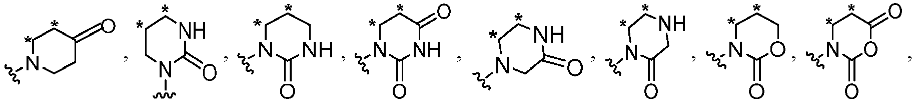 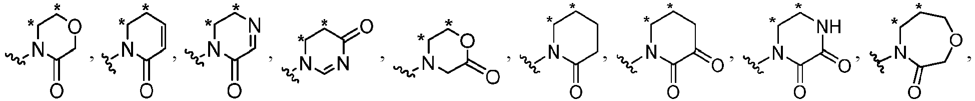 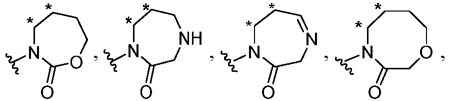 ** represents the site of fusing, 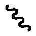 represents the attachment position of the L group;
more preferably, the ring A substituted by Rc(s) is

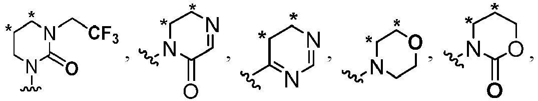

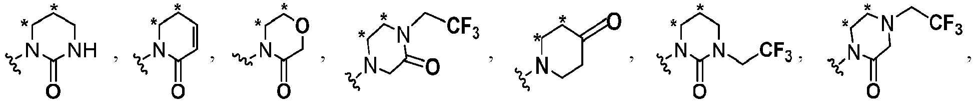

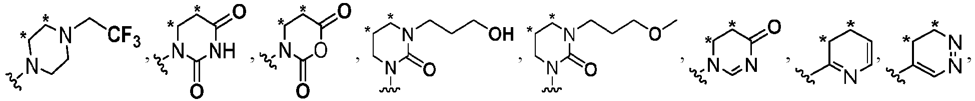

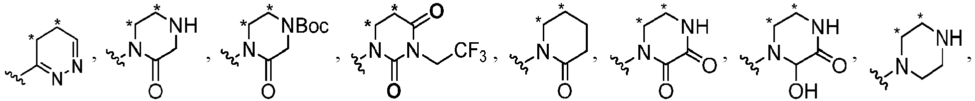

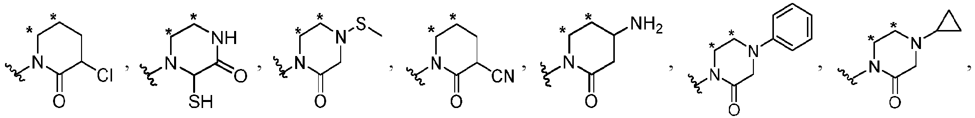

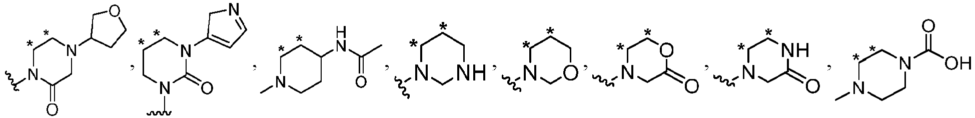

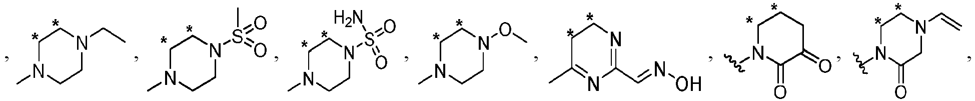

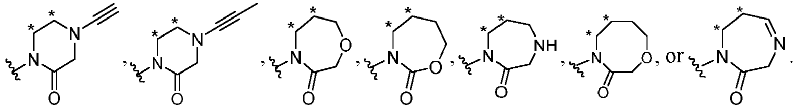

12. The compound according to any of claims 1-11, or a prodrug, tautomer, stereoisomer, metabolite, isotope derivative thereof or a mixture thereof, or a pharmaceutically acceptable salt thereof, co-crystal, polymorph or solvate thereof, wherein 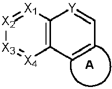 is selected from:

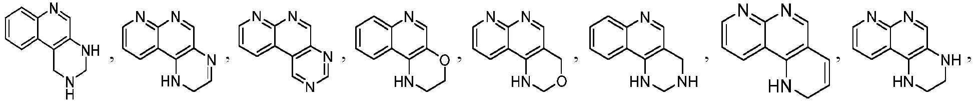

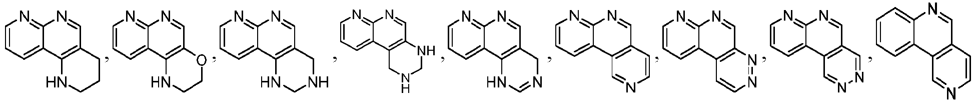

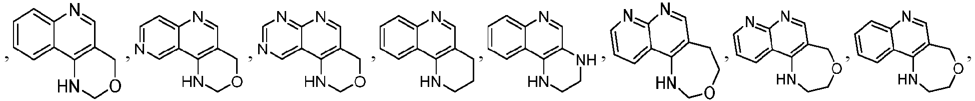

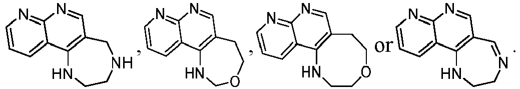

13. The compound according to any of claims 1-12, or a prodrug, tautomer, stereoisomer, metabolite, isotope derivative thereof or a mixture thereof, or a pharmaceutically acceptable salt thereof, co-crystal, polymorph or solvate thereof, wherein ring B is C₅-₁₀cycloalkyl, 3-20 membered heterocyclyl, C₆-₁₄aryl or 5-16 membered heteroaryl; preferably, ring B is C₅-₇cycloalkyl, 5-7 membered monocyclic heterocyclyl, 5-14 membered spiro-heterocyclyl, 5-14 membered fused-heterocyclyl, C₆-₁₀aryl, 5-6 membered monocyclic-heteroaryl or 5-14 membered fused-heteroaryl;
preferably, ring B is C₆cycloalkyl, C₇cycloalkyl, 6-membered monocyclic heterocyclyl, 7-membered monocyclic heterocyclyl, 4-membered/4-membered spiro-heterocyclyl, 4-membered/5-membered spiro-heterocyclyl, 5-membered/4-membered spiro-heterocyclyl, 5-membered/5-membered spiro-heterocyclyl, 4-membered/6-membered spiro-heterocyclyl, 6-membered/4-membered spiro-heterocyclyl, 5-membered/6-membered spiro-heterocyclyl, 6-membered/5-membered spiro-heterocyclyl, 6-membered/6-membered spiro-heterocyclyl, 4-membered/4-membered fused-heterocyclyl, 4-membered/5-membered fused-heterocyclyl, 5-membered/4-membered fused-heterocyclyl, 5-membered/5-membered fused-heterocyclyl, 5-membered/6-membered fused-heterocyclyl, 6-membered/5-membered fused-heterocyclyl, 4-membered/6-membered fused-heterocyclyl, 6-membered/4-membered fused-heterocyclyl, 6-membered/6-membered fused-heterocyclyl, phenyl, naphthyl, 5-membered monocyclic-heteroaryl, 6-membered monocyclic-heteroaryl, 5-membered/5-membered fused-heteroaryl, 5-membered/6-membered fused-heteroaryl, 6-membered/5-membered fused-heteroaryl, 6-membered/6-membered bicyclic fused-heteroaryl, the heteroatoms in the above-mentioned heterocyclyl, heteroaryl, fused-heterocyclyl, fused-heteroaryl are independently selected from O, N and S, the number of heteroatoms is 1, 2, or 3;
more preferably, ring B is

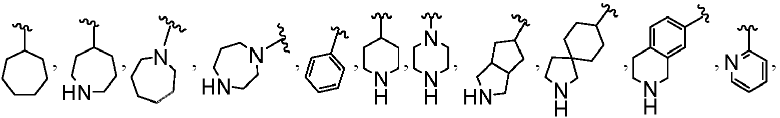

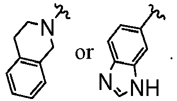

14. The compound according to any of claims 1-13, or a prodrug, tautomer, stereoisomer, metabolite, isotope derivative thereof or a mixture thereof, or a pharmaceutically acceptable salt thereof, co-crystal, polymorph or solvate thereof, wherein R_{D} is selected from -W-C(O)NR₂R₃, -W-C(O)NR₂OR₁, -W-OC(O)NR₂R₃, -W-NR₂C(O)R₄, -W-NR₂C(O)OR₁, -W-NR₂C(O)NR₂R₃, -W-S(O)₂R₄, -W-SO₂NR₂R₃, -W-NR₂S(O)₂R₄, -W-OS(O)₂R₄, -W-NR₂S(O)₂NR₂R₃, -W-OS(O)₂NR₂R₃, -W-P(O)(OR₁)₂, -W-P(S)(OR₁)₂, -W-O-P(S)(OR₁)₂, and -W-B(OH)₂; preferably, R_{D} is selected from -C₀₋₃alkylene-C(O)NHOH, -C₀₋₃alkylene-OC(O)NH₂, -C₀₋₃alkylene-SO₂NH₂, -C₀₋₃alkylene-NHS(O)₂H, -C₀₋₃alkylene-OS(O)₂H, -C₀₋₃alkylene-NHS(O)₂NH₂, -C₀₋₃alkylene-OS(O)₂NH₂, -C₀₋₃alkylene-P(O)(OH)₂, -C₀₋₃alkylene-P(S)(OH)₂, -C₀₋₃alkylene-O-P(S)(OH)₂, -C₀₋₃alkylene-B(OH)₂, -C₀₋₃alkyleneS(O)₂OH, -C₀₋₃alkylene-C(O)NH₂, -C₀₋₃alkylene-S(O)₂C₁₋₃alkyl, -C₀₋₃alkylene-OS(O)₂-C₁₋₃alkyl, -C₀₋₃alkyleneS(O)₂-C₁₋₃alkylene-C(O)OH, -C₀₋₃alkylene-S(O)₂C₁₋₃alkylene-NH₂, -C₀₋₃alkylene-S(O)₂NHCH₂C(O)OH, -C₀₋₃alkylene-NHS(O)₂CH₃, -C₀₋₃alkylene-S(O)₂ND₂, -C₀₋₃alkylene-S(O)₂NHNH₂, -C₀₋₃alkylene-S(O)₂NHSC₁₋₃alkyl, - C₀₋₃alkylene-NHS(O)₂C₁₋₃alkyl, -C₀₋₃alkylene-NH-C(O)C₁₋₃alkyl, -C₀₋₃alkylene-NH-CONH₂, -C₀₋₃alkylene-NH-COOC₁₋₃alkyl, and -C₀₋₃alkylene-S(O)₂NHOH;
preferably, R_{D} is selected from -methylene-C(O)NHOH, -methylene-OC(O)NH₂, -methylene-SO₂NH₂, -methylene-NHS(O)₂H, -methylene-OS(O)₂H, -methylene-NHS(O)₂NH₂, -methylene-OS(O)₂NH₂, -methylene-P(O)(OH)₂, - methylene-P(S)(OH)₂, -methylene-O-P(S)(OH)₂, -methylene-B(OH)₂, -C(O)NHOH, -OC(O)NH₂, -SO₂NH₂, - NHS(O)₂H, -OS(O)₂H, -NHS(O)₂NH₂, -OS(O)₂NH₂, -P(O)(OH)₂, -P(S)(OH)₂, -O-P(S)(OH)₂, -B(OH)₂; -S(O)₂OH, -C(O)NH₂, -S(O)₂C₁₋₃alkyl, -OS(O)₂C₁₋₃alkyl, -S(O)₂ C₁₋₃alkylene-C(O)OH, -S(O)₂C₁₋₃alkylene-NH₂, - S(O)₂NHCH₂C(O)OH, -NHS(O)₂CH₃, -S(O)₂ND₂, -S(O)₂NHNH₂, -methylene-NHS(O)₂C₁₋₃alkyl, -methylene-NH-C(O)C₁₋₃alkyl, -methylene-NH-CONH₂, -NH-COOC₁₋₃alkyl, and -S(O)₂NHOH;
most preferably, R_{D} is selected from -C(O)NHOH, -SO₂NH₂, -methylene-NHS(O)₂NH₂, -NHS(O)₂NH₂, -B(OH)₂, - P(O)(OH)₂, -OP(S)(OH)₂, -OS(O)₂NH₂, -S(O)₂OH, -C(O)NH₂, -S(O)₂CH₃, -OS(O)₂CH₃, -S(O)₂CH₂CH₂C(O)OH, - S(O)₂CH₂NH₂, -S(O)₂NHCH₂C(O)OH, -NHS(O)₂CH₃, -S(O)₂ND₂, -S(O)₂NHNH₂, -methylene-NHS(O)₂CH₃, - methylene-NH-C(O)CH₃, -methylene-NH-CONH₂, -NH-COOCH₃, and -S(O)₂NHOH.

15. The compound according to any of claims 1-14, or a prodrug, tautomer, stereoisomer, metabolite, isotope derivative thereof or a mixture thereof, or a pharmaceutically acceptable salt thereof, co-crystal, polymorph or solvate thereof, wherein R_{E} is each independently hydrogen, deuterium, halogen, oxo, oxime, -CN, -OH, -SH, -NO₂, -NH₂, C₁₋₆alkyl, C₂-₆alkenyl, C₂-₆alkynyl, C₁₋₆alkoxy, or C₃-₈cycloalkyl, the alkyl, alkenyl, alkynyl, alkoxy, or cycloalkyl is optionally substituted by one or more substituents selected from hydrogen, deuterium, halogen, oxo, oxime, -CN, -OH, -NO₂, -NH₂, C₁₋₆alkyl, phenyl, C₁₋₆alkoxy, C₁₋₆haloalkyl, C₁₋₆haloalkoxy, and halophenyl;
preferably, R_{E} is each independently hydrogen, deuterium, halogen, oxo, oxime, -CN, -OH, -SH, -NO₂, -NH₂, C₁₋₃alkyl, C₁₋₃alkoxy, or C₃₋₆cycloalkyl, the alkyl, alkoxy, or cycloalkyl is optionally substituted by one or more substituents selected from hydrogen, deuterium, halogen, oxo, oxime, -CN, -OH, -NO₂, -NH₂, C₁₋₃alkyl, C₁₋₃alkoxy, C₁₋₃haloalkyl, and C₁₋₃haloalkoxy;
preferably, R_{E} is each independently hydrogen, halogen, -OH, -SH, C₁₋₃alkyl, C₁₋₃alkoxy, or C₃₋₆cycloalkyl, the alkyl, alkoxy, or cycloalkyl is optionally substituted by one or more substituents selected from hydrogen, deuterium, halogen, oxo, -CN, -OH, -NO₂, -NH₂, C₁₋₃alkyl, C₁₋₃alkoxy, C₁₋₃haloalkyl, and C₁₋₃haloalkoxy;
further preferably, R_{E} is each independently hydrogen, F, Cl, Br, -OH, -SH, -methyl, ethyl, n-propyl, isopropyl, cyclopropyl, methoxy, methylthio, trifluoromethyl, difluoromethyl, monofluoromethyl, trifluoroethyl, difluoroethyl, monofluoroethyl, hydroxypropyl, hydroxyethyl, hydroxymethyl, methoxypropyl, methoxyethyl, or methoxymethyl; most preferably, R_{E} is each independently hydrogen, deuterium, F, Cl, carboxyl, -CN, -NH₂, methyl, methylthio, - CF₃, methoxy, -CH₂NH₂, -CH₂OH, -CH₂NHOH, -CH=NOH, -CH₂CH₂OH, -CHF₂, -C(O)OCH₃, -C(O)CH₃, 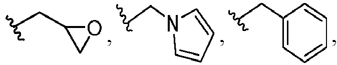 cyclopropyl, ethenyl, ethynyl, propynyl, ethyl, propenyl.

16. The compound according to any of claims 1-15, or a prodrug, tautomer, stereoisomer, metabolite, isotope derivative thereof or a mixture thereof, or a pharmaceutically acceptable salt thereof, co-crystal, polymorph or solvate thereof, wherein n4 is 1, 2, 3 or 4; preferably, n4 is 1, 2, or 3.

17. The compound according to any of claims 1-16, or a prodrug, tautomer, stereoisomer, metabolite, isotope derivative thereof or a mixture thereof, or a pharmaceutically acceptable salt thereof, co-crystal, polymorph or solvate thereof, wherein L is a bond, -O-, -S-, C₁₋₃alkylene, C₁₋₃alkyleneoxy, C₃₋₆cycloalkylene, C₁₋₃alkylenethio, C₂-₆alkenylene, or C₂-₆alkynylene, the alkylene, alkyleneoxy, cycloalkylene, alkylenethio, alkenylene, or alkynylene is optionally substituted by one or more substituents selected from deuterium, halogen, C₁₋₃alkyl, C₁₋₃alkoxy, oxo, -CN, -OH, and -NH₂;
preferably, L is a bond, -O-, -S-, methylene, ethylene, propylene, cyclopropylene, cyclobutylene, C₁₋₃alkyleneoxy, C₁₋₃alkylenethio, ethenylene, or ethynylene, the methylene, ethylene, propylene, cyclopropylene, cyclobutylene, alkyleneoxy, alkylenethio, ethenylene, or ethynylene is optionally substituted by one or more substituents selected from deuterium, halogen, methyl, ethyl, n-propyl, isopropyl, methoxy, ethoxy, propoxy, isopropoxy, oxo, -CN, -OH, and -NH₂;
more preferably, L is a bond, -O-, -S-, cyclopropylene, methylenemethoxy, methylenemethyl, methylene-OH, methylene-NH₂, ethylene, or propylene;
most preferably, L is a bond, -O-, -CH₂-,

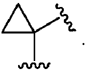

18. A compound, or a prodrug, tautomer, stereoisomer, metabolite, isotope derivative thereof or a mixture thereof, or a pharmaceutically acceptable salt thereof, co-crystal, polymorph or solvate thereof, wherein said compound is selected from:
| No. | Structure | No. | Structure | No. | Structure |
|---|---|---|---|---|---|
| 1 | 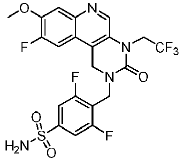 | 2 | 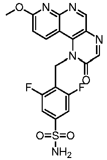 | 3 | 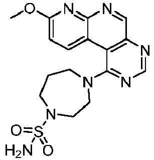 |
| 4 | 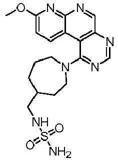 | 5 | 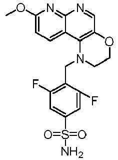 | 6 | 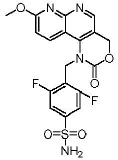 |
| 7 | 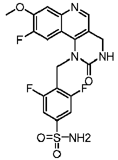 | 8 | 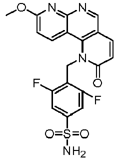 | 9 | 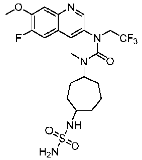 |
| 10 | 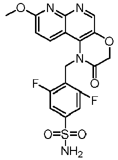 | 11 | 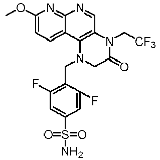 | 12 | 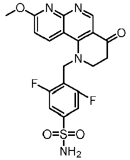 |
| 13 | 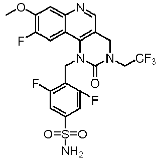 | 14 | 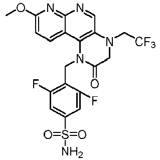 | 15 | 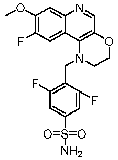 |
| 16 | 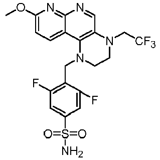 | 17 | 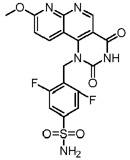 | 18 | 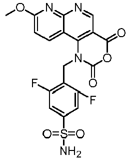 |
| 19 | 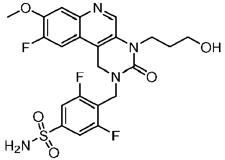 | 20 | 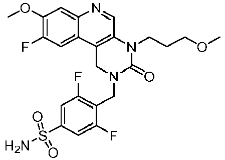 | 21 | 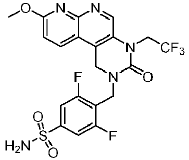 |
| 22 | 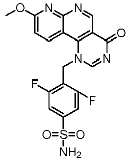 | 23 | 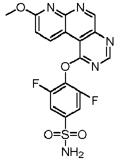 | 24 | 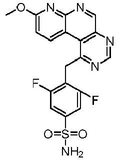 |
| 25 | 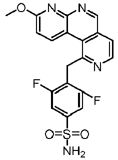 | 26 | 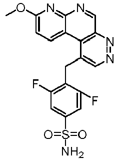 | 27 | 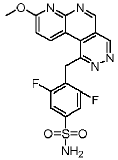 |
| 28 | 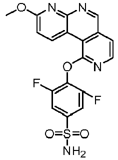 | 29 | 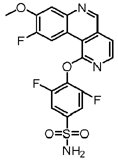 | 30 | 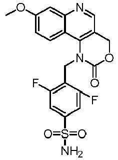 |
| 31 | 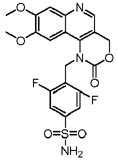 | 32 | 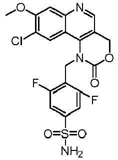 | 33 | 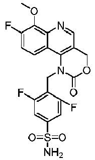 |
| 34 | 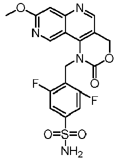 | 35 | 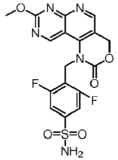 | 36 | 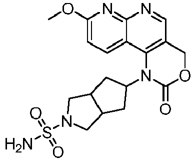 |
| 37 | 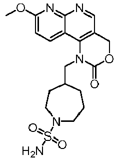 | 38 | 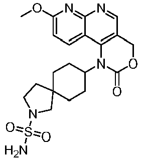 | 39 | 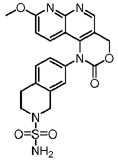 |
| 40 | 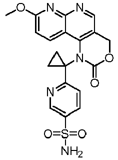 | 41 | 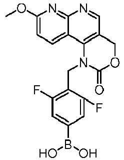 | 42 | 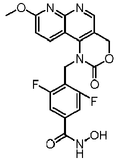 |
| 43 | 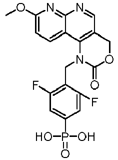 | 44 | 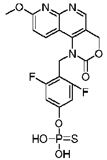 | 45 | 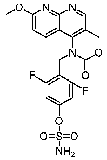 |
| 46 | 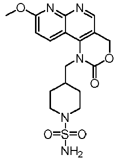 | 47 | 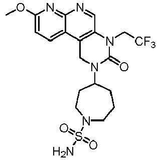 | 48 | 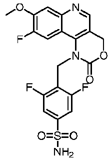 |
| 49 | 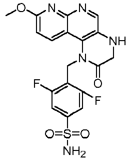 | 50 | 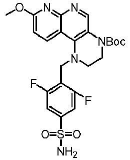 | 51 | 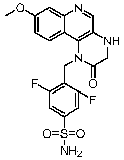 |
| 52 | 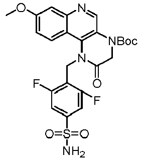 | 53 | 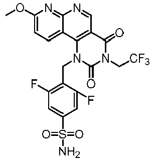 | 54 | 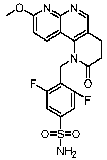 |
| 55 | 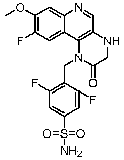 | 56 | 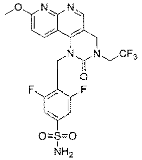 | 57 | 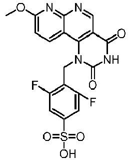 |
| 58 | 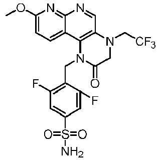 | 59 | 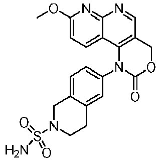 | 60 | 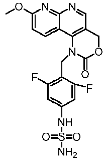 |
| 61 | 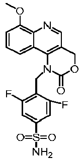 | 62 | 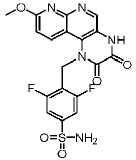 | 63 | 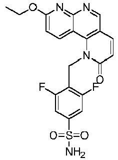 |
| 64 | 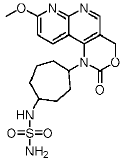 | 65 | 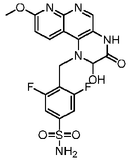 | 66 | 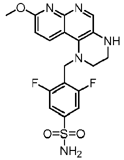 |
| 67 | 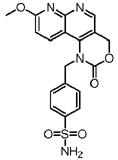 | 68 | 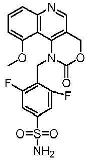 | 69 | 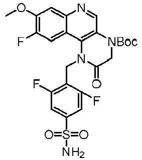 |
| 70 | 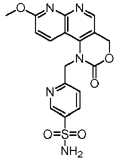 | 71 | 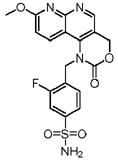 | 72 | 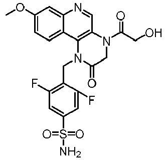 |
| 73 | 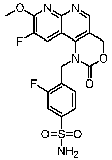 | 74 | 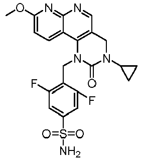 | 75 | 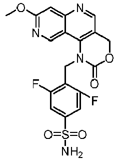 |
| 76 | 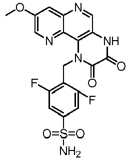 | 77 | 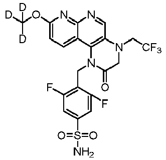 | 78 | 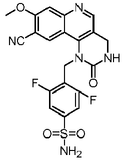 |
| 79 | 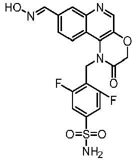 | 80 | 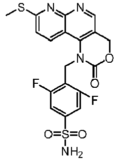 | 81 | 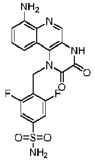 |
| 82 | 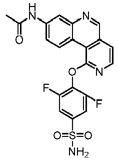 | 83 | 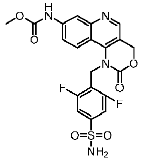 | 84 | 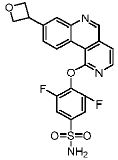 |
| 85 | 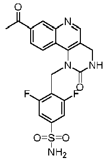 | 86 | 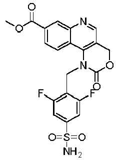 | 87 | 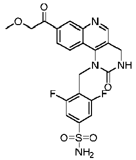 |
| 88 | 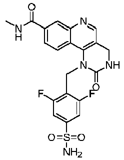 | 89 | 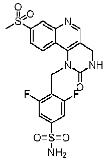 | 90 | 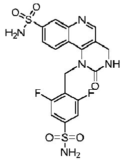 |
| 91 | 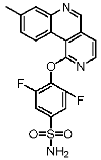 | 92 | 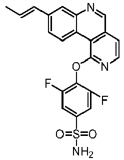 | 93 | 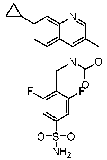 |
| 94 | 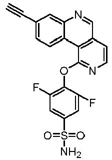 | 95 | 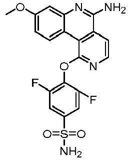 | 96 | 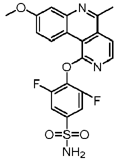 |
| 97 | 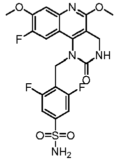 | 98 | 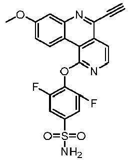 | 99 | 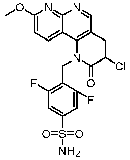 |
| 100 | 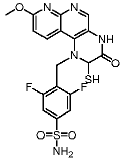 | 101 | 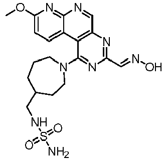 | 102 | 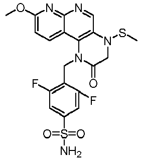 |
| 103 | 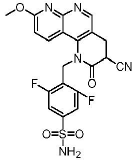 | 104 | 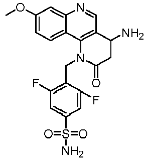 | 105 | 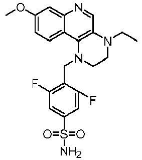 |
| 106 | 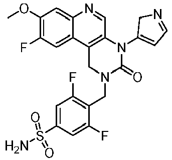 | 107 | 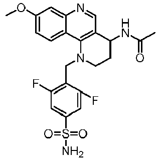 | 108 | 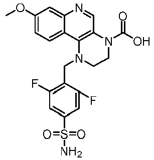 |
| 109 | 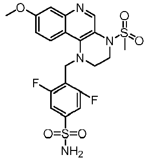 | 110 | 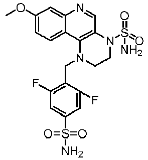 | 111 | 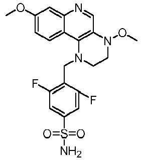 |
| 112 | 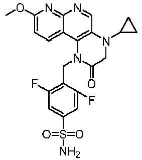 | 113 | 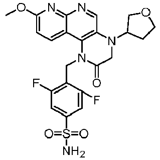 | 114 | 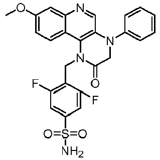 |
| 115 | 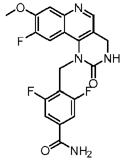 | 116 | 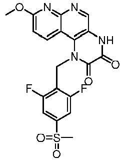 | 117 | 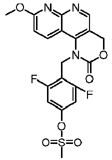 |
| 118 | 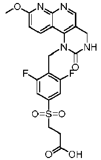 | 119 | 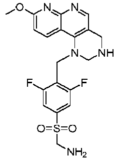 | 120 | 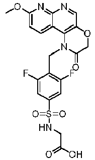 |
| 121 | 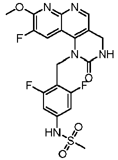 | 122 | 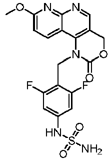 | 123 | 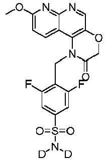 |
| 124 | 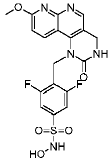 | 125 | 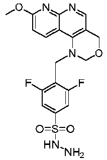 | 126 | 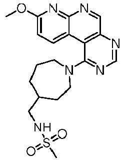 |
| 127 | 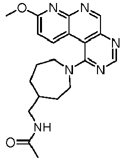 | 128 | 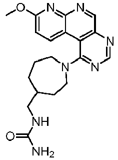 | 129 | 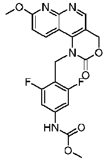 |
| 130 | 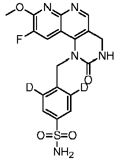 | 131 | 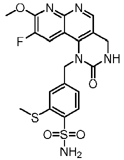 | 132 | 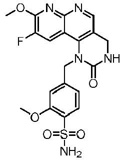 |
| 133 | 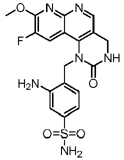 | 134 | 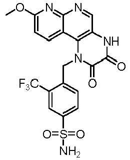 | 135 | 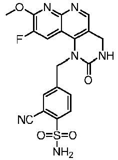 |
| 136 | 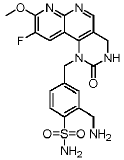 | 137 | 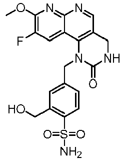 | 138 | 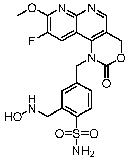 |
| 139 | 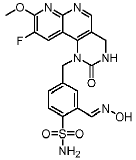 | 140 | 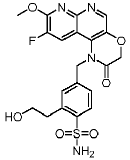 | 141 | 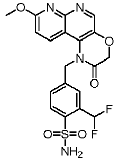 |
| 142 | 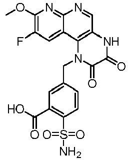 | 143 | 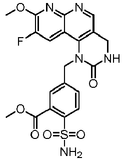 | 144 | 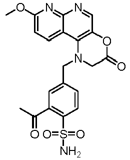 |
| 145 | 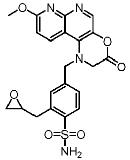 | 146 | 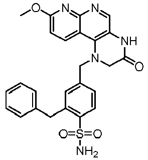 | 147 | 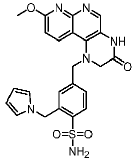 |
| 148 | 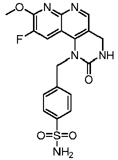 | 149 | 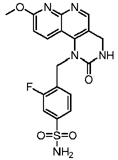 | 150 | 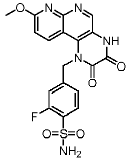 |
| 151 | 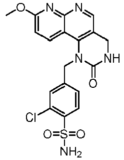 | 152 | 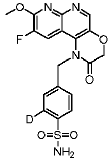 | 153 | 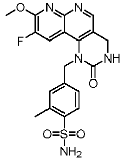 |
| 154 | 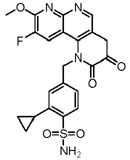 | 155 | 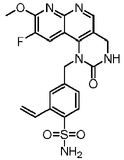 | 156 | 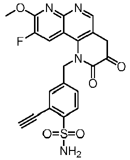 |
| 157 | 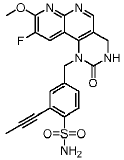 | 158 | 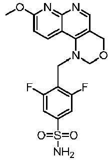 | 159 | 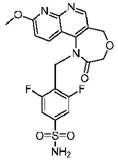 |
| 160 | 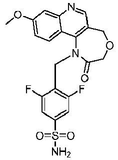 | 161 | 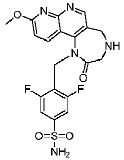 | 162 | 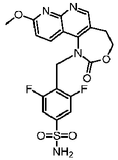 |
| 163 | 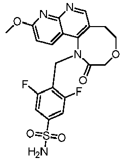 | 164 | 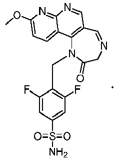 | | 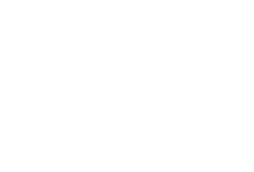 |

19. A pharmaceutical composition, containing the compound according to any of claims 1-18, a stereoisomer or tautomer of the compound or a mixture thereof, a pharmaceutically acceptable salt, co-crystal, polymorph or solvate of the compound, or a stable isotope derivative, metabolite or prodrug of the compound.

20. Use of the compound according to any of claims 1-18, a stereoisomer or tautomer of the compound or a mixture thereof, a pharmaceutically acceptable salt, co-crystal, polymorph or solvate of the compound, or a stable isotope derivative, metabolite or prodrug of the compound or the pharmaceutical composition according to claim 19 in the manufacture of a medicament for preventing and/or treating ENPP1-mediated diseases;
preferably, the ENPP1-mediated disease is cancer or tumour-related disease, or cardiovascular disease, more preferably, the ENPP1-mediated disease is pancreatic cancer, heart failure or myocardial infarction.
